# EUROPEAN PATENT APPLICATION

(11) **EP 4 840 883 A1**
(43) Date of publication of application: **18.11.2026**
(21) Application number: 25738462.8
(22) Date of filing: 08.01.2025
(51) Int. Cl.: C07K 16/28, C07K 16/46, A61P 35/00, C12N 15/13, A61K 39/395

(54) **ANTIBODY, AND DRUG CONJUGATE AND USE THEREOF**

(30) Priority: 08.01.2024 CN 202410027871; 04.06.2024 CN 202410720390
(71) Applicant: Velavigo (Shanghai) Limited, Shanghai 200131 (CN)
(72) Inventor: WANG, Wei, Shanghai 200131 (CN); LI, Jing, Shanghai 200131 (CN); GUAN, Lingyu, Shanghai 200131 (CN); YIN, Qun, Shanghai 200131 (CN); XU, Man, Shanghai 200131 (CN)
(74) Representative: Script Intellectual Property LLP
(86) International application number: PCT/CN2025/071288
(87) International publication number: WO 2025/148933

(57) **Abstract**

Provided are an antibody and an antibody-drug conjugate. More specifically, provided are an antibody and an antibody-drug conjugate (ADC) targeting TROP2 and/or NECTIN4, and a composition containing the antibody or the ADC and the therapeutic use thereof.

## Description

### TECHNICAL FIELD

The present invention relates to an antibody and an antibody-drug conjugate, and particularly to an antibody and an antibody-drug conjugate (ADC) targeting TROP2 and/or NECTIN4, and a composition comprising the antibody or the ADC, and therapeutic use thereof.

### BACKGROUND

Trop2 (trophoblast cell-surface antigen 2, human trophoblast cell surface glycoprotein antigen 2), also known as tumor-associated calcium signal transducer 2 (TACSTD2), epithelial glycoprotein 1 (EGP-1), gastrointestinal antigen 733-1 (GA733-1), and membrane surface marker 1 (M1S1), is a type I cell surface glycoprotein encoded by the TACSTD2 gene. Trop2 is highly expressed in human cancers, and signaling pathways mediated by it promote the growth, proliferation, and metastasis of tumor cells mainly by regulating the calcium ion signaling pathway and cyclin expression and reducing fibronectin adhesion. Thus, Trop2 has become an attractive therapeutic target for cancer therapy. However, since Trop2 is not only expressed in various cancer tissues such as ovarian cancer, pancreatic cancer, gastric cancer, breast cancer, but also in a large number of normal tissues such as breast, cervix, prostate, skin, stomach, and thymus, drugs targeting Trop2 have potential on-target toxicity. For example, when the skin and oral mucosa are greatly affected by the drugs, side effects such as stomatitis and rashes are more likely to occur.

The only Trop2 ADC drug currently on the market is Trodelvy^{®} from Gilead (sacituzumab govitecan; SG). In the ASCENT study, the incidence of grade 3 or higher adverse effects caused by SG reached 45%, and two side effects, neutropenia and diarrhea, were given a black box warning by the FDA. In addition, 9% of patients developed the rash and 5% of patients developed ocular toxicity. Datopotamab Deruxtecan (DS-1062, Dato-DXd) is a TROP2-targeted Dxd antibody-drug conjugate (ADC) jointly developed by Daiichi Sankyo and AstraZeneca. To control toxicity, Dato-DXd uses a moderate-affinity Trop2-targeted monoclonal antibody linked to a less-toxic DNA topoisomerase I inhibitor (DXd), and controls the DAR value at 4. However, in a toxicity test of cynomolgus monkeys, the highest non-severely toxic dose (HNSTD) of Dato-DXd was only 10 mg/kg due to pulmonary toxicity caused by Dxd, which is lower than the 30 mg/kg HNSTD of DS-8201 with a DAR value of 8 in the toxicology of the monkeys.

Nectin cell adhesion molecule 4 (Nectin4) is a type I transmembrane cell adhesion molecule belonging to the Nectin family. The protein was previously known as a homolog of a poliovirus receptor (PVR/CD155) and was also known as a poliovirus receptor-related (PRR) protein. In the physiological development process, the Nectin4 is specifically expressed during embryonic and fetal development, and is expressed at a low level in tissues in adulthood. It forms a physical link between adjacent cells and is essential for achieving intercellular communication, migration, and other important cellular processes. Nectin4 is over-expressed in various tumor cells, is used as a marker of cancer recurrence and metastasis, and is related to poor prognosis of various cancers, including urothelial cancer, breast cancer, ovarian cancer, pancreatic cancer, non-small cell lung cancer, gastric cancer, hepatocellular cancer, bladder cancer, and the like.

Compared with HER2, EGFR, Trop2, and other hot targets, there are currently few drugs targeting Nectin4, and only one ADC product, Padcev^{®}, is available globally. Padcev^{®} (Enfortumab Vedotin; EV) is a "first-in-class" drug developed jointly by Seagen and Astellas. However, since Nectin4 has certain expression in normal skin tissues, Padcev was given a black box warning of severe skin toxicity.

Nanobodies are small proteins consisting of single-chain antibody molecules, have high specificity and affinity, and have a smaller size, higher stability, and deeper tissue penetrability compared with traditional antibodies, which makes the nanobodies have a great potential in tumor therapy. The nanobodies can perform precise therapy by recognizing and targeting specific antigens on the surfaces of tumor cells, and can penetrate deep tumor tissues that are inaccessible to conventional antibodies. The nanobodies may be designed to deliver drugs or radioisotopes to the tumor cells so as to achieve the effect of killing the tumor cells. In addition, the nanobodies may also be used in a variety of other therapeutic modalities such as photodynamic therapy and immunodetection. Therefore, the application of nanobodies in the field of tumor therapy is attracting increasing attention, and the nanobodies are expected to become one of the important means for future tumor therapy (Bannas, Hambach, and Koch-Nolte 2017).

Antibody-based drugs have promoted advances in the field of cancer therapy, but many antibody-based drugs are still not ideal in terms of safety and efficacy. One of the major challenges faced by anti-cancer drugs is still drug selectivity, i.e., minimizing collateral damage of the drugs to normal cells while ensuring that the drugs effectively attack cancer. To overcome limitations of single-targeted drugs in this respect, strategies have been proposed to combine multiple monoclonal antibodies targeting different targets. However, published studies on different mAb combination therapies have shown that the combination effect varies widely depending on such factors as the combination of different targets. For example, simultaneous injection of trastuzumab (anti-HER2) and bevacizumab (anti-VEGF-A) has shown encouraging results in HER2+ metastatic breast cancer. However, bevacizumab in combination with cetuximab (anti-EGFR) did not lead to favorable results in the treatment of advanced colorectal cancer. See, e.g., Maruani A et al, Bispecifics and antibody-drug conjugates: A positive synergy. Drug Discov Today Technol. 2018 Dec; 30:55-61.

Thus, there is still an urgent need in this field for new drug molecules having improved tumor specificity and selectivity to reduce on-target toxicity, improve medication windows, and expand the therapeutic index.

### SUMMARY OF THE INVENTION

Analysis of immunohistochemical and bioinformatics data from clinical pathological samples (see, e.g., Annals of Oncology 28: 769-776, 2017; doi:10.1093/annonc/mdw678; and Front. Oncol. 12:858865. doi: 10.3389/fonc.2022.858865) revealed that Trop2 and Nectin4 proteins exhibit a co-expression pattern in many tumor tissues or cells, such as urothelial cancer, breast cancer, cervical squamous cell carcinoma, lung cancer, head and neck cancer, and thyroid cancer, while the co-expression ratio is relatively low in normal tissues. Therefore, the inventors proposed and designed an antibody molecule that targets Trop2 and Nectin4 simultaneously to reduce the on-target toxicity of the 2 targets and improve the therapeutic effect. On this basis, the inventors further provide an innovative antibody mode based on nanobodies, such that the molecular weight of the final antibody is only about 60% of that of a conventional four-chain antibody. This reduction enables a higher proportion of antibody molecules to be enriched in tumor tissues, thereby further improving the therapeutic effect. Based on these designs and findings, the inventors have established a multispecific antibody of the present invention, a drug-conjugate molecule thereof, and use thereof, particularly in cancer therapy.

Accordingly, in a first aspect, the present invention provides a multispecific antibody binding to Trop2 and Nectin4, a pharmaceutical composition thereof, and use thereof, wherein the antibody comprises at least one antigen-binding domain specifically binding to Trop2 and at least one antigen-binding domain specifically binding to Nectin4.

In a second aspect, the present invention provides an anti-Trop2 VHH domain and an anti-Trop2 antibody, an anti-Nectin4 VHH domain and an anti-Nectin4 antibody, and an antigen-binding molecule, immunoconjugate, and immunofusion comprising same, as well as a pharmaceutical composition thereof and use thereof.

In a third aspect, the present invention provides an antibody-drug conjugate (ADC) molecule comprising the antibody of the present invention, a pharmaceutical composition thereof, and use thereof.

In some embodiments, the antibody and the antibody-drug conjugate of the present invention have one or more of the following advantages:
(1) binding to target cells expressing human Nectin4 and/or Trop2 and having high avidity therefor;
(2) synergistically binding to target cells expressing human Nectin4 and Trop2;
(3) having a synergistic endocytosis effect on target cells expressing human Nectin4 and Trop2;
(4) having a synergistic killing effect on target cells expressing human Nectin4 and Trop2;
(5) having a remarkable bystander killing effect;
(6) having high anti-tumor efficacy, and having a stronger killing effect on tumor cells and a stronger inhibitory effect on the growth of tumors, particularly tumors with high expression of Nectin4 and/or Trop2; having significantly improved and even unexpected anti-tumor activity.

In some embodiments, the antibody and the antibody-drug conjugate of the present invention also have one or more of the following advantages:
(7) having an enhanced ability to resist the emergence of tumor drug resistance compared with Nectin4 and Trop2 single-target drugs;
(8) resisting tumor drug resistance caused by mutations in Nectin4 or Trop2 antigen epitopes;
(9) having anti-tumor activity against tumor cells resistant to Nectin4 and Trop2 single-target drugs (e.g., tumor cells expressing a high level of multi-drug resistance protein MDR1); and
(10) having improved tumor targeting distribution compared with Nectin4 or Trop2 single-target drugs.

In some embodiments, the single-target drug is a reference antibody Datopotamab, a reference antibody Enfortumab, or corresponding ADC drugs thereof.

In some embodiments, the antibody-drug conjugate of the present invention also has one or more of the following advantages:
(11) having better product uniformity;
(12) having low toxicity;
(13) having good physical and chemical stability; and
(14) having good druggability.

The present invention is further illustrated in the following drawings and specific embodiments. However, these drawings and specific embodiments should not be construed as limiting the scope of the present invention, and modifications easily conceived by those skilled in the art will be included in the spirit of the present invention and the protection scope of the appended claims.

### BRIEF DESCRIPTION OF THE DRAWINGS

FIG. 1A shows the endocytosis assay of exemplary anti-Trop2 VHH-Fc on BT-474 cells.
FIG. 1B shows the endocytosis assay of exemplary anti-Trop2 VHH-Fc on NCI-N87 cells.
FIG. 2A shows the endocytosis assay of exemplary anti-Nectin4 VHH-Fc on BT474 cells.
FIG. 2B shows the endocytosis assay of exemplary anti-Nectin4 VHH-Fc on NCI-N87 cells.
FIG. 3 shows the binding of exemplary anti-Trop2 VHH-Fc to NCI-N87 cells.
FIG. 4A shows the binding of exemplary anti-Nectin4 VHH-Fc antibodies to NCI-N87 cells.
FIG. 4B shows the binding of exemplary anti-Nectin4 VHH-Fc antibodies to BT474 cells.
FIG. 5A shows the binding of an exemplary anti-Nectin4 VHH-His antibody to CHO-K1-Human Nectin4 cells.
FIG. 5B shows the binding of an exemplary anti-Nectin4 VHH-His antibody to CHO-K1-Cyno Nectin4 cells.
FIG. 5C shows the non-specific binding of an exemplary anti-Nectin4 VHH-His antibody to CHO-K1 cells.
FIG. 6 shows the expression of a Trop2 antigen and a Nectin4 antigen on various target cells.
FIG. 7A shows the non-specific binding of an exemplary antibody V-hu21-Fc to paralogs Nectin1, 2, and 3 antigens, as well as a Trop1 antigen.
FIG. 7B shows the non-specific binding of an exemplary antibody V-hu23-Fc to paralogs Nectin1, 2, and 3 antigens and the specific binding of the exemplary antibody to a target antigen Nectin4.
FIG. 7C shows the non-specific binding of an exemplary antibody V-hu23-Fc to a paralog Trop1 antigen and the specific binding of the exemplary antibody to a target antigen Trop2.
FIG. 8A shows the binding of exemplary bispecific antibodies in single-chain forms to NCI-N87 cells.
FIG. 8B shows the binding of exemplary bispecific antibodies in double-chain forms to NCI-N87 cells.
FIG. 9A shows the binding of exemplary bispecific antibodies in single-chain forms to BT-474 cells.
FIG. 9B shows the binding of exemplary bispecific antibodies in double-chain forms to BT-474 cells.
FIG. 10A shows the binding of exemplary bispecific antibodies in double-chain forms to MDA-MB-468 cells.
FIG. 10B shows the binding of exemplary bispecific antibodies in double-chain forms to NCI-N87 cells.
FIG. 10C shows the binding of exemplary bispecific antibodies in double-chain forms to BT-474 cells.
FIG. 11 shows the binding of an exemplary antibody V-hu21-Fc to different tumor cells.
FIG. 12 shows the binding of an exemplary antibody V-hu23-Fc to different tumor cells.
FIG. 13A shows the endocytosis results of exemplary bispecific anti-Trop2/Nectin4 antibody molecules by BT-474 cells.
FIG. 13B shows the endocytosis results of exemplary bispecific anti-Trop2/Nectin4 antibody molecules by BT-474 cells.
FIG. 13C shows the endocytosis results of exemplary bispecific anti-Trop2/Nectin4 antibody molecules by NCI-N87 cells.
FIG. 13D shows the endocytosis results of exemplary bispecific anti-Trop2/Nectin4 antibody molecules by NCI-N87 cells.
FIG. 13E shows the endocytosis results of exemplary bispecific antibody molecules V-hu21-Fc and V-hu23-Fc by different target cells.
FIGs. 14A-14E show the comparison of the cell binding ability of ADCs with the corresponding bispecific antibodies.
FIG. 15A shows the targeted killing of target cells MDA-MB-468 by V-hu21-VA-Exd.
FIG. 15B shows the killing of non-target MKN45 cells by V-hu21-VA-Exd.
FIG. 15C shows the killing of target cells MDA-MB-468 by V-hu21-VA-Exd, V-hu23-VA-Exd, and V-hu24-VA-Exd.
FIG. 15D shows the killing of target cells MDA-MB-468 by V-hu23-Glu-Exd and V-hu24-Glu-Exd.
FIG. 15E shows the synergistic killing effect of the anti-Trop2 binding site and the anti-Nectin4 binding site of an antibody on double-positive NCI-H292 tumor cells.
FIG. 16A shows a bystander effect of V-hu21-VA-Exd.
FIG. 16B shows a bystander effect of V-hu23-Glu-Exd.
FIG. 17 shows the efficacy of multiple administrations of ADCs in an MDA-MB-468 subcutaneous xenograft mouse model.
FIG. 18 shows the efficacy of a single administration of ADCs in an MDA-MB-468 subcutaneous xenograft mouse model.
FIG. 19 shows the efficacy of multiple administrations of ADCs in an HT1376 subcutaneous xenograft mouse model.
FIG. 20 shows the efficacy of a single administration of ADCs in an HT1197 subcutaneous xenograft mouse model.
FIG. 21 shows the efficacy of a single administration of ADCs in an MDA-MB-453 subcutaneous xenograft model.
FIG. 22 shows the efficacy of multiple administrations of an ADC in a human head and neck cancer *in vivo* xenograft tumor model.
FIG. 23 shows the efficacy of a single administration of an ADC in a human endometrial cancer *in vivo* xenograft tumor model.
FIG. 24 shows the efficacy of a single administration of an ADC in a human cervical cancer *in vivo* xenograft tumor model.
FIG. 25 shows the efficacy of two administrations of ADCs in a murine colon cancer *in vivo* graft tumor model.
FIG. 26 shows the co-expression of Trop2 and Nectin4 in various tumors as revealed by bioinformatics analysis. In the figures, TPM (Transcripts Per Million) was used to quantify the gene expression level, showing the expression abundance of Trop2 and Nectin4 in different types of tumor tissues. FIG. 26A shows exemplary tumor types with double-positive expression of Trop2 and Nectin4 according to the median TPM in the patient population. FIG. 26B shows the expression distribution of Trop2 and Nectin4 observed in patient populations of specific tumor types.
FIG. 27 shows the biodistribution of a bispecific antibody in a human HT1376 xenograft tumor mouse model. FIG. 27A shows the quantification of tumor accumulation of an antibody over time by the fluorescence ratio of tumor to background. FIG. 27B shows the distribution of antibodies in organ tissues 72 h after injection of fluorescently labeled antibody proteins, as assessed by the fluorescence ratio of tumor to muscle.

### DETAILED DESCRIPTION OF THE INVENTION

Unless otherwise defined, all technical and scientific terms used herein have the same meaning as commonly understood by a person of ordinary skill in the art to which the present invention belongs. All publications, patent applications, patents, and other references mentioned herein are incorporated by reference in their entirety. In addition, the materials, methods, and examples described herein are illustrative only and are not intended to be limiting. Other features, objectives, and advantages of the present invention will be apparent from the specification and drawings and from the appended claims.

### Definitions

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

Herein, the term "comprise" or "include" used herein, unless otherwise indicated, also encompasses the situation where the entirety consists of the described elements, integers, or steps. For example, when referring to an antibody variable region "comprising" a specific sequence, it is also intended to encompass an antibody variable region consisting of the specific sequence.

Herein, the term "antigen-binding molecule" refers to a protein or polypeptide that comprises an antigen-binding domain or antigen-binding site capable of binding to a target antigen. When the target antigen is TROP2 and/or NECTIN4, the antigen-binding molecule binding to TROP2 and/or NECTIN4 is also referred to as a TROP2-binding molecule, a NECTIN4-binding molecule, or a TROP2/NECTIN4-binding molecule. The antigen-binding molecule includes, for example, an antibody and an antigen-binding fragment thereof, as well as various fusions constructed on the basis of the antibody or the antigen-binding fragment, such as a VHH-Fc antibody, a multi/bispecific antibody, and a chimeric antigen receptor (CAR). It will be appreciated by those skilled in the art that the antigen-binding site of an antibody typically comprises amino acid residues from a "complementary determining region" or "CDR".

Herein, the term "antibody" refers to a polypeptide comprising at least an immunoglobulin light chain variable region or heavy chain variable region that specifically recognizes and binds to an antigen. The term encompasses various antibody structures, including but not limited to, monoclonal antibodies, single-chain antibodies or multi-chain antibodies, monospecific or multispecific antibodies (e.g., bispecific antibodies), single-domain antibodies, heavy-chain antibodies, chimeric or humanized antibodies, intact antibodies, and antibody fragments, as long as they exhibit the desired antigen-binding activity.

Herein, "intact antibody" refers to an immunoglobulin molecule comprising at least two heavy (H) chains and two light (L) chains. Each heavy chain consists of a heavy chain variable region (abbreviated herein as VH) and a heavy chain constant region. Each light chain consists of a light chain variable region (abbreviated herein as VL) and a light chain constant region.

Herein, the terms "antibody fragment" and "antigen-binding fragment" are used interchangeably and refer to a molecule different from an intact antibody, which comprises a portion of the intact antibody and can bind to an antigen to which the intact antibody binds. Examples of the antibody fragment include, but are not limited to, an Fv, a Fab, a Fab', a Fab'-SH, a F(ab')2; a diabody; a linear antibody; a single-chain antibody (e.g., an scFv); a single-domain antibody; a Camelid antibody (a heavy-chain antibody) or a fragment thereof (e.g., VHH); and a monospecific antibody, a bispecific antibody, or a multispecific antibody formed from antibody fragments. Reference herein to the term "antibody" is equivalent to "antibody or antibody fragment thereof", unless otherwise stated herein or clearly contradicted by context. In some embodiments according to the present invention, the antibody fragment comprises a cysteine residue portion for forming an interchain disulfide bond between the heavy chain and the heavy chain, e.g., cysteine residues of the hinge region of the antibody, to provide an amino acid residue site useful for sulfhydryl conjugation chemistry. In some other embodiments according to the present invention, the antibody fragment comprises a cysteine residue introduced into the Fc region to provide an amino acid residue site useful for sulfhydryl conjugation chemistry.

Herein, the terms "antigen-binding site" and "antigen-binding domain" are used interchangeably and refer to a region in an antibody molecule that actually binds to an antigen. The antigen-binding site for the antibody of the present invention is preferably provided by a variable domain (i.e., "VHH") from a heavy-chain antibody.

Herein, the term "multispecific" refers to an antigen-binding molecule (e.g., an antibody) having at least two antigen-binding sites that bind to different antigenic epitopes, e.g., bind to different epitopes on different antigens and/or different epitopes on the same antigen. Accordingly, "monospecific" refers to the ability to bind to only one type of epitope. "Bispecific" refers to the ability to bind to two different types of epitopes.

Herein, the expression "valence" or "valent" in relation to antibodies refers to the total number of antigen-binding sites in an antibody molecule, or the number of antigen-binding sites having the same antigen-binding specificity. For example, a tetravalent antibody means that the antibody molecule comprises a total of 4 antigen-binding sites; the antibody molecule may be a bispecific antibody of the "2 + 2 valent" type, i.e., the antibody has two different antigen-binding specificities, wherein 2 identical antigen-binding sites are present for each antigen-binding specificity, respectively.

The term "on-target/off tumor toxicity" refers to the fact that, in addition to tumor cells, normal tissue cells also express tumor-associated antigens targeted by the antibody, and thus bind to the antibody to cause damage.

Herein, the terms "TROP2" and "Trop2" are used interchangeably and refer to trophoblast cell surface glycoprotein antigen 2. Unless otherwise stated, the term includes any variant of human Trop2, including sequence variants, particularly naturally occurring variants, allelic variants, as well as post-translationally modified variants and conformational variants, and encompasses species homologs thereof. In addition, it should be understood that the term encompasses not only TROP2 naturally or recombinantly expressed by a cell or TROP2 expressed on a natural or recombinant cell, but also a recombinantly expressed fusion protein comprising the extracellular domain of TROP2. An example of the TROP2 is a human TROP2 protein comprising the amino acid sequence under UniProtKB - P09758, or a recombinant protein comprising the extracellular domain of the protein (in particular, the amino acid sequence of amino acids 31-274). Another example of the TROP2 is a monkey TROP2 protein comprising the amino acid sequence under NCBI -XP_005543292.2, or a recombinant protein comprising the extracellular domain of the protein. Herein, unless otherwise specified, the term "TROP2" or "Trop2" refers to Trop2 derived from a human. In the embodiments of the present disclosure, "antigen-binding specificity for Trop2", i.e., "antigen-binding domain specifically binding to TROP2", is preferably provided by a VHH domain.

Herein, the term "TROP2-positive" cell refers to a cell that exhibits positive cell surface expression of TROP2, such as a cancer cell, an engineered cancer cell, or an engineered non-tumor cell. The expression level of TROP2 on the cell surface can be determined by any conventional method known in the art for determining the expression level of cell surface antigens, for example, an FACS detection method or an immunofluorescence staining method. TROP2 has significantly higher expression levels on various tumor cells than on normal tissues/cells, for example, HT1376 (human bladder cancer cells) and MDA-MB-468 (human breast cancer cells). Preferably, herein, the TROP2-positive cells are TROP2-positive tumor cells.

Herein, the terms "NECTIN4" and "Nectin4" are used interchangeably and refer to Nectin cell adhesion molecule 4. Unless otherwise stated, the term includes any variant of human NECTIN4, including sequence variants, particularly naturally occurring variants, allelic variants, as well as post-translationally modified variants and conformational variants, and encompasses species homologs thereof. In addition, it should be understood that the term encompasses not only NECTIN4 naturally or recombinantly expressed by a cell or NECTIN4 expressed on a natural or recombinant cell, but also a recombinantly expressed fusion protein comprising the extracellular domain of NECTIN4. An example of the NECTIN4 is a human NECTIN4 protein comprising the amino acid sequence under UniProtKB-Q96NY8, or a recombinant protein comprising the extracellular domain of the protein (in particular, the amino acid sequence of amino acids 32-349). Another example of the NECTIN4 is a monkey NECTIN4 protein comprising the amino acid sequence under NCBI -_ XP_005541277.1, or a recombinant protein comprising the extracellular domain of the protein. Herein, unless otherwise specified, the term "NECTIN4" refers to NECTIN4 derived from a human. In the embodiments of the present disclosure, "antigen-binding specificity for NECTIN4", i.e., "antigen-binding domain specifically binding to NECTIN4", is preferably provided by a VHH domain.

Herein, the term "NECTIN4-positive" cell refers to a cell that exhibits positive cell surface expression of NECTIN4, such as a cancer cell, an engineered cancer cell, or an engineered non-tumor cell. The expression level of NECTIN4 on the cell surface can be determined by any conventional method known in the art for determining the expression level of cell surface antigens, for example, an FACS detection method or an immunofluorescence staining method. NECTIN4 has significantly higher expression levels on various tumor cells than on normal tissues/cells, for example, HT1376 (human bladder cancer cells) and BT474 (human breast ductal carcinoma cells). Preferably, the NECTIN4-positive cells are NECTIN4-positive tumor cells.

Herein, the term "affinity" or "binding affinity" refers to the strength of the sum of all noncovalent interactions between a single binding site of a molecule (e.g., an antibody) and its binding partner (e.g., an antigen epitope). "Binding affinity" reflects the intrinsic binding affinity of a 1:1 interaction between members of a binding pair. Generally, the binding affinity can be expressed by a binding dissociation equilibrium constant (K_{D}) and can be measured by common methods known in the art, such as surface plasmon resonance (SPR) technique.

Herein, the term "avidity" or "binding avidity" refers to the combined strength of interactions between multiple binding sites of a molecule (antibody) and the same target. Thus, one requirement for avidity is: the polyvalence of the molecule (e.g., the antibody) to a target.

Herein, the term "immunoglobulin" refers to a protein having the structure of a naturally occurring antibody. For example, an IgG immunoglobulin is a heterotetrameric glycoprotein of about 150,000 Daltons consisting of two light chains and two heavy chains that are disulfide-bonded. Each immunoglobulin heavy chain has a heavy chain variable region (VH), also referred to as a heavy chain variable domain, followed by three heavy chain constant domains (CH1, CH2, and CH3) from the N-terminus to the C-terminus. Similarly, each immunoglobulin light chain has a light chain variable region (VL), also referred to as a light chain variable domain, followed by a light chain constant domain (CL) from the N-terminus to the C-terminus. The heavy chains of an immunoglobulin can be assigned to one of 5 classes, α (IgA), δ (IgD), ε (IgE), γ (IgG), or µ (IgM) based on the types of constant regions thereof, in which some classes can be further divided into subclasses, such as γ1 (IgG1), γ2 (IgG2), γ3 (IgG3), γ4 (IgG4), α1 (IgA1), and α2 (IgA2). The light chains of the immunoglobulin can also be divided into one of the two classes, κ or λ, based on the amino acid sequences of constant domains thereof.

Herein, the term "isotype" refers to the class of antibodies determined according to the heavy chain constant regions of the antibodies. For example, the antibodies according to the present invention may be IgA (e.g., IgA1 or IgA2), IgG1, IgG2 (e.g., IgG2a or IgG2b), IgG3, IgG4, IgE, IgM, and IgD antibodies, and have heavy chain constant regions of the immunoglobulin class. Furthermore, the present invention contemplates not only antibodies having native sequence constant regions, but also antibodies comprising variant sequence constant regions.

Herein, the term "variable region" or "variable domain" refers to a domain of a heavy chain or light chain of an antibody involved in the binding of the antibody to an antigen. In the case of the heavy-chain antibody, for example, a heavy-chain antibody from Camelidae, a single VH domain (herein, also referred to as the VHH domain) may be sufficient to provide antigen-binding specificity. The VHH domain, like the heavy chain variable region and the light chain variable region of a conventional IgG antibody, comprises four conserved framework regions (FRs) and three complementary determining regions (CDRs), arranged in the order FR1-CDR1-FR2-CDR2-FR3-CDR3-FR4. In some aspects according to the present invention, one or more residues in variable regions of the antibody may be modified, for example, one or more CDR regions and/or one or more framework regions are subjected to residue modifications, particularly conservative residue substitutions, to obtain antibody variants that still substantially retain at least one biological property (e.g., antigen-binding ability) of the parent antibody. In still other aspects, the variable regions of the antibody may be modified by CDR grafting. Since CDR sequences are responsible for most of the antibody-antigen interactions, recombinant antibody variants that simulate the properties of known antibodies can be constructed. In such antibody variants, CDR sequences from known antibodies are grafted onto the framework regions of different antibodies with different properties, and one to several residue mutations, such as back mutations, may be performed as needed to refine the desired properties of the antibody. Properties of the mutated and/or modified antibodies or ADCs comprising same, such as target antigen-binding properties or other desired functional properties, e.g., endocytic activity, pharmacokinetics, and *in vivo* tumor killing activity, can be assessed in an *in vitro* or *in vivo* assay. Thus, variants of any of the variable regions (e.g., VHH) presented herein are also contemplated by the present invention.

Herein, "complementary determining region" or "CDR region" or "CDR" or "hypervariable region" is a region in an antibody variable domain that is highly variable in sequence and forms a structurally defined loop ("hypervariable loop") and/or contains antigen-contacting residues ("antigen-contacting sites"). CDRs are primarily responsible for binding to antigen epitopes. In the VHH domain of the antibody of the present invention, the CDRs are numbered sequentially from the N-terminus, and are generally referred to as CDR1, CDR2, and CDR3. The CDR sequences in a defined VHH domain can be determined using schemes well known in the art, for example, by defining the region ranges of the CDRs and combined ranges thereof using Kabat, AbM, Chothia, Contact, and IMGT schemes. Unless otherwise stated, the term "CDR" or "CDR sequence" used herein encompasses CDR sequences determined by any one of the schemes and combinations thereof. In addition, it is known in the art that although CDRs vary from antibody to antibody, only a limited number of amino acid positions within the CDRs are directly involved in antigen binding. The smallest overlapping region can be determined using at least two of the Kabat, Chothia, AbM, and Contact schemes, thereby providing a "minimal binding unit" for antigen binding. Such a minimal binding unit may be a sub-portion of the CDR. As will be understood by those skilled in the art, residues in the rest of the CDR sequences can be determined by the antibody structure and protein folding. Thus, variants of any of the CDRs presented herein are also contemplated by the present invention. For example, in a variant of the CDR, the amino acid residues of the minimal binding unit may remain unchanged, while the remaining CDR residues may be substituted.

Unless otherwise stated, residue positions of antibody variable regions and CDRs (including heavy chain variable region residues) are numbered according to the Kabat numbering system.

Herein, the terms "VHH" and "VHH domain" are used interchangeably, are used to refer to a heavy chain variable domain derived from a heavy-chain antibody lacking a light chain, sometimes also referred to as a single variable domain fragment (sVD). Thus, the VHH differs from the conventional VH of a four-chain immunoglobulin in that it does not need to be paired with a light chain variable domain to form an antigen-binding site. Such a VHH molecule may be derived from antibodies produced in Camelidae species such as camels, alpacas, dromedaries, llamas, and guanacos. Species other than Camelidae may also produce heavy-chain antibodies naturally devoid of light chains, and such VHHs are also within the scope of the present invention. In some cases, for therapeutic applications of antibodies or molecules derived therefrom, it is desirable to reduce their immunogenicity. Thus, preferably, in one embodiment, the antibody of the present invention comprises a humanized variable region (e.g., a VHH domain).

Herein, the term "half-life extension domain" refers to a chemical structure capable of endowing a molecule (e.g., an antibody) to which it binds with an increased circulating half-life following administration to an animal. Such a chemical structure includes, for example, flexible hydrophilic molecules (e.g., carbohydrates or PEG (polyethylene glycol)), immunoglobulin Fc regions, serum albumin, serum albumin-binding domains (e.g., small organic molecules, fatty acids, peptides, and proteins capable of binding to the serum albumin), or serum albumin binding peptides. The half-life extension domain may be conjugated with the antibody of the present invention by chemical conjugation or fusion, depending on its specific properties. Preferably, the half-life extension domain for use in the present invention is a peptide or polypeptide structure.

Herein, "serum albumin binding peptide" refers to a peptide or polypeptide that is capable of binding to the serum albumin so as to act as the half-life extension domain. Examples of such peptides or polypeptides include, but are not limited to, fragments derived from anti-serum albumin antibodies, such as Fab, single-chain Fab, scFv, single variable domain fragments (sVD), VHH, and bacterial albumin-binding domains and peptides, such as albumin-binding domains from Streptococcal protein G (SPG for short). Such peptides or polypeptides may be conjugated to the antibody molecule of the present invention by genetic fusion, thereby avoiding chemical coupling moieties. See, e.g., Expert Opin Biol Ther. 2016 Jul; 16(7):903-15. doi: 10.1517/14712598. 2016. 1165661.

Herein, the terms "immunoglobulin Fc region", "Fc region", and "Fc domain" are used interchangeably to define a C-terminus region of an immunoglobulin heavy chain, which comprises at least a portion of a constant region. The term includes Fc regions of native sequences and variant Fc regions. The Fc regions that can be used in the antibody of the present invention include, but are not limited to, Fc regions of IgG1, IgG2, IgG3, or IgG4 having native or variant sequences. Unless otherwise stated herein, amino acid residues in the Fc region or the heavy chain constant region are numbered according to the EU numbering system (also referred to as the EU Index) as described in, for example, Kabat et al., SEQuences of Proteins of Immunological Interest, 5th Ed., Public Health Service, National Institutes of Health, Bethesda, MD, 1991. Herein, the term "Fc region" or "Fc domain" does not comprise a heavy chain variable region VH and a light chain variable region VL as well as a heavy chain constant region CH1 and a light chain constant region CL of an immunoglobulin, but may comprise a CH2 domain and a CH3 domain, and comprises or does not comprise an immunoglobulin hinge region. For example, in some examples, the Fc region may comprise or consist of, from the N-terminus to the C-terminus, a CH2 domain and a CH3 domain. In other examples, the Fc region may comprise or consist of, from the N-terminus to the C-terminus, an immunoglobulin hinge region or a portion thereof, a CH2 domain, and a CH3 domain.

Herein, the term "native sequence Fc region" encompasses various naturally occurring immunoglobulin Fc region sequences, such as Fc region sequences of various Ig subclasses and allotypes thereof (Gestur Vidarsson et al., IgG subclasses and allotypes: from structure to effector functions, 20 October 2014, doi: 10.3389/fimmu.2014.00520.). In some embodiments, the heavy chain Fc region of human IgG has an amino acid sequence extending from Cys226 or from Pro230 to the carboxyl terminus of the heavy chain. However, the C-terminal lysine (Lys447) of the Fc region may or may not be present. In some embodiments, the heavy chain Fc region of human IgG carries a hinge sequence or a part of the hinge sequence of a native immunoglobulin at the N-terminus, e.g., according to EU numbering, the sequence from E216 to T225 or the sequence from D221 to T225.

Herein, the term "variant sequence Fc region" refers to an Fc region polypeptide comprising modifications relative to the native sequence Fc region polypeptide. The modifications may be the additions, deletions, or substitutions of amino acid residues. The substitutions may include substitutions of naturally occurring amino acids and non-naturally occurring amino acids. The purpose of the modifications may be to alter the binding of the Fc region to its receptor and the effector functions elicited thereby, or the purpose of the modifications may be to prevent undesired heavy chain mismatches, or to introduce site-directed amino acid modifications that can be used to conjugate other active molecules.

Herein, the term "effector function" refers to biological activities attributed to an immunoglobulin Fc region that vary with immunoglobulin isotype. Examples of immunoglobulin effector functions include: Fc receptor binding, C1q binding and complement-dependent cytotoxicity (CDC), and antibody-dependent cell-mediated cytotoxicity (ADCC). Depending on the intended use of the antibody molecule, the Fc region of the antibody may be modified such that the antibody has altered effector functions, such as reduced or eliminated Fcγ receptor binding, relative to an antibody molecule having a wild-type Fc region.

Herein, the term "flexible linker peptide", "peptide linker", and "linker peptide" are used interchangeably and refer to a short amino acid sequence consisting of amino acids, such as glycine (G) and/or serine (S) and/or threonine residues (T) used alone or in combination, or a hinge region derived from an immunoglobulin.

Herein, the "percent identity (%)" of an amino acid sequence refers to the percentage of amino acid residues in a candidate sequence that are the same as those of a specific amino acid sequence shown in the specification when aligning the candidate sequence with the specific amino acid sequence shown in this specification, with gaps introduced if necessary to achieve maximum percent sequence identity and without considering any conservative substitutions as part of sequence identity. In some embodiments, the present invention considers variants of the antibody molecule of the present invention that have a considerable degree of identity to the antibody molecule and the sequence thereof specifically disclosed herein; for example, the identity is at least 80%, 85%, 90%, 95%, 97%, 98%, or 99% or higher. The variants may comprise conservative modifications.

For polypeptide sequences, "conservative modifications" include substitutions of, deletions of, or additions to a polypeptide sequence that result in the substitution of a certain amino acid with a chemically similar amino acid. Conservative substitution tables providing functionally similar amino acids are well known in the art. The following 8 groups contain amino acids that are conservatively substituted with each other: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M) (see, for example, Creighton, Proteins (1984)). In some embodiments, the term "conservative sequence modification" is particularly used to refer to an amino acid modification that does not significantly affect or alter the desired properties (e.g., binding characteristics and/or internalization characteristics) of an antibody containing the amino acid sequence.

Herein, the term "bind to" or "specifically bind to" means that the binding effect is selective for antigens and may be distinguished from undesired or non-specific interactions. The ability of an antigen-binding site to bind to a specific antigen can be determined by an enzyme-linked immunosorbent assay (ELISA) or conventional binding assays known in the art, e.g., by detecting the ability of the antibody to bind to the antigen by the ELISA assay described in the examples, or by detecting the ability of the antibody to bind to cells expressing the antigen on the surfaces by the FACS assay described in the examples, or by detecting the affinity constant K_{D} using the SPR technique described in the examples.

Herein, the term "epitope" refers to a portion of an antigen to which an antibody specifically binds. Epitopes may consist of contiguous and/or non-contiguous amino acids that form conformational space units. Different antibodies that bind to the same antigen can be subjected to epitope binning by competitive binding tests. When the antibody to be tested blocks the binding of a reference antibody to the antigen (e.g., TROP2 or NECTIN4) by 50% or more in the competitive binding test, and conversely, when the reference antibody blocks the binding of the antibody to the antigen (e.g., TROP2 or NECTIN4) by 50% or more in the competitive binding test, the antibody to be tested and the reference antibody can be considered to be "antibodies competing for binding". The antibody that competes for binding and the reference antibody can bind to the same epitope region, e.g., the same epitope, adjacent epitopes, or overlapping epitopes. The competitive binding test can be performed by methods known in the art, such as direct or indirect solid-phase radioimmunoassay (RIA), direct or indirect solid-phase enzyme immunoassay (EIA), and sandwich competition assay, or using the methods described in the examples herein.

Herein, "humanized" antibody refers to a chimeric antibody comprising amino acid residues from non-human CDRs and amino acid residues from human FRs. In some embodiments, in the humanized antibody, all or substantially all CDRs (e.g., CDRs) correspond to those of a non-human antibody, and all or substantially all FRs correspond to those of a human antibody. A humanized antibody may optionally comprise at least a portion of an antibody constant region derived from a human antibody. "Humanized form" of an antibody (e.g., a non-human antibody) refers to an antibody that has been humanized. Herein, in some embodiments, the humanized antibody of the present invention has framework region sequences "derived from" a specific human germline sequence. As used herein, "derived from" means that the amino acid sequence of the framework region of the antibody has at least 85% or 90% identity to the amino acid sequence of the corresponding framework region encoded by a human germline immunoglobulin gene, and the antibody retains the antigen-binding activity.

Herein, an amino acid sequence (e.g., VHH) is said to have "cross-reactivity" to two different antigens or antigenic determinants if it is specific for the two different antigens or antigenic determinants (e.g., TROP2 or NECTIIN4 from different mammalian species, e.g., human TROP2 or human NECTIN4, cynomolgus monkey TROP2 or cynomolgus monkey NECTIN4). It would be advantageous for the antibody to have human-monkey species cross-reactivity, in particular having similar human-monkey antigen-binding affinity, and this property may facilitate preclinical drug development of the antibody, such as toxicological detection of ADC molecules formed from the antibodies. In some embodiments, the antibody of the present invention preferably has human-monkey species cross-reactivity.

Herein, the terms "endocytosis" and "internalization" are used interchangeably and refer to the process whereby a ligand/receptor complex is internalized and delivered into the cytosol or transferred to a suitable intracellular compartment, triggered by binding of the ligand to the corresponding receptor on the cell surface. In some embodiments, the antibodies of the present invention trigger TROP2 and/or NECTIN4 receptor-mediated endocytosis upon binding to TROP2 and/or NECTIN4 expressed on the cell surface. Herein, the endocytic activity of the antibody can be characterized by determining the endocytosis and the endocytosis rate, for example, using the methods described in the examples. In some embodiments, the antibodies of the present invention with endocytic activity can be used as a tool for transporting an anti-tumor drug into cancer cells in the ADCs of the present invention.

Herein, the term "host cell" refers to a cell into which an exogenous polynucleotide has been introduced, including the progeny of such cells. Host cells include "transformants" and "transformed cells", which include primary transformed cells and progeny derived therefrom. Host cells are any type of cell system that can be used to produce the antibody molecule of the present invention, including eukaryotic cells, e.g., mammalian cells, insect cells, and yeast cells, as well as prokaryotic cells, e.g., *Escherichia coli* cells. Host cells include cultured cells, as well as cells within a transgenic animal, a transgenic plant, or a cultured plant tissue or animal tissue.

Herein, the term "expression vector" refers to a vector comprising a recombinant polynucleotide, which comprises an expression control sequence effectively linked to a nucleotide sequence to be expressed. The expression vector comprises sufficient cis-regulatory elements for expression, and other elements for expression may be provided by the host cell or in an *in vitro* expression system. The expression vector includes all those known in the art, including cosmids, plasmids (e.g., naked or contained in liposomes), and viruses (e.g., lentiviruses, retroviruses, adenoviruses, and adeno-associated viruses) into which recombinant polynucleotides are incorporated.

Herein, when referring to "synergistic endocytosis", "synergistic binding", and "synergistic killing", the expression "synergistic" means that the antibody of the present invention having dual-targeting binding sites for TROP2 and NECTIN4 or an ADC molecule based thereon has an increased endocytosis, binding, or killing effect as compared to a reference antibody having the corresponding single-targeting binding site for TROP2 or NECTIN4 (e.g., a parent antibody of TROP2 or NECTIN4) or an ADC molecule based thereon, when tested under the same conditions.

Herein, the term "immunoconjugate" generally refers to a molecule formed by conjugating one or more immunoglobulin-related molecules or fragments thereof (e.g., the antibody or the fragment thereof) with one or more additional molecules. An immunoconjugate typically comprises at least one non-proteinaceous chemical structural moiety, for example, a chemical linker for achieving the conjugation. In some cases, the additional molecule may be a proteinaceous molecule, e.g., a peptide, polypeptide, or protein. In some cases, the additional molecule may also be a non-proteinaceous molecule, e.g., a chemical toxin. In some cases, the additional molecule may be an immunoglobulin-related molecule or a fragment thereof. In some cases, the additional molecule may be different from an immunoglobulin-related molecule or a fragment thereof. The one or more additional molecules may be the same or different from each other. For example, the additional molecule may be a target-binding element and/or an effector element, such as a chemotherapeutic agent, a toxin, a drug (e.g., an immunotherapeutic agent), a radioactive element, a probe, or a signaling molecule.

Herein, the terms "individual" and "subject" are used interchangeably and refer to mammals. The mammals include, but are not limited to, domesticated animals (e.g., cows, sheep, cats, dogs, and horses), primates (e.g., human and non-human primates, such as monkeys), rabbits, and rodents (e.g., mice and rats). In particular, the individual is a human.

Herein, the term "treatment", "treat", or "treating" refers to a clinical intervention intending to alter the natural progress of a disease in an individual being treated. Desired therapeutic effects include, but are not limited to, preventing occurrence or recurrence of diseases, alleviating symptoms, reducing any direct or indirect pathological outcomes of diseases, preventing metastasis, delaying disease progression, improving or alleviating disease conditions, and alleviating or improving prognosis. In the context of tumor or cancer treatment, "treatment" encompasses anti-tumor biological effects that can be caused by human intervention (e.g., by administration of drugs), including but not limited to, for example, a decrease in tumor volume, a decrease in the number of tumor cells, a decrease in tumor cell proliferation, or a decrease in tumor cell viability.

Herein, the terms "cancer" and "tumor" are used interchangeably and refer to or describe a physiological condition in mammals that is typically characterized by unregulated cell growth. Examples of cancers include, but are not limited to, carcinoma, solid tumors, and liquid tumors. In certain embodiments, the cancer suitable for treatment with the antibody or the immunoconjugate or the immunofusion of the present invention includes TROP2-positive and/or NECTIN4-positive tumors/cancers, including metastatic forms thereof.

As used herein, "antibody-drug conjugate (ADC)" refers to a compound/molecule obtained by linking the antibody to a (small-molecule) drug via a linker.

The term "linker" refers to a structural fragment that links a drug (e.g., a small-molecule drug) to an antibody moiety. It should be understood that the linker, prior to linking to the antibody or the antigen-binding fragment thereof, has a functional group that can form a bond with a functional group of the antibody or the antigen-binding fragment thereof.

The term "linker-payload" refers to a compound formed by a payload, such as a drug (e.g., a small-molecule drug), linked to a linker.

The term "alkyl" described herein refers to a fully saturated branched or unbranched hydrocarbon group. Alkyl preferably comprises 1-16 carbon atoms, e.g., 1-12 carbon atoms, 1-10 carbon atoms, 1-6 carbon atoms, or 1-4 carbon atoms. Representative examples of alkyl include, but are not limited to, methyl, ethyl, *n*-propyl, isopropyl, *n*-butyl, *sec*-butyl, isobutyl, *tert*-butyl, *n-*pentyl, isopentyl, neopentyl, *n*-hexyl, 3-methylhexyl, 2,2-dimethylpentyl, 2,3-dimethylpentyl, *n-*heptyl, *n*-octyl, *n*-nonyl, *n*-decyl, and the like.

The term "alkenyl" refers to a linear or branched hydrocarbyl group comprising 2-16 carbon atoms and at least one double bond and no triple bond. The alkenyl group preferably comprises 2-12 carbon atoms, 2-10 carbon atoms, 2-8 carbon atoms, 2-6 carbon atoms, or 2-4 carbon atoms. Representative examples of alkenyl include, but are not limited to, ethenyl, propenyl, butenyl, pentenyl, hexenyl, and the like.

The term "alkynyl" refers to a linear or branched hydrocarbyl group comprising 2-16 carbon atoms and at least one triple bond. The alkynyl group preferably comprises 2-12 carbon atoms, 2-10 carbon atoms, 2-8 carbon atoms, 2-6 carbon atoms, or 2-4 carbon atoms. Representative examples of alkynyl include, but are not limited to, ethynyl, propynyl, butynyl, pentynyl, hexynyl, and the like.

The term "halogen" or "halo" refers to fluorine (-F), chlorine (-Cl), bromine (-Br), or iodine (-I).

The term "haloalkyl" refers to alkyl, as defined herein, substituted with one or more halogen groups as defined herein. The haloalkyl may preferably be monohaloalkyl, dihaloalkyl, or polyhaloalkyl (including perhaloalkyl). The monohaloalkyl group may contain one iodine, bromine, chlorine, or fluorine in the alkyl. The dihaloalkyl and polyhaloalkyl groups may contain two or more identical halogen atoms in the alkyl or contain a combination of different halogen groups. Preferably, the polyhaloalkyl comprises up to 12, 10, 8, 6, 4, 3, or 2 halogen groups. Nonlimiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, and dichloropropyl. The perhaloalkyl refers to alkyl in which all hydrogen atoms are substituted with halogen atoms.

The term "haloalkenyl" refers to alkenyl, as defined herein, substituted with one or more halogen groups as defined herein. The term "haloalkynyl" refers to alkynyl, as defined herein, substituted with one or more halogen groups as defined herein. The meaning of "halo" as defined for "haloalkyl" is applicable to "haloalkenyl" and "haloalkynyl".

The term "amino acid" refers to naturally occurring amino acids and synthetic amino acids. The amino acids may be either L isomers or D isomers. The conventional amino acids referred to herein are written following conventional usage. See, e.g., Immunology-A Synthesis (2nd Edition, E. S. Golub and D. R. Gren, Eds., Sinauer Associates, Sunderland, Mass. (1991)), which is incorporated herein by reference. Besides, in the present disclosure, the amino acids are generally represented by single-letter and three-letter abbreviations known in the art. For example, the amino acid may be selected from phenylalanine (Phe; F), tyrosine (Tyr; Y), leucine (Leu; L), glycine (Gly; G), alanine (Ala; A), valine (Val; V), lysine (Lys; K), citrulline (Cit), serine (Ser; S), glutamic acid (Glu; E), aspartic acid (Asp; D), asparagine (Asn), isoleucine (Ile), arginine (Arg), proline (Pro), and glutamine (Gln).

The term "penturonic acid" refers to a compound formed by oxidizing the primary hydroxyl group of a pentose to a carboxyl group. Examples of penturonic acids include, but are not limited to, xyluronic acid and arabinuronic acid.

The term "hexuronic acid" refers to a compound formed by oxidizing the primary hydroxyl group of a hexose to a carboxyl group. Examples of hexuronic acids include, but are not limited to, glucuronic acid, galacturonic acid, and mannuronic acid.

The term "pentose", also known as five-carbon sugar, refers to a monosaccharide containing five carbon atoms. Pentoses include D- and/or L-pentoses. Examples of pentoses include, but are not limited to, xylose, arabinose, ribose, and deoxyribose.

The term "hexose", also known as six-carbon sugar, refers to a monosaccharide containing six carbon atoms. Hexoses include D- and/or L-hexoses. Examples of hexoses include, but are not limited to, glucose, galactose, mannose, and fructose.

The term "optional" or "optionally" means that the subsequently described event or circumstance may or may not occur, and the description includes instances where the event or circumstance occurs and instances where the event or circumstance does not occur. For example, when a group or structure is "optionally substituted", the group or structure may or may not be substituted.

The term "pharmaceutically acceptable salt" refers to salts that retain the biological effects and properties of the ADCs of the present invention and are not biologically or otherwise undesired. The ADCs of the present invention may exist in their pharmaceutically acceptable salt forms, including acid addition salts and base addition salts. In the present invention, pharmaceutically acceptable non-toxic acid addition salts refer to salts formed by the ADCs of the present invention and organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. Pharmaceutically acceptable non-toxic base addition salts refer to salts formed by the ADCs of the present invention and organic or inorganic bases including, but not limited to, alkali metal salts (e.g., lithium, sodium, or potassium salts), alkaline earth metal salts (e.g., calcium or magnesium salts), and organic base salts (e.g., ammonium salts formed by reaction with N group-containing organic bases).

The term "solvate" refers to an association compound formed by one or more solvent molecules and the antibody-drug conjugate (ADC) of the present invention. Solvents for forming solvates include, but are not limited to, water, methanol, ethanol, isopropanol, ethyl acetate, tetrahydrofuran, N,N-dimethylformamide, dimethylsulfoxide, and the like.

"Pharmaceutically acceptable" and "pharmaceutical" are used interchangeably herein, provided that there is no contradiction according to the context.

The term "drug-to-antibody ratio" or "DAR" refers to the ratio of the drug moiety (D) conjugated to the Ab moiety described herein to the Ab moiety in the antibody-drug conjugate molecule. In some embodiments described herein, DAR may be determined by p in formula I; for example, DAR may be 1-16, e.g., 2-16, 4-16, 5-12, 6-10, 2-8, 3-8, 2-6, 4-6, or 6-10, e.g., 2, 3, 4, 5, 6, 7, 8, 9, 10, 11, 12, 13, 14, or 15. DAR may also be calculated as the average DAR of the molecular population in the product, i.e., the overall ratio of the drug moiety (D) conjugated to the Ab moiety described herein to the Ab moiety in the product as measured by detection methods (e.g., by conventional methods such as mass spectrometry, ELISA assay, electrophoresis, and/or HPLC), and such a DAR is referred to herein as average DAR. In some embodiments, the average DAR value of the conjugate of the present invention is 1-16, e.g., 2-16, 4-16, 5-12, 6-10, 2-8, 3-8, 2-6, 4-6, or 6-10, e.g., 1.0-8.0 or 2.0-6.0, e.g., 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, and ranges with any two of these numerical values as endpoints. It should be understood that when referring to average DAR values, the ADC of the present invention refers to a population of ADC molecules or a mixture of ADC molecules comprising ADC molecules having the same and/or different DARs.

The term "therapeutic agent" described herein encompasses any substance that is effective in preventing or treating a tumor, e.g., cancer, including a chemotherapeutic agent, a cytokine, an angiogenesis inhibitor, a cytotoxic agent, other antibodies, a small-molecule drug, or an immunomodulator (e.g., an immunosuppressant).

The term "cytotoxic agent" used herein refers to a substance that inhibits or prevents cell functions and/or causes cell death or cell destruction.

"Chemotherapeutic agents" include chemical compounds useful in the treatment of cancer or immune system diseases.

The term "drug" refers to an organic compound capable of modulating biological processes, particularly altering or preventing pathological processes.

The term "small-molecule drug" refers to an organic compound having a low molecular weight and capable of modulating biological processes, particularly altering or preventing pathological processes. "Small molecule" is defined as a molecule with a molecular weight of less than 10 kD, usually less than 2 kD, preferably less than 1 kD, and more preferably less than 500 D. The small-molecule drug includes, but is not limited to, organic molecules, organic molecules containing inorganic components, molecules containing radioactive atoms, synthetic molecules, peptide mimetics, and antibody mimetics. As therapeutic agents, small molecules can penetrate cells better, are less susceptible to degradation, and are less likely to induce an immune response compared with large molecules.

The term "immunomodulator" used herein refers to a natural or synthetic active agent or drug that suppresses or modulates (e.g., activates) an immune response. The immune response may be a humoral response or a cellular response. The immunomodulator includes an immunosuppressant or an immunoagonist. In some embodiments, the immunomodulator of the present invention includes an immune checkpoint inhibitor or an immune checkpoint agonist.

"Anti-tumor compound" is a pharmaceutically active compound having an effect on tumors, including but not limited to, cytotoxic agents or chemotherapeutic agents, such as the cytotoxic agents disclosed in WO2021/173773 and US5658920, such as a camptothecin compound exatecan (topoisomerase I inhibitor Exatecan), Dxd (a novel derivative of topoisomerase I inhibitor Exatecan), and an auristatin compound such as monomethyl auristatin E (MMAE). The structure is shown below:

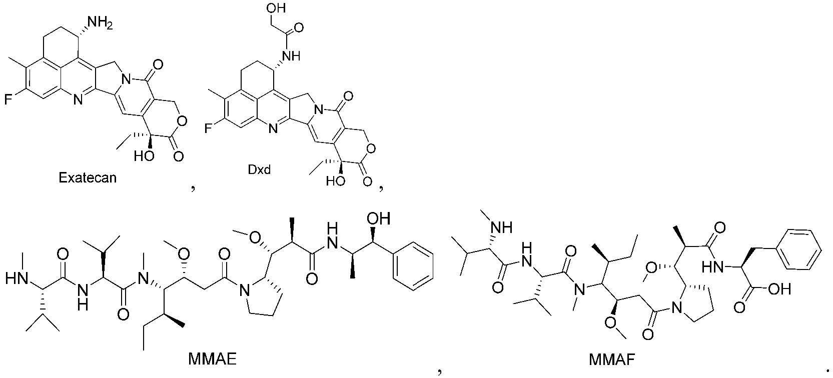

The term "effective amount" refers to an amount or dosage of the antibody, or ADC molecule, or composition, or combination of the present invention which generates expected effects in a patient in need of treatment or prevention after being administered to the patient in a single dose or multiple doses. "Therapeutically effective amount" and "prophylactically effective amount" may be included, depending on desired effects.

"Therapeutically effective amount" refers to an amount effective to achieve a desired therapeutic result at a necessary dose for a necessary period of time. The therapeutically effective amount is also such an amount that any toxic or adverse effect of the antibody, or ADC molecule, or composition, or combination is inferior to the therapeutically beneficial effect. The "therapeutically effective amount" preferably inhibits a measurable parameter (e.g., tumor volume) by at least about 30%, and even more preferably by at least about 40%, 45%, 50%, 55%, 60%, 65%, 70%, 75%, 80%, 85%, 90%, or even 100%, relative to untreated subjects.

"Prophylactically effective amount" refers to an amount effective to achieve a desired prophylactic result at a necessary dose for a necessary period of time. Generally, since a prophylactic dose is administered in a subject before or at an earlier stage of a disease, the prophylactically effective amount will be less than the therapeutically effective amount.

The term "anti-tumor effect" refers to a biological effect that can be demonstrated by a variety of means, including but not limited to, for example, a decrease in tumor volume, a decrease in the number of tumor cells, a decrease in tumor cell proliferation, or a decrease in tumor cell viability.

The term "pharmaceutical auxiliary material" refers to diluents, adjuvants (e.g., Freund's adjuvants (complete and incomplete)), excipients, carriers, stabilizers, or the like, which are co-administered with active substances.

The term "pharmaceutical composition" refers to such a composition that exists in a form allowing the effective biological activity of an active ingredient contained therein and does not contain additional ingredients having unacceptable toxicity to a subject to which the composition is administered.

The term "pharmaceutical combination", "combination product", "drug combination", or "combined product" refers to a non-fixed combination product or a fixed combination product, including but not limited to, a kit and a pharmaceutical composition. The term "non-fixed combination" means that the active ingredients (e.g., (i) the antibody molecule or ADC molecule of the present invention, and (ii) an additional therapeutic agent) are administered, either simultaneously or sequentially (without specific time limitation or at identical or different time intervals), to a patient as separate entities, wherein such administration provides two or more prophylactically or therapeutically effective active agents in the patient. In some embodiments, the antibody molecule or ADC molecule of the present invention and the additional therapeutic agent used in the pharmaceutical combination are administered at levels that do not exceed their levels when used alone. The term "fixed combination" means that two or more active agents are administered to a patient simultaneously in the form of a single entity. The dose and/or time intervals of two or more active agents are preferably selected such that the combined use of the components can result in a therapeutic effect on the disease or condition, which is superior to the effect achieved by the use of any one of the ingredients alone. The ingredients may each take a separate formulation form and such separate formulation forms may be the same or different.

The term "combination therapy" or "co-therapy" refers to the administration of two or more therapeutic agents or therapeutic modalities (e.g., radiotherapy or surgery) to treat the disease described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (such as tablets, capsules, powders, and liquids). The powder and/or liquid may be reconstituted or diluted to a desired dose before administration. In addition, such administration further includes using all types of therapeutic agents at approximately the same time or in a sequential manner at different times. In any case, the therapeutic regimen will provide the beneficial effect of the pharmaceutical combination in the treatment of conditions or symptoms described herein.

As used herein, "prevention", "prevent", or "preventing" includes the inhibition of the development or progression of symptoms of a disease or condition, or a specific disease or condition. In some embodiments, subjects with a family history of cancer are candidates for preventive regimens. Generally, in the context of cancer, the term "prevention", "prevent", or "preventing" refers to the administration of a drug prior to the onset of signs or symptoms of cancer, particularly in subjects at risk of cancer.

### I. Multispecific Antibody of Present Invention

Through intensive research, the inventors found that, by designing an antibody molecule that targets Trop2 and Nectin4 simultaneously, the on-target toxicity of the 2 targets can be reduced and the anti-tumor therapeutic effect can be improved. Accordingly, in a first aspect, the present invention provides a multispecific antibody binding to Trop2 and Nectin4, wherein the antibody comprises at least one antigen-binding domain specifically binding to Trop2 and at least one antigen-binding domain specifically binding to Nectin4. In some aspects, to prolong the half-life of the antibody of the present invention in the animal circulation, the antibody of the present invention further comprises a half-life extension domain, e.g., a serum albumin binding peptide or an immunoglobulin Fc region. The antibody of the present invention may take any suitable form, e.g., a single-chain or double-chain form, in which domains on the same chain may be linked by a peptide linker or directly linked as required.

The components of the multispecific antibody of the present invention are described in detail below. It will be understood by those skilled in the art that any combination of any of the technical features of these components is within the contemplation of the present invention, unless otherwise expressly stated to the contrary. Furthermore, it will be understood by those skilled in the art that the antibody (including any form of the antibody) of the present invention may comprise any such combination of features, unless otherwise expressly stated to the contrary.

### Antigen-binding domain

Although high-affinity antibodies were favored in early antibody screening, more and more studies showed that high-affinity antibodies are not equivalent to high efficacy. For example, high-affinity antibodies that bind to a rapidly internalizing target may be rapidly eliminated from circulation. In addition, high affinity may also cause the antibodies to be tightly bound or rapidly internalized by peripheral cells of the tumor tissue initially encountered, thereby limiting penetration of the antibodies into the tumor. Conversely, an antibody with excessively low affinity may also exhibit a low tumor retention rate due to ineffective binding to its receptor, although such an antibody may increase its tumor penetration efficiency. Therefore, depending on the specific application impact of the antibodies or antibody-based molecules, it may be more advantageous in some cases to screen and employ antibodies with moderate affinity that can allow for efficient delivery of the antibodies throughout the tumor, while allowing for accumulation of the antibodies in the tumor. Herein, "moderate affinity" means that the binding affinity K_{D} value of an antigen-binding domain for its target epitope is equal to or greater than 1 nM, but less than 500 nM, e.g., as determined by the surface plasmon resonance (SPR) technique.

In terms of the antibody targeting Trop2 and Nectin4 of the present invention, the inventors found during research that, in some cases (e.g., using the antibody as an ADC vehicle), additional advantages in terms of efficacy and safety may be advantageously conferred to the multispecific antibody of the present invention by selecting Trop2 and Nectin4 antigen-binding domains with moderate binding affinities, and by adjusting the valence of the Trop2 and Nectin4 antigen-binding domains in the antibody. For example, reducing the valence and the affinity of the Trop2-binding domain may help to reduce systemic toxicity of the antibody when being administered to an individual; while increasing avidity may play a role in balancing antibody efficacy and toxicity by comprising the moderate-affinity Nectin4-binding domain having a relatively high valence (e.g., bivalence). Furthermore, by selecting the antigen-binding domain in the antibody as a nanobody-based VHH domain, the enrichment rate and the enrichment amount of the antibody in tumor tissues can be further improved.

Thus, in some embodiments, the present invention provides a multispecific antibody comprising a Trop2-binding domain and a Nectin4-binding domain. In some embodiments, the Trop2 antigen-binding domain of the antibody according to the present invention has a moderate binding affinity for Trop2 (preferably human Trop2), for example, with a binding affinity K_{D} value of 50 × 10⁻⁸ M to 0.5 × 10⁻⁸ M, or 50 × 10⁻⁸ M to 1 × 10⁻⁸ M, optionally 15 × 10⁻⁸ M to 1 × 10⁻⁸ M. In some embodiments, the Nectin4 antigen-binding domain of the antibody according to the present invention has a moderate binding affinity for Nectin4 (preferably human Nectin4), for example, with a binding affinity K_{D} value of 50 × 10⁻⁸ M to 0.5 × 10⁻⁸ M, or 50 × 10⁻⁸ M to 1 × 10⁻⁸ M, optionally 30 × 10⁻⁸ M to 1 × 10⁻⁸ M. In some embodiments, the Trop2 and Nectin4 antigen-binding domains each have an antigen-binding affinity K_{D} value of 50 × 10⁻⁸ M to 0.5 × 10⁻⁸ M. In some embodiments, the Trop2-binding domain has a binding affinity K_{D} value of 10 × 10⁻⁸ M to 1 × 10⁻⁸ M for Trop2, and wherein the Nectin4-binding domain has a binding affinity of 30 × 10⁻⁸ M to 1 × 10⁻⁸ M, optionally 20 × 10⁻⁸ M to 1 × 10⁻⁸ M for Nectin4.

In some embodiments, the present invention provides a multispecific antibody comprising a Trop2 antigen-binding domain and a Nectin4 antigen-binding domain, wherein the ratio of the number or valence of the Trop2 antigen-binding domain to the Nectin4 antigen-binding domain is 1:1 or 1:2. In some embodiments, the valence of the multispecific antibody according to the present invention (i.e., the total number of the antigen-binding domains) is 2-5, preferably no more than 4. In some embodiments, the multispecific antibody according to the present invention is a trivalent antibody comprising 1 said Trop2-binding domain having moderate affinity and 2 said Nectin4-binding domains having moderate affinity. In some other embodiments, the multispecific antibody according to the present invention is a tetravalent antibody comprising 2 said Trop2-binding domains having moderate affinity and 2 said Nectin4-binding domains having moderate affinity.

In some embodiments, the antibody of the present invention is an "isolated" antibody. Herein, "isolated" antibodies refer to artificial antibodies, recombinantly produced antibodies, and antibodies that have been at least partially separated from components in the natural environment in which they were produced. In some embodiments, the isolated antibodies are purified to greater than 95% or 99% pure as determined, for example, by electrophoresis (e.g., SDS-PAGE, isoelectric focusing (IEF), or capillary electrophoresis) or chromatography (e.g., ion exchange or reverse-phase HPLC).

### Trop2 antigen-binding domain

In the multispecific antibody according to the present invention, in some embodiments, preferably, the antigen-binding site that specifically binds to TROP2 is provided by a VHH domain (also abbreviated herein as VHH^{Trop2}).

In some embodiments, the VHH^{Trop2} domain according to the present invention comprises CDR1, CDR2, and CDR3 sequences in a variable region having the amino acid sequence selected from SEQ ID NOs: 5, 9, 13, 29, 30, 98, and 99. Preferably, the CDRs are defined according to AbM, Chothia, Kabat, IMGT, or any combination thereof. Still preferably, the CDRs are defined according to Kabat or AbM or a combination thereof, and more preferably, the CDRs are defined according to AbM. It should be understood that the CDRs may also be defined according to any other methods known in the art.

In some embodiments, the VHH^{TROP2} domain according to the present invention comprises CDR1, CDR2, and CDR3 sequences, wherein the CDR1, CDR2, and CDR3 sequences:
(i) comprise or consist of the amino acid sequences of SEQ ID NOs: 6, 7, and 8, respectively;
(ii) comprise or consist of the amino acid sequences of SEQ ID NOs: 10, 11, and 12, respectively;
(iii) comprise or consist of the amino acid sequences of SEQ ID NOs: 14, 15, and 16, respectively; or
(iv) comprise or consist of the amino acid sequences of SEQ ID NOs: 31, 32, and 33, respectively.

In some embodiments, the VHH^{TROP2} domain according to the present invention comprises: a VHH variable region sequence or a variant thereof of any one of the exemplary antibodies of the present invention, such as an antibody or a fragment thereof, e.g., a humanized antibody, having the same CDR sequences as one of the exemplary antibodies and having the same or different framework region sequences. In some cases, preferably, the framework sequence is subjected to one to several residue mutations, for example, to remove post-translational modifications (PTMs) and/or to deimmunize. The retention or improvement of the antigen-binding properties or other functional properties of the mutated antibody can be assessed in an *in vitro* or *in vivo* assay.

In some embodiments, the VHH^{TROP2} domain according to the present invention comprises a variable region sequence having the amino acid sequence selected from SEQ ID NOs: 5, 9, 13, 29, 30, 98, and 99. In still further embodiments, the VHH^{TROP2} domain according to the present invention comprises an amino acid sequence having at least 80%, 85%, 90%, 95%, or 99% identity to the amino acid sequence selected from SEQ ID NOs: 5, 9, 13, 29, 30, 98, and 99 and retaining the ability to specifically bind to TROP2. In still further embodiments, the VHH^{TROP2} domain according to the present invention comprises an amino acid sequence having one or more (preferably 1-10, and more preferably 1-5) amino acid additions, deletions, and/or substitutions (e.g., conservative substitutions) compared to the amino acid sequence selected from SEQ ID NOs: 5, 9, 13, 29, 30, 98, and 99 and retaining the ability to specifically bind to TROP2. Preferably, the amino acid additions, deletions, and/or substitutions do not occur in the CDR regions.

In some preferred embodiments, the VHH^{TROP2} domain according to the present invention comprises CDR1-3 sequences of SEQ ID NOs: 10-12. In some embodiments, the VHH^{TROP2} domain comprises the amino acid sequence of SEQ ID NO: 9, or an amino acid sequence having at least 95%, 96%, 97%, 98%, or 99% sequence identity thereto. Preferably, the VHH^{TROP2} domain comprises or consists of the amino acid sequence set forth in SEQ ID NO: 9.

In some preferred embodiments, the VHH^{TROP2} domain according to the present invention comprises CDR1-3 sequences of SEQ ID NOs: 31-33. In some embodiments, the VHH^{TROP2} domain comprises the amino acid sequence of SEQ ID NO: 29, 30, 98, or 99, or an amino acid sequence having at least 95%, 96%, 97%, 98%, or 99% sequence identity thereto. Preferably, the VHH^{TROP2} domain comprises or consists of the amino acid sequence set forth in SEQ ID NO: 29 or 30.

### Nectin4 antigen-binding domain

In the multispecific antibody according to the present invention, in some embodiments, preferably, the antigen-binding site that specifically binds to NECTIN4 is provided by a VHH domain (also abbreviated herein as VHH^{Nectin4}).

In some embodiments, the VHH^{Nectin4} domain according to the present invention comprises CDR1, CDR2, and CDR3 sequences in a variable region having the amino acid sequence selected from SEQ ID NOs: 17, 21, 25, 34, 35, 38, and 100-106. Preferably, the CDRs are defined according to AbM, Chothia, Kabat, IMGT, or any combination thereof. Still preferably, the CDRs are defined according to Kabat or AbM or a combination thereof, and more preferably, the CDRs are defined according to AbM. It should be understood that the CDRs may also be defined according to any other methods known in the art.

In some embodiments, the VHH^{NECTIN4} domain according to the present invention comprises CDR1, CDR2, and CDR3 sequences, wherein the CDR1, CDR2, and CDR3 sequences:
(i) comprise or consist of the amino acid sequences of SEQ ID NOs: 18, 19, and 20, respectively;
(ii) comprise or consist of the amino acid sequences of SEQ ID NOs: 22, 23, and 24, respectively;
(iii) comprise or consist of the amino acid sequences of SEQ ID NOs: 26, 27, and 28, respectively;
(iv) comprise or consist of the amino acid sequences of SEQ ID NOs: 18, 36, and 20, respectively; or
(v) comprise or consist of the amino acid sequences of SEQ ID NOs: 18, 37, and 20, respectively.

In some embodiments, the VHH^{NECTIN4} domain according to the present invention comprises: a VHH variable region sequence or a variant thereof of any one of the exemplary antibodies of the present invention, such as an antibody or a fragment thereof, e.g., a humanized antibody, having the same CDR sequences as one of the exemplary antibodies and having the same or different framework region sequences. In some cases, preferably, the framework sequence is subjected to one to several residue mutations, for example, to remove post-translational modifications (PTMs) and/or to deimmunize. The retention or improvement of the antigen-binding properties or other functional properties of the mutated antibody can be assessed in an *in vitro* or *in vivo* assay.

In some embodiments, the VHH^{NECTIN4} domain according to the present invention comprises a variable region sequence having the amino acid sequence selected from SEQ ID NOs: 17, 21, 25, 34, 35, 38, and 100-106. In still further embodiments, the VHH^{NECTIN4} domain according to the present invention comprises an amino acid sequence having at least 80%, 85%, 90%, 95%, or 99% identity to the amino acid sequence selected from SEQ ID NOs: 17, 21, 25, 34, 35, 38, and 100-106 and retaining the ability to specifically bind to NECTIN4. In still further embodiments, the VHH^{NECTIN4} domain according to the present invention comprises an amino acid sequence having one or more (preferably 1-10, and more preferably 1-5) amino acid additions, deletions, and/or substitutions (e.g., conservative substitutions) compared to the amino acid sequence selected from SEQ ID NOs: 17, 21, 25, 34, 35, 38, and 100-106 and retaining the ability to specifically bind to NECTIN4. Preferably, the amino acid additions, deletions, and/or substitutions do not occur in the CDR regions.

In some preferred embodiments, the VHH^{NECTIN4} domain according to the present invention comprises CDR1-3 sequences of SEQ ID NOs: 22-24. In some embodiments, the VHH^{NECTIN4} domain comprises the amino acid sequence of SEQ ID NO: 21, or an amino acid sequence having at least 95%, 96%, 97%, 98%, or 99% sequence identity thereto. Preferably, the VHH^{NECTIN4} domain comprises or consists of the amino acid sequence set forth in SEQ ID NO: 21.

In some preferred embodiments, the VHH^{NECTIN4} domain according to the present invention comprises a CDR1 sequence of SEQ ID NO: 18, a CDR2 sequence of SEQ ID NO: 36, and a CDR3 sequence of SEQ ID NO: 20. In some embodiments, the VHH^{NECTIN4} domain comprises the amino acid sequence of SEQ ID NO: 34, 38, 102, 104, or 106, or an amino acid sequence having at least 95%, 96%, 97%, 98%, or 99% sequence identity thereto. Preferably, the VHH^{NECTIN4} domain comprises or consists of the amino acid sequence set forth in SEQ ID NO: 34 or 38.

In some preferred embodiments, the VHH^{NECTIN4} domain according to the present invention comprises a CDR1 sequence of SEQ ID NO: 18, a CDR2 sequence of SEQ ID NO: 37, and a CDR3 sequence of SEQ ID NO: 20. In some embodiments, the VHH^{NECTIN4} domain comprises the amino acid sequence of SEQ ID NO: 35, 103, or 105, or an amino acid sequence having at least 95%, 96%, 97%, 98%, or 99% sequence identity thereto. Preferably, the VHH^{NECTIN4} domain comprises or consists of the amino acid sequence set forth in SEQ ID NO: 35.

### Half-life extension domain

In addition to the aforementioned antigen-binding domains, in some embodiments, the multispecific antibody according to the present invention may further comprise a half-life extension domain to adjust the circulating half-life of the antibody after administration to an animal. Preferably, the half-life extension domain for use in the antibody of the present invention is a peptide or polypeptide structure, and thus may be conjugated to the antibody of the present invention by genetic fusion. Useful peptide or polypeptide half-life extension domains include, but are not limited to, an immunoglobulin Fc region, serum albumin, or a serum albumin binding peptide. In some embodiments, preferably, the antibody of the present invention comprises an immunoglobulin Fc region, particularly where the antibody adopts a double-chain configuration. In some embodiments, preferably, the antibody of the present invention comprises a serum albumin binding peptide, particularly where the antibody adopts a single-chain configuration. The half-life extension domain may be linked to the C- or N-terminus of the TROP2-binding domain or to the C- or N-terminus of NECTIN4.

### Immunoglobulin Fc region

The half-life extension domain may comprise an immunoglobulin Fc region. The immunoglobulin Fc region may be any immunoglobulin Fc region. The Fc region is a C-terminal constant domain of an immunoglobulin, which interacts with cell surface Fc receptors and some proteins of the complement system. The immunoglobulin Fc region typically comprises two or three heavy chain constant domains (referred to as CH2, CH3, and CH4) and a hinge region, and typically exists in a dimerized form. The two chains in the dimerized Fc region may be linked by disulfide bonds in the hinge region. Fc regions from the immunoglobulin isotypes IgG1, IgG2, and IgG4 are capable of binding to FcRn receptors and undergoing FcRn-mediated recycling to provide a long circulating half-life. The position at which the IgG interacts with FcRn has been determined to be in the Fc region covering a portion of the CH2 and CH3 domains.

The immunoglobulin Fc region for use in the multispecific antibody of the present invention may be from any immunoglobulin Fc region. In some embodiments, the immunoglobulin Fc region comprises at least an immunoglobulin CH2 domain and CH3 domain. In some embodiments, the immunoglobulin Fc region further comprises a hinge region or a portion thereof. In some embodiments, the immunoglobulin Fc region comprises or consists of, from the N-terminus to the C-terminus, an immunoglobulin hinge region or a portion thereof, a CH2 domain, and a CH3 domain. In some embodiments, the immunoglobulin Fc region comprises or consists of, from the N-terminus to the C-terminus, a CH2 domain and a CH3 domain. In some embodiments, the immunoglobulin Fc region is preferably from IgG1, IgG2, or IgG4, or a subtype thereof. Preferably, the immunoglobulin Fc region comprises an Fc region sequence from a human.

The immunoglobulin Fc region may be fused to the C- or N-terminus of other domains (i.e., TROP2- or NECTIN4-binding domain, e.g., VHH^{TROP2} or VHH^{NECTIN4}). The immunoglobulin Fc may be fused to other domains via a peptide linker, or directly fused to other domains. In embodiments where the immunoglobulin Fc region is fused to other domains at its N-terminus, the fusion is preferably performed by an immunoglobulin hinge region sequence.

In some cases, the immunoglobulin Fc region comprising a hinge region sequence is preferred, which may, for example, promote dimerization of the antibody polypeptide chains and/or provide a cysteine residue for conjugating to other active molecules. Such a hinge sequence may correspond substantially or in part to the hinge region of IgG1, IgG2, IgG3, or IgG4. For example, the hinge region sequence may include all or part of the core hinge region and all or part of the lower hinge region. The core hinge region has the amino acid sequence CPPC in IgG1, IgG2, and IgG3, and has the sequence CPSC in IgG4. Preferably, the hinge region contains at least one disulfide bond linking the two Fc chains. In some embodiments, the hinge region sequence comprises a hinge region sequence from E216 to T225 or a hinge region sequence from D221 to T225 (according to EU numbering) of IgG1, or a corresponding hinge region sequence from other immunoglobulin isotypes. In some embodiments, the hinge region sequence comprises the amino acid sequence of SEQ ID NO: 96 or 97.

The immunoglobulin Fc region for use in the multispecific antibody of the present invention may be a native Fc region sequence. Alternatively, the Fc region may comprise a mutation relative to the native Fc sequence. The mutation includes a substitution, an insertion, and/or a deletion. Such a mutation may be made with the purpose of introducing desired therapeutic properties. For example, to promote heterodimerization, a Knob-into-Hole (KiH) mutation may be introduced into the CH3 domain. In this case, one Fc chain is designed to contain a large protruding residue (i.e., Knob), while the other Fc chain is designed to contain a complementary pocket (i.e., Hole). The suitable position for the KiH mutation is known in the art. Exemplary KiH mutations include, but are not limited to, Knob mutation T366W in combination with Hole mutations T366S, L368A, and Y407V; and Knob mutation T366Y in combination with Hole mutation Y407T. Where the multispecific antibody of the present invention comprises an asymmetric double-chain structure, it is preferred that the Fc region comprises the KiH mutation that promotes proper heterodimerization of the antibody polypeptide chains. Additionally or alternatively, cysteine mutations may be introduced into the Fc region to increase disulfide bond linkage in the dimerized Fc region. For example, a mutation S354C is introduced into one Fc chain, and a mutation Y349C is introduced into the other Fc chain.

In addition, the Fc region may also contain a mutation that alters effector function, depending on the particular application of the antibody or antibody-based molecule. For example, where the effector function is not required, the Fc region may comprise a mutation that reduces or eliminates effector function. In some cases (e.g., where the antibody of the present invention is used as an ADC vehicle), preferably, the Fc region comprises a mutation that reduces or eliminates the binding of the Fc region to the Fcγ receptor, for example, a LALA mutation from lysine (L) to alanine (A) at positions 234 and 235 of the Fc region, to reduce Fcγ receptor-mediated off-target cytotoxicity. Additionally or alternatively, the mutation may be introduced into the Fc region to increase binding to FcRn and/or remove protease sites, and/or amino acid modifications that can be used for conjugating to active molecules can be introduced into the Fc region. Additionally or alternatively, the Fc region may be mutated for antibody production reasons, e.g., to remove or replace amino acids that may undergo post-translational modifications (e.g., glycosylation), thereby providing improved druggability and developability of therapeutic antibodies.

In some embodiments, the multispecific antibody according to the present invention comprises an Fc region from IgG, e.g., an Fc region of IgG1, IgG2, or IgG4, preferably an Fc region from human IgG1 or human IgG4. Preferably, the Fc region comprises the amino acid sequence selected from SEQ ID NOs: 83-87 and 111-117, or an amino acid sequence having at least 90%, 95%, 96%, 97%, 98%, 99%, or more identity thereto.

### Serum albumin binding peptide

The half-life extension domain may comprise a serum albumin binding peptide. As is known in the art, serum albumin has a long circulating half-life. Attachment to albumin is a well-known strategy to improve the *in vivo* circulating half-life of therapeutic molecules. The albumin can be linked non-covalently by using a specific albumin-binding domain, or linked covalently by conjugation or direct gene fusion.

The albumin-binding domain for use in the multispecific antibody of the present invention is preferably a peptide or polypeptide binding to serum albumin. Such a peptide or polypeptide is also referred to herein as a "serum albumin binding peptide". Examples of serum albumin binding peptides that can be used in the present invention include, for example, antibodies and antibody fragments that specifically bind to serum albumin, such as Albudab^{®} based on single-domain antibodies (O'Connor-Semmes et al., Clin Pharmacol Ther. Dec. 2014; 96(6):704-12); and artificial proteins based on natural proteins that bind to serum albumin (e.g., Streptococcal protein G), such as Albumod^{®}. Other examples of serum albumin binding peptides that may be used in the present invention include, for example, a number of ISVDs binding to human serum albumin as described in WO 04/041865, WO 06/122787, and WO 2012/175400, with the ISVD being selected from serum albumin-binding moieties of Alb-1, Alb-3, Alb-4, Alb-5, Alb-6, Alb-7, Alb-8, Alb-9, Alb-10, and Alb-23.

The serum albumin binding peptide may be fused to the C- or N-terminus of other domains (i.e., TROP2- or NECTIN4-binding domain, e.g., VHH^{TROP2} or VHH^{NECTIN4}). The serum albumin binding peptide may be fused to other domains via a peptide linker, or directly fused to other domains. Preferably, the serum albumin binding peptide is fused to other domains via a peptide linker. The sequence or optimal length of available peptide linkers can be easily determined from the linkage position. In some embodiments, preferably, the peptide linker is 5-15 amino acids in length, and more preferably, the peptide linker comprises the amino acid sequence G4S or (G4S)2.

In some embodiments, the serum albumin binding peptide for use in the multispecific antibody of the present invention is an antibody or antibody fragment specifically binding to serum albumin, particularly a single-chain antibody or single-domain antibody. In some embodiments, the serum albumin binding peptide is a VHH domain (also abbreviated herein as VHH^{SA}) specifically binding to serum albumin. Preferably, the serum albumin is human serum albumin (HSA). Preferably, the serum albumin binding peptide binds to human serum albumin with affinities ranging from micromolar to picomolar.

In some embodiments, the multispecific antibody according to the present invention comprises a VHH domain specifically binding to serum albumin. In some embodiments, the VHH^{SA} comprises CDR1, CDR2, and CDR3 in a variable region sequence having the amino acid sequence selected from SEQ ID NOs: 39 and 40. The CDRs may be defined according to AbM, Chothia, Kabat, IMGT, or any combination thereof. In some embodiments, the VHH^{SA} comprises the amino acid sequence of SEQ ID NO: 39 or 40 or an amino acid sequence having at least 90%, 95%, 96%, 97%, 98%, 99%, or more identity thereto. In some embodiments, the VHH^{SA} comprises or consists of the amino acid sequence of SEQ ID NO: 40.

### Peptide linker

In the multispecific antibody according to the present invention, the linkage between the antibody components (i.e., the antigen-binding domain and optionally the half-life binding domain) may be done with a peptide linker.

The peptide linker that can be used in the antibody of the present invention is not particularly limited. The peptide linker sequence is generally flexible. It may consist mainly of amino acids, such as glycine, alanine, and serine, which do not have large side chains that may limit flexibility. Alternatively, it may consist of sequences from the immunoglobulin hinge region. The sequence or optimal length of available peptide linkers can be readily determined by those skilled in the art, depending on the linkage position and the components to be linked.

Suitable peptide linkers may be 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids in length, or longer. In some cases, the peptide linker sequence may be short in length, e.g., less than about 20 or 15 amino acids in length, e.g., 2-15 amino acids in length or 5-10 amino acids in length.

Suitable peptide linker sequences include, but are not limited to, G4S (SEQ ID NO: 88); (G4S)2 (SEQ ID NO: 89); (G4S)3 (SEQ ID NO: 90); GGGSG (SEQ ID NO: 118); GGSGG (SEQ ID NO: 119); GSGGG (SEQ ID NO: 120); GSGGGP (SEQ ID NO: 121); GGEPS (SEQ ID NO: 122); GGEGGGP (SEQ ID NO: 123) and GGEGGGSEGGGS (SEQ ID NO: 124); (G4S)n (SEQ ID NO: 91), wherein n is an integer equal to or greater than 1; TS(G4S)n (SEQ ID NO: 92), wherein n is an integer equal to or greater than 1; G(G4S)n (SEQ ID NO: 93), wherein n is an integer equal to or greater than 1; (G4)n (SEQ ID NO: 94), wherein n is an integer equal to or greater than 1; and (GRPGS)n (SEQ ID NO: 95), wherein n is an integer equal to or greater than 1. Peptide linkers that can be used in the antibody molecule of the present invention may also be, for example, but are not limited to, the following amino acid sequences: (G₃S)₂ (SEQ ID NO: 125), (G₄S)₂ (SEQ ID NO: 89), (G₃S)₃ (SEQ ID NO: 126), (G₄S)₃ (SEQ ID NO: 90), (G₃S)₄ (SEQ ID NO: 127), (G₄S)₄ (SEQ ID NO: 128), (G₃S)₅ (SEQ ID NO: 129), (G₄S)₅ (SEQ ID NO: 130), (G₃S)₆ (SEQ ID NO: 131), (G₄S) (SEQ ID NO: 88), GGG (SEQ ID NO: 132), DGGGS (SEQ ID NO: 133), TGEKP (SEQ ID NO: 134), GGRR (SEQ ID NO: 135), EGKSSGSGSESKVD (SEQ ID NO: 136), KESGSVSSEQLAQFRSLD (SEQ ID NO: 137), GGRRGGGS (SEQ ID NO: 138), LRQRDGERP (SEQ ID NO: 139), LRQKDGGGSERP (SEQ ID NO: 140), and GSTSGSGKPGSGEGSTKG (SEQ ID NO: 141). Alternatively, a computer program may be used to simulate three-dimensional structures of proteins and peptides, or a suitable flexible linker peptide is rationally designed by a phage display method.

In some embodiments, the peptide linker for use in the antibody of the present invention is a flexible linker peptide of 5-50 amino acids, preferably a linker peptide comprising a glycine (G) and/or serine (S) and/or threonine (T) residue. In one embodiment, the peptide linker is 5-50 amino acids in length, e.g., 5, 10, 15, 20, 25, or 30 amino acids in length, or has an amino acid length falling between any two integers. In some embodiments, the peptide linker comprises the amino acid sequence (G₄S)ₙ (SEQ ID NO: 91), wherein n is an integer equal to or greater than 1; for example, n is an integer of 2, 3, 4, 5, 6, or 7. In still further embodiments, the peptide linker is a hinge region from an immunoglobulin.

In some preferred embodiments, in the multispecific antibody according to the present invention, the peptide linker for linking the antigen-binding domain and the half-life extension domain (if present) comprises the amino acid sequence G4S (SEQ ID NO: 88) or (G4S)2 (SEQ ID NO: 89).

### Form of multispecific antibody

The multispecific antibody according to the present invention may take any suitable form, e.g., a single-chain or double-chain form.

In some embodiments, the present invention provides a multispecific antibody in a single-chain form. In some embodiments, the multispecific antibody according to the present invention comprises a single polypeptide chain, wherein the polypeptide chain comprises at least one (preferably 1 or 2) Nectin4-binding domain and at least one (preferably 1 or 2) Trop2-binding domain and optionally at least one half-life extension domain, wherein the half-life extension domain is a serum albumin binding peptide; preferably, the domains are linked to each other via a peptide linker.

In some embodiments, the present invention provides a multispecific antibody in a double-chain form. In some embodiments, the multispecific antibody according to the present invention comprise a first polypeptide chain and a second polypeptide chain, wherein the first polypeptide chain and the second polypeptide chain each comprise at least one (preferably 1 or 2) Nectin4-binding domain and/or at least one (preferably 1 or 2) Trop2-binding domain and at least one (preferably 1) half-life extension domain, wherein the half-life extension domain is an immunoglobulin Fc region; preferably, domains on the same chain are linked to each other via a peptide linker or directly linked to each other.

### Single-chain form

In some embodiments, the present invention provides a multispecific antibody comprising a single polypeptide chain. In some embodiments, the polypeptide chain comprises, from the N-terminus to the C-terminus:

(VHH^{A})ₙ₁-(VHH^{B})ₙ₂-(HLE)ₙ₃-(VHH^{A})ₙ₄-(VHH^{B})ₙ₅-(HLE)ₙ₆, (I)

wherein n1, n2, n3, n4, n5, and n6 are each independently selected from integers of 0, 1, and 2; VHH^{A} and VHH^{B} represent VHH domains binding to an antigen A and an antigen B, respectively, wherein A and B are different from each other and are independently selected from Trop2 and Nectin4; HLE represents a serum albumin binding peptide as a half-life extension domain; the symbol "-" represents linkage via a peptide linker or direct linkage, preferably a peptide linker having 5-15 amino acids in length.

The multispecific antibody may have any suitable valency. In some cases, preferably, the multispecific antibody has a valency of 2 to 6 (i.e., n1 + n2 + n4 + n5 = 2 to 6), more preferably, no more than 4 (i.e., n1 + n2 + n4 + n5 = 2 to 4).

The number of VHH^{Nectin4} domains and the number of VHH^{Trop2} domains in the multispecific antibody may be equal or different. In some embodiments, preferably, the number of VHH^{Trop2} domains is no more than 4, preferably no more than 3, such as 1-2. In some embodiments, preferably, the number of VHH^{Nectin4} domains is no more than 4, preferably no more than 3, such as 1-2. In some embodiments, preferably, the ratio of the number of VHH^{Trop2} domains to the number of VHH^{Nectin4} domains is preferably 1:1 or 1:2. In one embodiment, the number of VHH^{Trop2} domains is 1 or 2, and the number of VHH^{Nectin4} domains is 1 or 2.

The multispecific antibody may or may not comprise an HLE domain as desired; for example, the antibody may comprise 0 or 1 HLE.

In some preferred embodiments, the present invention provides a multispecific antibody of the above formula I, wherein n1, n2, n3, n4, n5, and n6 are each independently selected from integers of 0, 1, and 2; and wherein n1 + n4 = 1 or 2, n2 + n5 = 1 or 2, and n3 + n6 = 0 or 1.

In some more preferred embodiments, the present invention provides a multispecific antibody comprising a single polypeptide chain, wherein the polypeptide chain comprises, from the N-terminus to the C-terminus:
(i) VHH^{A}-VHH^{B},
(ii) VHH^{A}-VHH^{B}-HLE,
(iii) VHH^{B}-VHH^{A}-VHH^{B},
(iv) VHH^{A}-VHH^{B}-VHH^{B},
(v) VHH^{A}-HLE-VHH^{B}-VHH^{B}, or
(vi) VHH^{A}-VHH^{A}-HLE-VHH^{B}-VHH^{B}, wherein preferably, A represents Trop2 and B represents Nectin4.

In the embodiment of the above antibody in a single-chain form, preferably, the HLE represents an anti-serum albumin VHH domain (VHH^{SA}). The VHH^{SA} domain may be any VHH^{SA} domain described herein or known in the art. For example, as an example, the VHH^{SA} domain may comprise the amino acid sequence selected from SEQ ID NOs: 39 and 40, or an amino acid sequence having at least 85%, 90%, 95%, or 99% identity thereto, or having one or more (preferably 1-10, and more preferably 1-5) amino acid additions, deletions, and/or substitutions. In some embodiments, preferably, the HLE comprises or consists of the amino acid sequence of SEQ ID NO: 40.

In the embodiment of the above antibody in a single-chain form, preferably, the domains are linked to each other via a peptide linker. Preferably, the peptide linker is 5-15 amino acids in length, and more preferably, comprises the amino acid sequence G4S or the amino acid sequence (G4S)2.

As understood by those skilled in the art, in the single-chain multispecific antibody of the present invention, a purification tag (e.g., His tag) or other helper peptide sequences may be optionally added at the N-terminus or C-terminus of the polypeptide chain in order to facilitate subsequent purification or linking to other active molecules. The addition may be achieved through a peptide linker (e.g., AAA) or through direct linkage.

The multispecific antibody having a polypeptide chain of formula (I) is preferably a trivalent bispecific antibody or a tetravalent bispecific antibody.

### Symmetric double-chain form

In some embodiments, the present invention provides a multispecific antibody comprising two identical polypeptide chains. In some embodiments, the present invention provides a multispecific antibody comprising a polypeptide chain shown below, wherein the polypeptide chain comprises, from the N-terminus to the C-terminus:

(VHH^{A})ₙ₁-(VHH^{B})ₙ₂-HLE-(VHH^{A})ₙ₃-(VHH^{B})ₙ₄, (II)

wherein n1, n2, n3, and n4 are each independently selected from integers of 0, 1, and 2;

VHH^{A} and VHH^{B} represent VHH domains binding to an antigen A and an antigen B, respectively, wherein A and B are different from each other and are independently selected from Trop2 and Nectin4; HLE represents an immunoglobulin Fc region as a half-life extension domain, particularly a human IgG1 or human IgG4 Fc region; the symbol "-" represents linkage via a peptide linker or direct linkage, preferably a peptide linker having 5-15 amino acids in length.

The multispecific antibody may have any suitable valency. In some cases, preferably, the multispecific antibody has a valency of 2 to 6 (i.e., 2 × (n1 + n2 + n3 + n4) = 2 to 6), more preferably, no more than 4.

The number of VHH^{Nectin4} domains and the number of VHH^{Trop2} domains in the multispecific antibody may be equal or different. In some embodiments, preferably, the number of VHH^{Trop2} domains is no more than 4, preferably no more than 3, such as 1-2. In some embodiments, preferably, the number of VHH^{Nectin4} domains is no more than 4, preferably no more than 3, such as 1-2. In some embodiments, preferably, the ratio of the number of VHH^{Trop2} domains to the number of VHH^{Nectin4} domains is preferably 1:1 or 1:2. In one embodiment, the number of VHH^{Trop2} domains is 1 or 2, and the number of VHH^{Nectin4} domains is 1 or 2.

In some preferred embodiments, n3 and n4 are 0, and the polypeptide chain having formula (II) comprises, from the N-terminus to the C-terminus: (VHH^{A})ₙ₁-(VHH^{B})ₙ₂-HLE, wherein n1 and n2 are each independently selected from integers of 1 and 2.

In some more preferred embodiments, n3 and n4 are 0, n1 and n2 are 1, and the polypeptide chain having formula (II) comprises, from the N-terminus to the C-terminus: VHH^{A}-VHH^{B}-HLE, such as VHH^{Nectin4}-VHH^{Trop2}-Fc or VHH^{Trop2}-VHH^{Nectin4}-Fc.

In some further preferred embodiments, the polypeptide chain having formula (II) comprises, from the N-terminus to the C-terminus, VHH^{Nectin4}-VHH^{Trop2}-Fc.

In the above multispecific antibodies, preferably, the antigen-binding domains on the polypeptide chain (i.e., VHH^{Trop2} domain and VHH^{Nectin4} domain) are linked to each other via a peptide linker. Preferably, the peptide linker is 5-15 amino acids in length, and more preferably, comprises the amino acid sequence G4S or the amino acid sequence (G4S)2. In the above multispecific antibody, preferably, the Fc region comprises a hinge region or a portion thereof and is directly linked to a domain located at its N-terminus.

The multispecific antibody having the above polypeptide chain of formula (II) may associate to form a homodimer as a result of dimerization of the immunoglobulin Fc region, thereby producing a multispecific binding molecule in a double-chain form. In some embodiments, the Fc region comprises an amino acid sequence from human IgG1 or IgG4. In some embodiments, the Fc region comprises a mutation that reduces or eliminates Fcγ receptor binding, such as a LALA mutation.

The multispecific antibody having a polypeptide chain of formula (II) is preferably a tetravalent bispecific antibody.

### Asymmetric double-chain form

In some embodiments, the present invention provides a multispecific antibody comprising two different polypeptide chains. In some embodiments, the present invention provides a multispecific antibody comprising a first polypeptide chain and a second polypeptide chain, wherein
the first polypeptide chain comprises, from the N-terminus to the C-terminus: (VHH^{A})ₙ₁-(VHH^{B})ₙ₂-HLE-(VHH^{A})ₙ₃-(VHH^{B})ₙ₄, (III)
the second polypeptide chain comprises, from the N-terminus to the C-terminus: (VHH^{B})ₘ₁-(VHH^{A})ₘ₂-HLE-(VHH^{B})ₘ₃-(VHH^{A})ₘ₄, (IV)
wherein n1, n2, n3, and n4, as well as m1, m2, m3, and m4, are each independently selected from integers of 0, 1, and 2;
VHH^{A} and VHH^{B} represent VHH domains binding to an antigen A and an antigen B, respectively, wherein A and B are different from each other and are independently selected from Trop2 and Nectin4; HLE represents an immunoglobulin Fc region as a half-life extension domain, particularly a human IgG1 or IgG4 Fc region; the symbol "-" represents linkage via a peptide linker or direct linkage, preferably a peptide linker having 5-15 amino acids in length.

The multispecific antibody may have any suitable valency. In some cases, preferably, the multispecific antibody has a valency of 2 to 6 (i.e., n1 + n2 + n3 + n4 + m1 + m2 + m3 + m4 = 2 to 6), more preferably, no more than 4.

The number of VHH^{Nectin4} domains and the number of VHH^{Trop2} domains in the multispecific antibody may be equal or different. In some embodiments, preferably, the number of VHH^{Trop2} domains is no more than 4, preferably no more than 3, such as 1-2. In some embodiments, preferably, the number of VHH^{Nectin4} domains is no more than 4, preferably no more than 3, such as 1-2. In some embodiments, preferably, the ratio of the number of VHH^{Trop2} domains to the number of VHH^{Nectin4} domains is preferably 1:1 or 1:2. In one embodiment, the number of VHH^{Trop2} domains is 1 or 2, and the number of VHH^{Nectin4} domains is 1 or 2.

In some preferred embodiments, n3, n4, m3, and m4 are 0, and the multispecific antibody comprises a first polypeptide chain and a second polypeptide chain, wherein
the first polypeptide chain comprises, from the N-terminus to the C-terminus: (VHH^{A})ₙ₁-(VHH^{B})ₙ₂-HLE,
the second polypeptide chain comprises, from the N-terminus to the C-terminus: (VHH^{B})ₘ₁-(VHH^{A})ₘ₂-HLE,
wherein n1 and n2, as well as m1 and m2, are each independently selected from integers of 0, 1, and 2, with the proviso that n1 and n2 are not simultaneously 0, m1 and m2 are not simultaneously 0, and the sum of n1 and m2, as well as the sum of n2 and m1, are greater than or equal to 1.

In some more preferred embodiments, n3, n4, m3, and m4 are 0, and m1 = 0 and m2 = 1, or m2 = 0 and m1 = 1; the multispecific antibody comprises a first polypeptide chain and a second polypeptide chain, wherein
the first polypeptide chain comprises, from the N-terminus to the C-terminus: (VHH^{A})ₙ₁-(VHH^{B})ₙ₂-HLE,
the second polypeptide chain comprises, from the N-terminus to the C-terminus: (VHH^{B})ₘ₁-HLE_{;} or (VHH^{A})ₘ₂-HLE,
wherein n1, m1, and m2 are each independently selected from integers of 1 and 2, and n2 is an integer of 0 or 1.

In some further preferred embodiments, n3, n4, m3, and m4 are 0, n1 + m2 = 1 or 2, n2 + m1 = 1 or 2, and m1 = 0 and m2 = 1, or m2 = 0 and m1 = 1; the multispecific antibody comprises a first polypeptide chain and a second polypeptide chain, wherein
the first polypeptide chain comprises, from the N-terminus to the C-terminus: (VHH^{A})-HLE,
the second polypeptide chain comprises, from the N-terminus to the C-terminus: (VHH^{B})-HLE;
   or
the first polypeptide chain comprises, from the N-terminus to the C-terminus: (VHH^{A})-(VHH^{B})-HLE,
the second polypeptide chain comprises, from the N-terminus to the C-terminus: (VHH^{A})-HLE;
   or
the first polypeptide chain comprises, from the N-terminus to the C-terminus: (VHH^{A})-(VHH^{A})-HLE,
the second polypeptide chain comprises, from the N-terminus to the C-terminus: (VHH^{B})-HLE;
   or
the first polypeptide chain comprises, from the N-terminus to the C-terminus: (VHH^{A})-(VHH^{B})-HLE,
the second polypeptide chain comprises, from the N-terminus to the C-terminus: (VHH^{B})-HLE.

In some further preferred embodiments, the multispecific antibody comprises a first polypeptide chain and a second polypeptide chain, wherein:
the first polypeptide chain comprises, from the N-terminus to the C-terminus: (VHH^{A})-HLE,
the second polypeptide chain comprises, from the N-terminus to the C-terminus: (VHH^{B})-HLE;
   or
the first polypeptide chain comprises, from the N-terminus to the C-terminus: (VHH^{A})-(VHH^{B})-HLE,
the second polypeptide chain comprises, from the N-terminus to the C-terminus: (VHH^{A})-HLE;
   or
the first polypeptide chain comprises, from the N-terminus to the C-terminus: (VHH^{A})-(VHH^{A})-HLE,
the second polypeptide chain comprises, from the N-terminus to the C-terminus: (VHH^{B})-HLE;
wherein A represents NECTIN4, and B represents TROP2.

In some most preferred embodiments, the multispecific antibody comprises a first polypeptide chain and a second polypeptide chain, wherein:
the first polypeptide chain comprises, from the N-terminus to the C-terminus: VHH^{Nectin4}-VHH^{Trop2}-Fc; and
the second polypeptide chain comprises, from the N-terminus to the C-terminus: VHH^{Nectin4}-Fc.

Due to dimerization of the immunoglobulin Fc region, the first and second polypeptide chains of the above multispecific antibody may associate to form a heterodimer, thereby producing a multispecific binding molecule in a double-chain form. Preferably, in order to promote heterodimerization of the first and second polypeptide chains, a Knob-into-hole mutation, such as (T366W/T366S, L368A, Y407V) mutations or (T366Y/Y407T) mutations, may be introduced into the Fc regions of the first and second polypeptide chains. Preferably, the Fc region comprises an amino acid sequence from human IgG1 or IgG4; preferably, the Fc region further comprises a mutation that reduces or eliminates Fcγ receptor binding, such as a LALA mutation.

In some embodiments, the multispecific antibody according to the present invention comprises a first Fc region and a second Fc region, wherein the first Fc region and the second Fc region comprise amino acid mutations that promote the formation of the Fc dimer. In some embodiments, the first Fc region comprises T366W and S354C, and the second Fc region comprises T366S, L368A, Y407V, and Y349C, or vice versa; alternatively, the first Fc region comprises T366W, and the second Fc region comprises T366S, L368A, and Y407V, or vice versa; alternatively, the first Fc region comprises T366Y, and the second Fc region comprises Y407T, or vice versa. In some further embodiments, the first Fc region and the second Fc region each further comprise a mutation that reduces or eliminates the binding of the Fc region to FcγR, e.g., a L234AL235A mutation. In some embodiments, preferably, the first and second Fc regions each comprise the amino acid sequence selected from SEQ ID NOs: 111-116 or an amino acid sequence having at least 90%, 95%, 96%, 97%, 98%, 99%, or more identity thereto.

In the above multispecific antibodies, preferably, the antigen-binding domains on the polypeptide chain (i.e., VHH^{Trop2} domain and VHH^{Nectin4} domain) are linked to each other via a peptide linker. Preferably, the peptide linker is 5-15 amino acids in length, and more preferably, comprises the amino acid sequence G4S or the amino acid sequence (G4S)2. In the above multispecific antibody, preferably, the Fc region comprises a hinge region or a portion thereof and is directly linked to a domain located at its N-terminus.

The multispecific antibody having first and second polypeptide chains of formula (III) and formula (IV) is preferably a trivalent bispecific antibody or a tetravalent bispecific antibody.

### Exemplary antigen-binding domain combination

In some embodiments of the above multispecific antibodies in single-chain and double-chain forms according to the present invention, preferably, the multispecific antibody according to the present invention comprises a combination of TROP2 and NECTIN4 antigen-binding domains as follows:
(a) the antigen-binding domain specifically binding to Nectin4 comprises 3 CDRs in the amino acid sequence of SEQ ID NO: 25, and the antigen-binding domain specifically binding to Trop2 comprises 3 CDRs in the amino acid sequence of SEQ ID NO: 13;
(b) the antigen-binding domain specifically binding to Nectin4 comprises 3 CDRs in the amino acid sequence of SEQ ID NO: 21, and the antigen-binding domain specifically binding to Trop2 comprises 3 CDRs in the amino acid sequence of SEQ ID NO: 9;
(c) the antigen-binding domain specifically binding to Nectin4 comprises 3 CDRs in the amino acid sequence of SEQ ID NO: 25, and the antigen-binding domain specifically binding to Trop2 comprises 3 CDRs in the amino acid sequence of SEQ ID NO: 9;
(d) the antigen-binding domain specifically binding to Nectin4 comprises 3 CDRs in the amino acid sequence of SEQ ID NO: 35, and the antigen-binding domain specifically binding to Trop2 comprises 3 CDRs in the amino acid sequence selected from SEQ ID NOs: 29 and 30; or
(e) the antigen-binding domain specifically binding to Nectin4 comprises 3 CDRs in the amino acid sequence selected from SEQ ID NOs: 34 and 38, and the antigen-binding domain specifically binding to Trop2 comprises 3 CDRs in the amino acid sequence selected from SEQ ID NOs: 29 and 30.

In some embodiments of the multispecific antibodies in single-chain and double-chain forms according to the present invention, more preferably, the multispecific antibody according to the present invention comprises a combination of TROP2 and NECTIN4 antigen-binding domains as follows:
(a) the antigen-binding domain specifically binding to Nectin4 comprises the amino acid sequence of SEQ ID NO: 21, and the antigen-binding domain specifically binding to Trop2 comprises the amino acid sequence of SEQ ID NO: 9;
(b) the antigen-binding domain specifically binding to Nectin4 comprises the amino acid sequence of SEQ ID NO: 35, and the antigen-binding domain specifically binding to Trop2 comprises the amino acid sequence selected from SEQ ID NOs: 29 and 30; or
(c) the antigen-binding domain specifically binding to Nectin4 comprises the amino acid sequence selected from SEQ ID NOs: 34 and 38, and the antigen-binding domain specifically binding to Trop2 comprises the amino acid sequence selected from SEQ ID NOs: 29 and 30.

In some embodiments of the multispecific antibodies in single-chain and double-chain forms according to the present invention, still preferably, the multispecific antibody according to the present invention comprises a combination of TROP2 and NECTIN4 antigen-binding domains as follows:
(a) the antigen-binding domain specifically binding to Nectin4 comprises the amino acid sequence selected from SEQ ID NO: 34, and the antigen-binding domain specifically binding to Trop2 comprises the amino acid sequence selected from SEQ ID NO: 29; or
(b) the antigen-binding domain specifically binding to Nectin4 comprises the amino acid sequence selected from SEQ ID NO: 38, and the antigen-binding domain specifically binding to Trop2 comprises the amino acid sequence selected from SEQ ID NO: 30.

### Exemplary multispecific antibody

In some embodiments, the present invention provides a multispecific antibody, wherein the antibody is in a symmetric double-chain form, and wherein a polypeptide chain of the antibody comprises the amino acid sequence of SEQ ID NO: 53 or 54 or an amino acid sequence having at least 95%, 96%, 97%, 98%, or 99% identity thereto, preferably the amino acid sequence of SEQ ID NO 53.

In some embodiments, the present invention provides a multispecific antibody, wherein the antibody is in an asymmetric double-chain form, and wherein the antibody comprises a first polypeptide chain and a second polypeptide chain selected from the group consisting of:
(i) first and second polypeptide chains comprising the amino acid sequences set forth in SEQ ID NOs: 55 and 56 or amino acid sequences having at least 95%, 96%, 97%, 98%, or 99% identity thereto, respectively,
(ii) first and second polypeptide chains comprising the amino acid sequences set forth in SEQ ID NOs: 57 and 58 or amino acid sequences having at least 95%, 96%, 97%, 98%, or 99% identity thereto, respectively,
(iii) first and second polypeptide chains comprising the amino acid sequences set forth in SEQ ID NOs: 59 and 60 or amino acid sequences having at least 95%, 96%, 97%, 98%, or 99% identity thereto, respectively,
(iv) first and second polypeptide chains comprising the amino acid sequences set forth in SEQ ID NOs: 61 and 62 or amino acid sequences having at least 95%, 96%, 97%, 98%, or 99% identity thereto, respectively,
(v) first and second polypeptide chains comprising the amino acid sequences set forth in SEQ ID NOs: 63 and 64 or amino acid sequences having at least 95%, 96%, 97%, 98%, or 99% identity thereto, respectively,
(vi) first and second polypeptide chains comprising the amino acid sequences set forth in SEQ ID NOs: 65 and 66 or amino acid sequences having at least 95%, 96%, 97%, 98%, or 99% identity thereto, respectively,
(vii) first and second polypeptide chains comprising the amino acid sequences set forth in SEQ ID NOs: 67 and 68 or amino acid sequences having at least 95%, 96%, 97%, 98%, or 99% identity thereto, respectively,
(viii) first and second polypeptide chains comprising the amino acid sequences set forth in SEQ ID NOs: 69 and 70 or amino acid sequences having at least 95%, 96%, 97%, 98%, or 99% identity thereto, respectively,
(ix) first and second polypeptide chains comprising the amino acid sequences set forth in SEQ ID NOs: 71 and 72 or amino acid sequences having at least 95%, 96%, 97%, 98%, or 99% identity thereto, respectively,
(x) first and second polypeptide chains comprising the amino acid sequences set forth in SEQ ID NOs: 73 and 74 or amino acid sequences having at least 95%, 96%, 97%, 98%, or 99% identity thereto, respectively, and
(xi) first and second polypeptide chains comprising the amino acid sequences set forth in SEQ ID NOs: 75 and 76 or amino acid sequences having at least 95%, 96%, 97%, 98%, or 99% identity thereto, respectively;
preferably, the first and second polypeptide chains comprise the amino acid sequences of SEQ ID NOs: 61/62, 69/70, or 71/72, respectively.

In some embodiments, the present invention provides a multispecific antibody, wherein the antibody is in a single-chain form, wherein a polypeptide chain of the antibody comprises the amino acid sequence selected from SEQ ID NOs: 41-52 or an amino acid sequence having at least 95%, 96%, 97%, 98%, or 99% identity thereto.

### Properties of multispecific antibody of present invention

The multispecific antibody of the present invention may have one or more of the following properties:
(i) binding to a tumor cell co-expressing TROP2 and NECTIN4 with high avidity;
(ii) having species cross-reactivity with cynomolgus monkey TROP2 and NECTIN4;
(iii) having TROP2 and/or NECTIN4 receptor-mediated endocytic activity;
(iv) exhibiting synergistic binding on a tumor cell co-expressing TROP2 and NECTIN4 antigens; and
(v) exhibiting a synergistic endocytosis effect on a tumor cell co-expressing TROP2 and NECTIN4 antigens.

In some aspects, the multispecific antibody of the present invention binds to a tumor cell co-expressing TROP2 and NECTIN4 with high avidity. The EC50 value and/or maximum level for the binding of the antibody of the present invention to TROP2- and/or NECTIN4-positive tumor cells can be determined by FACS or ELSA assays (e.g., the assays described in the examples) and optionally compared to a reference antibody to reflect the cell binding avidity of the antibody. In some embodiments, the multispecific antibody of the present invention exhibits a synergistic binding effect on a tumor cell co-expressing TROP2 and NECTIN4 compared to TROP2 and NECTIN4 monospecific binding molecules.

In some embodiments, the multispecific antibody of the present invention exhibits cross-reactivity with human and monkey TROP2 and NECTIN4. In some embodiments, the antibody of the present invention binds to human TROP2 with a KD value that is approximately comparable to the KD value with which the antibody binds to monkey TROP2, e.g., with a KD ratio therebetween being between 1 and 10, e.g., between 1 and 5, more preferably between about 1 and 3. In still further embodiments, the antibody binds to human TROP2 with a KD value that is approximately comparable to the KD value with which the antibody binds to monkey TROP2, e.g., with a KD ratio therebetween (human/monkey KD ratio or monkey/human KD ratio) being between 1 and 10, e.g., between 1 and 5, more preferably between about 1 and 3. In some embodiments, the antibody of the present invention binds to human NECTIN4 with a KD value that is approximately comparable to the KD value with which the antibody binds to monkey NECTIN4, e.g., with a KD ratio therebetween being between 1 and 5, more preferably between about 1 and 3. In still further embodiments, the antibody binds to human NECTIN4 with a KD value that is approximately comparable to the KD value with which the antibody binds to monkey NECTIN4, e.g., with a KD ratio therebetween (human/monkey KD ratio or monkey/human KD ratio) being between 1 and 10, e.g., between 1 and 5, more preferably between about 1 and 3.

In some embodiments, the multispecific antibody of the present invention has a TROP2 and/or NECTIN4 receptor-mediated endocytic activity. The evaluation of the endocytic activity of antibodies can be performed in a cell-based assay, such as the assay described in the examples. In some embodiments, in a TROP2- and/or NECTIN4-positive cell-based assay, the antibody to be tested is incubated with TROP2- and/or NECTIN4-positive cells (in particular TROP2- and/or NECTIN4-positive tumor cells) at 37 °C for a period of time (e.g., 2 hours or 4 hours), and then the change in the amount of antibodies bound to the cell surface relative to a negative control maintained at 4 °C for the same period of time is fluorescently detected by flow cytometry to determine the endocytosis and/or endocytosis rate of the antibody. In some embodiments, the internalization mediated by the multispecific antibody of the present invention exhibits greater selectivity for the tumor cell co-expressing both targets when compared to a TROP2 monospecific binding molecule or a NECTIN4 monospecific binding molecule, thereby minimizing the adverse effects of the antibody in normal tissues that do not exhibit significant levels of co-expression of TROP2 and NECTIN4, or a high expression of TROP2 alone, or a high expression of NECTIN4 alone. In some embodiments, the multispecific antibody of the present invention exhibits a synergistic endocytosis effect on a tumor cell co-expressing TROP2 and NECTIN4 compared to TROP2 and NECTIN4 monospecific binding molecules.

In still further embodiments, the multispecific antibody of the present invention further has one or more of the following properties: (iv) good developability; (v) good stability; and (vi) advantageous pharmacokinetic properties.

In some embodiments, the multispecific antibody of the present invention may reach a purity of 90% or 95% or more as determined by SEC-HPLC after one-step purification of the antibody product produced from recombinant mammalian cells using protein A affinity chromatography.

In still further embodiments, the multispecific antibody of the present invention has good stability. In some embodiments, compared to pre-storage, the antibody of the present invention has no change in antigen binding activity after being left at room temperature for 14 days, as determined by the assay in the examples.

In still further embodiments, the multispecific antibody of the present invention has serum stability. The serum stability of the antibody can be determined by measuring the circulating half-life of the antibody in animals according to the animal tests in the examples.

### II. Anti-Trop2 VHH Domain and Anti-Trop2 Antibody of Present Invention

In a second aspect, the present invention provides an anti-Trop2 VHH domain binding to Trop2 and an anti-Trop2 antibody comprising the VHH domain. In some embodiments, the anti-Trop2 VHH domain according to the present invention comprises CDR1, CDR2, and CDR3 sequences in the amino acid sequence selected from SEQ ID NOs: 5, 9, 13, 29, 30, 98, and 99;
still preferably, the CDR1, CDR2, and CDR3 sequences:
   (i) comprise or consist of the amino acid sequences of SEQ ID NOs: 6, 7, and 8, respectively;
   (ii) comprise or consist of the amino acid sequences of SEQ ID NOs: 10, 11, and 12, respectively;
   (iii) comprise or consist of the amino acid sequences of SEQ ID NOs: 14, 15, and 16, respectively; or
   (iv) comprise or consist of the amino acid sequences of SEQ ID NOs: 31, 32, and 33, respectively;
still preferably, the VHH domain comprises the amino acid sequence selected from SEQ ID NOs: 5, 9, 13, 29, 30, 98, and 99, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence;
most preferably, the VHH domain comprises or consists of the amino acid sequence selected from SEQ ID NOs: 5, 9, 13, 29, 30, 98, and 99.

The anti-Trop2 antibody according to the present invention may comprise or consist of the anti-Trop2 VHH domain according to the present invention, and may have any suitable antibody structure, including but not limited to, a single- or multi-chain antibody, a monospecific or multispecific antibody (e.g., bispecific antibody), a linear antibody, a single-domain antibody, a heavy-chain antibody, a chimeric antibody, or a humanized antibody. In some embodiments, the anti-Trop2 antibody according to the present invention comprises an immunoglobulin Fc region linked to the VHH domain (i.e., having the form VHH-Fc). In some embodiments, the anti-Trop2 antibody according to the present invention is a heavy-chain antibody. Herein, the term "heavy-chain antibody (hcAb)" refers to an antibody without a light chain, which may comprise, from the N-terminus to the C-terminus, VHH-CH2-CH3 or VHH-CH1-CH2-CH3; and may form a homodimer, such as a heavy-chain dimer antibody without a light chain. In some embodiments, the anti-Trop2 antibody according to the present invention further comprises an anti-Nectin4 binding domain, and is preferably the anti-Trop2/NECTIN4 multispecific antibody according to the present invention described in section I above.

The anti-Trop2 VHH domain according to the present invention has a good tumor targeting property, a tumor tissue penetrating ability, and a target cell endocytosis ability, and has immunological cross-reactivity with human and monkey species. The anti-Trop2 VHH domain according to the present invention or the anti-Trop2 antibody according to the present invention may therefore be used as a targeting block for conjugates or complexes, and conjugated or coupled to, for example, chemotherapeutic agents, toxins, drugs (e.g., immunotherapeutic agents), radioactive elements, probes, signaling molecules, or the like to provide better applications such as tumor killing, immunomodulation, or disease detection.

### III. Anti-NECTIN4 VHH Domain and Anti-NECTIN4 Antibody of Present Invention

In a third aspect, the present invention provides an anti-NECTIN4 VHH domain binding to NECTIN4 and an anti-NECTIN4 antibody comprising the VHH domain. In some embodiments, the anti-Nectin4 VHH domain according to the present invention comprises CDR1, CDR2, and CDR3 sequences in the amino acid sequence selected from SEQ ID NOs: 17, 21, 25, 34, 35, 38, and 100-106;
preferably, the CDR1, CDR2, and CDR3 sequences:
   (i) comprise or consist of the amino acid sequences of SEQ ID NOs: 18, 19, and 20, respectively;
   (ii) comprise or consist of the amino acid sequences of SEQ ID NOs: 22, 23, and 24, respectively;
   (iii) comprise or consist of the amino acid sequences of SEQ ID NOs: 26, 27, and 28, respectively;
   (iv) comprise or consist of the amino acid sequences of SEQ ID NOs: 18, 36, and 20, respectively; or
   (v) comprise or consist of the amino acid sequences of SEQ ID NOs: 18, 37, and 20, respectively;
still preferably, the VHH domain comprises the amino acid sequence selected from SEQ ID NOs: 17, 21, 25, 34, 35, 38, and 100-106, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence;
most preferably, the VHH domain comprises or consists of the amino acid sequence selected from SEQ ID NOs: 17, 21, 25, 34, 35, 38, and 100-106.

The anti-NECTIN4 antibody according to the present invention may comprise or consist of the anti-Nectin4 VHH domain according to the present invention, and may have any suitable antibody structure, including but not limited to, a single- or multi-chain antibody, a monospecific or multispecific antibody (e.g., bispecific antibody), a linear antibody, a single-domain antibody, a heavy-chain antibody, a chimeric antibody, or a humanized antibody. In some embodiments, the anti-NECTIN4 antibody according to the present invention comprises an immunoglobulin Fc region linked to the VHH domain (i.e., having the form VHH-Fc). In some embodiments, the anti-NECTIN4 antibody according to the present invention is a heavy-chain antibody. In some embodiments, the anti-NECTIN4 antibody according to the present invention further comprises an anti-TROP2 binding domain, and is preferably the anti-Trop2/NECTIN4 multispecific antibody according to the present invention described in section I above.

The anti-NECTIN4 VHH domain according to the present invention has a good tumor targeting property, a tumor tissue penetrating ability, and a target cell endocytosis ability, and has immunological cross-reactivity with human and monkey species. The anti-NECTIN4 VHH domain according to the present invention or the anti-NECTIN4 antibody according to the present invention may therefore be used as a targeting block for conjugates or complexes, and conjugated or coupled to, for example, chemotherapeutic agents, toxins, drugs (e.g., immunotherapeutic agents), radioactive elements, probes, signaling molecules, or the like to provide better applications such as tumor killing, immunomodulation, or disease detection.

### IV. Production and purification of antibody of present invention

In a fourth aspect, the present invention provides a method for producing the antibody of the present invention. To produce the antibody of the present invention, the polypeptide chains of the antibody of the present invention can be obtained, for example, by solid state peptide synthesis (e.g., Merrifield solid phase synthesis) or recombinant production, and assembled under suitable conditions.

For recombinant production, polynucleotides encoding any one or more polypeptide chains of the antibody are isolated and inserted into one or more vectors for further cloning and/or expression in host cells. The polynucleotides can be easily isolated and sequenced using conventional methods. In one embodiment, polynucleotides encoding one or more polypeptide chains of the antibody of the present invention are provided. In one further embodiment, the present invention provides a vector, preferably an expression vector, comprising one or more polynucleotides of the present invention. Therefore, in one embodiment, the present invention provides a method for producing the antibody of the present invention, which comprises: culturing a host cell comprising the polynucleotides encoding the polypeptide chains under conditions suitable for the expression of the polypeptide chains of the antibody; and assembling the polypeptide chains to produce the antibody under conditions suitable for the assembly of the polypeptide chains into the antibody.

Methods well known to those skilled in the art can be used to construct expression vectors. The expression vectors include, but is not limited to, viruses, plasmids, cosmids, a λ phages, or yeast artificial chromosomes (YAC).

In one embodiment, the present invention also provides a host cell comprising one or more polynucleotides of the present invention. In some embodiments, provided is a host cell comprising the expression vector of the present invention. Suitable host cells include prokaryotic microorganisms such as *E. coli*, eukaryotic microorganisms such as filamentous fungi or yeast, or various eukaryotic cells such as Chinese hamster ovary cells (CHO) and insect cells. A mammalian cell line suitable for suspension culture may be used. Examples of useful mammalian host cell lines include the SV40-transformed monkey kidney CV1 line (COS-7), human embryonic kidney line (HEK293 or 293F cells), baby hamster kidney cells (BHK), monkey kidney cells (CV1), African green monkey kidney cells (VERO-76), human cervical cancer cells (HELA), canine kidney cells (MDCK), buffalo rat liver cells (BRL 3A), human lung cells (W138), human liver cells (HepG2), CHO cells, NSO cells, and myeloma cell lines such as YO, NS0, P3X63, and Sp2/0. In a preferred embodiment, the host cell is a CHO cell or an HEK293 cell.

The antibody prepared by the method as described herein can be purified by known existing techniques such as high performance liquid chromatography, ion exchange chromatography, gel electrophoresis, affinity chromatography, and size exclusion chromatography. After purification, the purity of the antibody of the present invention can be determined by any one of a variety of well-known analytical methods including size exclusion chromatography, gel electrophoresis, high performance liquid chromatography, and the like. The antibody provided herein can be identified, screened, or characterized for physical/chemical properties and/or biological activity through a variety of assays known in the art.

In a preferred embodiment, the antibody of the present invention shows good production properties, in particular, good expression yield and good by-product profile when recombinantly produced in mammalian host cells, such as CHO cells.

### V. Antigen-Binding Molecule, Immunofusion, Immunoconjugate, and Antibody-Drug Conjugate (ADC)

In a fifth aspect, the present invention provides an antigen-binding molecule, immunofusion, or immunoconjugate produced by fusing or conjugating the antibody of the present invention to a heterologous molecule.

In one embodiment, the antigen-binding molecule according to the present invention comprises at least one (e.g., 1, 2, or 3) VHH^{Trop2} domain of the present invention that specifically binds to Trop2 described herein. In one embodiment, the antigen-binding molecule according to the present invention comprises at least one (e.g., 1, 2, or 3) VHH^{NECTIN4} domain of the present invention that specifically binds to Nectin4 described herein. The antigen-binding molecule according to the present invention may be, including but not limited to, a monospecific or multispecific protein comprising the VHH domain. As an example, the antigen-binding molecule is a chimeric antigen receptor.

In one embodiment, the present invention provides an immunofusion comprising the antibody of the present invention. In one embodiment of the immunofusion according to the present invention, the antibody (or the antigen-binding fragment thereof) of the present invention is linked to a heterologous peptide or polypeptide molecule directly or via an amino acid peptide linker. The heterologous peptides or polypeptides that can be mentioned include, but are not limited to, proteins or polypeptides conferring additional functional activity to the fusion, or tag peptides facilitating the purification or detection of the immunofusion.

In one embodiment, the present invention provides an immunoconjugate comprising the antibody of the present invention or the antigen-binding molecule of the present invention. In one embodiment of the immunoconjugate according to the present invention, the antibody (or the antigen-binding fragment thereof) of the present invention or the antigen-binding molecule of the present invention is conjugated to a therapeutic agent, a diagnostic agent, or a detectable agent. In the conjugates, linkers can be used to covalently link different entities of the conjugates. Suitable linkers include chemical linkers or peptide linkers. Advantageously, the linkers are "cleavable linkers" that facilitate the release of the polypeptides following delivery to a target site. For example, acid-labile linkers, peptidase-sensitive linkers, photolabile linkers, dimethyl linkers, or disulfide-containing linkers can be used.

In embodiments of conjugation to a therapeutic agent, the therapeutic agent suitable for the conjugate includes, but is not limited to, a cytotoxin (e.g., a cytostatic agent or a cytotoxic agent), a drug, or a radioisotope.

In embodiments of conjugation to a diagnostic agent or a detectable agent, such conjugates can be used as part of a clinical testing method (e.g., to determine the efficacy of a specific therapy) for monitoring or predicting the onset, development, progression, and/or severity of a disease or disorder. Such diagnosis and detection can be achieved by conjugating the antibody to the detectable agent, which includes, but is not limited to, a variety of enzymes, such as horseradish peroxidase; prosthetic groups, such as streptavidin/biotin and avidin/biotin; fluorescent substances; luminescent substances; radioactive substances; and positron-emitting metals and non-radioactive paramagnetic metal ions used in various positron emission tomography techniques.

In some embodiments, the therapeutic agent suitable for the conjugate includes, but is not limited to, a drug (e.g., an anti-tumor drug); in some other embodiments, the diagnostic agent suitable for the conjugate includes, but is not limited to, a radioactive diagnostic agent, a fluorescent substance, or a luminescent substance.

### Antibody-drug conjugate (ADC)

In some preferred embodiments, the immunoconjugate according to the present invention is an antibody-drug conjugate (ADC).

In some embodiments, the present invention provides an antibody-drug conjugate having formula (I):

Ab-(L-D)ₚ (I)

or a pharmaceutically acceptable salt or solvate thereof,
wherein:
   Ab is the antibody or the antigen-binding molecule of the present invention, e.g., the antibody or the fragment thereof (e.g., an antigen-binding fragment) specifically binding to Trop2 and/or Nectin4 (e.g., human Trop2 and/or human Nectin4) described above;
   L is a linker;
   D is a drug, such as an anti-tumor compound; and
   p is an integer selected from 1 to 16, such as an integer selected from 1-10, 1-9, 2-8, 4-10, 6-8, 3-7, 4-6, and 2-6, such as 1, 2, 3, 4, 5, 6, 7, 8, 9, 10, or 12. In some embodiments, Ab is the multispecific antibody binding to Trop2 and Nectin4 according to the present disclosure. In some particularly preferred embodiments, Ab is a double-chain multispecific antibody, wherein:
      (a) the antibody is in a symmetric double-chain form, and a polypeptide chain of the antibody comprises the amino acid sequence set forth in SEQ ID NO: 53 or an amino acid sequence having at least 90%, 91%, 92%, 93%, 95%, 96%, 97%, 98%, or 99% identity thereto; or
      (b) the antibody is in an asymmetric double-chain form, and the antibody comprises a first polypeptide chain and a second polypeptide chain, wherein the first polypeptide chain and the second polypeptide chain comprise the amino acid sequences set forth in SEQ ID NOs: 61/62, 69/70, or 71/72, or amino acid sequences having at least 90%, 91%, 92%, 93%, 95%, 96%, 97%, 98%, or 99% identity thereto, respectively.

In some embodiments, D in formula (I) of the present invention may be any anti-tumor compound and is not particularly limited as long as it is a compound that has an anti-tumor effect and has a substituent or partial structure capable of being linked to a linker structure. For example, the anti-tumor compound may be a pharmaceutically active compound that acts on a tumor. For an anti-tumor compound, it is preferable that a part or all of the linker is cleaved in a tumor cell to release the anti-tumor compound portion, thereby exhibiting an anti-tumor effect. When the linker is cleaved at the site of linkage to the drug, the anti-tumor compound is released in an unmodified structure and exhibits its original anti-tumor effect.

In some embodiments, the anti-tumor compound may be, for example, a cytotoxic agent, such as camptothecin compounds, e.g., Exatecan, Dxd, and auristatin compounds, e.g., monomethyl auristatin E (MMAE).

In some embodiments, D has a structure represented by formula (D-1a) or formula (D-1b): 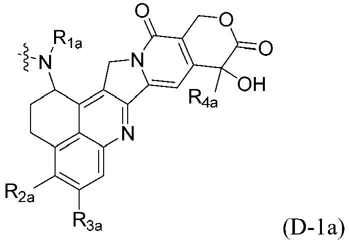
wherein R₁ₐ is selected from H and C₁-C₆ alkyl;
R₂ₐ is selected from H, halogen, C₁-C₆ alkyl, C₁-C₆ haloalkyl, -OR₅ₐ, and -SR₅ₐ;
R₃ₐ is selected from H, halogen, CN, C₁-C₆ alkyl, C₁-C₆ haloalkyl, and -OR₅ₐ; and
R₄ₐ and R₅ₐ are independently selected from H and C₁-C₄ alkyl; 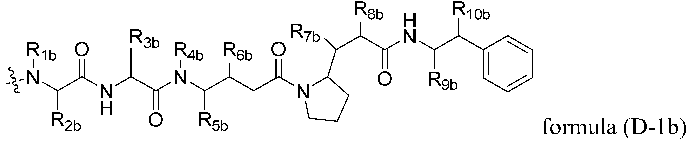
wherein R_{1b}, R_{2b}, R_{3b}, R_{4b}, R_{5b}, and R_{8b} are each independently selected from C₁₋₈ alkyl; preferably C₁₋₄ alkyl, such as methyl, ethyl, n-propyl, isopropyl, n-butyl, isobutyl, or *sec*-butyl;
R_{6b} and R_{7b} are each independently selected from C₁₋₈ alkoxy, such as methoxy, ethoxy, or propoxy;
R_{9b} is selected from C₁₋₈ alkyl and COOH; preferably C₁₋₄ alkyl, such as methyl, ethyl, n-propyl, isopropyl, n-butyl, isobutyl, or *sec*-butyl; and
R_{10b} is selected from OH and H.

In some embodiments, R₁ₐ is H; R₂ₐ is C₁-C₆ alkyl; R₃ₐ is halogen, preferably -F; R₄ₐ is C₁-C₄ alkyl, preferably ethyl.

In some embodiments, R_{1b}, R_{4b}, and R_{8b} are each independently selected from C₁₋₂ alkyl, preferably methyl;
R_{2b}, R_{3b}, and R_{5b} are each independently selected from C₃₋₄ alkyl;
R_{6b} and R_{7b} are each independently selected from C₁₋₂ alkoxy; and
R_{9b} is selected from C₁₋₄ alkyl, and R_{10b} is OH; or R_{9b} is COOH, and R_{10b} is H.

In some embodiments, D has a structure represented by formula (D-2a) or formula (D-2b): 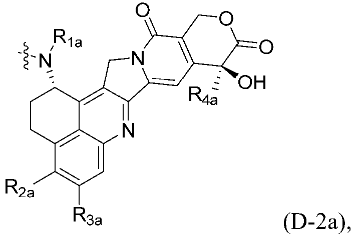 wherein R₁ₐ, R₂ₐ, R₃ₐ, and R₄ₐ are as defined above; or 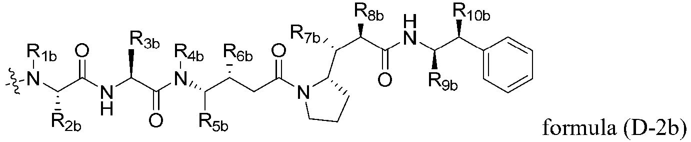 wherein R_{1b}, R_{2b}, R_{3b}, R_{4b}, R_{5b}, R_{6b}, R_{7b}, R_{8b}, R_{9b}, and R_{10b} are as defined above.

In some embodiments, D has a structure represented by formula (D-3a) or (D-3b):

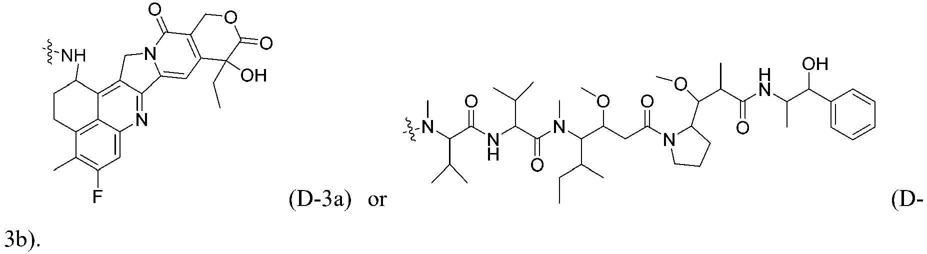

In some embodiments, D has a structure represented by formula (D-4a) or (D-4b):

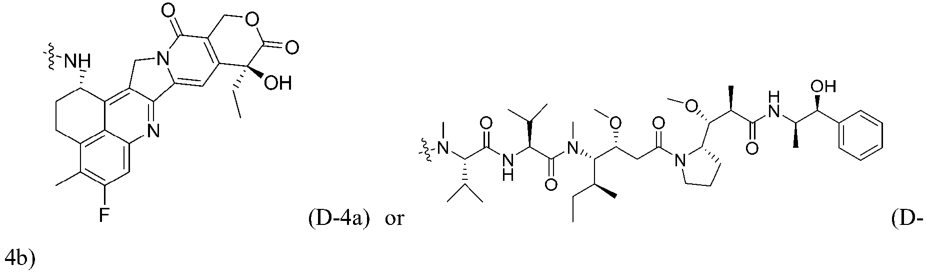

In some embodiments, -L- has the following structure: -Z-L₁-L₂-L₃-
wherein
Z is selected from 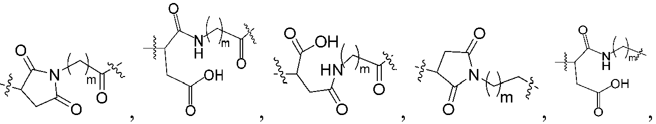 and 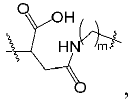 wherein m is independently an integer selected from 1-10, such as 1, 2, 3, 4, 5, 6, 7, or 8;
L₁ is selected from absence, 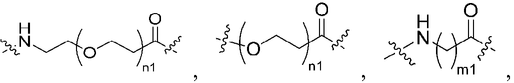 and 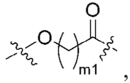 wherein n1 and m1 are each independently an integer selected from 0-20, for example, an integer selected from 0-12, such as 1, 2, 3, 4, 5, 6, 7, or 8;
L₂ is an amino acid residue or a peptide residue consisting of 2-8 amino acids; and
L₃ is selected from: 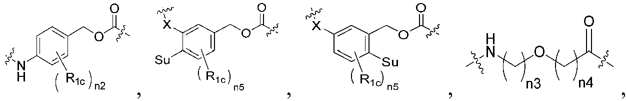 and 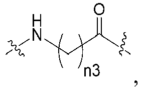 wherein X is selected from -NH-, -O-, and -S-; each R_{1c} is independently selected from C₁₋₈ alkyl, C₁₋₈ haloalkyl-, C₁₋₈ alkoxy, halogen, nitro, and cyano; each Su is independently selected from pentose, penturonic acid, hexose, and hexuronic acid; n2 is 0, 1, 2, 3, or 4; n5 is 0, 1, 2, or 3; n3 and n4 are independently 1, 2, 3, 4, 5, or 6;

It should be understood that in the above -Z-L₁-L₂-L₃-, Z is linked to S on Ab, and L₃ is linked to D.

In some embodiments,
Z is selected from 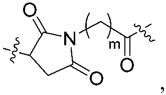 wherein m is an integer selected from 1-10, such as 1, 2, 3, 4, 5, 6, 7, or 8;
L₁ is selected from absence, 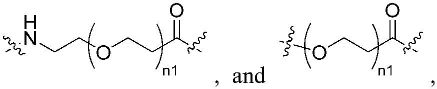 wherein n1 is independently an integer selected from 0-12, such as 1, 2, 3, 4, 5, 6, 7, or 8;
L₂ is a peptide residue consisting of 2-8 amino acids; and
L₃ is selected from: 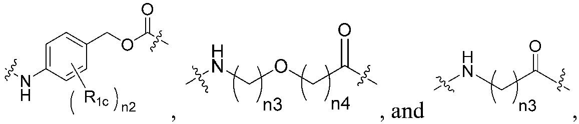 wherein R_{1c} is selected from: H and C₁-C₆ alkyl; n2 is 1, 2, 3, or 4; and n3 and n4 are independently 1, 2, 3, 4, 5, or 6.

In some embodiments, Z is selected from 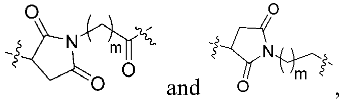 wherein m is 1, 2, 3, 4, 5, 6, 7, or 8.

In some embodiments, Z is selected from 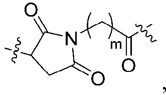 wherein m is 1, 2, 3, 4, 5, 6, 7, or 8.

In some embodiments, Z is selected from 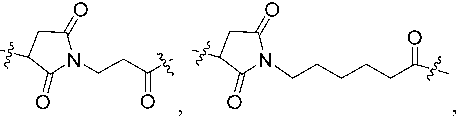 and

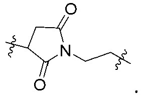

In some embodiments, Z is selected from

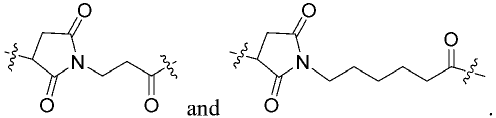

In some embodiments, L₁ is selected from absence, 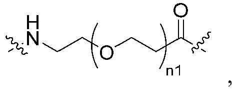 and 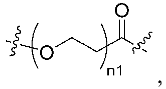 wherein n1 is independently an integer selected from 0-12, such as 1, 2, 3, 4, 5, 6, 7, or 8.

In some embodiments, L₁ is absent or

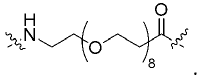

In some embodiments, L₂ is an amino acid residue or a peptide residue consisting of 2, 3, 4, 5, 6, 7, or 8 amino acids.

In some embodiments, L₂ is a peptide residue consisting of 2, 3, 4, 5, 6, or 7 amino acids.

In some embodiments, L₂ is an amino acid residue or a peptide residue consisting of 2, 3, or 4 amino acids.

The amino acid constituting L₂ is not particularly limited, and is, for example, an L- or D-amino acid, preferably an L-amino acid. Furthermore, in addition to α-amino acids, the amino acids may also be amino acids with structures such as β-alanine, ε-aminocaproic acid, and γ-aminobutyric acid. In addition, the amino acids may be unnatural amino acids such as N-methylated amino acids.

In some embodiments, the amino acid residues or the amino acids constituting the peptide residue are each independently selected from glycine (Gly), valine (Val), alanine (Ala), lysine (Lys), citrulline (Cit), glutamine (Gln), glutamic acid (Glu), phenylalanine (Phe), leucine (Leu), tyrosine (Tyr), serine (Ser), aspartic acid (Asp), asparagine (Asn), isoleucine (Ile), arginine (Arg), proline (Pro), methionine (Met), tryptophan (Trp), cysteine (Cys), histidine (His), and threonine (Thr), wherein the amino acid residues or the amino acids are optionally substituted with one or more C₁₋₆ alkyl.

In some embodiments, the amino acid residues or the amino acids constituting the peptide residue are each independently selected from glycine (Gly), valine (Val), alanine (Ala), lysine (Lys), citrulline (Cit), glutamine (Gln), glutamic acid (Glu), phenylalanine (Phe), aspartic acid (Asp), asparagine (Asn), arginine (Arg), and threonine (Thr).

In some embodiments, the amino acid residues or the amino acids constituting the peptide residue are each independently selected from glycine (Gly), valine (Val), alanine (Ala), phenylalanine (Phe), glutamic acid (Glu), and citrulline (Cit).

In some embodiments, L₂ is selected from -Ala-, -Val-, -Gly-, -Val-Ala-, -Gly-Gly-Phe-Gly-, -Val-Cit-, and -Glu-Val-Cit-.

In some embodiments, L ₂ is selected from -Gly-, -Val-Ala-, and -Gly-Gly-Phe-Gly-.

In some embodiments, L₂ is selected from -Val-Ala-, -Gly-Gly-Phe-Gly-, -Val-Cit-, and -Glu-Val-Cit-.

It should be understood that L₂ is linked to L₁ or Z through the amino group of the amino acid on the left and to L₃ through the carbonyl group of the amino acid on the right, which is consistent with the explanation below.

In some embodiments, L₃ is selected from: 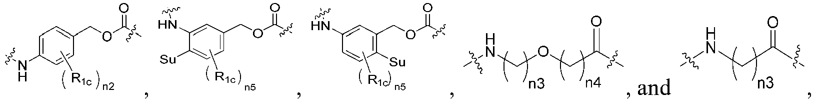 wherein each R _{1c} is independently selected from C₁₋₈ alkyl, C₁₋₈ haloalkyl-, C₁₋₈ alkoxy, halogen, nitro, and cyano; each Su is independently selected from 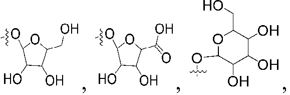 and 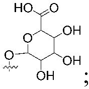 n2 is 0, 1, 2, 3, or 4; n5 is 0, 1, 2, or 3; and n3 and n4 are independently 1, 2, 3, 4, 5, or 6.

In some embodiments, L₃ is selected from: 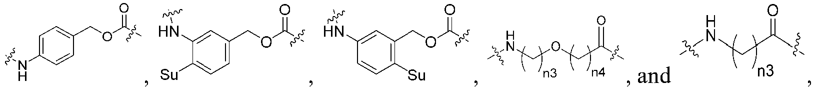 wherein the variables are as defined herein.

In some embodiments, L₃ is selected from: 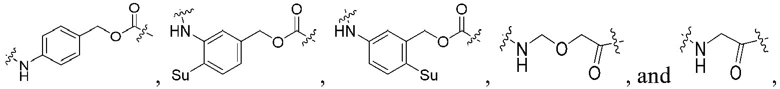 wherein the variables are as defined herein.

In some embodiments, L₃ is selected from: 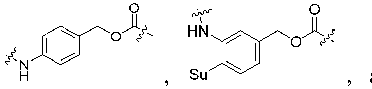 and 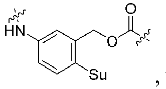 wherein the variables are as defined herein.

In some embodiments, Su is selected from xylose, arabinose, xyluronic acid, arabinuronic acid, glucose, galactose, mannose, glucuronic acid, galacturonic acid, and mannuronic acid.

In some embodiments, Su is

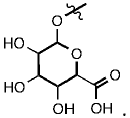

In some embodiments, each Su is independently:

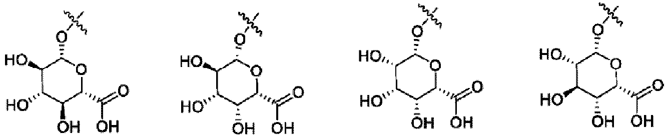

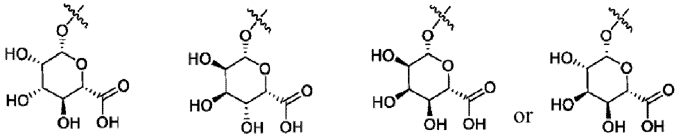

In some embodiments, each Su is independently

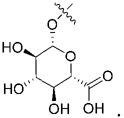

In some embodiments, each Su is independently

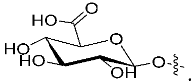

In some embodiments, L₃ is selected from: 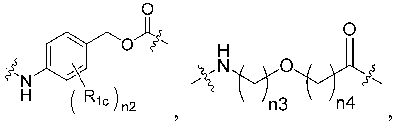 and 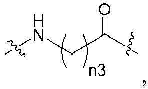 wherein R_{1c} is selected from: H and C₁-C₆ alkyl; n2 is 1, 2, 3, or 4; n3 and n4 are independently 1, 2, 3, 4, 5, or 6.

In some embodiments, L₃ is selected from:

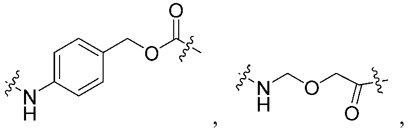

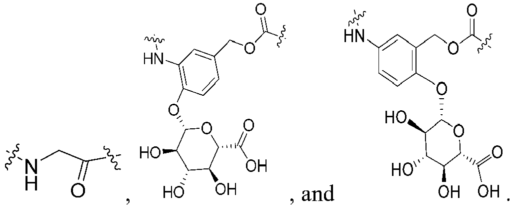

In some embodiments, L₃ is selected from: 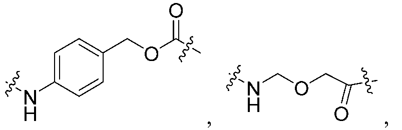 and

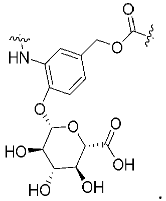

In some embodiments, L₃ is selected from: 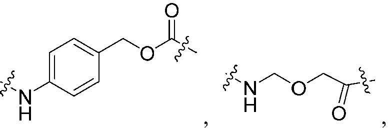 and

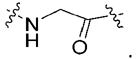

It should be understood that L₃ is linked to L₂ through the amino group on the left and to D through the carbonyl group on the right, which is consistent with the explanation below.

In some embodiments, Z is selected from 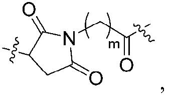 wherein m is 1, 2, 3, 4, 5, 6, 7, or 8;
L₁ is selected from absence, 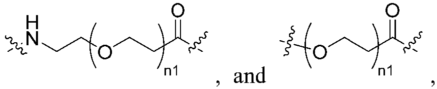 wherein n1 is independently an integer selected from 0-12; and
L₃ is selected from: 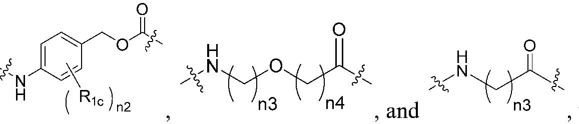 wherein R_{1c} is selected from H and C₁-C₆ alkyl; n2 is 1, 2, 3, or 4; and n3 and n4 are independently 1, 2, 3, 4, 5, or 6.

In some embodiments, -Z-L₁-L₂-L₃- is selected from the following structures:

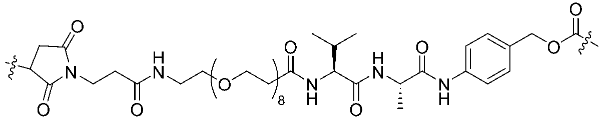

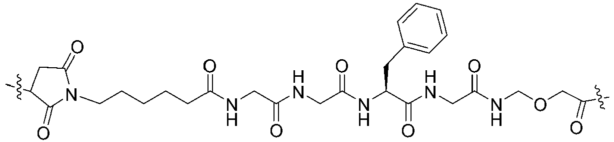

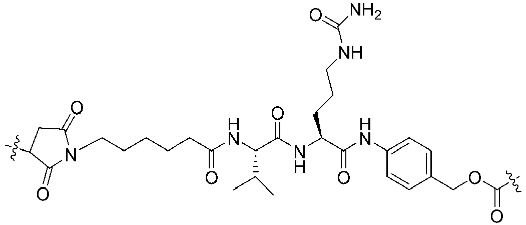

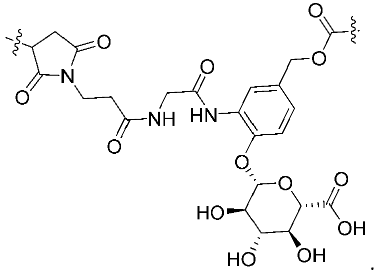

It should be understood that, without particular description and without contradiction according to the context, for the ADCs of the present invention, the bond on the left side of the divalent group as shown herein is linked to Ab or a group near the Ab end, and the bond on the right side of the divalent group is linked to D or a group near the D end. For example, when L₂ is 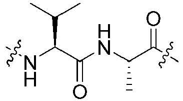 , the amino group on the left is linked to L₁, and the carbonyl group on the right is linked to L₃.

In some embodiments, the antibody-drug conjugate has an average DAR of 2-10, 6-10, 4-8, 7-9, or 2-4.

In some embodiments, formula (I) is represented by formula (I'),

Ab-(S-L-D)p (I')

wherein Ab, L, D, and p are as defined in formula (I) above;

It should be understood that S in formula (I') is the sulphur from the antibody Ab.

In some embodiments, the antibody-drug conjugate is selected from: 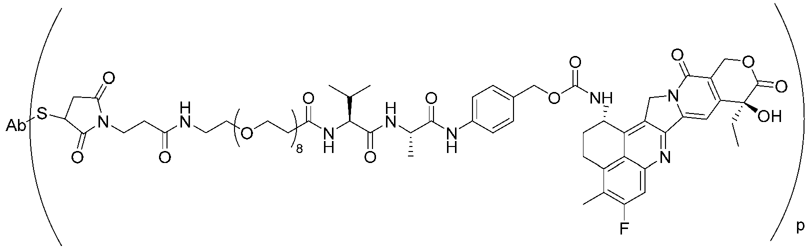 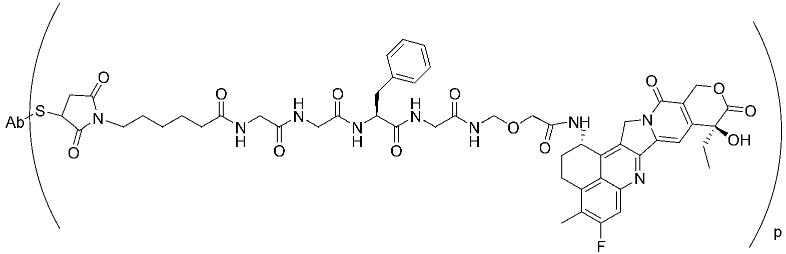 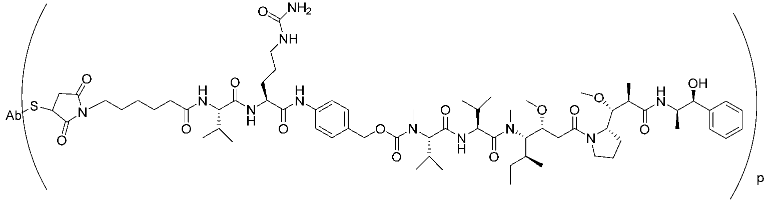 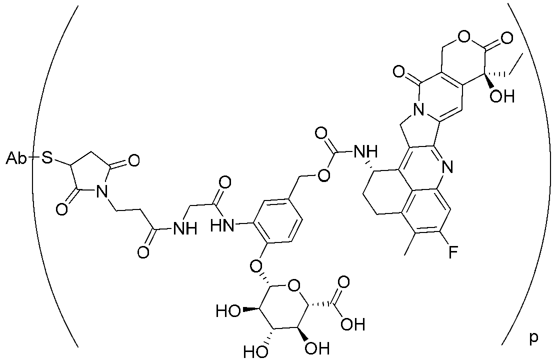 wherein Ab is the antibody of the present invention, preferably V-15, V-hu-21, V-hu-23, or V-hu-24; p is as defined above; preferably, the antibody-drug conjugate has an average DAR of, for example, 2-10, 6-10, 4-8, 7-9, or 2-4.

It should be understood that the S atom linked to Ab in the above ADC is derived from antibody Ab. Ab opens disulfide bonds under the action of a reducing agent such as TCEP to generate a sulfhydryl group (-SH), which is then linked to a terminal functional group of the linker, such as a maleimide moiety.

### VI. Pharmaceutical Composition, Pharmaceutical Combination, and Kit

In one aspect, the present invention provides a composition, e.g., a pharmaceutical composition, comprising the antibody as described herein formulated together with a pharmaceutically acceptable carrier. As used herein, the "pharmaceutically acceptable carrier" includes any and all solvents, dispersion media, isotonic agents, absorption delaying agents, and the like that are physiologically compatible. The pharmaceutical composition of the present invention is suitable for intravenous, intramuscular, subcutaneous, parenteral, rectal, spinal, or epidermal administration (e.g., by injection or infusion). In some embodiments, the antibody of the present invention or the immunoconjugate or immunofusion of the present invention is the only active ingredient in the pharmaceutical composition. In some other embodiments, the pharmaceutical composition may comprise the antibody as described herein or the immunoconjugate or immunofusion of the present invention and more than one therapeutic agent.

In another aspect, the present invention also provides a pharmaceutical combination comprising the antibody as described herein or the immunoconjugate or immunofusion of the present invention and more than one therapeutic agent.

The therapeutic agent suitable for use in the pharmaceutical composition and the pharmaceutical combination of the present invention may be a therapeutic agent selected from any one of the following categories (i)-(iv): (i) drugs that enhance antigen presentation (e.g., tumor antigen presentation); (ii) drugs that enhance effector cell responses (e.g., activation and/or mobilization of B cells and/or T cells); (iii) drugs that reduce immunosuppression; and (iv) drugs that have anti-tumor effects.

The pharmaceutical composition of the present invention may comprise a "therapeutically effective amount" or a "prophylactically effective amount" of the antibody of the present invention. "Therapeutically effective amount" refers to an amount effective to achieve a desired therapeutic result at a necessary dose for a necessary period of time. The therapeutically effective amount can be varied according to a variety of factors such as disease state, age, gender, and weight of the individual. The therapeutically effective amount is an amount at which any toxic or adverse effect is inferior to the therapeutically beneficial effect. The "therapeutically effective amount" preferably inhibits a measurable parameter (e.g., tumor growth rate) by at least about 20%, more preferably at least about 40%, even more preferably at least about 60%, and still more preferably at least about 80%, relative to untreated subjects. The ability of the antibody of the present invention to inhibit a measurable parameter (e.g., tumor volume) can be evaluated in an animal model system that predicts efficacy in human tumors. "Prophylactically effective amount" refers to an amount effective to achieve a desired prophylactic result at a necessary dose for a necessary period of time. Generally, since a prophylactic dose is used in a subject before or at an earlier stage of a disease, the prophylactically effective amount will be less than the therapeutically effective amount.

A kit comprising the antibody as described herein is also within the scope of the present invention. The kit may comprise one or more other elements, including, for example, a package insert; other reagents, such as a label or a reagent for conjugation; a pharmaceutically acceptable carrier; and a device or other materials for administration to a subject.

### VII. Use and Method

Based on the good targeting of the antibody of the present invention or the immunoconjugate or immunofusion of the present invention to tumor cells expressing TROP2 and/or NECTIN4, as well as the other advantageous properties as mentioned above, the present invention also provides use and a method of the antibody of the present invention or the immunoconjugate or immunofusion of the present invention for the treatment and prevention of diseases associated with TROP2 and/or NECTIN4.

TROP2 and NECTIN4 are overexpressed on the surface of cancer tissue cells from a variety of origins, thereby being suitable targets for the development of cancer immunotherapies. In one aspect, the present invention thus provides use of the immunoconjugate or conjugate or the pharmaceutically acceptable salt or solvate thereof of the present invention, and the antibody or the antigen-binding fragment thereof of the present invention, in the prevention and/or treatment of a TROP2- and/or NECTIN4-positive tumor (e.g., cancer) in a subject. In the use, the immunoconjugate or conjugate or the pharmaceutically acceptable salt or solvate thereof of the present invention, or the antibody or the antigen-binding fragment thereof of the present invention may be administered to the subject as the sole active agent, or may be administered to the subject in combination with additional therapies or therapeutic agents. The additional therapies and therapeutic agents include, for example, drugs that target antigens on the surface of tumor cells to destroy tumors by binding and/or blocking these molecules; and drugs that activate the immune system of the subject to spontaneously destroy the tumors.

In yet another aspect, the present invention further provides a method for preventing or treating a TROP2- and/or NECTIN4-positive tumor (e.g., cancer) in a subject, comprising administering to the subject in need thereof the immunoconjugate or conjugate or the pharmaceutically acceptable salt or solvate thereof of the present invention, or administering to the subject in need thereof the antibody or the antigen-binding fragment thereof of the present invention.

The TROP2- and/or NECTIN4-positive tumor suitable for use in the method and use of the present invention may be selected from a variety of solid tumors.

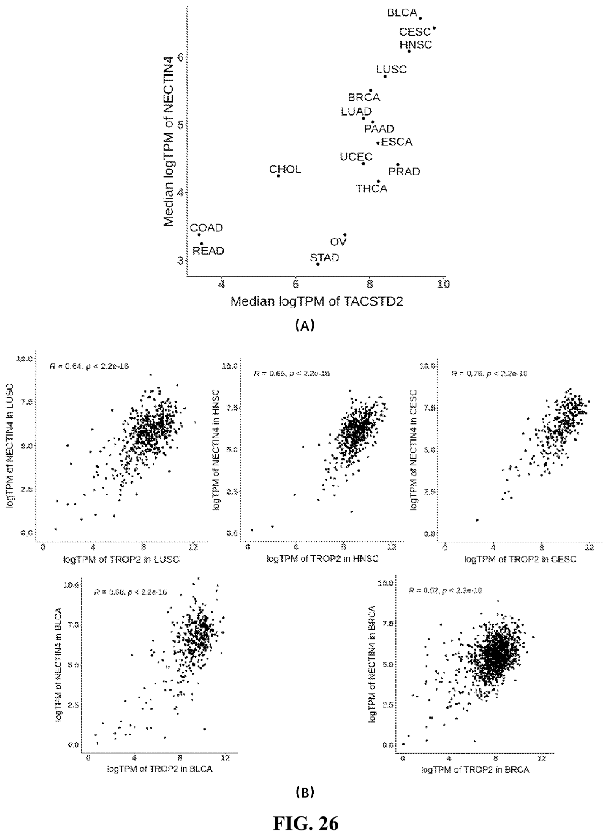

By applying TCGA tumor transcriptome data, expression level correlation analysis (Pearson correlation analysis) performed on clinical pathological samples from human tumor patients has revealed the expression pattern of TROP2 and NECTIN4 proteins in a variety of human tumor tissues. Tumor types with a double-positive expression trend of TROP2 and NECTIN4 include, but are not limited to, bladder cancer (e.g., BLCA (bladder urothelial carcinoma)), cervical cancer (e.g., CESC (cervical squamous cell carcinoma)), head and neck cancer (e.g., HNSC (head and neck squamous cell carcinoma)), lung cancer (e.g., LUSC (lung squamous cell carcinoma) and LUAD (lung adenocarcinoma)); breast cancer (e.g., BRCA (breast invasive carcinoma)); pancreatic cancer (e.g., PAAD (pancreatic adenocarcinoma)); esophageal cancer (e.g., ESCA (esophageal carcinoma)); prostate cancer (e.g., PRAD (prostate adenocarcinoma)); bile duct cancer (e.g., CHOL (cholangiocarcinoma)); endometrioid cancer (e.g., UCEC (uterine corpus endometrial carcinoma)); thyroid cancer (e.g., THCA (thyroid carcinoma)); ovarian cancer (e.g., OV (ovarian serous cystadenocarcinoma)); colorectal cancer (e.g., COAD (colon adenocarcinoma) and READ (rectum adenocarcinoma,)); and gastric cancer (e.g., STAD (stomach adenocarcinoma)). See, FIG. 26. These cancer types are all contemplated by the present disclosure.

In some embodiments, the tumor for use in the method and use of the present invention is selected from: urothelial cancer; bladder cancer (e.g., BLCA); cervical cancer (e.g., CESC); head and neck cancer (e.g., HNSC); lung cancer (e.g., LUSC and LUAD); breast cancer (e.g., BRCA); pancreatic cancer (e.g., PAAD); esophageal cancer (e.g., ESCA); prostate cancer (e.g., PRAD); bile duct cancer (e.g., CHOL); endometrioid cancer (e.g., UCEC); thyroid cancer (e.g., THCA); ovarian cancer (e.g., OV); colorectal cancer (e.g., COAD and READ); and gastric cancer (e.g., STAD).

In some embodiments, the tumor for use in the method and use of the present invention is selected from: cervical cancer, endometrial cancer, head and neck cancer (e.g., head and neck squamous cell carcinoma), breast cancer (e.g., triple-negative breast cancer), gastric cancer, bladder cancer (e.g., muscle-invasive bladder cancer), thyroid cancer, ovarian cancer, lung cancer (e.g., non-small cell lung cancer), colorectal cancer, and urothelial cancer (e.g., metastatic urothelial cancer).

The TROP2- and/or NECTIN4-positive tumor suitable for use in the method and use of the present invention may be a cancer at an early, intermediate, or advanced stage, or may be a metastatic cancer. In addition, the TROP2- and/or NECTIN4-positive tumor suitable for use in the method and use of the present invention may be a tumor that has previously undergone treatment and developed immune escape.

In some embodiments, the TROP2- and/or NECTIN4-positive tumor as treated according to the method of the present invention has at least 10%, 20%, 30%, 40%, 50%, 60%, 70%, 75%, 80%, 85%, 90%, 95%, or 100% TROP2- and/or NECTIN4-positive cells. Expression levels of TROP2 and/or NECTIN4 on tumor biopsies can be assessed by immunohistochemistry. High percentages of TROP2- and/or NECTIN4-positive cells have been detected on various cancer biopsies, e.g., bladder cancer.

Preferably, in some embodiments, the methods according to the present invention are used for treating tumors with a high percentage of TROP2- and/or NECTIN4-positive cells, e.g., tumors having at least 25%, 50%, 75%, or 100% TROP2- and/or NECTIN4-positive cells. In some embodiments, the application of the method of the present invention in the cancer induces tumor regression.

In some embodiments, the method according to the present invention may also be used for treating cancers with a percentage of TROP2- and/or NECTIN4-positive cells of lower than 25% or 20%. In some embodiments, the application of the method of the present invention in the cancer results in tumor growth inhibition.

In any one of the above embodiments of the method of the present invention, administration of the antibody or the binding fragment thereof according to the present invention and the immunoconjugate or conjugate or the pharmaceutically acceptable salt or solvate thereof according to the present invention may comprise 1) a therapeutic measure, which cures, slows, and alleviates symptoms of the diagnosed pathological condition or disease, and/or stops progression of the diagnosed pathological condition or disease; or 2) a prophylactic or preventative measure, which prevents and/or slows the progression of the pathological condition or disease. Thus, in the method of the present invention, the subject may be an individual already suffering from the disease, an individual susceptible to the disease, or an individual for whom the disease is to be prevented. The individual will benefit from the therapeutic measure or the prophylactic measure and exhibit an alleviation or amelioration in the occurrence, recurrence, or progression of the disease, disorder, condition, and/or symptom as compared to an individual not receiving the treatment. In some embodiments, the present invention relates to the treatment of the disease or disorder. In some other embodiments, the present invention relates to the prevention of the disease or disorder.

The antibody or the binding fragment thereof according to the present invention and the immunoconjugate or conjugate or the pharmaceutically acceptable salt or solvate thereof according to the present invention, and optionally additional therapeutic agents in combination therewith can be administered by any suitable method, including parenteral, intratumoral, and intranasal administration. Parenteral infusion includes intramuscular, intravenous, intra-arterial, intraperitoneal, or subcutaneous administration. Various administration schedules are encompassed herein, including but not limited to, single administration or multiple administrations at multiple time points, bolus injection, and pulse infusion.

In order to prevent or treat diseases, the appropriate dosage of the antibody or the binding fragment thereof according to the present invention and the immunoconjugate or conjugate or the pharmaceutically acceptable salt or solvate thereof according to the present invention (when used alone or in combination with one or more additional therapeutic agents) will depend on types of diseases to be treated, particular types of drugs used, severity and progression of the disease, purpose of administration (prophylactic or therapeutic) of the drug, previous treatments, clinical histories of patients, responses to the antibody, and the discretion of an attending physician.

In some embodiments, the present invention also provides use of the immunoconjugate or conjugate or the pharmaceutically acceptable salt or solvate thereof of the present invention, and the antibody or the antibody fragment of the present invention, as a medicament or in the manufacture of a medicament. In some embodiments, the medicament is a medicament for use in the aforementioned treatment and prevention methods.

### VIII. Preparation of ADC Molecule of Present Invention

In another aspect, the present invention provides a method for preparing an ADC by using the antibody of the present invention. The "ADC" in the present invention is defined as an antibody conjugated to an active substance (D) having biological and/or pharmaceutical activity via a linker (L). The method comprises allowing the antibody (Ab) of the present invention to be conjugated to one or more active substances D via one or more linkers (L) defined herein. Preferably, the linker-active substance is site-specifically conjugated to the antibody.

In some embodiments, the method comprises preparing an Ab for an ADC, and comprises culturing a host cell comprising a nucleic acid encoding the Ab (e.g., any one and/or more polypeptide chains) or an expression vector comprising the nucleic acid, as provided above, under conditions suitable for expressing the Ab or a chain thereof, and optionally recovering the Ab from the host cell (or host cell culture medium).

In some embodiments, the method comprises the following steps:
(a) adding the antibody Ab to a buffer, adding a reducing agent, and then incubating the mixture;
(b) adding a linker-payload to the reaction solution in step (a) for conjugating, so as to obtain a crude product; and
(c) optionally purifying the crude product to obtain the antibody-drug conjugate of the present invention;
wherein Ab is as defined above.

It should be understood that the linker-payload is reacted with the Ab to provide the -L-D moiety in the compound of formula I, and where -L-D is clearly defined, the structure of the linker-payload can be determined according to the prior art.

In some embodiments, the buffer in step a) is a PBS buffer, preferably at a pH of 5.0-9.0, e.g., 6.0-8.0.

In some embodiments, the reducing agent in step a) is TCEP.

In some embodiments, the linker-payload has the following structure: Z'-L₁-L₂-L₃-D, wherein L₁, L₂, L₃, and D are as defined above, Z' is 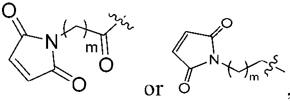 , and m is as defined above.

In some embodiments, the method for synthesizing an ADC in which Z is 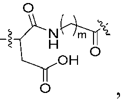 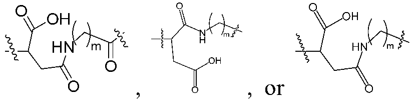 further comprises an additional hydrolysis step of opening the maleimide ring.

In some embodiments, the steps are performed under the specific reaction conditions disclosed in the examples.

It should be noted that embodiments in which ranges or specific values for the specific reaction conditions disclosed in the examples are varied by 100%, 80%, 60%, 40%, 20%, or 10% are also contemplated by the present invention.

In one aspect, the present invention further provides the following group A embodiments:
A1. A multispecific antibody binding to Trop2 and Nectin-4, wherein the antibody comprises at least one antigen-binding domain specifically binding to Trop2 and at least one antigen-binding domain specifically binding to Nectin-4.
A2. The multispecific antibody according to embodiment A1, wherein the antibody has one or more characteristics selected from the following:
   (a) the Trop2 antigen-binding domain and the Nectin-4 antigen-binding domain are VHH domains;
   (b) antigen-binding affinity K_{D} values of the Trop2 antigen-binding domain and the Nectin-4 antigen-binding domain are separately 50 × 10⁻⁸ M to 0.5 × 10⁻⁸ M;
   (c) the Trop2 antigen-binding domain and the Nectin-4 antigen-binding domain in the antibody have a valence ratio of 1:1 or 1:2; and
   (d) the antibody is a trivalent or tetravalent bispecific antibody.
A3. The multispecific antibody according to any one of embodiments A1-A2, wherein the antigen-binding domain specifically binding to Trop2 is a VHH domain, and
   wherein the VHH domain comprises CDR1, CDR2, and CDR3 sequences in the amino acid sequence selected from SEQ ID NOs: 5, 9, 13, 29, and 30;
   still preferably, the CDR1, CDR2, and CDR3 sequences:
      (i) comprise or consist of the amino acid sequences of SEQ ID NOs: 6, 7, and 8, respectively;
      (ii) comprise or consist of the amino acid sequences of SEQ ID NOs: 10, 11, and 12, respectively;
      (iii) comprise or consist of the amino acid sequences of SEQ ID NOs: 14, 15, and 16, respectively; or
      (iv) comprise or consist of the amino acid sequences of SEQ ID NOs: 31, 32, and 33, respectively;
   still preferably, the VHH domain comprises the amino acid sequence selected from SEQ ID NOs: 5, 9, 13, 29, and 30, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 5, 9, 13, 29, and 30.
A4. The multispecific antibody according to any one of embodiments A1-A3, wherein the antigen-binding domain specifically binding to Nectin-4 is a VHH domain, and
   wherein the VHH domain comprises CDR1, CDR2, and CDR3 sequences in the amino acid sequence selected from SEQ ID NOs: 17, 21, 25, 34, 35, and 38;
   still preferably, the CDR1, CDR2, and CDR3 sequences:
      (i) comprise or consist of the amino acid sequences of SEQ ID NOs: 18, 19, and 20, respectively;
      (ii) comprise or consist of the amino acid sequences of SEQ ID NOs: 22, 23, and 24, respectively;
      (iii) comprise or consist of the amino acid sequences of SEQ ID NOs: 26, 27, and 28, respectively;
      (iv) comprise or consist of the amino acid sequences of SEQ ID NOs: 18, 36, and 20, respectively; or
      (v) comprise or consist of the amino acid sequences of SEQ ID NOs: 18, 37, and 20, respectively;
   still preferably, the VHH domain comprises the amino acid sequence selected from SEQ ID NOs: 17, 21, 25, 34, 35, and 38, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 17, 21, 25, 34, 35, and 38.
A5. The multispecific antibody according to any one of embodiments A1-A4, wherein the antibody further comprises:
   (a) a half-life extension domain, wherein preferably, the half-life extension domain is selected from a serum albumin binding peptide and an immunoglobulin Fc region; and/or
   (b) a linker, wherein preferably, the linker is 5-15 amino acids in length, and more preferably, the linker comprises the amino acid sequence (G₄S)ₙ, wherein n = 1, 2, or 3.
A6. The multispecific antibody according to any one of embodiments A1-A5, wherein the antibody is a single-chain or double-chain antibody comprising at least one (preferably 1 or 2) Nectin4-binding domain and at least one (preferably 1 or 2) Trop2-binding domain.
A7. The multispecific antibody according to any one of embodiments A1-A6, wherein the antibody comprises two identical polypeptide chains, wherein the polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
   wherein n1, n2, n3, and n4 are each independently selected from integers of 0, 1, and 2; preferably, n3 and n4 are 0, and n1 and n2 are 1;
   wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain; the symbol "-" represents linkage via a linker or direct linkage.
A8. The multispecific antibody according to embodiment A7, wherein the polypeptide chain comprises, from the N-terminus to the C-terminus, VHH^{A}-VHH^{B}-HLE, and wherein VHH^{A} represents a Nectin4-binding domain and VHH^{B} represents a Trop2-binding domain.
A9. The multispecific antibody according to any one of embodiments A1-A6, wherein the antibody comprises two different polypeptide chains, wherein
   the first polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
   the second polypeptide chain comprises, from the N-terminus to the C-terminus, (VHH^{B})ₘ₁-(VHH^{A})ₘ₂-HLE-(VHH^{B})ₘ₃-(VHH^{A})ₘ₄,
   wherein n1, n2, n3, and n4, as well as m1, m2, m3, and m4, are each independently selected from integers of 0, 1, and 2, and preferably, wherein n3, n4, m3, and m4 are 0, n1 + m2 = 1 or 2, and n2 + m1 = 1 or 2; still preferably, m1 = 0 and m2 = 1, or m2 = 0 and m1 = 1;
   wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain, and preferably, comprises a knob-into-hole mutation; the symbol "-" represents linkage via a linker or direct linkage.
A10. The multispecific antibody according to embodiment A9, wherein
   the first polypeptide chain comprises, from the N-terminus to the C-terminus, VHH^{A}-VHH^{B}-HLE,
   the second polypeptide chain comprises, from the N-terminus to the C-terminus, VHH^{A}-HLE,
   wherein VHH^{A} represents a Nectin4-binding domain and VHH^{B} represents a Trop2-binding domain.
A11. The multispecific antibody according to any one of embodiments A7-A11, wherein the immunoglobulin Fc region is an Fc region from IgG, such as an Fc region from human IgG1 or IgG4; preferably, the Fc region comprises a mutation that reduces or eliminates the binding of the Fc region to FcγR.
A12. The multispecific antibody according to any one of embodiments A1-A6, wherein the antibody comprises a single polypeptide chain, wherein the polypeptide chain comprises, from the N-terminus to the C-terminus, (VHH^{A})ₙ₁-(VHH^{B})ₙ₂-(HLE)ₙ₃-(VHH^{A})ₙ₄-(VHH^{B})ₙ₅-(HLE)ₙ₆,
   wherein n1, n2, n3, n4, n5, and n6 are each independently selected from integers of 0, 1, and 2, and preferably, n1 + n4 = 1 or 2, n2 + n5 = 1 or 2, and n3 + n6 = 0 or 1,
   wherein VHH^{A} and VHH^{B} represent VHH domains binding to an antigen A and an antigen B, respectively, wherein A and B are different from each other and are each independently selected from Nectin4 and Trop2; HLE represents a serum albumin binding peptide as a half-life extension domain; the symbol "-" represents linkage via a linker or direct linkage,
   preferably, the polypeptide chain comprises, from the N-terminus to the C-terminus:
      (i) VHH^{A}-VHH^{B},
      (ii) VHH^{A}-VHH^{B}-HLE,
      (iii) VHH^{B}-VHH^{A}-VHH^{B},
      (iv) VHH^{A}-VHH^{B}-VHH^{B},
      (v) VHH^{A}-HLE-VHH^{B}-VHH^{B}, or
      (vi) VHH^{A}-VHH^{A}-HLE-VHH^{B}-VHH^{B}, wherein preferably, A represents Trop2 and B represents Nectin4,
   still preferably, the HLE represents an anti-serum albumin VHH domain, and is optionally selected from the amino acid sequences of SEQ ID NOs: 39 and 40, or amino acid sequences having at least 85%, 90%, 95%, or 99% identity thereto or having one or more (preferably 1-10, and more preferably 1-5) amino acid additions, deletions, and/or substitutions.
A13. The multispecific antibody according to any one of embodiments A1-A12, wherein
   (a) the antigen-binding domain specifically binding to Nectin4 comprises the amino acid sequence of SEQ ID NO: 25, and the antigen-binding domain specifically binding to Trop2 comprises the amino acid sequence of SEQ ID NO: 13;
   (b) the antigen-binding domain specifically binding to Nectin4 comprises the amino acid sequence of SEQ ID NO: 21, and the antigen-binding domain specifically binding to Trop2 comprises the amino acid sequence of SEQ ID NO: 9;
   (c) the antigen-binding domain specifically binding to Nectin4 comprises the amino acid sequence of SEQ ID NO: 25, and the antigen-binding domain specifically binding to Trop2 comprises the amino acid sequence of SEQ ID NO: 9;
   (d) the antigen-binding domain specifically binding to Nectin4 comprises the amino acid sequence of SEQ ID NO: 35, and the antigen-binding domain specifically binding to Trop2 comprises the amino acid sequence selected from SEQ ID NOs: 29 and 30; or
   (e) the antigen-binding domain specifically binding to Nectin4 comprises the amino acid sequence selected from SEQ ID NOs: 34 and 38, and the antigen-binding domain specifically binding to Trop2 comprises the amino acid sequence selected from SEQ ID NOs: 29 and 30.
A14. The multispecific antibody according to any one of embodiments A1-A6, wherein the antibody is in a double-chain form, wherein
   (a) the antibody is in a symmetric double-chain form, and the polypeptide chain of the antibody comprises the amino acid sequence of SEQ ID NO: 53 or 54 or an amino acid sequence having at least 95%, 96%, 97%, 98%, or 99% identity thereto, and preferably comprises the amino acid sequence of SEQ ID NO: 53; or
   (b) the antibody is in an asymmetric double-chain form, and the antibody comprises a first polypeptide chain and a second polypeptide chain selected from the group consisting of:
      (i) first and second polypeptide chains comprising the amino acid sequences set forth in SEQ ID NOs: 55 and 56 or amino acid sequences having at least 95%, 96%, 97%, 98%, or 99% identity thereto, respectively,
      (ii) first and second polypeptide chains comprising the amino acid sequences set forth in SEQ ID NOs: 57 and 58 or amino acid sequences having at least 95%, 96%, 97%, 98%, or 99% identity thereto, respectively,
      (iii) first and second polypeptide chains comprising the amino acid sequences set forth in SEQ ID NOs: 59 and 60 or amino acid sequences having at least 95%, 96%, 97%, 98%, or 99% identity thereto, respectively,
      (iv) first and second polypeptide chains comprising the amino acid sequences set forth in SEQ ID NOs: 61 and 62 or amino acid sequences having at least 95%, 96%, 97%, 98%, or 99% identity thereto, respectively,
      (v) first and second polypeptide chains comprising the amino acid sequences set forth in SEQ ID NOs: 63 and 64 or amino acid sequences having at least 95%, 96%, 97%, 98%, or 99% identity thereto, respectively,
      (vi) first and second polypeptide chains comprising the amino acid sequences set forth in SEQ ID NOs: 65 and 66 or amino acid sequences having at least 95%, 96%, 97%, 98%, or 99% identity thereto, respectively,
      (vii) first and second polypeptide chains comprising the amino acid sequences set forth in SEQ ID NOs: 67 and 68 or amino acid sequences having at least 95%, 96%, 97%, 98%, or 99% identity thereto, respectively,
      (viii) first and second polypeptide chains comprising the amino acid sequences set forth in SEQ ID NOs: 69 and 70 or amino acid sequences having at least 95%, 96%, 97%, 98%, or 99% identity thereto, respectively,
      (ix) first and second polypeptide chains comprising the amino acid sequences set forth in SEQ ID NOs: 71 and 72 or amino acid sequences having at least 95%, 96%, 97%, 98%, or 99% identity thereto, respectively,
      (x) first and second polypeptide chains comprising the amino acid sequences set forth in SEQ ID NOs: 73 and 74 or amino acid sequences having at least 95%, 96%, 97%, 98%, or 99% identity thereto, respectively, and
      (xi) first and second polypeptide chains comprising the amino acid sequences set forth in SEQ ID NOs: 75 and 76 or amino acid sequences having at least 95%, 96%, 97%, 98%, or 99% identity thereto, respectively;
      preferably, the first and second polypeptide chains comprise the amino acid sequences of SEQ ID NOs: 61/62, 69/70, or 71/72, respectively.
A15. The multispecific antibody according to any one of embodiments A1-A6, wherein the antibody is in a single-chain form, wherein the polypeptide chain of the antibody comprises the amino acid sequence selected from SEQ ID NOs: 41-52 or an amino acid sequence having at least 95%, 96%, 97%, 98%, or 99% identity thereto.
A16. An anti-Trop2 antibody, comprising a VHH domain specifically binding to Trop2,
   wherein the VHH domain comprises CDR1, CDR2, and CDR3 sequences in the amino acid sequence selected from SEQ ID NOs: 5, 9, 13, 29, and 30;
   still preferably, the CDR1, CDR2, and CDR3 sequences:
      (i) comprise or consist of the amino acid sequences of SEQ ID NOs: 6, 7, and 8, respectively;
      (ii) comprise or consist of the amino acid sequences of SEQ ID NOs: 10, 11, and 12, respectively;
      (iii) comprise or consist of the amino acid sequences of SEQ ID NOs: 14, 15, and 16, respectively; or
      (iv) comprise or consist of the amino acid sequences of SEQ ID NOs: 31, 32, and 33, respectively;
   still preferably, the VHH domain comprises the amino acid sequence selected from SEQ ID NOs: 5, 9, 13, 29, and 30, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 5, 9, 13, 29, and 30.
A17. An anti-Necti-4 antibody, comprising a VHH domain specifically binding to Nectin-4,
   wherein the VHH domain comprises CDR1, CDR2, and CDR3 sequences in the amino acid sequence selected from SEQ ID NOs: 17, 21, 25, 34, 35, and 38;
   preferably, the CDR1, CDR2, and CDR3 sequences:
      (i) comprise or consist of the amino acid sequences of SEQ ID NOs: 18, 19, and 20, respectively;
      (ii) comprise or consist of the amino acid sequences of SEQ ID NOs: 22, 23, and 24, respectively;
      (iii) comprise or consist of the amino acid sequences of SEQ ID NOs: 26, 27, and 28, respectively;
      (iv) comprise or consist of the amino acid sequences of SEQ ID NOs: 18, 36, and 20, respectively; or
      (v) comprise or consist of the amino acid sequences of SEQ ID NOs: 18, 37, and 20, respectively;
   still preferably, the VHH domain comprises the amino acid sequence selected from SEQ ID NOs: 17, 21, 25, 34, 35, and 38, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 17, 21, 25, 34, 35, and 38.
A18. A polynucleotide encoding the antibody according to any one of embodiments A1-A17.
A19. A vector, preferably an expression vector, comprising the polynucleotide according to embodiment A18.
A20. A host cell, comprising the polynucleotide according to embodiment A18 or the vector according to embodiment A19, wherein, for example, the host cell is a mammalian cell.
A21. A method for producing the antibody according to any one of embodiments A1-A17, comprising:
   culturing a host cell comprising a polynucleotide encoding a polypeptide chain under conditions suitable for the expression of the polypeptide chain of the antibody; and assembling the polypeptide chain to produce the antibody under conditions suitable for the assembly of the polypeptide chain into the antibody.
A22. An immunoconjugate or immunofusion comprising the antibody according to any one of embodiments A1-A17.
A23. The immunoconjugate according to embodiment A22, wherein the immunoconjugate is an antibody-drug conjugate represented by formula (I):

   Ab-(L-D)ₚ (I)

   or a pharmaceutically acceptable salt or solvate thereof,
   wherein:
   Ab is the antibody according to any one of embodiments A1-A17;
   L is a linker;
   D is a drug, preferably an anti-tumor compound; and
   p is an integer selected from 1 to 16, e.g., 1, 2, 3, 4, 5, 6, 7, 8, 9, or 10.
A24. The antibody-drug conjugate or the pharmaceutically acceptable salt or solvate thereof according to embodiment A23, wherein the anti-tumor compound is a cytotoxic agent, such as a camptothecin compound and an auristatin compound, such as exatecan, Dxd, or MMAE.
A25. The antibody-drug conjugate or the pharmaceutically acceptable salt or solvate thereof according to embodiment A23, wherein D has a structure represented by formula (D-1a) or formula (D-1b): 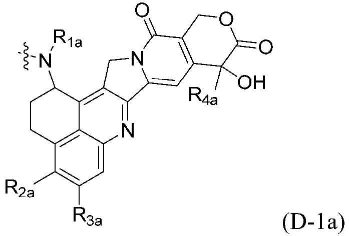
   wherein R₁ₐ is selected from H and C₁-C₆ alkyl;
   R₂ₐ is selected from H, halogen, C₁-C₆ alkyl, C₁-C₆ haloalkyl, -OR₅ₐ, and -SR₅ₐ;
   R₃ₐ is selected from H, halogen, CN, C₁-C₆ alkyl, C₁-C₆ haloalkyl, and -OR₅ₐ; and R₄ₐ and R₅ₐ are independently selected from H and C₁-C₄ alkyl;
      or 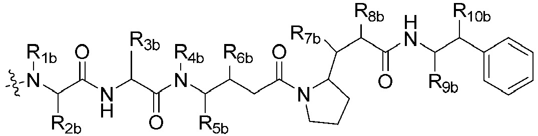 formula (D-1b)
   wherein R_{1b}, R_{2b}, R_{3b}, R_{4b}, R_{5b}, and R_{8b} are each independently selected from C₁₋₈ alkyl, such as methyl, ethyl, n-propyl, isopropyl, n-butyl, isobutyl, or *sec*-butyl;
   R_{6b} and R_{7b} are each independently selected from C₁₋₈ alkoxy, such as methoxy, ethoxy, or propoxy;
   R_{9b} is selected from C₁₋₈ alkyl and COOH, such as methyl, ethyl, n-propyl, isopropyl, n-butyl, isobutyl, or *sec*-butyl; and
   R_{10b} is selected from OH and H.
A26. The antibody-drug conjugate or the pharmaceutically acceptable salt or solvate thereof according to embodiment A25, wherein
   R₁ₐ is H; R₂ₐ is C₁-C₆ alkyl; R₃ₐ is halogen, preferably -F; R₄ₐ is C₁-C₄ alkyl, preferably ethyl;
   R_{1b}, R_{4b}, and R_{8b} are each independently selected from C₁₋₂ alkyl, preferably methyl;
   R_{2b}, R_{3b}, and R_{5b} are each independently selected from C₃₋₄ alkyl;
   R_{6b} and R_{7b} are each independently selected from C₁₋₂ alkoxy; and
   R_{9b} is selected from C₁₋₄ alkyl, and R_{10b} is OH; or R_{9b} is COOH, and R_{10b} is H.
A27. The antibody-drug conjugate or the pharmaceutically acceptable salt or solvate thereof according to embodiment A25, wherein D has a structure represented by formula (D-2a) or formula (D-2b): 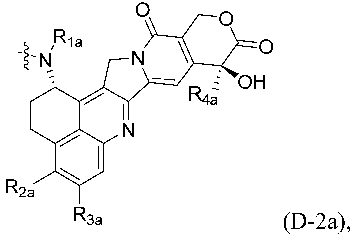 wherein R₁ₐ, R₂ₐ, R₃ₐ, and R₄ₐ are as defined in embodiment A25 or A26; 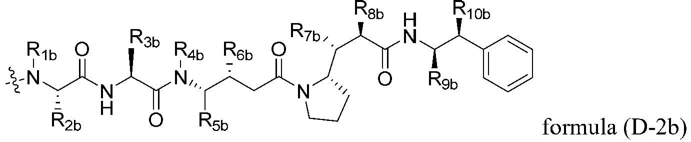 wherein R_{1b}, R_{2b}, R_{3b}, R_{4b}, R_{5b}, R_{6b}, R_{7b}, R_{8b}, R_{9b}, and R_{10b} are as defined in embodiment A25 or A26.
A28. The antibody-drug conjugate or the pharmaceutically acceptable salt or solvate thereof according to embodiment A25, wherein D has a structure represented by formula (D-3a) or formula (D-3b): 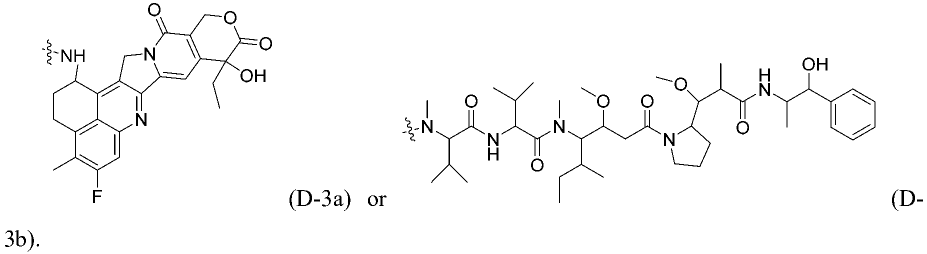
A29. The antibody-drug conjugate or the pharmaceutically acceptable salt or solvate thereof according to embodiment A25, wherein D has a structure represented by formula (D-4a) or formula (D-4b): 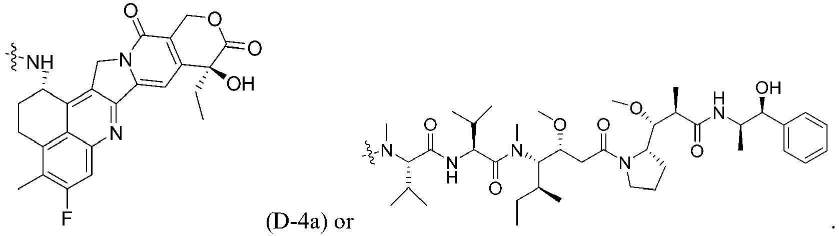
A30. The antibody-drug conjugate or the pharmaceutically acceptable salt or solvate thereof according to any one of embodiments A23-A29, wherein -L- has the following structure: -Z-L₁-L₂-L₃-,
   wherein
   Z is selected from 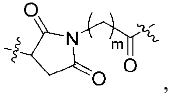 wherein m is an integer selected from 1 to 10, e.g., 1, 2, 3, 4, 5, 6, 7, or 8;
   L₁ is selected from absence, 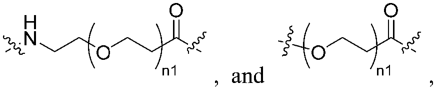 wherein n1 is independently an integer selected from 0 to 12, e.g., 1, 2, 3, 4, 5, 6, 7, or 8;
   L₂ is a peptide residue consisting of 2-8 amino acids; and
   L₃ is selected from: 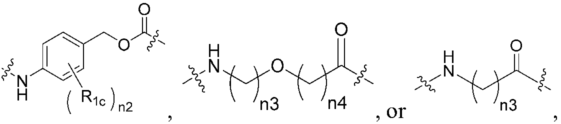 wherein R_{1c} is selected from: H and C₁-C₆ alkyl; n2 is 1, 2, 3, or 4; and n3 and n4 are independently 1, 2, 3, 4, 5, or 6.
A31. The antibody-drug conjugate or the pharmaceutically acceptable salt or solvate thereof according to embodiment A30,
   wherein L₂ is a peptide residue consisting of 2, 3, 4, 5, 6, or 7 amino acids.
A32. The antibody-drug conjugate or the pharmaceutically acceptable salt or solvate thereof according to embodiment A31,
   wherein Z is selected from 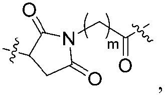 wherein m is 1, 2, 3, 4, 5, 6, 7, or 8;
   L₁ is selected from absence, 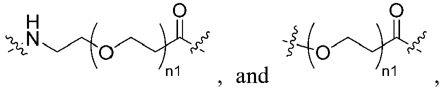 wherein n1 is independently an integer selected from 0-12; and
   L₃ is selected from: 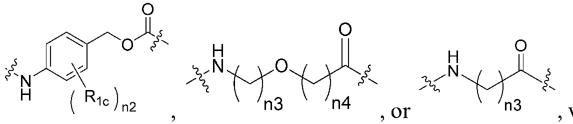 wherein R_{1c} is selected from H and C₁-C₆ alkyl; n2 is 1, 2, 3, or 4; and n3 and n4 are independently 1, 2, 3, 4, 5, or 6.
A33. The antibody-drug conjugate or the pharmaceutically acceptable salt or solvate thereof according to embodiment A31,
   Z is selected from 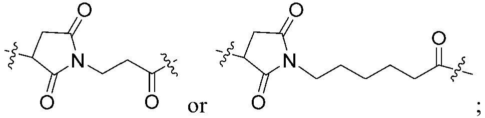
   L₁ is selected from absence and 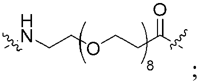
   L₂ is selected from -Val-Ala-, -Gly-Gly-Phe-Gly-, -Val-Cit-, and -Glu-Val-Cit-; and
   L₃ is selected from 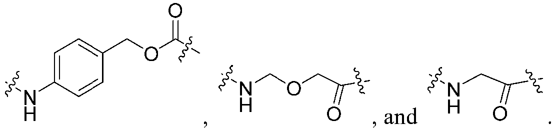
A34. The antibody-drug conjugate or the pharmaceutically acceptable salt or solvate thereof according to embodiment A31,
   wherein -Z-L₁-L₂-L₃- is selected from the following structure 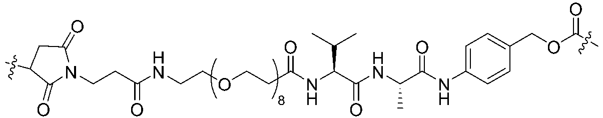 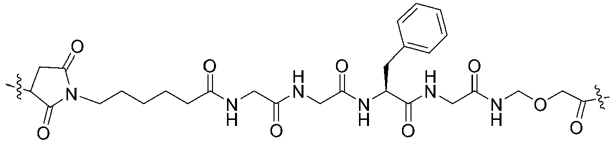 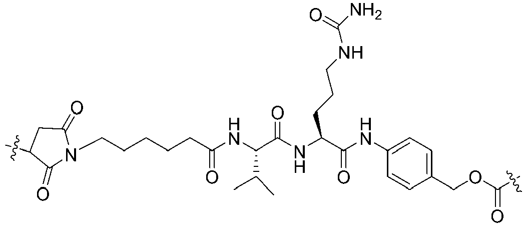
A35. The antibody-drug conjugate or the pharmaceutically acceptable salt or solvate thereof according to any one of embodiments A23-A34, having an average DAR value of 2-10, e.g., 4-8.
A36. The antibody-drug conjugate or the pharmaceutically acceptable salt or solvate thereof according to embodiment A23, wherein the antibody-drug conjugate is selected from: 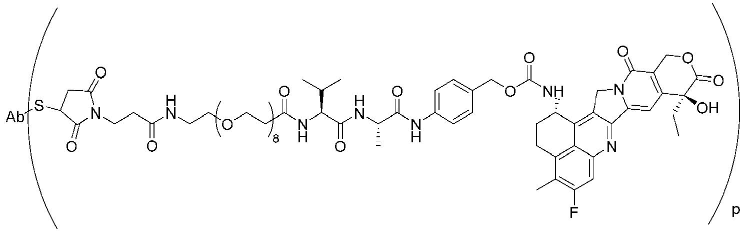 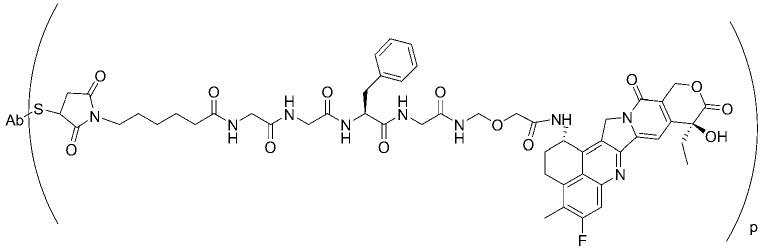 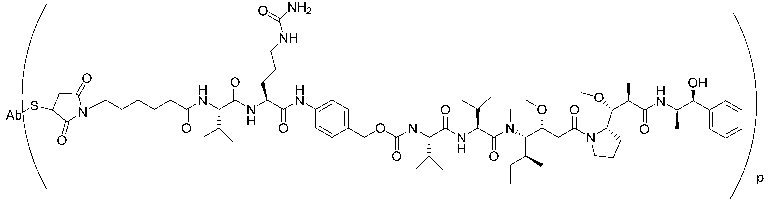
   wherein Ab and p are as defined in embodiment A23;
   preferably, Ab is a double-chain antibody, wherein
      (a) the antibody is in a symmetric double-chain form, and the polypeptide chain of the antibody comprises the amino acid sequence of SEQ ID NO: 53; or
      (b) the antibody is in an asymmetric double-chain form, and the antibody comprises first and second polypeptide chains comprising the amino acid sequences of SEQ ID NOs: 61/62, 69/70, or 71/72, respectively;
   preferably, the antibody-drug conjugate has an average DAR of 2-10 or 4-8.
A37. A pharmaceutical composition, comprising the antibody according to any one of embodiments A1-A17 or the immunoconjugate or immunofusion according to any one of embodiments A22-A36, and a pharmaceutically acceptable carrier.
A38. Use of the antibody according to any one of embodiments A1-A17 or the immunoconjugate or immunofusion according to any one of embodiments A22-A36 as a medicament or in the manufacture of a medicament.
A39. The use according to embodiment A38, wherein the medicament is used for treating and/or preventing cancer in an individual, wherein the cancer is a Trop2/Nectin-4 double-positive solid tumor; preferably, the tumor has at least 40% of cells exhibiting Trop2/Nectin-4 double-positive high-level expression.
In one aspect, the present invention further provides the following group B embodiments:
   B1. A multispecific antibody binding to Trop2 and Nectin-4, wherein the antibody comprises at least one antigen-binding domain specifically binding to Trop2 and at least one antigen-binding domain specifically binding to Nectin-4.
   B2. The multispecific antibody according to embodiment B1, wherein the antibody has one or more characteristics selected from the following:
      (a) the Trop2 antigen-binding domain and the Nectin-4 antigen-binding domain are VHH domains;
      (b) antigen-binding affinity K_{D} values of the Trop2 antigen-binding domain and the Nectin-4 antigen-binding domain are separately 50 × 10⁻⁸ M to 0.5 × 10⁻⁸ M;
      (c) the Trop2 antigen-binding domain and the Nectin-4 antigen-binding domain in the antibody have a valence ratio of 1:1 or 1:2; and
      (d) the antibody is a trivalent or tetravalent bispecific antibody.
   B3. The multispecific antibody according to any one of embodiments B1-B2, wherein the antigen-binding domain specifically binding to Trop2 is a VHH domain, and
      wherein the VHH domain comprises CDR1, CDR2, and CDR3 sequences in the amino acid sequence selected from SEQ ID NOs: 5, 9, 13, 29, and 30;
      still preferably, the CDR1, CDR2, and CDR3 sequences:
         (i) comprise or consist of the amino acid sequences of SEQ ID NOs: 6, 7, and 8, respectively;
         (ii) comprise or consist of the amino acid sequences of SEQ ID NOs: 10, 11, and 12, respectively;
         (iii) comprise or consist of the amino acid sequences of SEQ ID NOs: 14, 15, and 16, respectively; or
         (iv) comprise or consist of the amino acid sequences of SEQ ID NOs: 31, 32, and 33, respectively;
   still preferably, the VHH domain comprises the amino acid sequence selected from SEQ ID NOs: 5, 9, 13, 29, and 30, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 5, 9, 13, 29, and 30.
B4. The multispecific antibody according to any one of embodiments B1-B3, wherein the antigen-binding domain specifically binding to Nectin-4 is a VHH domain, and
   wherein the VHH domain comprises CDR1, CDR2, and CDR3 sequences in the amino acid sequence selected from SEQ ID NOs: 17, 21, 25, 34, 35, and 38;
   still preferably, the CDR1, CDR2, and CDR3 sequences:
      (i) comprise or consist of the amino acid sequences of SEQ ID NOs: 18, 19, and 20, respectively;
      (ii) comprise or consist of the amino acid sequences of SEQ ID NOs: 22, 23, and 24, respectively;
      (iii) comprise or consist of the amino acid sequences of SEQ ID NOs: 26, 27, and 28, respectively;
      (iv) comprise or consist of the amino acid sequences of SEQ ID NOs: 18, 36, and 20, respectively; or
      (v) comprise or consist of the amino acid sequences of SEQ ID NOs: 18, 37, and 20, respectively;
   still preferably, the VHH domain comprises the amino acid sequence selected from SEQ ID NOs: 17, 21, 25, 34, 35, and 38, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 17, 21, 25, 34, 35, and 38.
B5. The multispecific antibody according to any one of embodiments B1-B4, wherein the antibody further comprises:
   (a) a half-life extension domain, wherein preferably, the half-life extension domain is selected from a serum albumin binding peptide and an immunoglobulin Fc region; and/or
   (b) a linker, wherein preferably, the linker is 5-15 amino acids in length, and more preferably, the linker comprises the amino acid sequence (G₄S)ₙ, wherein n = 1, 2, or 3.
B6. The multispecific antibody according to any one of embodiments B1-B5, wherein the antibody is a single-chain or double-chain antibody comprising at least one (preferably 1 or 2) Nectin4-binding domain and at least one (preferably 1 or 2) Trop2-binding domain.
B7. The multispecific antibody according to any one of embodiments B1-B6, wherein the antibody comprises two identical polypeptide chains, wherein the polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
   wherein n1, n2, n3, and n4 are each independently selected from integers of 0, 1, and 2; preferably, n3 and n4 are 0, and n1 and n2 are 1;
   wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain; the symbol "-" represents linkage via a linker or direct linkage.
B8. The multispecific antibody according to embodiment B7, wherein the polypeptide chain comprises, from the N-terminus to the C-terminus, VHH^{A}-VHH^{B}-HLE, and wherein VHH^{A} represents a Nectin4-binding domain and VHH^{B} represents a Trop2-binding domain.
B9. The multispecific antibody according to any one of embodiments B1-B6, wherein the antibody comprises two different polypeptide chains, wherein
   the first polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
   the second polypeptide chain comprises, from the N-terminus to the C-terminus, (VHH^{B})ₘ₁-(VHH^{A})ₘ₂-HLE-(VHH^{B})ₘ₃-(VHH^{A})ₘ₄,
   wherein n1, n2, n3, and n4, as well as m1, m2, m3, and m4, are each independently selected from integers of 0, 1, and 2, and preferably, wherein n3, n4, m3, and m4 are 0, n1 + m2 = 1 or 2, and n2 + m1 = 1 or 2; still preferably, m1 = 0 and m2 = 1, or m2 = 0 and m1 = 1;
   wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain, and preferably, comprises a knob-into-hole mutation; the symbol "-" represents linkage via a linker or direct linkage.
B10. The multispecific antibody according to embodiment B9, wherein
   the first polypeptide chain comprises, from the N-terminus to the C-terminus, VHH^{A}-VHH^{B}-HLE,
   the second polypeptide chain comprises, from the N-terminus to the C-terminus, VHH^{A}-HLE,
   wherein VHH^{A} represents a Nectin4-binding domain and VHH^{B} represents a Trop2-binding domain.
B11. The multispecific antibody according to any one of embodiments B7-B11, wherein the immunoglobulin Fc region is an Fc region from IgG, such as an Fc region from human IgG1 or IgG4; preferably, the Fc region comprises a mutation that reduces or eliminates the binding of the Fc region to FcγR.
B12. The multispecific antibody according to any one of embodiments B1-B6, wherein the antibody comprises a single polypeptide chain, wherein the polypeptide chain comprises, from the N-terminus to the C-terminus, (VHH^{A})ₙ₁-(VHH^{B})ₙ₂-(HLE)ₙ₃-(VHH^{A})ₙ₄-(VHH^{B})ₙ₅-(HLE)ₙ₆,
   wherein n1, n2, n3, n4, n5, and n6 are each independently selected from integers of 0, 1, and 2, and preferably, n1 + n4 = 1 or 2, n2 + n5 = 1 or 2, and n3 + n6 = 0 or 1,
   wherein VHH^{A} and VHH^{B} represent VHH domains binding to an antigen A and an antigen B, respectively, wherein A and B are different from each other and are each independently selected from Nectin4 and Trop2; HLE represents a serum albumin binding peptide as a half-life extension domain; the symbol "-" represents linkage via a linker or direct linkage,
   preferably, the polypeptide chain comprises, from the N-terminus to the C-terminus:
      (i) VHH^{A}-VHH^{B},
      (ii) VHH^{A}-VHH^{B}-HLE,
      (iii) VHH^{B}-VHH^{A}-VHH^{B},
      (iv) VHH^{A}-VHH^{B}-VHH^{B},
      (v) VHH^{A}-HLE-VHH^{B}-VHH^{B}, or
      (vi) VHH^{A}-VHH^{A}-HLE-VHH^{B}-VHH^{B}, wherein preferably, A represents Trop2 and B represents Nectin4;
   still preferably, the HLE represents an anti-serum albumin VHH domain, and is optionally selected from the amino acid sequences of SEQ ID NOs: 39 and 40, or amino acid sequences having at least 85%, 90%, 95%, or 99% identity thereto or having one or more (preferably 1-10, and more preferably 1-5) amino acid additions, deletions, and/or substitutions.
B13. The multispecific antibody according to any one of embodiments B1-B12, wherein
   (a) the antigen-binding domain specifically binding to Nectin4 comprises the amino acid sequence of SEQ ID NO: 25, and the antigen-binding domain specifically binding to Trop2 comprises the amino acid sequence of SEQ ID NO: 13;
   (b) the antigen-binding domain specifically binding to Nectin4 comprises the amino acid sequence of SEQ ID NO: 21, and the antigen-binding domain specifically binding to Trop2 comprises the amino acid sequence of SEQ ID NO: 9;
   (c) the antigen-binding domain specifically binding to Nectin4 comprises the amino acid sequence of SEQ ID NO: 25, and the antigen-binding domain specifically binding to Trop2 comprises the amino acid sequence of SEQ ID NO: 9;
   (d) the antigen-binding domain specifically binding to Nectin4 comprises the amino acid sequence of SEQ ID NO: 35, and the antigen-binding domain specifically binding to Trop2 comprises the amino acid sequence selected from SEQ ID NOs: 29 and 30; or
   (e) the antigen-binding domain specifically binding to Nectin4 comprises the amino acid sequence selected from SEQ ID NOs: 34 and 38, and the antigen-binding domain specifically binding to Trop2 comprises the amino acid sequence selected from SEQ ID NOs: 29 and 30.
B14. The multispecific antibody according to any one of embodiments B1-B6, wherein the antibody is in a double-chain form, wherein
   (a) the antibody is in a symmetric double-chain form, and the polypeptide chain of the antibody comprises the amino acid sequence of SEQ ID NO: 53 or 54 or an amino acid sequence having at least 95%, 96%, 97%, 98%, or 99% identity thereto, and preferably comprises the amino acid sequence of SEQ ID NO: 53; or
   (b) the antibody is in an asymmetric double-chain form, and the antibody comprises a first polypeptide chain and a second polypeptide chain selected from the group consisting of:
      (i) first and second polypeptide chains comprising the amino acid sequences set forth in SEQ ID NOs: 55 and 56 or amino acid sequences having at least 95%, 96%, 97%, 98%, or 99% identity thereto, respectively,
      (ii) first and second polypeptide chains comprising the amino acid sequences set forth in SEQ ID NOs: 57 and 58 or amino acid sequences having at least 95%, 96%, 97%, 98%, or 99% identity thereto, respectively,
      (iii) first and second polypeptide chains comprising the amino acid sequences set forth in SEQ ID NOs: 59 and 60 or amino acid sequences having at least 95%, 96%, 97%, 98%, or 99% identity thereto, respectively,
      (iv) first and second polypeptide chains comprising the amino acid sequences set forth in SEQ ID NOs: 61 and 62 or amino acid sequences having at least 95%, 96%, 97%, 98%, or 99% identity thereto, respectively,
      (v) first and second polypeptide chains comprising the amino acid sequences set forth in SEQ ID NOs: 63 and 64 or amino acid sequences having at least 95%, 96%, 97%, 98%, or 99% identity thereto, respectively,
      (vi) first and second polypeptide chains comprising the amino acid sequences set forth in SEQ ID NOs: 65 and 66 or amino acid sequences having at least 95%, 96%, 97%, 98%, or 99% identity thereto, respectively,
      (vii) first and second polypeptide chains comprising the amino acid sequences set forth in SEQ ID NOs: 67 and 68 or amino acid sequences having at least 95%, 96%, 97%, 98%, or 99% identity thereto, respectively,
      (viii) first and second polypeptide chains comprising the amino acid sequences set forth in SEQ ID NOs: 69 and 70 or amino acid sequences having at least 95%, 96%, 97%, 98%, or 99% identity thereto, respectively,
      (ix) first and second polypeptide chains comprising the amino acid sequences set forth in SEQ ID NOs: 71 and 72 or amino acid sequences having at least 95%, 96%, 97%, 98%, or 99% identity thereto, respectively,
      (x) first and second polypeptide chains comprising the amino acid sequences set forth in SEQ ID NOs: 73 and 74 or amino acid sequences having at least 95%, 96%, 97%, 98%, or 99% identity thereto, respectively, and
      (xi) first and second polypeptide chains comprising the amino acid sequences set forth in SEQ ID NOs: 75 and 76 or amino acid sequences having at least 95%, 96%, 97%, 98%, or 99% identity thereto, respectively;
      preferably, the first and second polypeptide chains comprise the amino acid sequences of SEQ ID NOs: 61/62, 69/70, or 71/72, respectively.
B15. The multispecific antibody according to any one of embodiments B1-B6, wherein the antibody is in a single-chain form, wherein the polypeptide chain of the antibody comprises the amino acid sequence selected from SEQ ID NOs: 41-52 or an amino acid sequence having at least 95%, 96%, 97%, 98%, or 99% identity thereto.
B16. An anti-Trop2 antibody, comprising a VHH domain specifically binding to Trop2,
   wherein the VHH domain comprises CDR1, CDR2, and CDR3 sequences in the amino acid sequence selected from SEQ ID NOs: 5, 9, 13, 29, and 30;
   still preferably, the CDR1, CDR2, and CDR3 sequences:
      (i) comprise or consist of the amino acid sequences of SEQ ID NOs: 6, 7, and 8, respectively;
      (ii) comprise or consist of the amino acid sequences of SEQ ID NOs: 10, 11, and 12, respectively;
      (iii) comprise or consist of the amino acid sequences of SEQ ID NOs: 14, 15, and 16, respectively; or
      (iv) comprise or consist of the amino acid sequences of SEQ ID NOs: 31, 32, and 33, respectively;
   still preferably, the VHH domain comprises the amino acid sequence selected from SEQ ID NOs: 5, 9, 13, 29, and 30, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 5, 9, 13, 29, and 30.
B17. An anti-Necti-4 antibody, comprising a VHH domain specifically binding to Nectin-4,
   wherein the VHH domain comprises CDR1, CDR2, and CDR3 sequences in the amino acid sequence selected from SEQ ID NOs: 17, 21, 25, 34, 35, and 38;
   preferably, the CDR1, CDR2, and CDR3 sequences:
      (i) comprise or consist of the amino acid sequences of SEQ ID NOs: 18, 19, and 20, respectively;
      (ii) comprise or consist of the amino acid sequences of SEQ ID NOs: 22, 23, and 24, respectively;
      (iii) comprise or consist of the amino acid sequences of SEQ ID NOs: 26, 27, and 28, respectively;
      (iv) comprise or consist of the amino acid sequences of SEQ ID NOs: 18, 36, and 20, respectively; or
      (v) comprise or consist of the amino acid sequences of SEQ ID NOs: 18, 37, and 20, respectively;
   still preferably, the VHH domain comprises the amino acid sequence selected from SEQ ID NOs: 17, 21, 25, 34, 35, and 38, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence,
   most preferably, the VHH domain comprises or consists of the amino acid sequence selected from SEQ ID NOs: 17, 21, 25, 34, 35, and 38.
B18. A polynucleotide encoding the antibody according to any one of embodiments B1-B17.
B19. A vector, preferably an expression vector, comprising the polynucleotide according to embodiment B18.
B20. A host cell, comprising the polynucleotide according to embodiment B18 or the vector according to embodiment B19, wherein, for example, the host cell is a mammalian cell.
B21. A method for producing the antibody according to any one of embodiments B1-B17, comprising: culturing a host cell comprising a polynucleotide encoding a polypeptide chain under conditions suitable for the expression of the polypeptide chain of the antibody; and assembling the polypeptide chain to produce the antibody under conditions suitable for the assembly of the polypeptide chain into the antibody.
B22. An immunoconjugate or immunofusion comprising the antibody according to any one of embodiments B1-B17.
B23. The immunoconjugate according to embodiment B22, wherein the immunoconjugate is an antibody-drug conjugate represented by formula (I):

   Ab-(L-D)ₚ (I)

   or a pharmaceutically acceptable salt or solvate thereof, wherein:
   Ab is the antibody according to any one of embodiments B1-B17;
   L is a linker;
   D is a drug, preferably an anti-tumor compound; and
   p is an integer selected from 1 to 16, e.g., 1, 2, 3, 4, 5, 6, 7, 8, 9, or 10.
B24. The antibody-drug conjugate or the pharmaceutically acceptable salt or solvate thereof according to embodiment B23, wherein the anti-tumor compound is a cytotoxic agent, such as a camptothecin compound and an auristatin compound, such as exatecan, Dxd, or MMAE.
B25. The antibody-drug conjugate or the pharmaceutically acceptable salt or solvate thereof according to embodiment B23, wherein D has a structure represented by formula (D-1a) or formula (D-1b): 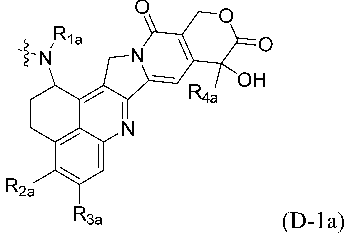
   wherein R₁ₐ is selected from H and C₁-C₆ alkyl;
   R₂ₐ is selected from H, halogen, C₁-C₆ alkyl, C₁-C₆ haloalkyl, -OR₅ₐ, and -SR₅ₐ;
   R₃ₐ is selected from H, halogen, CN, C₁-C₆ alkyl, C₁-C₆ haloalkyl, and -OR₅ₐ; and
   R₄ₐ and R₅ₐ are independently selected from H and C₁-C₄ alkyl;
      or 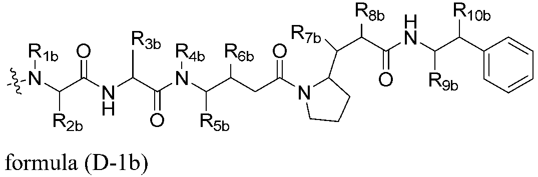
   wherein R_{1b}, R_{2b}, R_{3b}, R_{4b}, R_{5b}, and R_{8b} are each independently selected from C₁₋₈ alkyl, such as methyl, ethyl, *n*-propyl, isopropyl, *n*-butyl, isobutyl, or *sec*-butyl;
   R_{6b} and R_{7b} are each independently selected from C₁₋₈ alkoxy, such as methoxy, ethoxy, or propoxy;
   R_{9b} is selected from C₁₋₈ alkyl and COOH, such as methyl, ethyl, *n*-propyl, isopropyl, *n*-butyl, isobutyl, or *sec*-butyl; and
   R_{10b} is selected from OH and H.
B26. The antibody-drug conjugate or the pharmaceutically acceptable salt or solvate thereof according to embodiment B25, wherein
   R₁ₐ is H; R₂ₐ is C₁-C₆ alkyl; R₃ₐ is halogen, preferably -F; R₄ₐ is C₁-C₄ alkyl, preferably ethyl;
   R_{1b}, R_{4b}, and R_{8b} are each independently selected from C₁₋₂ alkyl, preferably methyl;
   R_{2b}, R_{3b}, and R_{5b} are each independently selected from C₃₋₄ alkyl;
   R_{6b} and R_{7b} are each independently selected from C₁₋₂ alkoxy; and
   R_{9b} is selected from C₁₋₄ alkyl, and R_{10b} is OH; or R_{9b} is COOH, and R_{10b} is H.
B27. The antibody-drug conjugate or the pharmaceutically acceptable salt or solvate thereof according to embodiment B25, wherein D has a structure represented by formula (D-2a) or formula (D-2b): 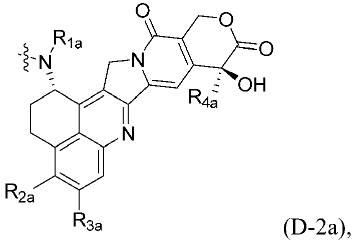 wherein R₁ₐ, R₂ₐ, R₃ₐ, and R₄ₐ are as defined in embodiment B25 or B26; 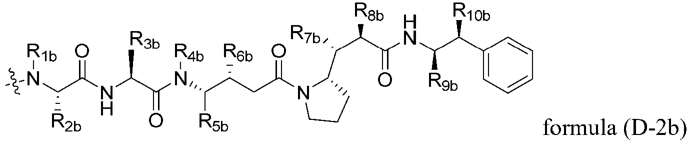 wherein R_{1b}, R_{2b}, R_{3b}, R_{4b}, R_{5b}, R_{6b}, R_{7b}, R_{8b}, R_{9b}, and R_{10b} are as defined in embodiment B25 or B26.
B28. The antibody-drug conjugate or the pharmaceutically acceptable salt or solvate thereof according to embodiment B25, wherein D has a structure represented by formula (D-3a) or formula (D-3b): 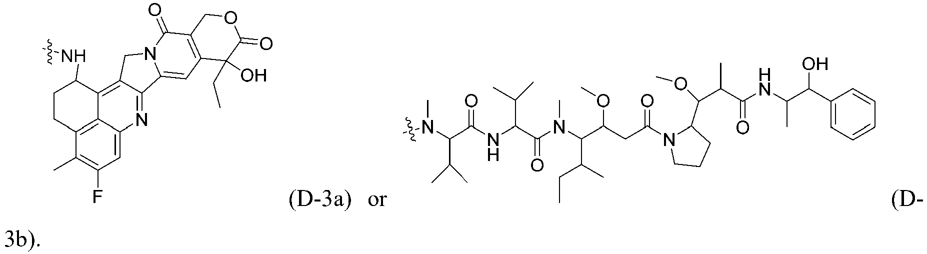
B29. The antibody-drug conjugate or the pharmaceutically acceptable salt or solvate thereof according to embodiment B25, wherein D has a structure represented by formula (D-4a) or formula (D-4b): 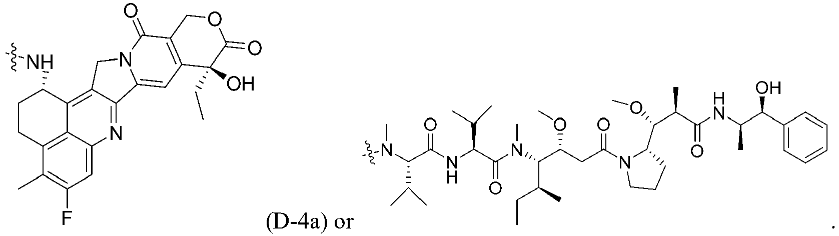
B30. The antibody-drug conjugate or the pharmaceutically acceptable salt or solvate thereof according to any one of embodiments B23-B29, wherein -L- has the following structure: -Z-L₁-L₂-L₃-,
   wherein
   Z is selected from 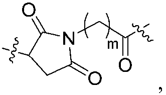 wherein m is an integer selected from 1 to 10, e.g., 1, 2, 3, 4, 5, 6, 7, or 8;
   L₁ is selected from absence, 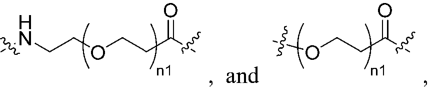 wherein n1 is independently an integer selected from 0 to 12, e.g., 1, 2, 3, 4, 5, 6, 7, or 8;
   L₂ is a peptide residue consisting of 2-8 amino acids; and
   L₃ is selected from: 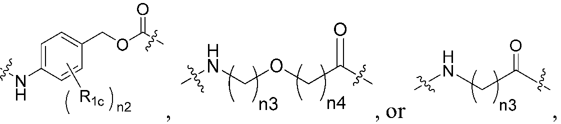 wherein R_{1c} is selected from: H and C₁-C₆ alkyl; n2 is 1, 2, 3, or 4; and n3 and n4 are independently 1, 2, 3, 4, 5, or 6.
B31. The antibody-drug conjugate or the pharmaceutically acceptable salt or solvate thereof according to embodiment B30,
   wherein L₂ is a peptide residue consisting of 2, 3, 4, 5, 6, or 7 amino acids.
B32. The antibody-drug conjugate or the pharmaceutically acceptable salt or solvate thereof according to embodiment B31,
   wherein Z is selected from 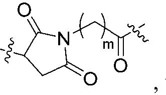 wherein m is 1, 2, 3, 4, 5, 6, 7, or 8;
   L₁ is selected from absence, 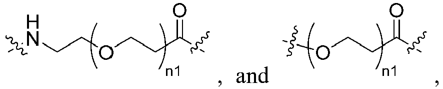 wherein n1 is independently an integer selected from 0-12; and
   L₃ is selected from: 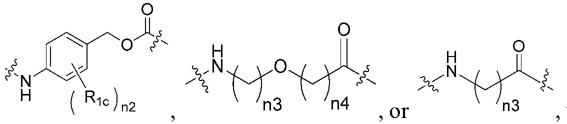 wherein R_{1c} is selected from H and C₁-C₆ alkyl; n2 is 1, 2, 3, or 4; and n3 and n4 are independently 1, 2, 3, 4, 5, or 6.
B33. The antibody-drug conjugate or the pharmaceutically acceptable salt or solvate thereof according to embodiment B31,
   Z is selected from 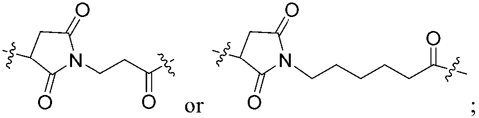
   L₁ is selected from absence or 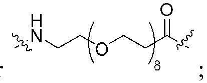
   L₂ is selected from -Val-Ala-, -Gly-Gly-Phe-Gly-, -Val-Cit-, and -Glu-Val-Cit-; and
   L₃ is selected from 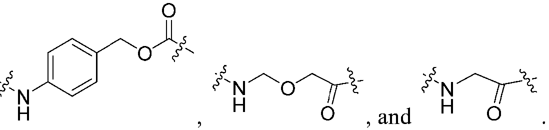
B34. The antibody-drug conjugate or the pharmaceutically acceptable salt or solvate thereof according to embodiment B31,
   wherein -Z-L₁-L₂-L₃- is selected from the following structure 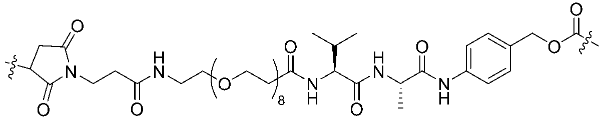 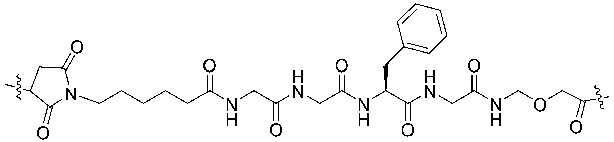 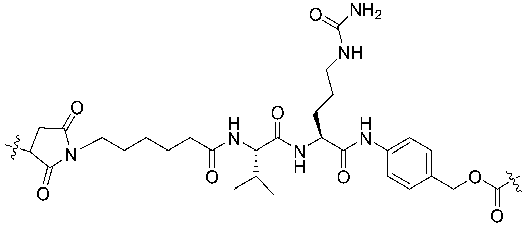 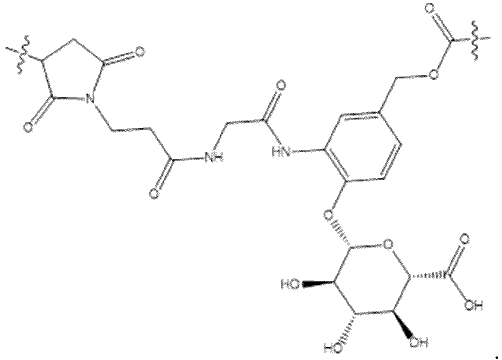
B35. The antibody-drug conjugate or the pharmaceutically acceptable salt or solvate thereof according to any one of embodiments B23-B34, having an average DAR value of 2-10, e.g., 4-8.
B36. The antibody-drug conjugate or the pharmaceutically acceptable salt or solvate thereof according to embodiment B23, wherein the antibody-drug conjugate is selected from: 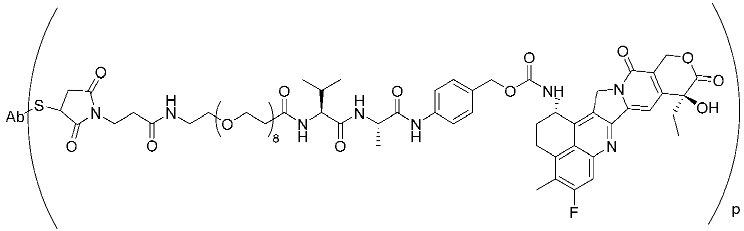 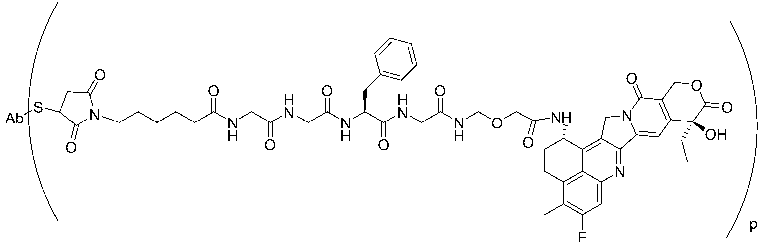 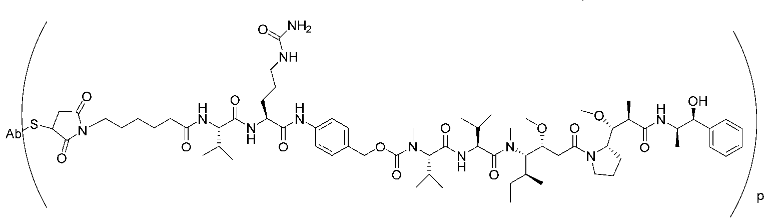 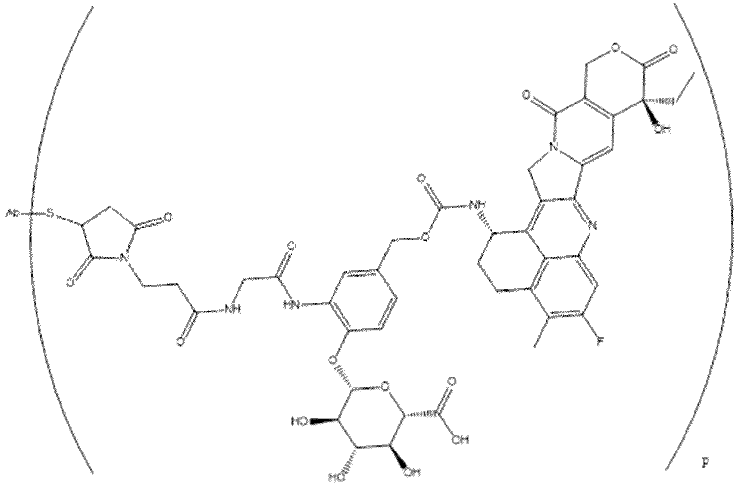
   wherein Ab and p are as defined in embodiment B23;
   preferably, Ab is a double-chain antibody, wherein
      (a) the antibody is in a symmetric double-chain form, and the polypeptide chain of the antibody comprises the amino acid sequence of SEQ ID NO: 53; or
      (b) the antibody is in an asymmetric double-chain form, and the antibody comprises first and second polypeptide chains comprising the amino acid sequences of SEQ ID NOs: 61/62, 69/70, or 71/72, respectively;
   preferably, the antibody-drug conjugate has an average DAR of 2-10 or 4-8.
B37. A pharmaceutical composition, comprising the antibody according to any one of embodiments B1-B17 or the immunoconjugate or immunofusion according to any one of embodiments B22-B36, and a pharmaceutically acceptable carrier.
B38. Use of the antibody according to any one of embodiments B1-B17 or the immunoconjugate or immunofusion according to any one of embodiments B22-B36 as a medicament or in the manufacture of a medicament.
B39. The use according to embodiment B38, wherein the medicament is used for treating and/or preventing cancer in an individual, wherein the cancer is a Trop2/Nectin-4 double-positive solid tumor; preferably, the tumor has at least 40% of cells exhibiting Trop2/Nectin-4 double-positive high-level expression.

In one aspect, the present invention further provides the following group C embodiments:
C1. A multispecific antibody binding to Trop2 and Nectin4, wherein the antibody comprises at least one antigen-binding domain specifically binding to Trop2 and at least one antigen-binding domain specifically binding to Nectin4.
C2. The multispecific antibody according to embodiment C1, wherein the antibody has one or more characteristics selected from the following:
   (a) the Trop2 and Nectin4 antigen-binding domains are VHH domains;
   (b) the Trop2 and Nectin4 antigen-binding domains each have an antigen-binding affinity K_{D} value of 50 × 10⁻⁸ M to 0.5 × 10⁻⁸ M;
   (c) the Trop2 antigen-binding domain and the Nectin4 antigen-binding domain in the antibody have a valence ratio of 1:1 or 1:2; and
   (d) the antibody is a trivalent or tetravalent bispecific antibody; and
      preferably, the antibody further has one or more characteristics selected from the following: compared with Nectin4 and Trop2 single-target drugs, the antibody exhibits, on a target tumor cell expressing human Nectin4 and Trop2, (e) a synergistic binding effect; (f) a synergistic endocytosis effect; and (g) improved targeting distribution to the tumor cell.
C3. The multispecific antibody according to any one of embodiments C1-C2, wherein the antigen-binding domain specifically binding to Trop2 is a VHH domain, and
   wherein the VHH domain comprises CDR1, CDR2, and CDR3 sequences in the amino acid sequence selected from SEQ ID NOs: 5, 9, 13, 29, 30, 98, and 99;
   still preferably, the CDR1, CDR2, and CDR3 sequences:
      (i) comprise or consist of the amino acid sequences of SEQ ID NOs: 6, 7, and 8, respectively;
      (ii) comprise or consist of the amino acid sequences of SEQ ID NOs: 10, 11, and 12, respectively;
      (iii) comprise or consist of the amino acid sequences of SEQ ID NOs: 14, 15, and 16, respectively; or
      (iv) comprise or consist of the amino acid sequences of SEQ ID NOs: 31, 32, and 33, respectively;
   still preferably, the VHH domain comprises the amino acid sequence selected from SEQ ID NOs: 5, 9, 13, 29, 30, 98, and 99, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence;
   most preferably, the VHH domain comprises or consists of the amino acid sequence selected from SEQ ID NOs: 5, 9, 13, 29, 30, 98, and 99.
C4. The multispecific antibody according to any one of embodiments C1-C3, wherein the antigen-binding domain specifically binding to Nectin4 is a VHH domain, and
   wherein the VHH domain comprises CDR1, CDR2, and CDR3 sequences in the amino acid sequence selected from SEQ ID NOs: 17, 21, 25, 34, 35, 38, and 100-106;
   still preferably, the CDR1, CDR2, and CDR3 sequences:
      (i) comprise or consist of the amino acid sequences of SEQ ID NOs: 18, 19, and 20, respectively;
      (ii) comprise or consist of the amino acid sequences of SEQ ID NOs: 22, 23, and 24, respectively;
      (iii) comprise or consist of the amino acid sequences of SEQ ID NOs: 26, 27, and 28, respectively;
      (iv) comprise or consist of the amino acid sequences of SEQ ID NOs: 18, 36, and 20, respectively; or
      (v) comprise or consist of the amino acid sequences of SEQ ID NOs: 18, 37, and 20, respectively;
   still preferably, the VHH domain comprises the amino acid sequence selected from SEQ ID NOs: 17, 21, 25, 34, 35, 38, and 100-106, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence;
   most preferably, the VHH domain comprises or consists of the amino acid sequence selected from SEQ ID NOs: 17, 21, 25, 34, 35, 38, and 100-106.
C5. The multispecific antibody according to any one of embodiments C1-C4, wherein the antibody further comprises:
   (a) a half-life extension domain, wherein preferably, the half-life extension domain is selected from a serum albumin binding peptide and an immunoglobulin Fc region; and/or
   (b) a peptide linker; preferably, the peptide linker is 5-15 amino acids in length, and more preferably, the peptide linker comprises the amino acid sequence (G₄S)ₙ, wherein n = 1, 2, or 3.
C6. The multispecific antibody according to any one of embodiments C1-C5, wherein the antibody is a single-chain or double-chain antibody comprising at least one (preferably 1 or 2) Nectin4-binding domain and at least one (preferably 1 or 2) Trop2-binding domain.
C7. The multispecific antibody according to any one of embodiments C1-C6, wherein the antibody comprises two identical polypeptide chains, wherein the polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
   wherein n1, n2, n3, and n4 are each independently selected from integers of 0, 1, and 2; preferably, n3 and n4 are 0, and n1 and n2 are 1;
   wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain; the symbol "-" represents linkage via a peptide linker or direct linkage.
C8. The multispecific antibody according to embodiment C7, wherein the polypeptide chain comprises, from the N-terminus to the C-terminus, VHH^{A}-VHH^{B}-HLE, and wherein VHH^{A} represents a Nectin4-binding domain and VHH^{B} represents a Trop2-binding domain.
C9. The multispecific antibody according to any one of embodiments C1-C6, wherein the antibody comprises two different polypeptide chains, wherein
   the first polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
   the second polypeptide chain comprises, from the N-terminus to the C-terminus, (VHH^{B})ₘ₁-(VHH^{A})ₘ₂-HLE-(VHH^{B})ₘ₃-(VHH^{A})ₘ₄,
   wherein n1, n2, n3, and n4, as well as m1, m2, m3, and m4 are each independently selected from integers of 0, 1, and 2, and preferably, wherein n3, n4, m3, and m4 are 0, n1 + m2 = 1 or 2, n2 + m1 = 1 or 2; still preferably, m1 = 0 and m2 = 1 or m2 = 0 and m1 = 1;
   wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain, and preferably, comprises a knob-into-hole mutation; the symbol "-" represents linkage via a peptide linker or direct linkage.
C10. The multispecific antibody according to embodiment C9, wherein
   the first polypeptide chain comprises, from the N-terminus to the C-terminus, VHH^{A}-VHH^{B}-HLE,
   the second polypeptide chain comprises, from the N-terminus to the C-terminus, VHH^{A}-HLE,
   wherein VHH^{A} represents a Nectin4-binding domain and VHH^{B} represents a Trop2-binding domain.
C11. The multispecific antibody according to any one of embodiments C7-C10, wherein the immunoglobulin Fc region is an Fc region from IgG, such as an Fc region from human IgG1 or IgG4; preferably, the Fc region comprises a mutation that reduces or eliminates the binding of the Fc region to FcγR.
C12. The multispecific antibody according to any one of embodiments C1-C6, wherein the antibody comprises a single polypeptide chain, wherein the polypeptide chain comprises, from the N-terminus to the C-terminus, (VHH^{A})ₙ₁-(VHH^{B})ₙ₂-(HLE)ₙ₃-(VHH^{A})ₙ₄-(VHH^{B})ₙ₅-(HLE)ₙ₆,
   wherein n1, n2, n3, n4, n5, and n6 are each independently selected from integers of 0, 1, and 2, and preferably, n1 + n4 = 1 or 2, n2 + n5 = 1 or 2, and n3 + n6 = 0 or 1,
   wherein VHH^{A} and VHH^{B} represent VHH domains binding to an antigen A and an antigen B, respectively, wherein A and B are different from each other and are each independently selected from Nectin4 and Trop2; HLE represents a serum albumin binding peptide as a half-life extension domain; the symbol "-" represents linkage via a peptide linker or direct linkage,
   preferably, the polypeptide chain comprises, from the N-terminus to the C-terminus:
      (i) VHH^{A}-VHH^{B},
      (ii) VHH^{A}-VHH^{B}-HLE,
      (iii) VHH^{B}-VHH^{A}-VHH^{B},
      (iv) VHH^{A}-VHH^{B}-VHH^{B},
      (v) VHH^{A}-HLE-VHH^{B}-VHH^{B}, or
      (vi) VHH^{A}-VHH^{A}-HLE-VHH^{B}-VHH^{B}, wherein preferably, A represents Trop2 and B represents Nectin4;
   still preferably, the HLE represents an anti-serum albumin VHH domain, and is optionally selected from the amino acid sequences of SEQ ID NOs: 39 and 40, or amino acid sequences having at least 85%, 90%, 95%, or 99% identity thereto or having one or more (preferably 1-10, and more preferably 1-5) amino acid additions, deletions, and/or substitutions.
C13. The multispecific antibody according to any one of embodiments C1-C12, wherein
   (a) the antigen-binding domain specifically binding to Nectin4 comprises the amino acid sequence of SEQ ID NO: 25, and the antigen-binding domain specifically binding to Trop2 comprises the amino acid sequence of SEQ ID NO: 13;
   (b) the antigen-binding domain specifically binding to Nectin4 comprises the amino acid sequence of SEQ ID NO: 21, and the antigen-binding domain specifically binding to Trop2 comprises the amino acid sequence of SEQ ID NO: 9;
   (c) the antigen-binding domain specifically binding to Nectin4 comprises the amino acid sequence of SEQ ID NO: 25, and the antigen-binding domain specifically binding to Trop2 comprises the amino acid sequence of SEQ ID NO: 9;
   (d) the antigen-binding domain specifically binding to Nectin4 comprises the amino acid sequence of SEQ ID NO: 35, and the antigen-binding domain specifically binding to Trop2 comprises the amino acid sequence selected from SEQ ID NOs: 29 and 30; or
   (e) the antigen-binding domain specifically binding to Nectin4 comprises the amino acid sequence selected from SEQ ID NOs: 34 and 38, and the antigen-binding domain specifically binding to Trop2 comprises the amino acid sequence selected from SEQ ID NOs: 29 and 30.
C14. The multispecific antibody according to any one of embodiments C1-C6, wherein the antibody is in a double-chain form, wherein
   (a) the antibody is in a symmetric double-chain form, and the polypeptide chain of the antibody comprises the amino acid sequence of SEQ ID NO: 53 or 54 or an amino acid sequence having at least 95%, 96%, 97%, 98%, or 99% identity thereto, and preferably comprises the amino acid sequence of SEQ ID NO: 53; or
   (b) the antibody is in an asymmetric double-chain form, and the antibody comprises a first polypeptide chain and a second polypeptide chain selected from the group consisting of:
      (i) first and second polypeptide chains comprising the amino acid sequences set forth in SEQ ID NOs: 55 and 56 or amino acid sequences having at least 95%, 96%, 97%, 98%, or 99% identity thereto, respectively,
      (ii) first and second polypeptide chains comprising the amino acid sequences set forth in SEQ ID NOs: 57 and 58 or amino acid sequences having at least 95%, 96%, 97%, 98%, or 99% identity thereto, respectively,
      (iii) first and second polypeptide chains comprising the amino acid sequences set forth in SEQ ID NOs: 59 and 60 or amino acid sequences having at least 95%, 96%, 97%, 98%, or 99% identity thereto, respectively,
      (iv) first and second polypeptide chains comprising the amino acid sequences set forth in SEQ ID NOs: 61 and 62 or amino acid sequences having at least 95%, 96%, 97%, 98%, or 99% identity thereto, respectively,
      (v) first and second polypeptide chains comprising the amino acid sequences set forth in SEQ ID NOs: 63 and 64 or amino acid sequences having at least 95%, 96%, 97%, 98%, or 99% identity thereto, respectively,
      (vi) first and second polypeptide chains comprising the amino acid sequences set forth in SEQ ID NOs: 65 and 66 or amino acid sequences having at least 95%, 96%, 97%, 98%, or 99% identity thereto, respectively,
      (vii) first and second polypeptide chains comprising the amino acid sequences set forth in SEQ ID NOs: 67 and 68 or amino acid sequences having at least 95%, 96%, 97%, 98%, or 99% identity thereto, respectively,
      (viii) first and second polypeptide chains comprising the amino acid sequences set forth in SEQ ID NOs: 69 and 70 or amino acid sequences having at least 95%, 96%, 97%, 98%, or 99% identity thereto, respectively,
      (ix) first and second polypeptide chains comprising the amino acid sequences set forth in SEQ ID NOs: 71 and 72 or amino acid sequences having at least 95%, 96%, 97%, 98%, or 99% identity thereto, respectively,
      (x) first and second polypeptide chains comprising the amino acid sequences set forth in SEQ ID NOs: 73 and 74 or amino acid sequences having at least 95%, 96%, 97%, 98%, or 99% identity thereto, respectively, and
      (xi) first and second polypeptide chains comprising the amino acid sequences set forth in SEQ ID NOs: 75 and 76 or amino acid sequences having at least 95%, 96%, 97%, 98%, or 99% identity thereto, respectively;
      preferably, the first and second polypeptide chains comprise the amino acid sequences of SEQ ID NOs: 61/62, 69/70, or 71/72, respectively.
C15. The multispecific antibody according to any one of embodiments C1-C6, wherein the antibody is in a single-chain form, wherein the polypeptide chain of the antibody comprises the amino acid sequence selected from SEQ ID NOs: 41-52 or an amino acid sequence having at least 95%, 96%, 97%, 98%, or 99% identity thereto.
C16. A VHH domain specifically binding to Trop2,
   wherein the VHH domain comprises CDR1, CDR2, and CDR3 sequences in the amino acid sequence selected from SEQ ID NOs: 5, 9, 13, 29, 30, 98, and 99;
   still preferably, the CDR1, CDR2, and CDR3 sequences:
      (i) comprise or consist of the amino acid sequences of SEQ ID NOs: 6, 7, and 8, respectively;
      (ii) comprise or consist of the amino acid sequences of SEQ ID NOs: 10, 11, and 12, respectively;
      (iii) comprise or consist of the amino acid sequences of SEQ ID NOs: 14, 15, and 16, respectively; or
      (iv) comprise or consist of the amino acid sequences of SEQ ID NOs: 31, 32, and 33, respectively;
   still preferably, the VHH domain comprises the amino acid sequence selected from SEQ ID NOs: 5, 9, 13, 29, 30, 98, and 99, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence;
   most preferably, the VHH domain comprises or consists of the amino acid sequence selected from SEQ ID NOs: 5, 9, 13, 29, 30, 98, and 99.
C17. A VHH domain specifically binding to Nectin4,
   wherein the VHH domain comprises CDR1, CDR2, and CDR3 sequences in the amino acid sequence selected from SEQ ID NOs: 17, 21, 25, 34, 35, 38, and 100-106;
   preferably, the CDR1, CDR2, and CDR3 sequences:
      (i) comprise or consist of the amino acid sequences of SEQ ID NOs: 18, 19, and 20, respectively;
      (ii) comprise or consist of the amino acid sequences of SEQ ID NOs: 22, 23, and 24, respectively;
      (iii) comprise or consist of the amino acid sequences of SEQ ID NOs: 26, 27, and 28, respectively;
      (iv) comprise or consist of the amino acid sequences of SEQ ID NOs: 18, 36, and 20, respectively; or
      (v) comprise or consist of the amino acid sequences of SEQ ID NOs: 18, 37, and 20, respectively;
   still preferably, the VHH domain comprises the amino acid sequence selected from SEQ ID NOs: 17, 21, 25, 34, 35, 38, and 100-106, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence;
   most preferably, the VHH domain comprises or consists of the amino acid sequence selected from SEQ ID NOs: 17, 21, 25, 34, 35, 38, and 100-106.
C18. An antibody, comprising the VHH domain specifically binding to Trop2 according to embodiment C16 and/or the VHH domain specifically binding to Nectin4 according to embodiment C17.
C19. An antigen-binding molecule, comprising the antibody according to embodiment C18.
C20. A polynucleotide encoding the antibody according to any one of embodiments C1-C15, the VHH domain according to embodiment C16 or C17, the antibody according to embodiment C18, or the antigen-binding molecule according to embodiment C19.
C21. A vector, preferably an expression vector, comprising the polynucleotide according to embodiment C20.
C22. A host cell, comprising the polynucleotide according to embodiment C20 or the vector according to embodiment C21, wherein, for example, the host cell is a mammalian cell.
C23. A method for producing the antibody according to any one of embodiments C1-C15 and C18, comprising:
   culturing a host cell comprising a polynucleotide encoding a polypeptide chain under conditions suitable for the expression of the polypeptide chain of the antibody; and assembling the polypeptide chain to produce the antibody under conditions suitable for the assembly of the polypeptide chain into the antibody.
C24. An immunoconjugate or immunofusion comprising the antibody according to any one of embodiments C1-C15 and C18.
25. An antibody-drug conjugate represented by formula (I):

   Ab-(L-D)ₚ (I)

   or a pharmaceutically acceptable salt or solvate thereof,
   wherein:
   Ab is the antibody according to any one of embodiments C1-C15 and C18 or the antigen-binding molecule according to embodiment C19;
   L is a linker;
   D is a drug, preferably an anti-tumor compound; and
   p is an integer selected from 1 to 16, e.g., 1, 2, 3, 4, 5, 6, 7, 8, 9, or 10.
C26. The antibody-drug conjugate or the pharmaceutically acceptable salt or solvate thereof according to embodiment C25, wherein the anti-tumor compound is a cytotoxic agent, such as a camptothecin compound and an auristatin compound, such as exatecan, Dxd, or MMAE.
C27. The antibody-drug conjugate or the pharmaceutically acceptable salt or solvate thereof according to embodiment C25, wherein D has a structure represented by formula (D-1a) or formula (D-1b): 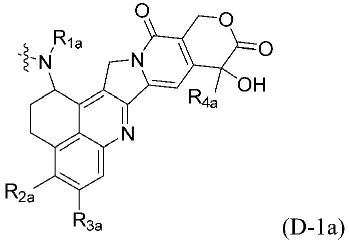
   wherein R₁ₐ is selected from H and C₁-C₆ alkyl;
   R₂ₐ is selected from H, halogen, C₁-C₆ alkyl, C₁-C₆ haloalkyl, -OR₅ₐ, and -SR₅ₐ;
   R₃ₐ is selected from H, halogen, CN, C₁-C₆ alkyl, C₁-C₆ haloalkyl, and -OR₅ₐ; and
   R₄ₐ and R₅ₐ are independently selected from H and C₁-C₄ alkyl;
      or 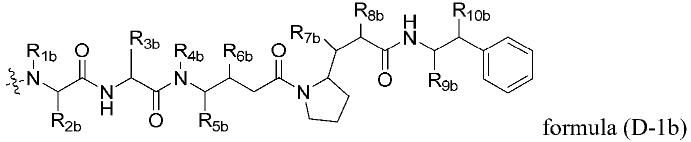
   wherein R_{1b}, R_{2b}, R_{3b}, R_{4b}, R_{5b}, and R_{8b} are each independently selected from C₁₋₈ alkyl, such as methyl, ethyl, *n*-propyl, isopropyl, *n*-butyl, isobutyl, or *sec*-butyl;
   R_{6b} and R_{7b} are each independently selected from C₁₋₈ alkoxy, such as methoxy, ethoxy, or propoxy;
   R_{9b} is selected from C₁₋₈ alkyl and COOH, such as methyl, ethyl, *n*-propyl, isopropyl, *n*-butyl, isobutyl, or *sec*-butyl; and
   R_{10b} is selected from OH and H.
C28. The antibody-drug conjugate or the pharmaceutically acceptable salt or solvate thereof according to embodiment C27, wherein
   R₁ₐ is H; R₂ₐ is C₁-C₆ alkyl; R₃ₐ is halogen, preferably -F; R₄ₐ is C₁-C₄ alkyl, preferably ethyl;
   R_{1b}, R_{4b}, and R_{8b} are each independently selected from C₁₋₂ alkyl, preferably methyl;
   R_{2b}, R_{3b}, and R_{5b} are each independently selected from C₃₋₄ alkyl;
   R_{6b} and R_{7b} are each independently selected from C₁₋₂ alkoxy; and
   R_{9b} is selected from C₁₋₄ alkyl, and R_{10b} is OH; or R_{9b} is COOH, and R_{10b} is H.
C29. The antibody-drug conjugate or the pharmaceutically acceptable salt or solvate thereof according to embodiment C27, wherein D has a structure represented by formula (D-2a) or formula (D-2b): 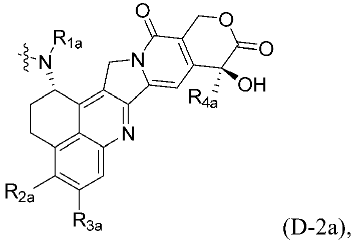 wherein R₁ₐ, R₂ₐ, R₃ₐ, and R₄ₐ are as defined in embodiment C27 or C28; 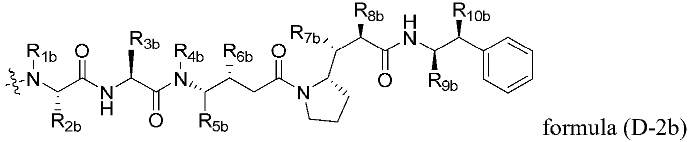 wherein R_{1b}, R_{2b}, R_{3b}, R_{4b}, R_{5b}, R_{6b}, R_{7b}, R_{8b}, R_{9b}, and R_{10b} are as defined in embodiment C27 or C28.
C30. The antibody-drug conjugate or the pharmaceutically acceptable salt or solvate thereof according to embodiment C27, wherein D has a structure represented by formula (D-3a) or formula (D-3b): 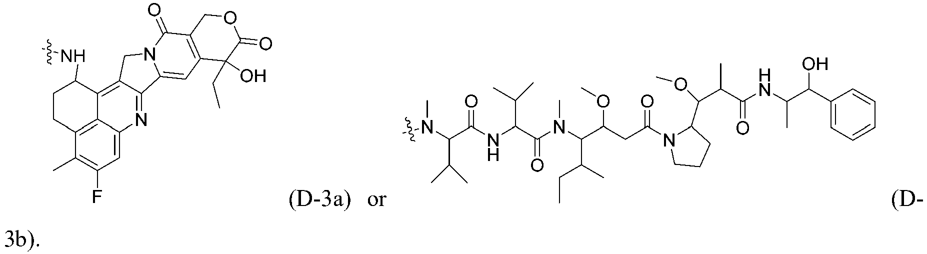
C31. The antibody-drug conjugate or the pharmaceutically acceptable salt or solvate thereof according to embodiment C27, wherein D has a structure represented by formula (D-4a) or formula (D-4b): 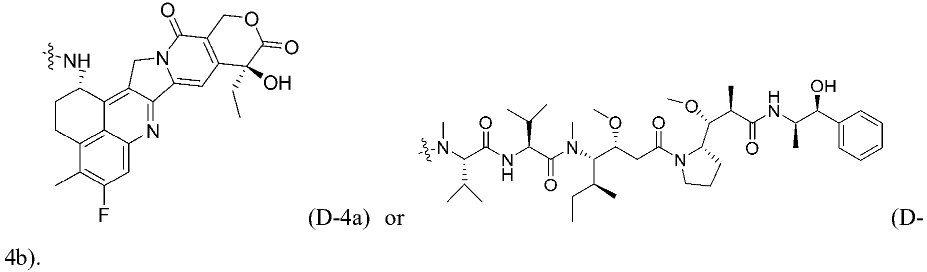
C32. The antibody-drug conjugate or the pharmaceutically acceptable salt or solvate thereof according to any one of embodiments C25-C31, wherein -L- has the following structure: -Z-L₁-L₂-L₃-, wherein
   Z is selected from 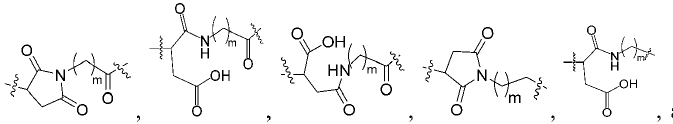 and 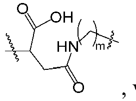 wherein m is independently an integer selected from 1-10, such as 1, 2, 3, 4, 5, 6, 7, or 8;
   L₁ is selected from absence, 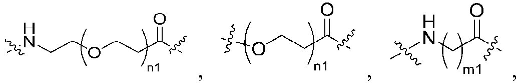 and 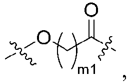 wherein n1 and m1 are each independently an integer selected from 0-20, for example, an integer selected from 0-12, such as 1, 2, 3, 4, 5, 6, 7, or 8;
   L₂ is an amino acid residue or a peptide residue consisting of 2-8 amino acids; and
   L₃ is selected from: 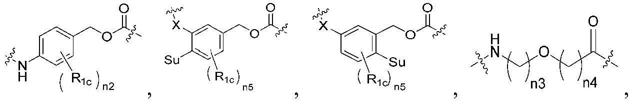 and 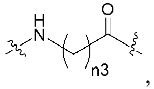 wherein X is selected from -NH-, -O-, and -S-; each R_{1c} is independently selected from C₁₋₈ alkyl, C₁₋₈ haloalkyl-, C₁₋₈ alkoxy, halogen, nitro, and cyano; each Su is independently selected from pentose, penturonic acid, hexose, and hexuronic acid; n2 is 0, 1, 2, 3, or 4; n5 is 0, 1, 2, or 3; n3 and n4 are independently 1, 2, 3, 4, 5, or 6.
C33. The antibody-drug conjugate or the pharmaceutically acceptable salt or solvate thereof according to embodiment C32,
   wherein Z is selected from 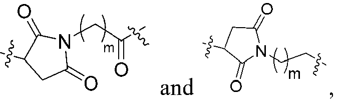 wherein m is 1, 2, 3, 4, 5, 6, 7, or 8;
   preferably, Z is selected from 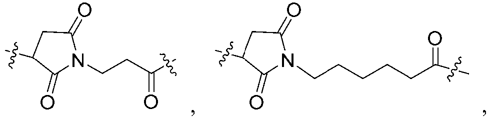 and 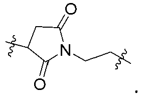
C34. The antibody-drug conjugate or the pharmaceutically acceptable salt or solvate thereof according to any one of embodiments C32-C33,
   wherein L₁ is selected from absence, 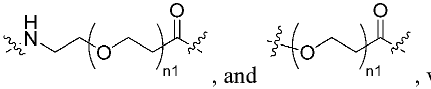 wherein n1 is independently an integer selected from 0-12, such as 1, 2, 3, 4, 5, 6, 7, or 8;
   preferably, L ₁ is absent or 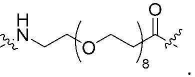
C35. The antibody-drug conjugate or the pharmaceutically acceptable salt or solvate thereof according to any one of embodiments C32-C34,
   wherein L₂ is an amino acid residue or a peptide residue consisting of 2, 3, **4,** 5, 6, or 7 amino acids; preferably, the amino acid residues or the amino acids constituting the peptide residue are each independently selected from glycine (Gly), valine (Val), alanine (Ala), lysine (Lys), citrulline (Cit), glutamine (Gln), glutamic acid (Glu), phenylalanine (Phe), leucine (Leu), tyrosine (Tyr), serine (Ser), aspartic acid (Asp), asparagine (Asn), isoleucine (Ile), arginine (Arg), proline (Pro), methionine (Met), tryptophan (Trp), cysteine (Cys), histidine (His), and threonine (Thr), wherein the amino acid residues or the amino acids are optionally substituted with one or more C₁₋₆ alkyl;
   more preferably, the amino acid residues or the amino acids constituting the peptide residue are each independently selected from glycine (Gly), valine (Val), alanine (Ala), phenylalanine (Phe), glutamic acid (Glu), and citrulline (Cit);
   still more preferably, L₂ is selected from -Ala-, -Val-, -Gly-, -Val-Ala-, -Gly-Gly-Phe-Gly-, - Val-Cit-, and -Glu-Val-Cit-.
C36. The antibody-drug conjugate or the pharmaceutically acceptable salt or solvate thereof according to any one of embodiments C32-C35,
   wherein L₃ is selected from: 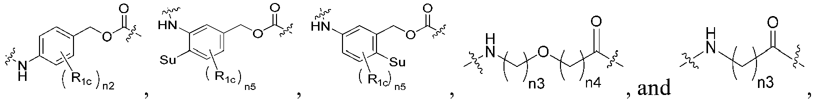
   wherein each R _{1c} is independently selected from C₁₋₈ alkyl, C₁₋₈ haloalkyl-, C₁₋₈ alkoxy, halogen, nitro, and cyano; each Su is independently selected from 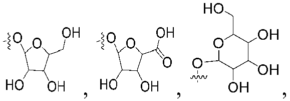 and 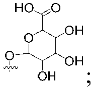 n2 is 0, 1, 2, 3, or 4; n5 is 0, 1, 2, or 3; and n3 and n4 are independently 1, 2, 3, 4, 5, or 6;
   preferably, L₃ is selected from: 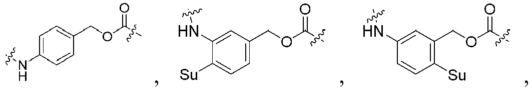 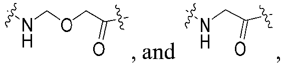
   wherein the variables are as defined above.
C37. The antibody-drug conjugate or the pharmaceutically acceptable salt or solvate thereof according to any one of embodiments C32-C36,
   wherein each Su is independently: 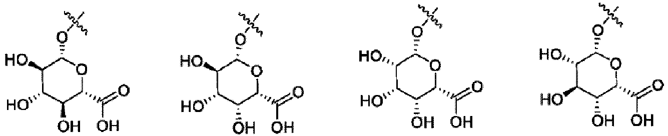 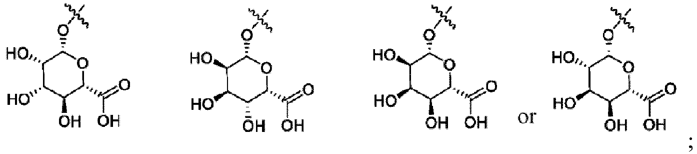
   more preferably, each Su is independently 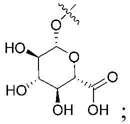
   still more preferably, each Su is independently 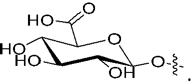
C38. The antibody-drug conjugate or the pharmaceutically acceptable salt or solvate thereof according to any one of embodiments C32-C36,
   wherein L₃ is selected from: 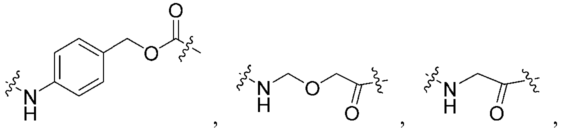 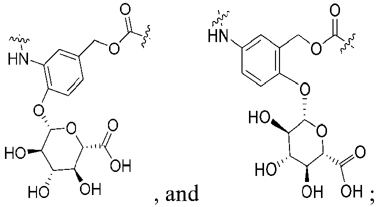
   preferably, L₃ is selected from: 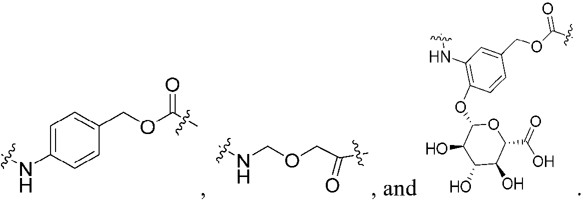
C39. The antibody-drug conjugate or the pharmaceutically acceptable salt or solvate thereof according to embodiment C35,
   Z is selected from 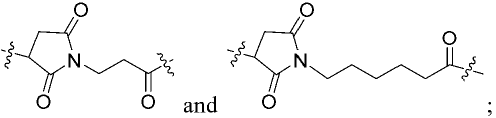
   L₁ is selected from absence and 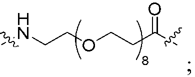
   L₂ is selected from -Gly-, -Val-Ala-, and -Gly-Gly-Phe-Gly; and
   L₃ is selected from 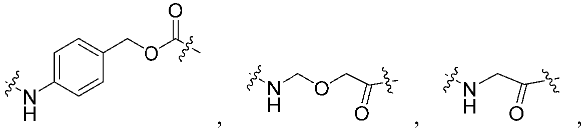 and 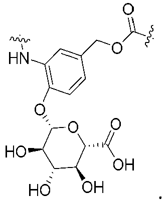
C40. The antibody-drug conjugate or the pharmaceutically acceptable salt or solvate thereof according to embodiment C35,
   wherein -Z-L₁-L₂-L₃- is selected from the following structure 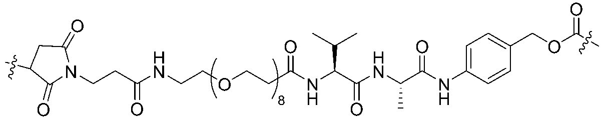 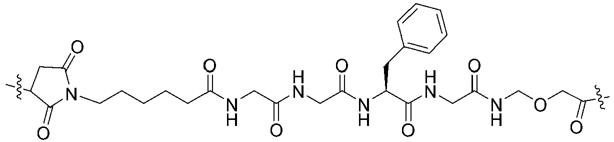 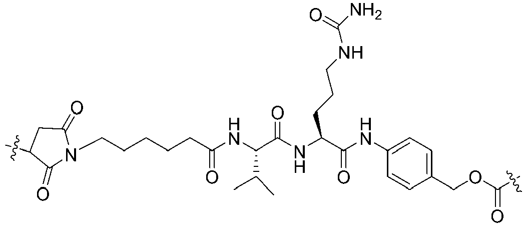 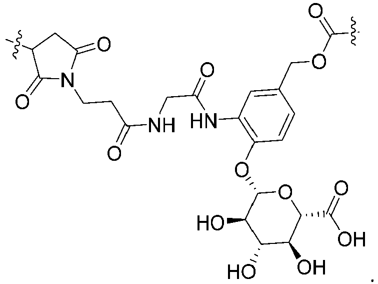
C41. The antibody-drug conjugate or the pharmaceutically acceptable salt or solvate thereof according to any one of embodiments C25-C40, having an average DAR value of 2-10, e.g., 4-8.
C42. The antibody-drug conjugate or the pharmaceutically acceptable salt or solvate thereof according to embodiment C25, wherein the antibody-drug conjugate is selected from: 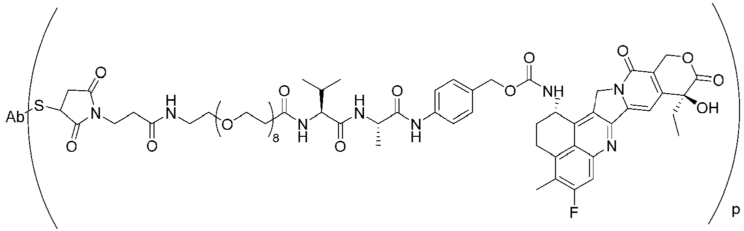 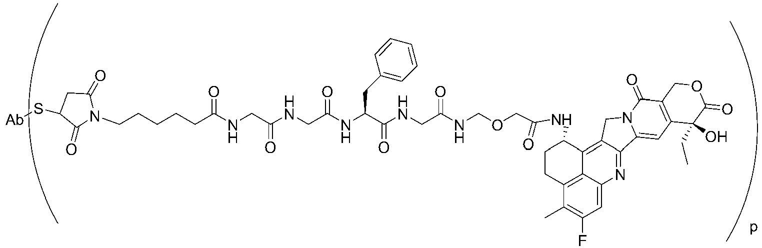 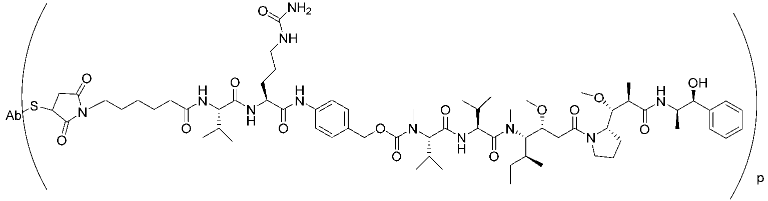 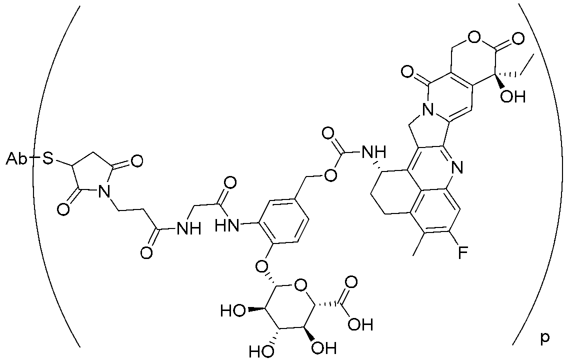
   wherein Ab and p are as defined in embodiment C25;
   preferably, Ab is a double-chain antibody, wherein
      (a) the antibody is in a symmetric double-chain form, and the polypeptide chain of the antibody comprises the amino acid sequence of SEQ ID NO: 53; or
      (b) the antibody is in an asymmetric double-chain form, and the antibody comprises first and second polypeptide chains comprising the amino acid sequences of SEQ ID NOs: 61/62, 69/70, or 71/72, respectively;
   preferably, the antibody-drug conjugate has an average DAR of 2-10 or 4-8.
C43. A pharmaceutical composition, comprising the antibody according to any one of embodiments C1-C15 or C18, the antigen-binding molecule according to embodiment C19, the immunoconjugate or immunofusion according to embodiment C24, or the antibody-drug conjugate or the pharmaceutically acceptable salt or solvate thereof according to any one of embodiments C25-C42, and a pharmaceutically acceptable carrier.
C44. Use of the antibody according to any one of embodiments C1-C15 or C18, the antigen-binding molecule according to embodiment C19, the immunoconjugate or immunofusion according to embodiment C24, or the antibody-drug conjugate or the pharmaceutically acceptable salt or solvate thereof according to any one of embodiments C25-C42 as a medicament or in the manufacture of a medicament.
C45. The use according to embodiment C44, wherein the medicament is used for treating and/or preventing cancer in an individual, wherein the cancer is a Trop2-positive, Nectin4-positive, or Trop2/Nectin4 double-positive solid tumor selected from, for example, urothelial cancer; bladder cancer (e.g., BLCA); cervical cancer (e.g., CESC); head and neck cancer (e.g., HNSC); lung cancer (e.g., LUSC and LUAD); breast cancer (e.g., BRCA); pancreatic cancer (e.g., PAAD); esophageal cancer (e.g., ESCA); prostate cancer (e.g., PRAD); bile duct cancer (e.g., CHOL); endometrioid cancer (e.g., UCEC); thyroid cancer (e.g., THCA); ovarian cancer (e.g., OV); colorectal cancer (e.g., COAD and READ); and gastric cancer (e.g., STAD), including primary, recurrent, and refractory cancers thereof.

Any or all of the features described above and throughout the present application may be combined in various embodiments of the present invention. The present invention will be further illustrated with reference to the following examples. However, it should be understood that the examples are for illustrative purposes, and should not be construed as constituting any limitation.

### Examples

### Materials and methods

### Reference antibodies and preparation thereof

In this example, the reference antibody Datopotamab (also known as V-BMK1) and the reference antibody Enfortumab (also known as V-BMK2) were used.

The "reference antibody Datopotamab" is an anti-Trop2 antibody constructed from the amino acid sequences of the heavy and light chain variable regions of the monospecific bivalent antibody Datopotamab (FDA UNII: BA3HQU7PO9) published by NCATS Inxight Drugs. In the context of reference antibody Datopotamab, unless otherwise specifically stated, the reference antibody will have a Fab antigen-binding domain as well as a human IgG1 heavy chain constant region and a kappa light chain.

The "reference antibody Enfortumab" is an anti-Nectin4 antibody constructed from the amino acid sequences of the heavy and light chain variable regions of the monospecific bivalent antibody Enfortumab (FDA UNII: U1HUE4W970) published by NCATS Inxight Drugs. In the context involving the reference antibody Enfortumab, unless otherwise specifically stated, the reference antibody will have a Fab antigen-binding domain, as well as a human IgG1 heavy chain constant region and a kappa light chain.

The heavy chain variable region and light chain variable region sequences of the two reference antibodies are set forth in SEQ ID NOs: 1-4, respectively.

The reference antibodies were prepared as follows. The coding sequence of the reference antibody was genetically synthesized from GENEWIZ (Shanghai, China), cloned into a pcDNA 3.4 expression vector, and transfected into Expi293F cells. The culture supernatant of the transfected cells was collected, and the antibodies were isolated and purified by a protein A column. The concentration of the purified antibody was measured by Nano Drop; the protein purity was determined by SDS-PAGE and analytical HPLC-SEC, and the antibodies were stored at -80 °C for later use.

### Antigens and preparation thereof

The extracellular segment sequence information (Gln 31-Tyr 274) of human Trop2 (UniProt_P09758-1) and cynomolgus monkey Trop2 (XP_005543292.2) and the extracellular segment sequence information (Gly 32-Ser 349) of human Nectin4 (UniProt_Q96NY8-1) and cynomolgus monkey Nectin4 (XP_005541277.1) were searched from a database, His tags were added at the C-termini, respectively, and gene synthesis was performed after human codon preference optimization. The sequences were separately subcloned into a pcDNA3.4 vector. After being verified as correct by Sanger sequencing, the plasmids were extracted for later use. Expi293F cells were transiently transfected using these protein eukaryotic expression vectors as constructed; protein expression supernatants were collected from the transfected cell cultures, and the target proteins were purified using a nickel column; SDS-PAGE experiments were performed to detect the purity of the proteins, and the purity was > 95%. The activities of the prepared human Trop2-His and human Nectin4-His recombinant antigen proteins were confirmed by ELISA using the above reference anti-Trop2 antibody (Datopotamab) and reference anti-Nectin 4 antibody (Enfortumab).

### Engineered cell lines recombinantly expressing antigens and preparation thereof

A CHO-K1 engineered cell line expressing human Trop2 and a CHO-K1 engineered cell line expressing human Nectin4 were prepared as follows.

The full-length genes of human Trop2 and human Nectin4 were separately inserted into the lentiviral expression vector pLVX-puro, and 293T cells were used to package lentiviruses, thereby infecting CHO-K1 cells. The cells were cultured in a medium containing 8 µg/mL puromycin for selection pressure to obtain a polyclonal cell strain. By limited dilution, a stably transfected CHO-K1-human Trop2 cell line and a CHO-K1-human Nectin4 cell line, which highly expressed Trop2 and Nectin4, were selected. The FACS assay was performed using the reference antibodies Datopotamab and Enfortumab, and the positive rate of the antigen expression in the stably transfected cell lines was > 90%.

### ELISA binding assay

The antigen was diluted to 0.5-1.0 µg/mL with PBS, plated at 100 µL/well, and incubated at 2-8 °C overnight or at 37 °C for 2 h. The plate was washed 3 times with 0.05% PBST by a plate washer, a blocking solution (1% BSA or 8% skimmed milk powder in PBS) was added at 250 µL/well, and the mixture was allowed to stand and be blocked for 1 h or more at room temperature. The samples to be tested were diluted to a proper concentration with the blocking solution. The plate was washed 3 times with 0.05% PBST by a plate washer, the diluted sample was added at 100 µL/well, and the mixture was allowed to stand for about 1 h at room temperature. The plate was washed 3 times with 0.05% PBST by a plate washer, secondary antibody-HRP diluted to the target dilution ratio was added at 100 µL/well, and the mixture was allowed to stand for about 0.5 h or 1 h at room temperature. The plate was washed 3 times with 0.05% PBST by a plate washer, TMB was added at 100 µL/well, the change in color was observed, and a stop solution was added at 100 µL/well as soon as the color reached the right level. OD450-OD650 values were read.

### FACS binding assay

Target cells were plated in a 96-well plate at a density of 1-5 × 10⁵ cells/well and centrifuged at 300 g for 5 min at 4 °C. The samples to be tested, diluted to an appropriate concentration, were added, and incubated for about 1 h at 2-8 °C. Centrifugation was performed at 4 °C, the supernatant was removed, and the residue was washed twice with 200 µL/well of FACS buffer (1% BSA or 2% FBS in PBS), and centrifuged at 4 °C. A flow secondary antibody diluted to the target dilution ratio was added at 100 µL/well, the cells were resuspended, and incubation was performed for about 0.5 h or 1 h at 2-8 °C in the dark. The cells were washed twice with 200 µL/well of FACS buffer, resuspended in 100 µL/well of FACS buffer, and tested on the instrument. The MFI of the cells was determined by a flow cytometer (Beckman Coulter).

### Endocytosis assay

Target cells were plated at 1-5 × 10⁵ cells/well, the sample to be tested, diluted to an appropriate concentration, was added, and the incubation was performed at 2-8 °C for about 0.5 h, so that the sample to be tested was bound with the cells. Centrifugation was performed at 800× g for 3 min at 4 °C, the supernatant was removed, and the cells were washed 2-3 times using a pre-cooled FACS buffer at 200 µL/well to remove the redundant unbound sample to be tested. The cells were resuspended using 100 µL/well of pre-cooled FACS buffer. The cells were evenly divided into 2 groups, which were incubated at 4 °C and 37 °C for 4 h, respectively. After the incubation was completed, the endocytosis assay was terminated by adding a FACS buffer under an ice bath. Centrifugation was performed at 800× g for 3 min at 4 °C, and the cells were washed 2-3 times using a pre-cooled FACS buffer at 200 µL/well. A flow secondary antibody diluted to the target dilution ratio was immediately added at 100 µL/well, the cells were resuspended, and incubation was performed for about 30 min to 1 h at 2-8 °C in the dark. The cells were washed 2-3 times with 200 µL/well of FACS buffer, and then the cells were resuspended by adding 100 µL/well of FACS buffer and tested on the instrument. The MFI of the cells was determined by a flow cytometer (Beckman Coulter). The internalization level of the antibody bound to the cell surface was calculated using the following formula:
endocytosis = MFI of sample incubated at 4 °C - MFI of sample incubated at 37 °C; 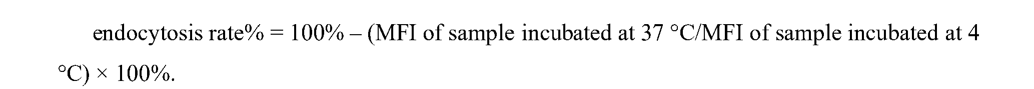 endocytosis rate% = 100% - (MFI of sample incubated at 37 °C/MFI of sample incubated at 4 °C) × 100%.

### SPR assay

The binding affinity of an antibody for an antigen was assayed by the surface plasmon resonance (abbreviated as SPR) technique.

Immobilized antibody for antigen assay: 10 µg/mL of an antibody was immobilized on a Protein A chip. The diluted antigen was injected at a flow rate of 30 µL/min, with a binding time of 120 s, followed by a dissociation time of 200 s. After each dissociation phase, 10 mM glycine (pH 2.0) was used for chip regeneration. The experimental data were analyzed using a 1:1 binding model.

Immobilized antigen for antibody assay: The concentration of an antigen was 10 µg/mL, and the antigen was immobilized on a CM5 chip. The diluted antibody was injected at a flow rate of 30 µL/min, with a binding time of 120 s, followed by a dissociation time of 200 s. After each dissociation, 10 mM glycine (pH 2.0) was used for regeneration. The experimental data were analyzed using a 1:1 binding model.

### SEC-HPLC

An appropriate amount of protein sample was loaded onto a TSK-gel G3000SWxL column (Tosoh Corporation) or a Zenix-C SEC-300 column (Sepax Technologies) at ambient column temperature. Isocratic elution was performed on the sample at a flow rate of 0.8 mL/min for 20 min using an Agilent 1260 HPLC system with a mobile phase (pH 6.8±0.1) consisting of 0.05 M sodium phosphate and 0.3 M sodium chloride. The eluted protein was assayed using UV absorbance at 280 nm.

### CEX-HPLC

An appropriate amount of protein sample was loaded onto a ProPac^{TM} WCX-10 BioLC column (Thermo SCIENTIFIC) at ambient column temperature. Gradient elution was performed on the sample at a flow rate of 0.9 mL/min for 70 min using an Agilent 1260 HPLC system with mobile phase A (pH 5.0±0.1) consisting of 2-methylpiperazine, imidazole, and Tris, and mobile phase B (pH 10.8±0.1) consisting of 100 mM sodium chloride, 2-methylpiperazine, imidazole, and Tris. The eluted protein was assayed using UV absorbance at 280 nm.

### HIC-HPLC

An appropriate amount of protein sample was loaded onto a MAbPac^{™} HIC-Butyl column (Thermo SCIENTIFIC) at ambient column temperature. Gradient elution was performed on the sample at a flow rate of 0.8 mL/min for 35 min using an Agilent 1260 HPLC system with mobile phase A and mobile phase B, wherein the mobile phase A (pH 6.0±0.1) consists of 1.5 M ammonium sulfate, 0.05 M sodium phosphate, and 5% isopropanol, and the mobile phase B (pH 6.0±0.1) consists of 0.05 M sodium phosphate and 5% isopropanol. The eluted protein was assayed using UV absorbance at 280 nm.

### Determination of thermostability by DSF

Based on the nano differential scanning fluorimetry (nanoDSF) technology, the temperature change range was 25-95 °C, the temperature change rate was 1 °C/min, and the denaturation temperature (Tm and Tonset) of an antibody protein and the aggregation onset temperature (Tagg) of the protein were accurately determined by using fluorescence spectroscopy at 280-450 nm and an intensity change in laser-scattered light at 266 nm or 473 nm, thereby evaluating the thermostability of the antibody protein.

### Example 1.1 VHH Screening

Candidate VHH sequences binding to Trop2 and Nectin4 were screened through preliminary property characterization using alpaca immunization and magnetic sorting technologies. Briefly, the recombinant antigen proteins human Trop2-His and human Nectin4-His prepared above were separately used to immunize alpacas at an interval of 14 days. From the second immunization, peripheral blood was collected seven days after each immunization, and the immune serum titer was monitored by ELISA binding assays. After the serum immune titer reached the standard of blood collection and library construction, the peripheral blood of the immunized alpacas was collected, and peripheral blood mononuclear cells (PBMCs) were isolated. Total RNA was extracted from PBMCs, and reverse transcription was performed using the PrimeScript^{™} II 1st Strand cDNA synthesis kit (Takara) with the RNA as a template to prepare cDNA. The first round of PCR amplification was performed with the cDNA as a template to produce nucleic acid fragments of conventional IgG (comprising VH) and heavy chain-only IgG (comprising VHH) lacking the CH1 domain. The two types of nucleic acids were separated on agarose gels, the coding nucleic acid comprising the VHH was extracted, purified, and then subjected to the second round of PCR amplification, and the resulting nucleic acids were separated and purified on the gel to recover the VHH fragments. The recovered VHH gene fragments and a linearized yeast display vector, pDisplay, were mixed and then co-transferred into yeast competent cells by electroporation to produce a yeast display library displaying VHH antibodies on the surface of yeast cells. Using streptavidin magnetic beads that had been previously incubated with a target antigen and thus bound to the antigen, the yeast cells binding to the target antigen were enriched from the constructed library by magnetic sorting.

The yeast liquid obtained by the magnetic bead sorting was applied on an SDCAA plate, and monoclonal cells were picked for culture. After the expression was induced for 48 h, the monoclonal cell cultures were incubated with Biotin-antigen followed by PE-Streptavidin. After the incubation was completed, flow cytometry (FACS) was performed to determine positive monoclonal yeast cells binding to the target antigen. Genomic DNA was extracted from the cultures of the obtained positive yeast cell clones for PCR amplification of antibody sequences and sequencing.

According to the sequencing results, candidate VHH sequences with large sequence differences were selected and separately linked to an expression vector pcDNA3.4 by fusing a human IgG1 sequence at the C-terminus. HEK-293F cells (hereinafter also referred to as "293F cells") were transiently transfected after the vector passed sequencing validation. The culture supernatant was collected, the binding property and the endocytosis property of the expression antibody were characterized, and finally, the anti-Trop2 VHH antibodies and the anti-Nectin4 VHH antibodies in Table 1 below were obtained after screening.

**Table 1. VHH antibodies and variable region sequences thereof**

| Anti-Trop2 antibody | VHH sequence | Anti-Nectin4 antibody | VHH sequence |
|---|---|---|---|
| A04 | SEQ ID NO: 5 | F09 (or F9) | SEQ ID NO: 17 |
| G08 | SEQ ID NO: 9 | F12 | SEQ ID NO: 21 |
| H03 | SEQ ID NO: 13 | D9 | SEQ ID NO: 25 |

### Example 1.2. In Vitro Characterization of Candidate VHHs

### Expression and purification of candidate VHH-Fc antibodies

The encoding gene of the VHH antibody sequence described above was synthesized and inserted into the expression vector pcDNA3.4 to fuse the antibody sequence to an hIgG1 Fc sequence (SEQ ID NO: 85) at the C-terminus. The constructed expression vector was transiently transfected into 293F cells. After the transfected cells were continuously cultured for 7 days, the culture supernatant was collected and filtered through a 0.45 µm filter membrane. The filtrate was transferred to a sterile centrifuge tube, the antibody was purified using a Protein A column, and the purity of the antibody product was determined by SEC-HPLC.

### Assay for antigen-binding property by ELISA

The binding of the candidate VHH-Fc antibodies to human and monkey antigens was assayed using ELISA binding assays. In a Trop2 ELISA binding assay, an ELISA plate was coated with a human Trop2-his antigen or cynomolgus monkey Trop2-his antigen, and the ELSIA assay was performed using an anti-hFc-HRP secondary antibody (Abcam, CAT# ab97225). In a Nectin4 ELISA binding assay, an ELISA plate was coated with a human Nectin4-his antigen or cynomolgus monkey Nectin4-his antigen, and the ELSIA assay was performed using an anti-hFc-HRP secondary antibody (Abcam, CAT# ab97225). The ELISA assay results are shown in Table 2 below.

**Table 2. Binding results of anti-Trop2 VHH-Fc by ELISA assay**

| Anti-Trop2 antibody | Human EC₅₀ pM | Cyno EC₅₀ pM | Ratio (C/H) | Anti-Nectin4 antibody | Human EC₅₀ pM | Cyno EC₅₀ pM | Ratio (C/H) |
|---|---|---|---|---|---|---|---|
| A04-Fc | 127.4 | 86.91 | 0.7 | F09-Fc | 82.98 | 73.78 | 0.9 |
| G08-Fc | 116.8 | 90.01 | 0.8 | F12-Fc | 228.1 | 200.4 | 0.9 |
| H03-Fc | 559.3 | 394.3 | 0.7 | D9-Fc | 344.4 | 287.3 | 0.8 |

As shown in the table above, in the Trop2 ELISA binding assays, all candidates showed relatively good binding to human and monkey Trop2 antigens, and the EC50 ratios for the binding of all candidates to monkey and human Trop2 antigens ranged from 0.7 to 0.8, indicating that the candidates have similar affinities for human and monkey Trop2 antigens.

Also as shown in the table above, in the Nectin4 ELISA binding assays, all candidates showed relatively good binding to human and monkey Nectin4 antigens, and the EC50 ratios for the binding of all candidates to monkey and human Nectin4 antigens ranged from 0.8 to 0.9, indicating that the candidates have similar affinities for human and monkey Nectin4 antigens.

### Assay for endocytosis property

According to the FACS binding preliminary experiments, 3 concentrations, 100 nM, 10 nM, and 2 nM, were selected as the concentrations used for the endocytosis assays, and the candidate anti-Trop2 VHH-Fc antibodies and anti-Nectin4 VHH-Fc antibodies were subjected to the endocytosis assays on NCI-N87 and BT474 tumor cells. In the assay, a VHH-Fc antibody to be tested with a concentration of 100 nM, 10 nM, or 2 nM was added to target cells at 2 × 10⁵ cells/well, and the mixture was incubated at 4 °C for 0.5 h; then, the cells were evenly divided into 2 groups, and these two groups were incubated at 4 °C and 37 °C for 4 h, respectively; and after the incubation was completed, the cells were incubated with anti-hIgG-Fc-PE (1:500 dilution) at 4 °C for 30 min in the dark, and immobilized in 1% paraformaldehyde (PFA) for the FACS assay.

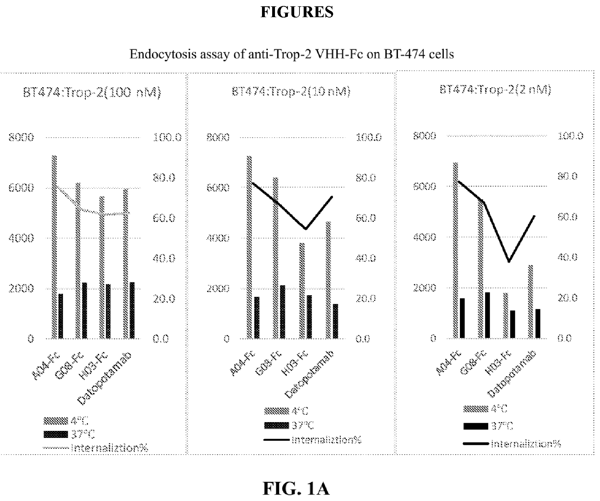

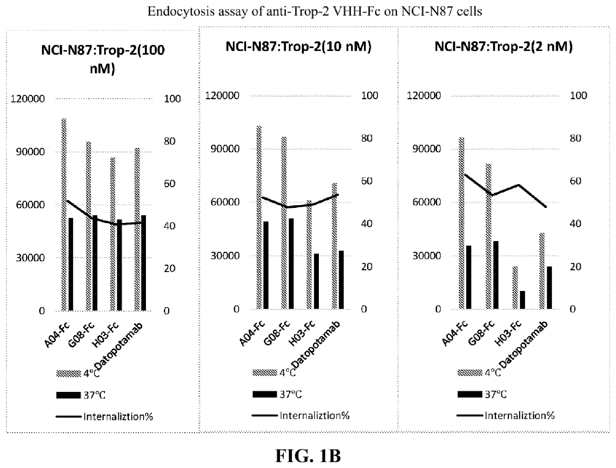

As shown in FIGs. 1A and 1B, the trends of the endocytosis assay results were substantially consistent at the three concentrations tested for the candidate anti-Trop2 VHH-Fc antibodies. Compared with V-BMK1, both of the candidates A04 and G08 showed better binding to the target cells and were not inferior to that of V-BMK1 in endocytosis, with endocytosis rates of 50% or more in the NCI-87 cells with a high expression level of Trop2 (Trop2^{hi}) and in the BT474 cells with a low expression level of Trop2 (Trop2^{low}) at a concentration of 2 nM. H03 showed slightly inferior binding to the target cells and endocytosis to those of V-BMK1, with an endocytosis rate of 54% in the NCI-87 cells with a high expression level of Trop2 (Trop2^{hi}) and 38% in the BT474 cells with a low expression level of Trop2 (Trop2^{low}) at a concentration of 2 nM.

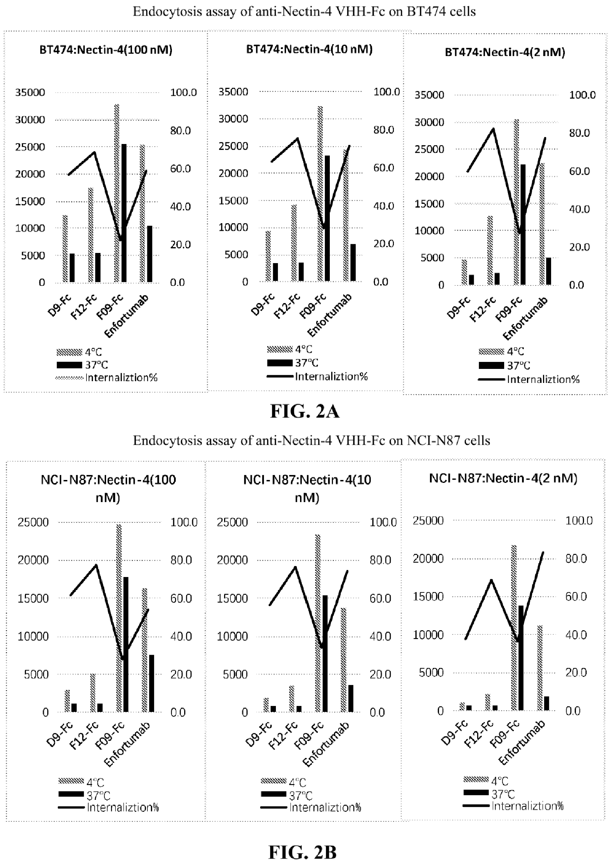

As shown in FIGs. 2A and 2B, the trends of the endocytosis assay results were substantially consistent at the three concentrations tested for the candidate anti-Nectin4 VHH-Fc antibodies. All candidates, F9-Fc, F12-Fc, and D9-Fc, were capable of binding to the target cells, but the differences in the target binding level and endocytosis rate among the candidates were large.

### Epitope binning

Epitope binning of the candidate anti-VHH-Fc antibodies and related reference antibodies (Datopotamab or Enfortumab) was assayed by the ELISA binding assay.

In the ELISA binding assay, the antibody to be tested, diluted to 1.0 µg/mL, was used for plate coating. An antigen (Trop2-his or Nectin4-his) was diluted to 300 ng/mL with a blocking solution, a competitor antibody was diluted to 30 µg/mL with the blocking solution, and equal volumes of the antigen and the competitor were mixed and incubated at room temperature for 1 h; in addition, the same volume of a mixture of the antigen and the blocking solution was used as a control for the competition experiment to calculate the inhibition rate of the competitor against the antibody to be tested. The prepared mixture was added to an ELISA plate at 100 µL/well, and the plate was incubated at room temperature for 1 h; anti-His antibody-HRP (Sino Biological /105327-MM02T-H) was used as a secondary antibody, and the plate was incubated at room temperature for another 1 h; and the plate was assayed on the instrument.

The ELISA results in Table 3 show that none of the anti-Trop2 candidates, A04-Fc, G08-Fc, and H03-Fc, bound to the same epitope as Datopotamab.

The ELISA results in Table 4 show that the anti-Nectin4 antibody F09-Fc exhibited weak signal inhibition against the binding of Enfortumab to the antigen Nectin4, indicating that the binding epitope of F09-Fc may be adjacent to Enfrotumab; while the anti-Nectin4 antibodies D9-Fc and F12-Fc were in different epitope bins from Enfortumab.

**Table 3. Anti-Trop2 epitope competition**

| | **Competitor** | | |
|---|---|---|---|
| **Coating** | A04-Fc | G08-Fc | H03-Fc |
| Datopotamab | -35 | -27 | -33 |

**Table 4. Anti-Nectin4 epitope competition**

| | **Competitor** | | |
|---|---|---|---|
| **Coating** | D9-Fc | F09-Fc | F12-Fc |
| Enfortumab | -18 | 57 | 11 |

### Assay for antigen-binding property by FACS

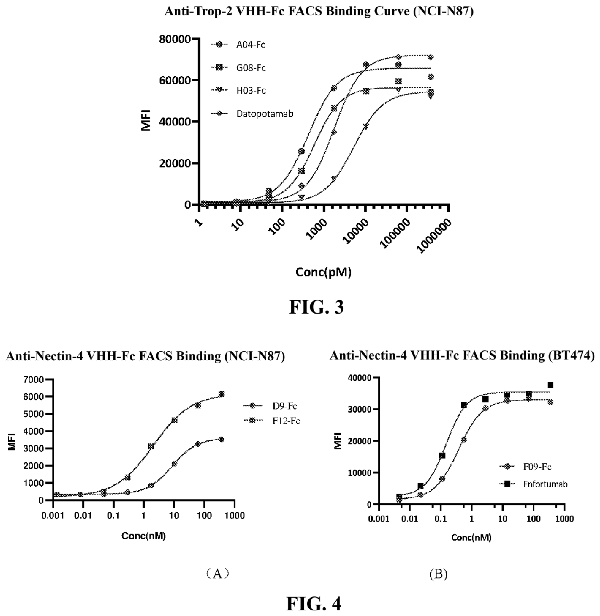

The binding of the anti-Trop2 VHH-Fc candidate antibody molecules, A04-Fc, G08-Fc, and H03-Fc, to target cells was assayed using the FACS binding assay. The assay was performed using the following conditions: NCI-N87 target cells (2 × 10⁵ cells/well) + VHH-Fc or reference antibody (375 nM, 6× dilution, at 4 °C for 1 h) + anti-hIgG Fc-PE secondary antibody (1:500, at 4 °C for 0.5 h). The FACS binding results are shown in FIG. 3. All three candidate antibodies showed good target cell binding properties.

The binding of the anti-Nectin4 VHH-Fc candidate antibody molecules, D9-Fc, F09-Fc, and F12-Fc, to target cells was assayed using the FACS binding assay. The assay was performed using the following conditions: NCI-N87 target cells (2 × 10⁵ cells/well) + D9-Fc or F12-Fc antibody or reference antibody (375 nM, 6× dilution, at 4 °C for 1 h) + anti-hIgG Fc-PE secondary antibody (1:500, at 4 °C for 0.5 h); or BT474 target cells (2 × 10⁵ cells/well) + F09-Fc or reference antibody (350 nM, 5× dilution, at 4 °C for 1 h) + anti-hIgG Fc-PE secondary antibody (1:500, at 4 °C for 1 h). The FACS binding results (FIG. 4 (A) and (B)) show that all three candidate antibodies showed good target cell binding properties.

### Example 1.3. Sequence Optimization and Characterization of VHH Components

### Sequence optimization of anti-Trop2 VHH components

The anti-Trop2 VHH sequence A04 was selected and subjected to sequence optimization.

The original VHH sequence was humanized using a "best-match method". Alignment and analysis were performed on amino acid sequences of VHH framework regions by using a human germline V gene database to select the optimal germline sequence. The humanized VHH sequence was generated by replacing the best-matched human CDR sequences with VHH CDR sequences. A plurality of residues of the framework regions were back-mutated, and post-translational modifications (PTMs) were removed. The humanized sequence was reverse-translated and sent to GENEWIZ (Shanghai, China) for gene synthesis. Then, the gene was constructed into the expression vector pcDNA 3.4, and the humanized VHHs were expressed in the form of a human IgG4 Half body by fusing a human IgG4 sequence (SEQ ID NO: 87) at the C-terminus, thereby obtaining VHH antibody proteins.

The VHH sequences of the PTM-removed and humanized antibodies for A04 are shown in Table 5 below, with underlined CDR sequences defined according to the AbM scheme.

**Table 5. Anti-Trop2 VHH sequences**

| Anti-Trop2 | SEQ ID NO. | Sequence |
|---|---|---|
| A04.m1 | 29 | QLQLVESGGGLVQPGGSLRLSCAASALTFKDYAMTW FRQAPGKEREGVSSVSSGGATTYADSVKGRFTISRDN AKNTVYLQMNSLQPEDTAVYYCAASWGYGLRSLTR KEYDYWGQGTQVTVSS |
| A04.m1.m10 | 98 | EVQLVESGGGLVQPGGSLRLSCAASALTFKDYAMTW FRQAPGKEREGVSSVSSGGATTYADSVKGRFTISRDN SKNTVYLQMNSLRAEDTAVYYCAASWGYGLRSLTR KEYDYWGQGTLVTVSS |
| A04.m1.m11 | 99 | QLQLVESGGGLVQPGGSLRLSCAASALTFKDYAMTW FRQAPGKEREGVSSVSSGGATTYADSVKGRFTISRDN SKNTVYLQMNSLRAEDTAVYYCAASWGYGLRSLTR KEYDYWGQGTLVTVSS |
| A04.m1.m12 | 30 | QLQLVESGGGLVQPGGSLRLSCAASALTFKDYAMTW FRQAPGKEREGVSSVSSGGATTYADSVKGRFTISRDN SKNTVYLQMNSLQPEDTAVYYCAASWGYGLRSLTRK EYDYWGQGTLVTVSS |

The expression and purification results of the A04 humanized antibodies expressed in the form of a VHH-hIgG4 half antibody are shown in Table 6 below.

**Table 6. Expression and purification results of A04.m1 humanized antibodies**

| Antibody | Concentration (mg/mL) | Purity% (SEC-HPLC) | **Yield** (mg/L) |
|---|---|---|---|
| A04.m1-hIgG4 | 1.19 | 96.5 | 119 |
| A04m1.m10-hIgG4 | 1.04 | 96.2 | 104 |
| A04m1.m11-hIgG4 | 1.87 | 95.7 | 187 |
| A04m1.m12-hIgG4 | 1.44 | 95.3 | 144 |

The obtained antibodies, A04.m1-hIgG4, A04m1.m10-hIgG4, A04m1.m11-hIgG4, and A04.m1.m12-hIgG4, were assayed by FACS, and the binding of four antibodies to the NCI-N87 target cells was similar to that of the parent antibody (Table 7). Conditions of the FACS binding assay were as follows: target cells (1 × 10⁵ cells/well) + sample (200 nM, 5× dilution, incubated at 4 °C for 1 h) + anti-Fc-PE (1:500, incubated at 4 °C for 0.5 h).

**Table 7. Binding activity of A04 humanized antibodies to NCI-N87**

| Antibody | A04.m1-hIgG4 | A04.m1.m10-hIgG4 | A04.m1.m11 -hIgG4 | A04.m1.m12 -hIgG4 |
|---|---|---|---|---|
| EC₅₀ nM | 37.26 | 21.94 | 21.06 | 20.96 |
| Maximum MFI value | 67375 | 58524 | 56913 | 60951 |

The human-monkey cross-reactivity of the selected A04 humanized antibodies was verified by the SPR assay (Table 8).

**Table 8. Binding affinity and kinetics of A04 humanized antibodies for human and cynomolgus monkey Trop2 antigens**

| **Ligand** | **Analyte** | **ka (1/Ms)** | **kd (1/s)** | **KD (M)** |
|---|---|---|---|---|
| Human Trop2-His | A04.m1-hIgG4 | 3.50E+06 | 1.18E-01 | 3.36E-08 |
| Human Trop2-His | A04m1.m10-hIgG4- | 1.09E+06 | 5.92E-02 | 5.46E-08 |
| Human Trop2-His | A04m1.m11-hIgG4- | 8.86E+05 | 3.15E-02 | 3.56E-08 |
| Human Trop2-His | A04m1.m12-hIgG4 | 8.90E+05 | 3.24E-02 | 3.65E-08 |
| Cyno Trop2-His | A04.m1-hIgG4 | 5.17E+05 | 2.13E-02 | 4.12E-08 |
| Cyno Trop2-His | A04m1.m10-hIgG4- | 6.60E+05 | 3.33E-02 | 5.05E-08 |
| Cyno Trop2-His | A04m1.m11-hIgG4- | 5.09E+05 | 1.97E-02 | 3.86E-08 |
| Cyno Trop2-His | A04m1.m12-hIgG4 | 4.79E+05 | 2.04E-02 | 4.26E-08 |

The VHH half antibodies prepared from 293F host cells as described above were analyzed for physicochemical properties using HIC-HPLC and DSF assays. The results are shown in Table 9 below.

**Table 9. DA data on physicochemical properties of A04 humanized antibodies**

| **Antibody** | **MW (KDa)** | **Concentration (mg/mL)** | **HIC-HPLC(%)** | **Tm Onset (°C)** | **Tagg(°C) (266 nm)** | Type of molecule |
|---|---|---|---|---|---|---|
| | | | **Main peak (NH₄)₂SO₄ (M)** | | | |
| A04.m1-hIgG4 | 39.20 | 1.19 | 0.55 | 58.28 | 43.49 | VHH-Fc half body |
| A04.m1.m10-hIgG4 | 39.20 | 1.04 | 0.63 | 51.33 | 42.18 | VHH-Fc half body |
| A04.m1.m11-hIgG4 | 39.20 | 1.87 | 0.51 | 54.10 | 42.01 | VHH-Fc half body |
| A04.m1.m12-hIgG4 | 39.20 | 1.44 | 0.50 | 56.76 | 46.44 | VHH-Fc half body |

### Sequence optimization of anti-Nectin 4 VHH components

The anti-Nectin4 VHH sequence F09 (hereinafter referred to as "F09") was selected and subjected to sequence optimization.

The original VHH sequence was humanized using a "best-match method". Alignment and analysis were performed on amino acid sequences of VHH framework regions by using a human germline V gene database to select the optimal germline sequence. The humanized VHH sequence was generated by replacing the best-matched human CDR sequences with VHH CDR sequences. A plurality of residues of the framework regions were back-mutated, and post-translational modifications (PTMs) were removed. The humanized sequence was reverse-translated and sent to GENEWIZ (Shanghai, China) for gene synthesis. Then, the gene was constructed into the expression vector pcDNA 3.4, and the humanized VHHs were expressed by fusing a His tag at the C-terminus. The antibody was purified using a nickel column, and the purity of the product was measured by SEC-HPLC, thereby obtaining a VHH-His antibody protein.

The humanized and optimized VHH sequences and their corresponding original VHH sequence are shown in Table 10 below, with underlined CDR sequences defined according to the AbM scheme.

**Table 10. Humanized and optimized VHH sequences**

| Anti-Nectin4 | SEQ ID NO. | Sequence |
|---|---|---|
| F09 (wt) | 17 | QVQLVESGGGLVQPGGSLRLSCAASGSTFGINVMGWYRQAP GKQREYVASITRDGSINYADSVRGRFTISRDNAKSTVYLQMN SLKPDDTAVYYCNADRLGPTGWGSRIELWGQGTQVTVSS |
| F09.m1 | 100 | EVQLVESGGGLVQPGGSLRLSCAASGSTFGINVMGWYRQAP GKQREYVASITRDGSINYADSVKGRFTISRDNSKNTVYLQMN SLRAEDTAVYYCNADRLGPTGWGSRIELWGQGTLVTVSS |
| F09.m2 | 101 | EVQLVESGGGLVQPGGSLRLSCAASGSTFGINVMGWYRQAP GKQREYVASITRDGSINYADSVRGRFTISRDNSKNTVYLQMN SLRAEDTAVYYCNADRLGPTGWGSRIELWGQGTLVTVSS |
| F09.m5 | 102 | QVQLVESGGGLVQPGGSLRLSCAASGSTFGINVMGWYRQAP GKQREYVASITREGSINYADSVRGRFTISRDNAKSTVYLQMN SLKPDDTAVYYCNADRLGPTGWGSRIELWGQGTQVTVSS |
| F09.m6 | 103 | QVQLVESGGGLVQPGGSLRLSCAASGSTFGINVMGWYRQAP GKQREYVASITRQGSINYADSVRGRFTISRDNAKSTVYLQMN SLKPDDTAVYYCNADRLGPTGWGSRIELWGQGTQVTVSS |
| F09.m1.m5 | 104 | EVQLVESGGGLVQPGGSLRLSCAASGSTFGINVMGWYRQAP GKQREYVASITREGSINYADSVKGRFTISRDNSKNTVYLQMN SLRAEDTAVYYCNADRLGPTGWGSRIELWGQGTLVTVSS |
| F09.m1.m6 | 105 | EVQLVESGGGLVQPGGSLRLSCAASGSTFGINVMGWYRQAP GKQREYVASITRQGSINYADSVKGRFTISRDNSKNTVYLQMN SLRAEDTAVYYCNADRLGPTGWGSRIELWGQGTLVTVSS |
| F09.m2.m5 | 34 | EVQLVESGGGLVQPGGSLRLSCAASGSTFGINVMGWYRQAP GKQREYVASITREGSINYADSVRGRFTISRDNSKNTVYLQMN SLRAEDTAVYYCNADRLGPTGWGSRIELWGQGTLVTVSS |
| F09.m2.m6 | 35 | EVQLVESGGGLVQPGGSLRLSCAASGSTFGINVMGWYRQAP GKQREYVASITRQGSINYADSVRGRFTISRDNSKNTVYLQMN SLRAEDTAVYYCNADRLGPTGWGSRIELWGQGTLVTVSS |

The expression and purification results of the F09 optimized antibodies expressed in the form of VHH-his are shown in Table 11 below.

**Table 11. Expression and purification results of F09 optimized antibodies**

| **Antibody** | **MW(KDa)** | **Concentration (mg/mL)** | **Purity% (SEC-HPLC)** | **Yield (mg/L)** |
|---|---|---|---|---|
| F09 | 14.18 | 1.06 | 85.74 | 113.59 |
| F09.m1 | 14.20 | 1.37 | 86.51 | 146.29 |
| F09.m2 | 14.22 | 1.20 | 85.97 | 128.21 |
| F09.m5 | 14.19 | 1.13 | 84.72 | 120.23 |
| F09.m6 | 14.19 | 0.98 | 90.59 | 104.83 |
| F09.m1.m5 | 14.21 | 1.11 | 83.8 | 130 |
| F09.m1.m6 | 14.21 | 0.82 | 89.9 | 96 |
| F09.m2.m5 | 14.24 | 1.10 | 83.7 | 129 |
| F09.m2.m6 | 14.24 | 1.14 | 89.5 | 133 |

The obtained antibodies were subjected to an FACS binding assay, and the obtained antibodies had a similar antigen-binding affinity to that of the parent antibody F09. The obtained antibodies were examined for binding affinity and kinetics for human and cynomolgus monkey Nectin4 antigens by the SPR assay, which showed comparable binding affinity and similar human-monkey cross-reactivity to those of the parent antibody (Table 12).

**Table 12. Binding affinity and kinetics of F09 optimized antibodies for human and cynomolgus monkey Nectin4 antigens**

| Immobilized ligand | Analyte | ka (1/Ms) | kd (1/s) | KD (M) |
|---|---|---|---|---|
| Human Nectin4-his | F09.m1.his | 3.44E+05 | 1.82E-02 | 5.29E-08 |
| Human Nectin4-his | F09.m2.his | 2.88E+05 | 1.15E-02 | 3.99E-08 |
| Human Nectin4-his | F09.m5.his | 2.91E+05 | 7.89E-03 | 2.71E-08 |
| Human Nectin4-his | F09.m6.his | 5.36E+05 | 7.47E-03 | 1.39E-08 |
| Human Nectin4-his | F09.m1.m5.his | 6.57E+04 | 1.20E-02 | 1.82E-07 |
| Human Nectin4-his | F09.m1.m6.his | 7.54E+04 | 1.14E-02 | 1.51E-07 |
| Human Nectin4-his | F09.m2.m5.his | 9.50E+04 | 7.54E-03 | 7.94E-08 |
| Human Nectin4-his | F09.m2.m6.his | 1.43E+05 | 7.73E-03 | 5.40E-08 |
| Human Nectin4-his | F09.his | 9.96E+04 | 1.14E-02 | 1.15E-07 |
| Cyno Nectin4-His | F09.m1.m5.his | 8.19E+04 | 1.48E-02 | 1.80E-07 |
| Cyno Nectin4-His | F09.m1.m6.his | 1.26E+05 | 1.34E-02 | 1.06E-07 |
| Cyno Nectin4-His | F09.m2.m5.his | 6.95E+04 | 9.43E-03 | 1.36E-07 |
| Cyno Nectin4-His | F09.m2.m6.his | 1.15E+05 | 7.97E-03 | 6.91E-08 |
| Cyno Nectin4-His | F09.his | 9.11E+04 | 1.44E-02 | 1.58E-07 |

After conversion of F09.m2m5 to Fc bivalent format, F09.m2m5-Fc still showed similar binding to that of F09-Fc when binding to the BT474 target cells by the FACS binding assay. The FACS assay was performed using the following conditions: BT474 target cells (2 × 10⁵ cells/well) + antibody sample (350 nM, 5× dilution, at 4 °C for 1 h) + anti-hIgG Fc-PE secondary antibody (1:500, at 4 °C for 1 h).

Based on the above data, F09.m2m5 was selected, the immunogenicity of the humanized VHH sequence was analyzed by the WeMol software, and the mutated residues were introduced into F09.m2m5 to obtain deimmunized sequences F09.m2m5-2 and F09.m2m5-4.

**Table 13. Deimmunized VHH sequences of F09.m2m5**

| Anti-Nectin4 | SEQ ID NO. | Sequence |
|---|---|---|
| F09.m2m5-2 | 38 | EVQLVESGGGLVQPGGSLRLSCAASGSTFGINVM GWYRQAPGKQREYVASITREGSINYADSVRGRFTI SRDNAKNTVYLQMNSLRPEDTAVYYCNADRLGP TGWGSRIELWGQGTLVTVSS |
| F09.m2m5-4 | 106 | EVQLVESGGGLVQPGGSLRLSCAASGSTFGINVM GWYRQAPGKQREYVASITREGSINYADSVRGRFTI SRDNAKNTVYLQMNSLKPEDTAVYYCNADRLGP TGWGSRIELWGQGTLVTVSS |

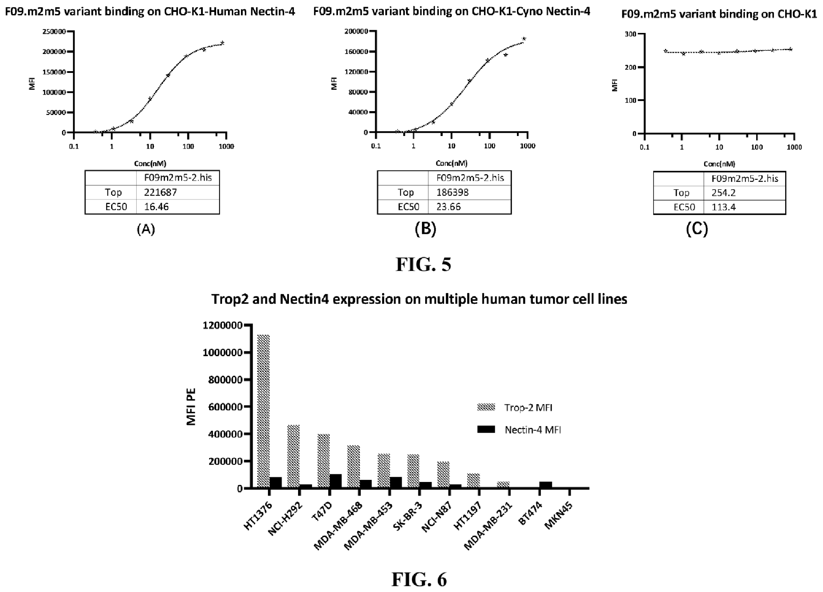

The cross-reactivity of the F09.m2m5 deimmunized sample was assayed by SPR and FACS. The SPR assay results are shown in Table 14. The FACS assay results are shown in FIG. 5. The sample remained cross-reactive with human Nectin4 and cyno Nectin4 engineered cell lines and was not cross-reactive with CHO-K1 cells. The physicochemical properties of F09.m2m5.his, F09.m2m5-2.his, and F09.m2m5-4.his were analyzed by SEC-HPLC, CEX-HPLC, HIC-HPLC, and DLS assays. The results are shown in Table 15 below. Both samples showed physicochemical properties with good developability.

**Table 14. Binding affinity and kinetics of deimmunized samples of F09.m2m5 for human and cynomolgus monkey Nectin4 antigens**

| Immobilized ligand | Analyte | ka (1/Ms) | kd (1/s) | KD (M) |
|---|---|---|---|---|
| Human Nectin4-his | F09m2m5-2.his | 8.75E+04 | 7.58E-03 | 8.66E-08 |
| Human Nectin4-his | F09m2m5-4.his | 1.03E+05 | 7.91E-03 | 7.71E-08 |
| Cyno Nectin4-His | F09m2m5-2.his | 6.48E+04 | 1.23E-02 | 1.90E-07 |
| Cyno Nectin4-His | F09m2m5-4.his | 2.16E+05 | 1.05E-02 | 4.88E-08 |

**Table 15. Data on physicochemical properties of F09.m2m5.his and deimmunized antibodies thereof**

| Antibody | Concentration (mg/mL) | CEX-HPLC(%) | | | HIC-HPLC(%) | Tm (°C) | Tagg (°C) 266 | Type of molecule |
|---|---|---|---|---|---|---|---|---|
| | | Acidic peak | Main peak | Basic peak | Main peak (NH₄)₂SO₄ (M) | | | |
| F09.m2m5.his | 1.1 | 19.1 | 77.2 | 3.7 | 0.94 | 67.87 | 60.29 | VHH-His |
| F09.m2m5-2.his | 1.4 | 20.51 | 71.25 | 8.24 | 1.08 | 65.66 | 54.5 | VHH-His |
| F09.m2m5-4.his | 1.51 | 19.7 | 75.8 | 4.5 | 1.09 | 65.73 | 42.58 | VHH-His |

### Example 1.4 Generation of Multispecific Anti-Trop2/Nectin4 Antibody Molecules

This example describes the structure of exemplary anti-Trop2/Nectin4 bispecific antibodies (BsAbs) and the design and construction of expression vectors.

### Design of multispecific antibody molecules

The following bispecific antibody molecule constructs were designed:
A. A single-chain form comprising, from the N-terminus to the C-terminus: (VHH^{A})ₙ₁-(VHH^{B})ₙ₂-(HLE)ₙ₃-(VHH^{A})ₙ₄-(VHH^{B})ₙ₅-(HLE)ₙ₆,
wherein n1, n2, n3, n4, n5, and n6 are each independently selected from integers of 0, 1, and 2; VHH^{A} and VHH^{B} represent VHH domains binding to an antigen A and an antigen B, respectively, wherein A and B are different from each other and are independently selected from Trop2 and Nectin4; HLE (Half-life extension) represents a serum albumin (SA) binding peptide as a half-life extension domain, particularly an anti-serum albumin VHH domain VHH^{SA}; the symbol "-" represents linkage via a peptide linker or direct linkage, preferably a peptide linker having 5-15 amino acids in length;
preferably, the ratio of VHH^{Trop2} domain to VHH^{Nectin4} domain is 1:1 or 1:2;
preferably, there are 1-2 VHH^{Trop2} domains, and there are 1-2 VHH^{Nectin4} domains; optionally, there are 0 or 1 HLE domain;
for example, the single-chain forms are shown in Table 16 below:

**Table 16. Anti-Trop2 and Nectin4 single-chain antibody forms**

| Formmat | Single polypeptide chain |
|---|---|
| 1 | VHH ^{Trop2}-VHH ^{Nectin4} |
| 2 | VHH ^{Trop2}-VHH ^{Nectin4}-VHH ^{SA} |
| 3 | VHH ^{Nectin4}-VHH ^{Trop2}-VHH ^{Nectin4} |
| 4 | VHH ^{Trop2}-VHH ^{Nectin4}-VHH ^{Nectin4} |
| 5 | VHH ^{Trop2}-VHH ^{SA}-VHH^{Nectin4}-VHH ^{Nectin4} |
| 6 | VHH ^{Trop2}-VHH^{Trop2}-VHH^{SA}-VHH ^{Nectin4}-VHH ^{Nectin4} |

For the single-chain antibodies, a purification tag (e.g., His tag) could optionally be added at the C-terminus of the antibody molecule for ease of subsequent purification, and the addition was achieved optionally via a small peptide linker (e.g., AAA) or via direct linkage.
B. A symmetric double-chain form consisting of two identical polypeptide chains, wherein each chain comprises, from the N-terminus to the C-terminus: (VHH^{A})ₙ₁-(VHH^{B})ₙ₂-HLE-(VHH^{A})ₙ₃-(VHH^{B})ₙ₄, wherein n1, n2, n3, and n4 are each independently selected from integers of 0, 1, and 2;
VHH^{A} and VHH^{B} represent VHH domains binding to an antigen A and an antigen B, respectively, wherein A and B are different from each other and are independently selected from Trop2 and Nectin4; HLE represents an immunoglobulin Fc region as a half-life extension domain, particularly a human IgG1 or human IgG4 Fc region; the symbol "-" represents linkage via a peptide linker or direct linkage, preferably a peptide linker having 5-15 amino acids in length;
preferably, the ratio of VHH^{Trop2} domain to VHH^{Nectin4} domain is 1:1 or 1:2;
preferably, there are 1-2 VHH^{Trop2} domains, and there are 1-2 VHH^{Nectin4} domains;
still preferably, each chain comprises, from the N-terminus to the C-terminus: (VHH^{A})ₙ₁-(VHH^{B})ₙ₂-HLE, wherein n1 and n2 are each independently selected from integers of 1 or 2, and more preferably, n1 and n2 are 1; for example, the symmetric double-chain forms are shown in Table 17 below:

**Table 17. Anti-Trop2 and Nectin4 symmetric double-chain antibody forms**

| Format | Antibody polypeptide chain |
|---|---|
| 1 | (VHH^{Nectin4}-VHH^{Trop2}-Fc) x2 |
| 2 | (VHH^{Trop2}-VHH^{Nectin4}- Fc) x2 |

Due to dimerization of the immunoglobulin Fc region, the first and second polypeptide chains could associate to form a homodimer, thereby producing a multispecific binding molecule in a double-chain form.
C. An asymmetric double-chain form consisting of two different polypeptide chains, wherein
the first polypeptide chain comprises, from the N-terminus to the C-terminus: (VHH^{A})ₙ₁-(VHH^{B})ₙ₂-HLE-(VHH^{A})ₙ₃-(VHH^{B})ₙ₄;
the second polypeptide chain comprises, from the N-terminus to the C-terminus: (VHH^{B})ₘ₁-(VHH^{A})ₘ₂-HLE-(VHH^{B})ₘ₃-(VHH^{A})ₘ₄,
wherein n1, n2, n3, and n4, as well as m1, m2, m3, and m4, are each independently selected from integers of 0, 1, or 2;
VHH^{A} and VHH^{B} represent VHH domains binding to an antigen A and an antigen B, respectively, wherein A and B are different from each other and are independently selected from Trop2 and Nectin4; HLE represents an immunoglobulin Fc region as a half-life extension domain, particularly a human IgG1 or IgG4 Fc region; the symbol "-" represents linkage via a peptide linker or direct linkage, preferably a peptide linker having 5-15 amino acids in length;
preferably, the ratio of VHH^{Trop2} domain to VHH^{Nectin4} domain is 1:1 or 1:2;
preferably, there are 1-2 VHH^{Trop2} domains, and there are 1-2 VHH^{Nectin4} domains;
still preferably, wherein
the first polypeptide chain comprises, from the N-terminus to the C-terminus: (VHH^{A})ₙ₁-(VHH^{B})ₙ₂-HLE,
the second polypeptide chain comprises, from the N-terminus to the C-terminus: (VHH^{B})ₘ₁-(VHH^{A})ₘ₂-HLE,
wherein n1 and n2, as well as m1 and m2, are each independently selected from integers of 0, 1, or 2, with the proviso that n1 and n2 cannot simultaneously be 0, m1 and m2 cannot simultaneously be 0, and the sum of n1 and m2, as well as the sum of n2 and m1, should be greater than or equal to 1;
more preferably, wherein:
   the first polypeptide chain comprises, from the N-terminus to the C-terminus: (VHH^{A})ₙ₁-(VHH^{B})ₙ₂-HLE,
   the second polypeptide chain comprises, from the N-terminus to the C-terminus: (VHH^{B})ₘ₁-HLE; or (VHH^{A})ₘ₂-HLE,
   wherein n1, m1, and m2 are each independently selected from integers of 1 or 2, and n2 is an integer of 0 or 1;
   still preferably,
   the first polypeptide chain comprises, from the N-terminus to the C-terminus: (VHH^{A})-HLE, the second polypeptide chain comprises, from the N-terminus to the C-terminus: (VHH^{B})-HLE;
      or
   the first polypeptide chain comprises, from the N-terminus to the C-terminus: (VHH^{A})-(VHH^{B})-HLE,
   the second polypeptide chain comprises, from the N-terminus to the C-terminus: (VHH^{A})-HLE;
      or
   the first polypeptide chain comprises, from the N-terminus to the C-terminus: (VHH^{A})-(VHH^{A})-HLE,
   the second polypeptide chain comprises, from the N-terminus to the C-terminus: (VHH^{B})-HLE;
      or
   the first polypeptide chain comprises, from the N-terminus to the C-terminus: (VHH^{A})-(VHH^{B})-HLE,
   the second polypeptide chain comprises, from the N-terminus to the C-terminus: (VHH^{B})-HLE;

For example, the double-chain forms are shown in Table 18 below:

**Table 18. Anti-Trop2 and Nectin4 asymmetric double-chain antibody forms**

| Format | Antibody polypeptide chain |
|---|---|
| 1 | First chain: VHH^{Trop2}-Fc |
| | Second chain: VHH^{Nectin4}-Fc |
| 2 | First chain: VHH^{Nectin4}-VHH^{Trop2}-Fc |
| | Second chain: VHH^{Nectin4}-Fc |
| 3 | First chain: VHH^{Nectin4}-VHH^{Nectin4}-Fc |
| | Second chain: VHH^{Trop2}-Fc |

Due to dimerization of the immunoglobulin Fc region, the first and second polypeptide chains could associate to form a heterodimer, thereby producing a multispecific binding molecule in a double-chain form. Preferably, in order to promote heterodimerization of the first and second polypeptide chains, a Knob-into-hole mutation, such as (T366W/T366S, L368A, Y407V) mutations or (T366Y/Y407T) mutations, could be introduced into the Fc regions of the first and second polypeptide chains.

### Construction of single-chain multispecific antibody molecules

Exemplary single-chain bispecific constructs shown in Tables 19 and 20 below were constructed, the corresponding amino acid sequences of which are provided in the sequence listing.

In Table 19, non-humanized VHH sequences were used as building blocks, wherein the amino acid sequence of VHH^{Trop2} is derived from G08 (hereinafter referred to as T1) and H03 (hereinafter referred to as T2), the amino acid sequences of which are set forth in SEQ ID NO: 9 and SEQ ID NO: 13, respectively; the amino acid sequence of VHH^{Nectin4} is derived from F12 (hereinafter referred to as N1) and D9 (hereinafter referred to as N2), the amino acid sequences of which are set forth in SEQ ID NO: 21 and SEQ ID NO: 25; the serum albumin binding peptide as the half-life extension domain (HLE) is derived from the VHH domain VHH^{SA}, specifically from the VHH^{SA} sequence MSA21 (hereinafter referred to as HLE1, SEQ ID NO: 39); the symbol "-" represents linkage between two VHH domains via a peptide linker.

**Table 19. Single-chain multispecific antibodies**

| **Antibody name** | **Format** | **Specific linking mode*** | **Specific domains used** |
|---|---|---|---|
| V-1 | VHH^{Trop2}-VHH ^{Nectin4} | T1-N1 | G08-F12 |
| V-2 | VHH^{Trop2}-VHH ^{Nectin4}-VHH ^{SA} | T1-N1-HLE1 | G08-F12-MSA21 |
| V-3 | VHH ^{Trop2}-VHH^{Nectin4} | T2-N2 | H03-D9 |
| V-4 | VHH ^{Trop2}-VHH ^{Nectin4}-VHH^{SA} | T2-N2-HLE1 | H03-D9-MSA21 |
| V-5 | VHH ^{Nectin4} -VHH ^{Trop2}-VHH ^{Nectin4} | N2-T2-N2 | D9-H03-D9 |
| V-6 | VHH ^{Nectin4}-VHH ^{Trop2}-VHH^{Nectin4} | N1-T1-N1 | F12-G08-F12 |
| V-7 | VHH ^{Nectin4}-VHH ^{Trop2}-VHH^{Nectin4} | N2-T1-N2 | D9-G08-D9 |
| V-8 | VHH^{Trop2}-VHH ^{Nectin4}-VHH ^{Nectin4} | T1-N1-N1 | G08-F12-F12 |
| V-9 | VHH ^{Trop2}-VHH ^{Nectin4}-VHH^{Nectin4} | T2-N2-N2 | H03-D9-D9 |

| | | | |
|---|---|---|---|
| *Peptide linker = G4S | | | |

In Table 20, humanized VHH sequences were used as building blocks, wherein the amino acid sequence of VHH^{Trop2} is derived from A04.m1 (hereinafter referred to as S1), the amino acid sequence of which is set forth in SEQ ID NO: 29; the amino acid sequence of VHH^{Nectin4} is derived from F09.m2m5 (hereinafter referred to as F1), the amino acid sequence of which is set forth in SEQ ID NO: 34; the serum albumin (SA) binding peptide as the half-life extension domain (HLE) is derived from the VHH domain VHH^{SA}, specifically from the VHH sequence ALB8 (hereinafter referred to as HLE2, SEQ ID NO: 40); the symbol "-" represents linkage between two VHH domains via a peptide linker.

**Table 20. Single-chain multispecific antibodies**

| Antibody ID (name) | Format | Specific linking mode | Specific domains used |
|---|---|---|---|
| V-hu2 (V-F1S1T1.2) | VHH^{Trop2}-VHH ^{Nectin4}-VHH ^{SA} | S1-F1-HLE2 | A04.m1-F09.m2m5-ALB8 |
| V-hu8 (V-F1F1S1T1.8) | VHH^{Trop2}-VHH^{SA}-VHH^{Nectin4}-VHH^{Nectin4} | S1-HLE2-F1-F1 | A04.m1-ALB8-F09.m2m5-F09.m2m5 |
| V-hu22 (V-F1F1S1S1T1.22) | VHH^{Trop2}-VHH^{Trop2}-VHH^{SA}-VHH^{Nectin4}-VHH^{Nectin4} | S1-S1-HLE2-F1-F1 | A04.m1-A04.m1-ALB8- F09.m2m5-F09.m2m5 |

| | | | |
|---|---|---|---|
| *Peptide linker = (G4S)2 | | | |

The single-chain antibodies in Tables 19 and 20 were constructed into pcDNA 3.4 expression vectors by adding a 6xHis tag at the C-terminus. The vectors were transfected into Expi293F cells; the cells were cultured for 3 days; and the culture supernatants of the transfected cells were collected, and the expressed antibodies were purified using a nickel column. The concentration of the antibodies was measured with Nano Drop. The protein purity was determined by SDS-PAGE and analytical HPLC-SEC, and the proteins were then stored at -80 °C.

### Construction of multi-chain multispecific antibody molecules

Exemplary multi-chain bispecific antibodies shown in Tables 21 and 22 below were constructed, the corresponding amino acid sequences of which are provided in the sequence listing.

In Table 21, non-humanized VHH sequences were used as building blocks, wherein the amino acid sequence of VHH^{Trop2} is derived from G08 (hereinafter referred to as T1) and H03 (hereinafter referred to as T2), the amino acid sequences of which are set forth in SEQ ID NO: 9 and SEQ ID NO: 13, respectively; the amino acid sequence of VHH^{Nectin4} is derived from F12 (hereinafter referred to as N1) and D9 (hereinafter referred to as N2), the amino acid sequences of which are set forth in SEQ ID NO: 21 and SEQ ID NO: 25; the immunoglobulin Fc region as the half-life extension domain (HLE) is derived from human IgG, specifically an hIgG1 Fc sequence comprising a knob mutation and a hole mutation ((T366Y/Y407T) or (T366W/T366S, L368A, Y407V)); the symbol "-" represents linkage via a peptide linker or direct linkage between two domains.

**Table 21. Multi-chain multispecific antibodies in Fc dimer form**

| Antibody ID | **Format** | Specific linking mode | Specific domains used |
|---|---|---|---|
| V-10 | First chain: VHH^{Trop2}-Fc | First chain: T1-Fc | G08-Fc |
| | Second chain: VHH^{Neetin4}-Fc | Second chain: N1-Fc | F12-Fc |
| V-13 | First chain: VHH^{Trop2}-Fc | First chain: T2-Fc | H03-Fc |
| | Second chain: VHH^{Neetin4}-Fc | Second chain: N2-Fc | D9-Fc |
| V-14 | First chain: VHH^{Nectin4}-VHH^{Trop2}-Fc | First chain: N2-T2-Fc | D9-H03-Fc |
| | Second chain: VHH^{Neetin4}-Fc | Second chain: N2-Fc | D9-Fc |
| V-15 | First chain: VHH^{Nectin4}-VHH^{Trop2}-Fc | First chain: N1-T1-Fc | F12-G08-Fc |
| | Second chain: VHH^{Neetin4}-Fc | Second chain: N1-Fc | F12-Fc |
| V-16 | First chain: VHH^{Nectin4}-VHH^{Trop2}-Fc | First chain: N2-T1-Fc | D9-G08-Fc |
| | Second chain: VHH^{Nectin4}-Fc | Second chain: N2-Fc | D9-Fc |
| V-17 | First chain: VHH^{Nectin4}-VHH^{Nectin4}-Fc | First chain: N1-N1-Fc | F12-F12-Fc |
| | | Second chain: T1-Fc | G08-Fc |
| | Second chain: VHH^{Trop2}-Fc | | |
| V-18 | First chain: VHH^{Nectin4}-VHH^{Nectin4}-Fc | First chain: N2-N2-Fc | D9-D9-Fc |
| | | Second chain: T2-Fc | H03-Fc |
| | Second chain: VHH^{Trop2}-Fc | | |

| | | | |
|---|---|---|---|
| Note: Two VHH domains are linked via a peptide linker G4S, and the VHH domain and the Fc domain are directly linked | | | |

In Table 22, humanized VHH sequences were used as building blocks, wherein the amino acid sequence of VHH^{Trop2} is derived from A04.m1 (hereinafter referred to as S1) or A04.m1m12 (hereinafter referred to as S2), the amino acid sequence of which is set forth in SEQ ID NO: 29 or SEQ ID NO: 30, respectively; the amino acid sequence of VHH^{Nectin4} is derived from F09.m2m5 (hereinafter referred to as F1) or F09.m2m5-2 (hereinafter referred to as F2), the amino acid sequence of which is set forth in SEQ ID NO: 34 or SEQ ID NO: 38, respectively; the immunoglobulin Fc region as the half-life extension domain (HLE) is derived from human IgG, specifically an hIgG1 sequence comprising or not comprising a knob mutation and a hole mutation ((T366Y/Y407T) or (T366W/T366S, L368A, Y407V)); the symbol "-" represents linkage via a peptide linker or direct linkage between two domains. The parental anti-Nectin4 antibody and parental anti-Trop2 antibody were also shown as controls in Table 22, wherein the parental anti-Nectin4 comprises a VHH^{Nectin4} domain (F2), the amino acid sequence of which is set forth in SEQ ID NO: 109; the parental anti-Trop2 comprises a VHH^{trop2} domain (S2) and a VHH^{RV} domain targeting the rotavirus (derived from DOI: 10.1053/j.gastro.2013.06.053), with the amino acid sequences of the first and second chains set forth in SEQ ID NOs: 107 and 108, respectively.

**Table 22. Multi-chain multispecific antibodies in Fc dimer form**

| Antibody ID (name) | Format | Specific linking mode | Specific domains used |
|---|---|---|---|
| V-hu21-Fc (V-F1S1.21 uIgG1 LALA) | First chain: VHH^{Nectin4}-VHH^{Trop2}-Fc | First chain: F1-S1-Fc | F09.m2m5-A04.m1-Fc |
| | | Second chain: F1-Fc | |
| | Second chain: VHH^{Nectin4}-Fc | | F09.m2m5-Fc |
| V-hu23-Fc (V-F2S2.23 uIgG1 LALA) | First chain: VHH^{Nectin4}-VHH^{Trop2}-Fc | First chain: F2-S2-Fc | F09.m2m5-2-A04.m1m12-Fc |
| | | Second chain: F2-Fc | |
| | Second chain: VHH^{Nectin4}-Fc | | F09.m2m5-2-Fc |
| V-hu24-Fc | First chain: VHH^{Nectin4}-VHH^{Trop2}-Fc | First chain: F2-S2-Fc | F09.m2m5-2-A04.m1m12-Fc |
| (V-F2S2.24 uIgG1 LALA) | Second chain: VHH^{Nectin4}-VHH^{Trop2}-Fc | Second chain: F2-S2-Fc | F09.m2m5-2-A04.m1m12-Fc |
| V-hu25-Fc (V-F2S2.25 uIgG1 LALA) | First chain: VHH^{Nectin4}-VHH^{Trop2}-Fc | First chain: F2-S2-Fc | F09.m2m5-2-A04.m1m12-Fc |
| | | Second chain: F2-S2-Fc | |
| | Second chain: VHH^{Nectin4}-VHH^{Trop2}-Fc | | F09.m2m5-2-A04.m1m12-Fc |
| V-hu28-Fc (V-F2S2.28 uIgG1 LALA) | First chain: VHH^{Nectin4}-VHH^{Nectin4}-Fc | First chain: F2-F2-Fc | F09.m2m5-2-F09.m2m5-2-Fc |
| | | Second chain: S2-Fc | |
| | Second chain: VHH^{Trop2}-Fc | | A04.m1m12- Fc |
| V-hu29-Fc (V-F2S2.29 uIgG1 LALA) | First chain: VHH^{Trop2}-Fc | First chain: S2-Fc | A04.m1m12-Fc |
| | Second chain: VHH^{Nectin4}-Fc | Second chain: F2-Fc | F09.m2m5-2-Fc |
| Parental anti-Nectin4 (V-F2.33 uIgG1 LALA | First chain: VHH^{Nectin4}-Fc | First chain: F2-Fc | F09.m2m5-2-Fc |
| | | Second chain: F2-Fc | F09.m2m5-2-Fc |
| | Second chain: VHH^{Nectin4}-Fc | | |
| Parental anti-Trop2 # (V-S2Fr1.34-uIgG1 LALA) | First chain: VHH^{RV}- | - | RV-RV-Fc |
| | VHH^{Rv}-Fc | | A04.m1m12-Fc |
| | Second chain: VHH^{Trop2}-Fc | | |

| | | | |
|---|---|---|---|
| *Two VHH domains are linked via a peptide linker (G4S)2, and the VHH domain and the Fc domain are directly linked or linked via G4S | | | |

In the present invention, V-hu21-Fc, V-hu22-Fc, V-hu23-Fc, V-hu24-Fc, V-hu25-Fc, V-hu26-Fc, V-hu27-Fc, V-hu28-Fc, and V-hu29-Fc are also referred to as V-hu21, V-hu22, V-hu23, V-hu24, V-hu25, V-hu26, V-hu27, V-hu28, and V-hu29, respectively.

The first/second polypeptide chains of the double-chain antibodies in the Fc dimer form shown in Tables 21 and 22 were constructed into pcDNA 3.4 expression vectors (if the antibody was of a symmetric structure, one type of vector was generated; and if the antibody was of an asymmetric structure, two types of vectors comprising genes encoding the first and second polypeptide chains, respectively, were generated), and the vectors were transfected into HEK293F cells. The cells were cultured for 3 days, and the culture supernatants of the transfected cells were collected and loaded onto a Protein A column (MabSelect PrismA, Cytiva) for purification. The antibodies were eluted with an acetic acid-sodium acetate solution (pH 3.5) and immediately neutralized with 2 M Tris. The concentration of the antibodies was measured with Nano Drop. The protein purity was determined by SDS-PAGE and analytical HPLC-SEC, and the proteins were then stored at -80 °C.

### Construction of control bispecific antibodies

Control bispecific antibodies V-11 and V-12 were constructed using the amino acid sequences of variable regions from the reference antibodies Datopotamab and Enfortumab. The antibody V-11 consists of a Fab portion of Datopotamab linked to an Fc region at the C-terminus and an scFv portion of Enfortumab linked to the Fc region at the C-terminus. The amino acid sequences (SEQ ID NOs: 77-79) of two heavy chains and one light chain of the antibody are provided in the sequence listing. The antibody V-12 consists of an scFv portion of Datopotamab linked to an Fc region at the C-terminus and a Fab portion of Enfortumab linked to the Fc region at the C-terminus. The amino acid sequences (SEQ ID NOs: 80-82) of two heavy chains and one light chain of the antibody are provided in the sequence listing. The control antibodies V-11 and V-12 were produced and purified by using substantially the same method as described above. The concentration of the antibodies was measured with Nano Drop. The protein purity was determined by SDS-PAGE and analytical HPLC-SEC, and the proteins were then stored at -80 °C.

### Example 1.5 Characterization of Multispecific Anti-Trop2/Nectin4 Antibody Molecules

### Analysis of target antigen expression levels on tumor cells

The expression of the Trop2 antigen and the Nectin4 antigen on various target tumor cells was assayed using the reference antibodies Datopotamab and Enfortumab by the FACS binding assay.

The test results are shown in FIG. 6. In the tested MKN45 tumor cells, the two antigens exhibited very low expression levels, such that MKN45 tumor cells belong to antigen-negative cells; in the remaining tested tumors, the remaining tumor cells exhibited a high expression level of Trop2, which was significantly higher than the expression level of Nectin4, except that BT474 cells had a very low expression level of Trop2. Comparison of the expression levels of Nectin4 among different tumors shows that the expression of Nectin4 was very low in HT1197 and MDA-MB-231, and the expression level of Nectin4 was relatively moderate in NCI-N87, SK-BR-3, and BT474, while the expression level of Nectin4 was relatively high in the remaining tumors.

### Non-specific binding analysis

The binding of the constructed and prepared multispecific antibody molecules as described above to antigen homologs was assayed by the ELISA binding assay. The ELISA binding assay was performed using the following conditions: antigen Trop1/2 or Nectin1/2/3/4 (1 µg/mL, coating at 2-8 °C overnight) + antibody sample to be tested (200 nM, 6× dilution, RT for 1 h) + Anti-Fc-HRP secondary antibody (1:5000, RT for 1 h).

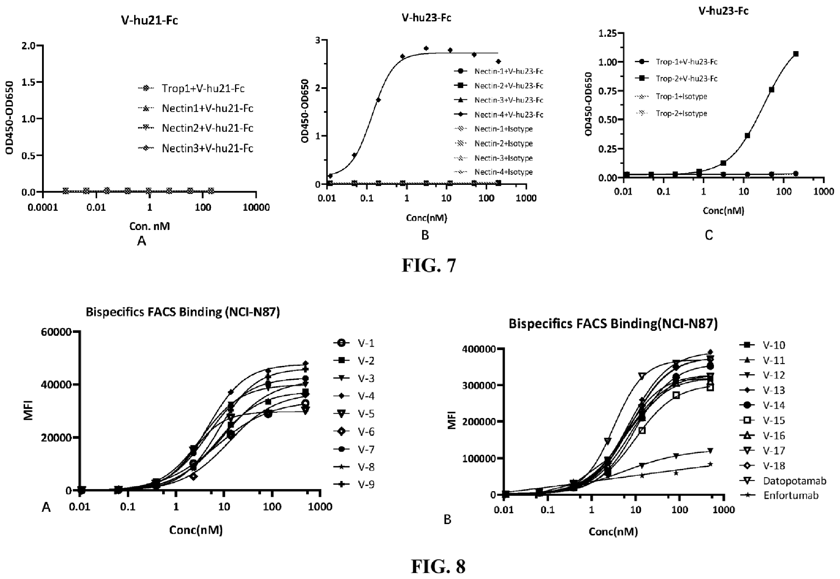

The results show that the multispecific antibody molecules did not specifically bind to the homologs Trop1 and Nectin1/2/3. The results of the non-specific binding analysis for the antibodies V-hu21-Fc and V-hu23-Fc are shown in FIG. 7.

### Binding activity to tumor cells

Based on the above analysis of antigen expression on tumor cells, cell lines NCI-N87 (Trop2^{hi}, Nectin4^{med}), BT474 (Trop2^{low}, Nectin4^{hi}), and MDA-MB-468 (Trop2^{hi}, Nectin4^{hi}) with different target antigen expression levels were selected, and the binding abilities of the bispecific antibodies (abbreviated as "V BsAbs") of the present invention on different target cells were assayed by the FACS binding assay.

The FACS binding assay was performed on the single-chain antibodies using the following conditions: target cells (1-2 × 10⁵ cells/well) + sample (200 nM, 3× dilution, at 4 °C for 1 h) + anti-His-APC secondary antibody (Biolegend, 1:200, at 4 °C for 1 h).

The FACS binding assay was performed on the double-chain antibodies in the Fc dimer form using the following conditions: target cells (2 × 10⁵ cells/well) + sample (200 nM, 3× dilution, at 4 °C for 1 h) + anti-human IgG Fc-PE secondary antibody (Thermo, 1:500, at 4 °C for 0.5 h).

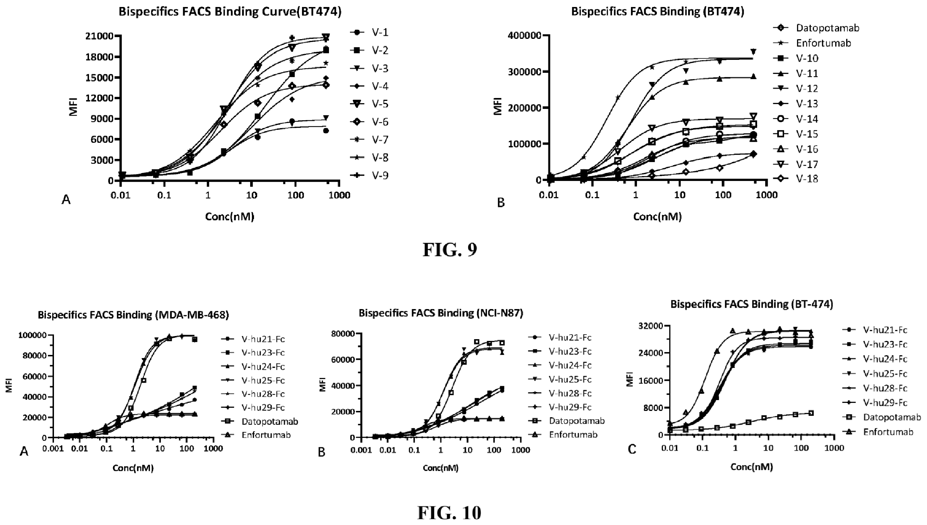

In the NCI-N87 cell line with high expression of Trop2, the binding of the non-humanized V BsAbs and the Fc-containing V BsAbs (V-10 and V-13 to V-18) of the present invention is shown in FIGs. 8A-8B. In the BT474 cell line with low expression of Trop2 and relatively high expression of Nectin4, the binding of the non-humanized V BsAbs and the Fc-containing V BsAbs (V-10 and V-13 to V-18) of the present invention is shown in FIGs. 9A-9B.

In MDA-MB-468 and NCI-87 tumor cells with high expression of Trop2, the symmetric humanized V BsAbs (V-hu24-Fc and V-hu25-Fc) with 2 Trop2-binding sites and 2 Nectin4-binding sites of the present invention had high binding activities comparable to that of the control antibody Datopotamab; the asymmetric humanized V BsAbs (V-hu21-Fc, hu23-Fc, and hu28-Fc) with 1 Trop2-binding site and 2 Nectin4-binding sites of the present invention showed comparable binding activities, which were all higher than that of the control antibody Enfortumab, and more distinct from Enfortumab in the target tumor cells NCI-87 with a lower level of Nectin4; furthermore, the humanized V BsAb (V-hu29-Fc) with only 1 Trop2-binding site and 1 Nectin4-binding site showed a low binding activity similar to that of Enfortumab (FIGs. 10A and 10B). In the BT474 cells with low expression of Trop2 and high expression of Nectin4, the humanized V BsAbs of the present invention showed similar binding behaviors to that of the control, Enfortumab (FIG. 10C).

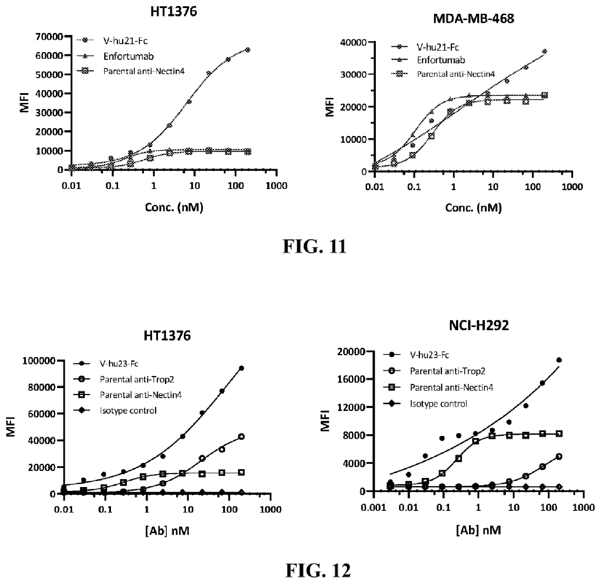

The humanized V-hu21-Fc in the Fc dimer form was selected, and the binding activity of the antibody to a variety of different tumor cells was further assayed by the FACS binding assay. In the assay, the anti-Nectin4 monospecific bivalent antibody parental anti-Nectin4 was used as a control. The FACS binding assay was performed using the following conditions: target cells (2 × 10⁵ cells/well) + sample (200 nM, 3× dilution, at 4 °C for 1 h) + anti-human IgG Fc-PE secondary antibody (Thermo, 1:500, at 4 °C for 0.5 h). The results are shown in FIG. 11.

The humanized V-hu23-Fc in the Fc dimer form was selected, and the binding activity of the antibody to HT1376 and NCI-H292 (human mucoepidermoid pulmonary carcinoma, CBP60187, Nanjing Cobioer Biosciences Co., Ltd.) tumor cells was further assayed by the FACS binding assay. In the assay, parental anti-Trop2 and parental anti-Nectin4 were used as controls. The FACS binding assay was performed using the following conditions: target cells (3 × 10⁵ cells/well) + sample (200 nM, 3× dilution, at 4 °C for 1 h) + anti-human IgG Fc-PE secondary antibody (Thermo, 1:500, at 4 °C for 0.5 h). The results are shown in FIG. 12.

The results show that compared with the parent control molecules, V-21-Fc and V-23-Fc showed synergistic binding effects on the cells that were positive for both targets.

### Internalization assay

The internalization abilities of the bispecific antibody molecules of the present invention on different target cells NCI-N87, BT-474, HT1376, MDA-MB-468, and NCI-H292 were assayed using an endocytosis assay.

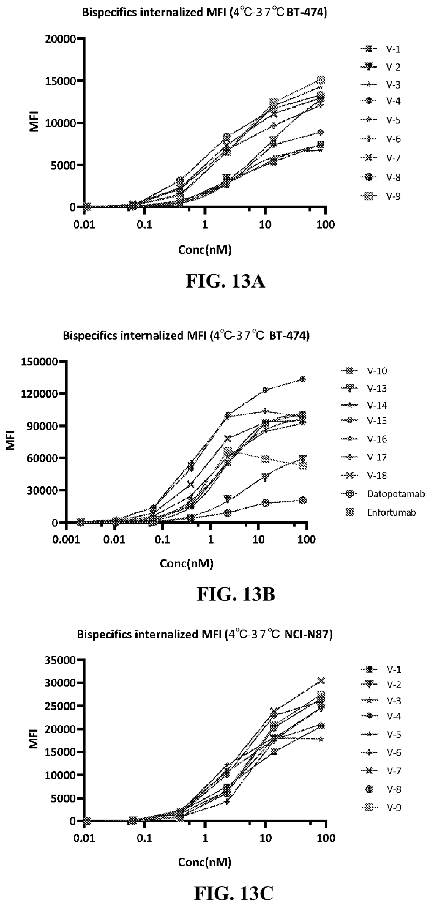

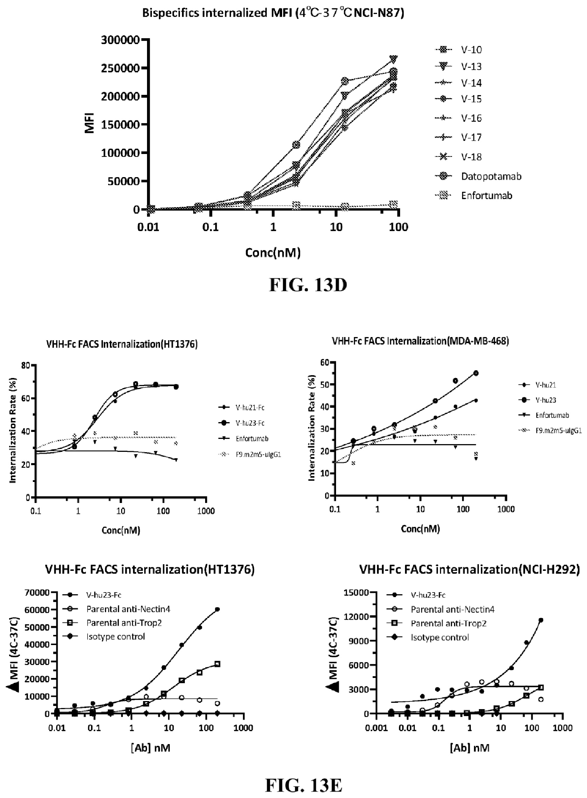

The endocytosis ability of the V BsAbs of the present invention was assayed by selecting the cell lines expressing different target antigens described above. As shown in FIGs. 13A-13E, all the tested V BsAbs showed good endocytic activity, and V-21-Fc and V-hu23-Fc showed synergistic endocytosis effects on the tumor cells (HT1376 and NCI-H292) that were positive for both targets compared with the parent control molecules parental anti-Trop2 and parental anti-Nectin4.

### Binding kinetics by SPR assay

The affinity of the bispecific antibody molecule V-hu21 for human and cynomolgus monkey Trop2 antigens and Nectin4 antigens (Sino Biological) was assayed by the SPR method. The antibody (10 µg/mL) was captured with a Protein A chip for 15 s, and then an antigen diluted in 2-fold gradient was injected at a flow rate of 30 µL/min, with a binding time of 120 s, followed by a dissociation time of 200 s. After each dissociation phase, 10 mM glycine (pH 2.0) was used for chip regeneration. The experimental data were analyzed using a 1:1 binding model.

The affinity results of the candidate molecule V-hu21-Fc for human and cynomolgus monkey Trop2 are shown in Table 23. The results show that the candidate molecule V-hu21-Fc had cross-reactivity with cynomolgus monkey Trop2 and exhibited similar binding and dissociation rates to the human Trop2 antigen and cynomolgus monkey Trop2 antigen.

**Table 23. Affinity of V-hu21-Fc for human and cynomolgus monkey Trop2 antigens**

| Immobilized substance | Analyte | ka (1/Ms) | kd (1/s) | KD (M) |
|---|---|---|---|---|
| V-hu21-Fc | Human Trop2 | 3.45E+05 | 2.09E-02 | 6.06E-08 |
| V-hu21-Fc | Cyno Trop2 | 1.58E+05 | 9.81E-03 | 6.21E-08 |
| V-BMK1 (Datopotamab) | Human Trop2 | 1.32E+05 | 3.15E-03 | 2.38E-08 |
| V-BMK1 (Datopotamab) | Cyno Trop2 | 1.10E+05 | 3.57E-03 | 3.25E-08 |

The affinity results of the candidate molecule V-hu21-Fc for human and cynomolgus monkey Nectin4 are shown in Table 24. The results show that the candidate molecule V-hu21-Fc had cross-reactivity with cynomolgus monkey Nectin4 and exhibited similar binding and dissociation rates to the human Nectin4 antigen and cynomolgus monkey Nectin4 antigen.

**Table 24. Affinity of V-hu21-Fc for human and cynomolgus monkey Nectin4 antigens**

| Capture | Analyte | ka (1/Ms) | kd (1/s) | KD (M) |
|---|---|---|---|---|
| V-hu21-Fc | Human Nectin4 | 5.08E+04 | 9.86E-03 | 1.94E-07 |
| V-hu21-Fc | Cyno Nectin4 | 2.54E+05 | 1.50E-02 | 5.89E-08 |
| V-BMK2 (Enfortumab) | Human Nectin4 | 4.12E+05 | 4.72E-03 | 1.14E-08 |
| V-BMK2 (Enfortumab) | Cyno Nectin4 | 3.83E+05 | 8.31E-03 | 2.17E-08 |

Furthermore, the affinity of the bispecific antibody molecule V-hu23-Fc for human and cynomolgus monkey Trop2 antigens or human and cynomolgus monkey Nectin4 antigens was also assayed by the SPR method. The results are shown in Tables 25 and 26 below.

**Table 25. Affinity of V-hu23-Fc for human and cynomolgus monkey Trop2 antigens**

| **Immobilized substance** | **Analyte** | **ka (1/Ms)** | **kd (1/s)** | **KD (M)** |
|---|---|---|---|---|
| V-hu23-Fc | Human Trop2-His | 1.14E+05 | 9.14E-03 | 8.05E-08 |
| V-hu23-Fc | Cyno Trop2-His | 9.95E+04 | 7.99E-03 | 8.03E-08 |
| V-BMK1 (Datopotamab) | Human Trop2-His | 1.31E+05 | 3.59E-03 | 2.75E-08 |
| V-BMK1 (Datopotamab) | Cyno Trop2-His | 1.10E+05 | 3.54E-03 | 3.23E-08 |

**Table 26. Affinity of V-hu23-Fc for human and cynomolgus monkey Nectin4 antigens**

| **Immobilized substance** | **Analyte** | **ka (1/Ms)** | **kd (1/s)** | **KD (M)** |
|---|---|---|---|---|
| V-hu23-Fc | Human Nectin4-His | 4.34E+04 | 5.94E-03 | 1.37E-07 |
| V-hu23-Fc | Cyno Nectin4-His | 3.77E+04 | 7.18E-03 | 1.90E-07 |
| V-BMK2-Enfortumab | Human Nectin4-His | 3.13E+05 | 3.93E-03 | 1.26E-08 |
| V-BMK2-Enfortumab | Cyno Nectin4-His | 3.26E+05 | 7.38E-03 | 2.27E-08 |

### Developability assessment

The candidate molecules V-hu21, hu23, and hu24-Fc were analyzed for physicochemical properties by SEC-HPLC, CEX-HPLC, HIC-HPLC, and DLS assays. The samples showed physicochemical properties with developability, as shown in Table 27.

**Table 27. DA data on physicochemical properties of V-hu21, hu23, and hu24-Fc**

| Sample name | Concentration (mg/mL) | SEC-HPLC (%) | CEX-HPLC(%) | | | HIC-HPLC(%) | Tm (°C) | Tagg (°C) 266 |
|---|---|---|---|---|---|---|---|---|
| | | Main peak | Acidic peak | Main peak | Basic peak | Main peak (NH4)₂So₄ (M) | | |
| V-hu21-Fc | 8.97 | 98.7 | 9.1 | 87.0 | 4.0 | 0.43 | 54.9 | 60.4 |
| V-hu23-Fc | 1.23 | 95.7 | 17.2 | 78.2 | 4.6 | 0.67 | 61.8 | 65.0 |
| V-hu24-Fc | 0.94 | 92.1 | 24.3 | 72.2 | 3.5 | 0.63 | 55.8 | 63.4 |

### Example 2. Preparation and Characterization of Antibody-Drug Conjugate (ADC)

The linker-payload used in the examples of the present invention is known in the art and/or commercially available. When the depicted structure is not in accordance with the actual situation, modifications to the structure should be permitted according to the actual situation.

### Materials and methods

| **Name** | **Manufacturer** | **Catalog Number** |
|---|---|---|
| Mal-Gly-EXD-D-glucuronic acid | MedChemExpress | HY- 153179 |
| Mal-PEG8-Val-Ala-PAB-Exatecan | MedChemExpress | HY-147271 |
| Deruxtecan | MedChemExpress | HY-13631E |
| Constant Temperature Oscillation Metal Bath | Dlab | HCM100-pro |
| Centrifuge | Thermo Fisher Scientific | Cat# 75002420 |
| Amicon Utral-15 | Millipore | UFC903096 |
| TECP | aladdin | T107252 |

### General synthetic method A

5.45 mg/mL of an antibody (e.g., the corresponding bispecific antibody prepared according to the above antibody preparation example) in a buffer at pH 5.5 containing 20 mM His-hac and 150 mM NaCl was placed in an Eppendorf tube. 6 molar equivalents of TCEP (Tris(2-carboxyethyl) phosphine hydrochloride, 5 mM concentration) was added to the antibody buffer (the molar ratio of TCEP:antibody = 6:1). The Eppendorf tube containing the reaction mixture was placed on a shaker (×500 rpm), and the mixture was reacted at 37 °C for 2 h. An additional 6 molar equivalents of TCEP (5 mM) was added to the mixture. The reaction mixture was placed on a shaker (500 rpm) and then shaken at 37 °C for 2 h. Ultrafiltration (MWCO 30 kd) was then performed to remove TECP, and the buffer was supplemented with 20 mM His-hac buffer to the original volume. 6 molar equivalents of linker-payload (5 mg/mL, in DMA) was added dropwise to the fully reduced antibody, while the DMA concentration was maintained below 20% (v/v). The reaction was maintained on a shaker at room temperature for 2 h (linker-payload:antibody = 6:1). The conversion rate was detected by HIC-HLPC, and purification was performed when the conversion was completed. The reaction mixture was transferred to an ultrafiltration tube (MWCO 30 kd), and 10 mM His-hac (pH 6) was added until the total volume was 450 µL. The sample was centrifuged at 10,000 rpm for 5 min. About 200 µL of the solution remained, which was supplemented with His-hac to 450 µL. The flow-through substances were discarded. The washing step was repeated 6 times. The remaining solution was transferred, and 200 µL of His-hac was added. The filter membrane was rinsed by pipetting up and down, and the ADC conjugate product was obtained through combination.

Pro A resin purification (40 g/L resin loading capacity) was performed. The gravity column packed with the resin was washed with 10 CV of PBS, and then the sample was loaded onto the column. The column was first washed with at least 10 CV of PBS buffer containing 10% DMSO and then washed with at least 50 CV of PBS buffer to completely remove the free payload. The column was eluted with a pH 3 acetic acid solution (concentration 50 mM), and the eluate was immediately neutralized to pH 5.5 to pH 6.0 with 2 M tris buffer (pH 12.0). The elution step was repeated and the flow-through concentration (Nanodrop, A280) was measured until the conjugate was completely eluted. The ADC solutions were combined in an Eppendorf tube. If the final concentration was lower than the expected concentration, the mixture was concentrated to the desired concentration using an ultrafiltration membrane (MWCO 30 kd).

The purity was determined by SEC-HPLC, the average DAR value was determined by HIC-HPLC, and the free linker-payload was determined by RP-HPLC.

### General synthetic method B

An antibody (e.g., the corresponding bispecific antibody prepared according to the above antibody preparation example) at pH = 7.4 in PBS was placed in an Eppendorf tube. 5.7 molar equivalents of TCEP (5 mM concentration) and 2.85 molar equivalents of ZnCl₂ (5 mM concentration) were added to the antibody buffer (TCEP:antibody:ZnCl₂ = 5.7:1:2.85). The Eppendorf tube containing the reaction mixture was placed on a shaker (×500 rpm) at 6 °C for 16 h. 12 molar equivalents of linker-payload (5 mg/mL, in DMA) was added dropwise to the fully reduced antibody, while the DMA concentration was maintained at 10% (v/v). The reaction was maintained on a shaker at 6 °C for 2 h (linker-payload:antibody = 12:1). The conversion rate was detected by HIC-HLPC, and purification was immediately performed when the conversion was completed. The reaction mixture was transferred to an ultrafiltration tube (MWCO 30 kd), and PBS (pH 7.4) was added until the total volume was 450 µL. The sample was centrifuged at 10,000 rpm for 5 min. About 200 µL of the solution remained, which was supplemented with PBS to the original volume. The flow-through liquid was discarded. The washing step was repeated 10 times. 200 µL of PBS was added at the last time, and the filter membrane was rinsed by pipetting up and down. The remaining solutions were combined to obtain the ADC product. If the final concentration was lower than the expected concentration, the mixture was concentrated to the desired concentration using an ultrafiltration membrane (MWCO 30 kd). The purity of ADC was determined by SEC-HPLC, the average DAR value was determined by HIC-HPLC, and the content of the free linker-payload was determined by RP-HPLC.

### General synthetic method C

5.45 mg/mL of an antibody (e.g., the corresponding bispecific antibody prepared according to the above antibody preparation example) in a buffer at pH 5.5 containing 20 mM His-hac and 150 mM NaCl was placed in an Eppendorf tube. 6 molar equivalents of TCEP (Tris(2-carboxyethyl) phosphine hydrochloride, 5 mM concentration) was added to the antibody buffer (TCEP:antibody = 6:1). The Eppendorf tube containing the reaction mixture was placed on a shaker (×500 rpm), and the mixture was reacted at 37 °C for 2 h. An additional 6 molar equivalents of TCEP (5 mM) was added to the mixture. The reaction mixture was placed on a shaker (500 rpm) and then shaken at 37 °C for 2 h. Ultrafiltration (MWCO 30 kd) was then performed to remove TECP, and the buffer was supplemented with 20 mM His-hac buffer to the original volume. 8 molar equivalents of linker-payload (5 mg/mL, in DMA) was added dropwise to the fully reduced antibody, while the DMA concentration was maintained below 20% (v/v). The reaction was maintained on a shaker at room temperature for 2 h (linker-payload:antibody = 8:1). The conversion rate was detected by HIC-HLPC, and purification was performed when the conversion was completed. The reaction mixture was transferred to an ultrafiltration tube (MWCO 30 kd), and 10 mM His-hac (pH 6) containing 10% DMA was added until the total volume was 450 µL. The sample was centrifuged at 10,000 rpm for 5 min. About 200 µL of the solution remained, which was supplemented with His-hac to 450 µL. The flow-through substances were discarded. The washing step was repeated 10 times. Then, 10 mM His-hac (pH 6) was added until the total volume was 450 µL. The washing step was repeated 5 times. Finally, the remaining solution was transferred, and 200 µL of His-hac was added. The filter membrane was rinsed by pipetting up and down. The transferred solution and the filter membrane washing solution were combined to obtain the ADC product.

The purity of ADC was determined by SEC-HPLC, the average DAR value was determined by HIC-HPLC, and the content of the free linker-payload was determined by RP-HPLC.

### General methods and/or parameters for determining or detecting ADCs

### Size exclusion chromatography (SEC) method (for detecting ADC purity).

### Parameters of SEC-HPLC method

| **Chromatographic conditions** | |
|---|---|
| **Column compartment** | 25±1°C |
| **Flow rate** | 0.8 mL/mL |
| **Run time** | 20 min |
| **Sample injection volume** | At least 40 µg |
| **Elution type** | Isocratic elution |
| **Column** | Zenix-C SEC-150, 7.8*300mm 150* |
| **Mobile phase:** | 50mM PB +300 mM NaCl, pH 6.8 ±0.1 |

### Reverse-phase HPLC (RP HPLC) method (for detecting free drug)

### Parameters of RP HPLC method

| **Column** | **Agilent ZORBAX StableBond 300 C3 column, 4.6 × 150 mm, 5 µm** |
|---|---|
| **Detection wavelength** | 280 nm; 214 nm (optional) |
| **Temperature of column compartment:** | 60 °C |
| **Temperature of sampler:** | 15 °C |
| **Flow rate:** | 1.0 mL/min |
| **Sample injection volume:** | 2µg |
| **Mobile phase** | **Mobile phase** A: a solution of 0.1% TFA in H₂O |
| | **Mobile phase** B: a solution of 0.1% TFA in ACN |

Elution was performed according to the following table.

| **Time (min)** | **Mobile phase A (%)** | **Mobile phase B (%)** |
|---|---|---|
| **0.0** | 75.0 | 25.0 |
| **2.0** | 75.0 | 25.0 |
| **10.0** | 5.0 | 95.0 |
| **12.0** | 5.0 | 95.0 |
| **14.0** | 5.0 | 95.0 |
| **16.0** | 80.0 | 20.0 |
| **18.0** | 80.0 | 20.0 |

### HIC-HPLC method (for detecting free antibody and DAR distribution)

### HIC-HPLC conditions:

| **Column** | **MAbPac^{™} HIC-Butyl HPLC column, 5 µm, 4.6 × 100 mm,Thermo SCIENTIFIC** |
|---|---|
| **Detection wavelength** | 280 nm; 214 nm (optional) |
| **Temperature of column compartment:** | 25°C |
| **Temperature of sampler:** | 15°C |
| **Flow rate:** | 0.6 mL/min |
| **Sample injection volume:** | 30-50µg |
| **Mobile phase** | **Mobile phase** A: 1.5 M (NH₄)₂SO₄ and 50 mM PB, pH 7.4 |
| | **Mobile phase** B: 50 mM PB, 25% IPA, pH 7.4 |

Elution was performed according to the following table.

| **Time (min)** | **Mobile phase A (%)** | **Mobile phase B (%)** |
|---|---|---|
| **0.0** | 100.0 | 0.0 |
| **7.0** | 100.0 | 0.0 |
| **23.0** | 0.0 | 100.0 |
| **26.0** | 0.0 | 100.0 |
| **26.5** | 100.0 | 0.0 |
| **35.0** | 100.0 | 0.0 |

### Example 2.1 Preparation of V-hu21-VA-Exd

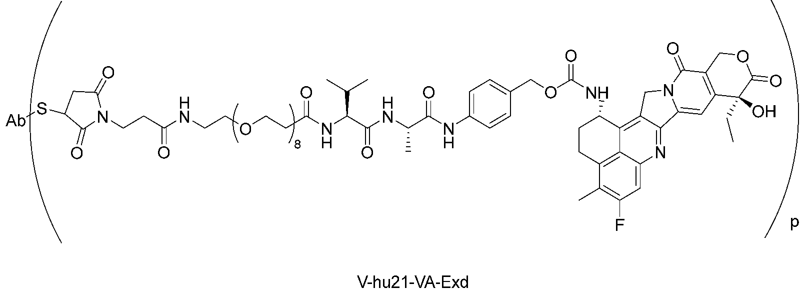

Ab is bispecific antibody V-hu21; p is mainly 4, and the obtained V-hu21-VA-Exd is determined to have an average DAR of 4.0.

V-hu21-VA-Exd was prepared according to general synthetic method A using the bispecific antibody V-hu21 prepared according to the method in the above example and linker-payload Mal-PEG8-Val-Ala-PAB-Exatecan (CAS No.: 2679821-39-5; Cat. No. HY-147271).

### Example 2.2 Preparation of V-hu21-GGFG-Dxd

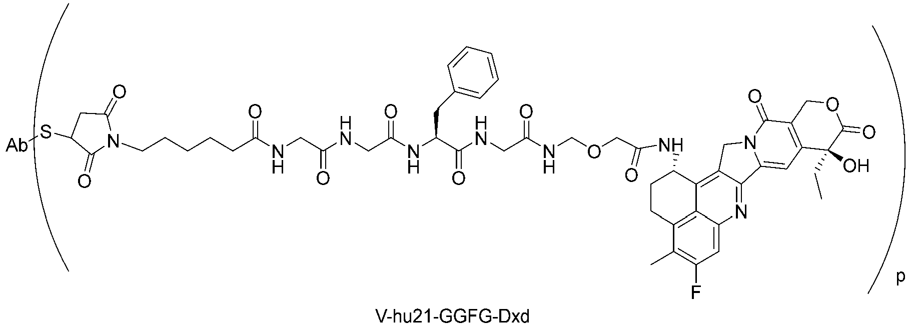

Ab is bispecific antibody V-hu21; p is mainly 4, and the obtained V-hu21-GGFG-Dxd is determined to have an average DAR of 4.0.

V-hu21-GGFG-Dxd was prepared according to general synthetic method A using the bispecific antibody V-hu21 prepared according to the method in the above example and linker-payload Deruxtecan (CAS No.: 1599440-13-7; MCE, HY-13631E). The product purity was 95%.

### Example 2.3 Preparation of V-15-GGFG-Dxd

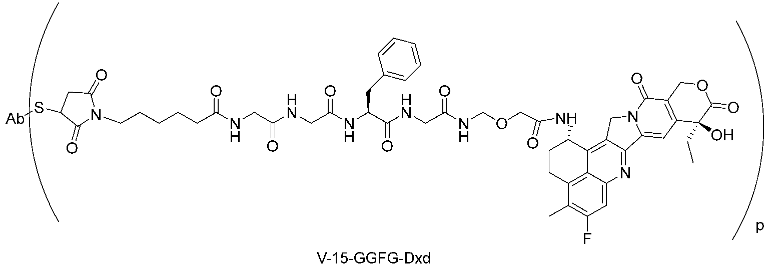

Ab is bispecific antibody V-15; p is mainly 4, and the obtained V-15-GGFG-Dxd is determined to have an average DAR of 4.0.

V-15-GGFG-Dxd was prepared according to general synthetic method A using the bispecific antibody V-15 prepared according to the method in the above example and linker-payload Deruxtecan (CAS No.: 1599440-13-7; MCE, HY-13631E). The product had an average DAR value of 4.0, a yield of 60%, and an SEC-HPLC purity of 95%.

### Example 2.4 Preparation of V-hu23-Glu-Exd

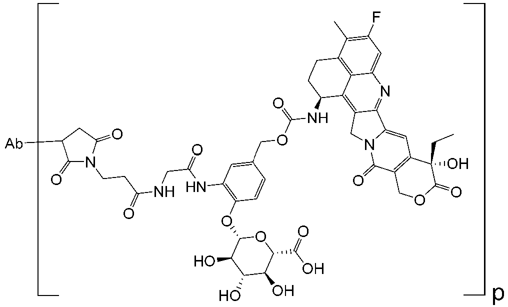

Ab is bispecific antibody V-hu23; p is mainly 4, and the obtained V-hu23-Glu-Exd is determined to have an average DAR of 4.0.

V-hu23-Glu-Exd was prepared according to general synthetic method C using the bispecific antibody V-hu23 prepared according to the method in the above example and linker-payload Mal-Gly-EXD-D-glucuronic acid (CAS No.: 2763252-25-9; MCE, HY-153179). The product had an average DAR value of 4.0 and a yield of 86%.

### Example 2.5 Preparation of Additional ADCs

V-15-VA-Exd, V-hu23-VA-Exd, or V-hu24-VA-Exd was separately prepared according to the method described in Example 2.1 using different antibodies (V-15, V-hu23, or V-hu24) instead of V-hu21.

V-hu24-Glu-Exd was prepared according to the method described in Example 2.4 using a different antibody (V-hu24) instead of V-hu23.

Some information regarding the ADCs prepared in the examples of the present application and the control ADC (commercially available Dato-Dxd) is provided in the table below (Table 28). Dato-Dxd could also be prepared according to general synthetic method B described above.

**Table 28. ADC basic information**

| **Molecule ID** | **Linker-paylaod type and DAR value** | **Conjugation Strategy** | **Extinction coefficient** | **MW** | **ConC.** | **Average Dar value** | **Purity (%)** |
|---|---|---|---|---|---|---|---|
| Dato-Dxd | GGFG-DXD /DAR4 | Cysteine | 1.52 | 150.14 | 1.86 | 3.8 | 99 |
| V-15 -GGFG-DXD | GGFG-Dxd/DAR4 | Cysteine | 1.74 | 92.89 | 3.85 | 4.0 | 95 |
| V-15-VA-Exd | PEG-VA-PAB-EXD/DAR4 | Cysteine | 1.74 | 94.37 | 1.47 | 4.0 | >95 |
| V-hu21-GGFG-DXD | GGFG-Dxd/DAR4 | Cysteine | 1.68 | 96.30 | 2.63 | 4.0 | 95 |
| V-hu21-VA-EXD | PEG-VA-PAB-EXD/DAR4 | Cysteine | 1.68 | 97.48 | 1 | 3.8 | 99 |
| V-hu23-VA-Exd | PEG-VA-PAB-EXD/DAR4 | Cysteine | 1.69 | 97.3 | 1.29 | 4.0 | >95 |
| V-hu24-VA-Exd | PEG-VA-PAB-EXD/DAR4 | Cysteine | 1.68 | 111.3 | 1.48 | 3.9 | >95 |
| V-hu23-Glu-Exd | Mal-Gly-ExD-D-glucuronic acid /DAR4 | Cysteine | 1.69 | 96 | 2.3 | 4.0 | >95 |
| V-hu24-Glu-Exd | Mal-Gly -ExD-D-glucuronic acid /DAR4 | Cysteine | 1.69 | 110.5 | 2.27 | 4.0 | >95 |

In the present disclosure, when referring to linker-payload, Glu is also referred to as Gluc; for example, Glu-Exd is also referred to as Gluc-Exd.

### Example 3. In Vitro Assay of ADCs

### Example 3.1 In Vitro Binding Assay of ADCs

### Method:

1) The target cells NCI-N87 (TCHu130, the Cell Bank of the Chinese Academy of Sciences) or MDA-MB-468 (TCHu136, the Cell Bank of the Chinese Academy of Sciences) were plated at 1-3 × 10⁵ cells/well, and the diluted test antibody or ADC prepared above (100 nM, 3-fold dilution) was added and incubated at 2-8 °C for about 1 h.
2) The mixture was centrifuged at 4 °C, and the supernatant was removed. The cells were resuspended in 200 µL/well of FACS Buffer (1% BSA in PBS) and centrifuged at 4 °C. The cells were resuspended in 200 µL/well of FACS Buffer again and centrifuged at 4 °C.
3) The cells were resuspended in 100 µL/well of flow secondary antibody Anti-Hu IgG Fc-PE (ThermoFisher Scientific) diluted to the target dilution ratio, and incubated at 2-8 °C for about 1 h in the dark.
4) After the mixture was washed twice with 200 µL/well of FACS Buffer, the cells were resuspended in 100 µL/well of FACS Buffer.
5) The MFI of the cells was determined by a flow cytometer (Beckman Coulter).

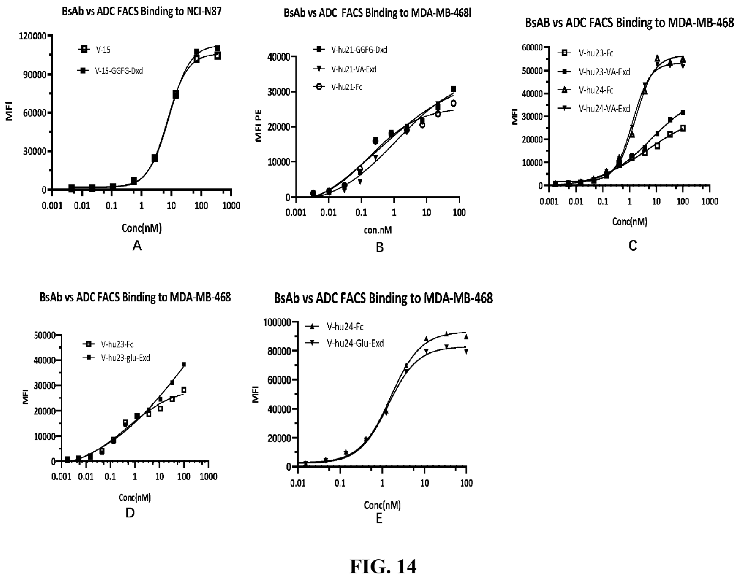

The results are shown in FIGs. 14A-14E. Each ADC molecule exhibited similar cell binding ability to its corresponding naked antibody.

### Example 3.2 In Vitro Killing Assay

### Method 1

1) MDA-MB-468 tumor cells (TCHu136, the Cell Bank of the Chinese Academy of Sciences) or MKN45 tumor cells (CBP60488, Nanjing Cobioer Biosciences Co., Ltd.) as the target cells were plated at 3000-6000 cells/well and at 100 µL/well and incubated in a 5% CO2 incubator at 37 °C overnight; the sample to be tested was diluted with a complete medium to a proper concentration (the starting concentration was 400 nM) and added to the plated 96-well culture plate at 100 µL/well; and the plate was incubated in the 5% CO2 incubator at 37 °C for 3-6 days. 2) CCK-8 was added at 20 µL/well, and the plate was incubated in the 5% CO2 incubator at 37 °C for 1-5 h. 3) The OD450 was read.

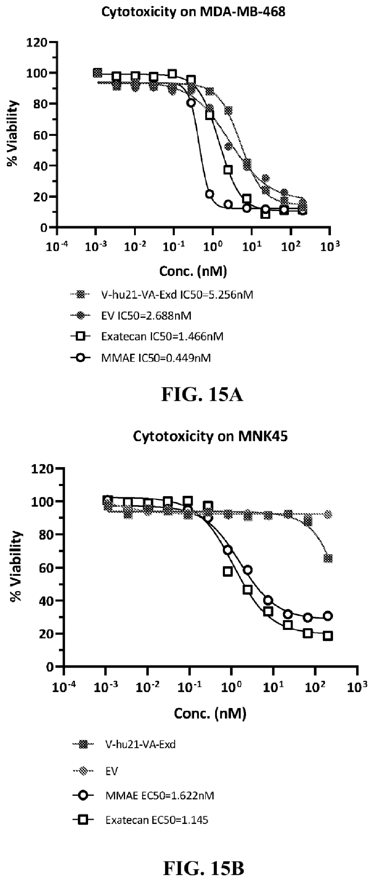

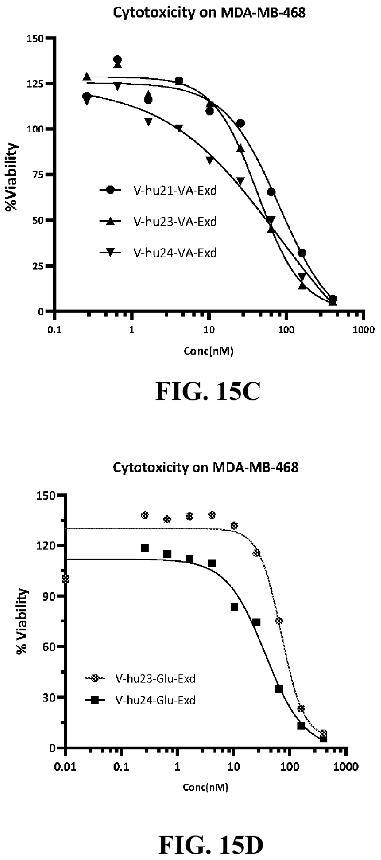

V-hu21-VA-Exd and EV (i.e., Enfortumab Vedotin) were cytotoxic to Nectin4⁺Trop2⁺ MDA-MB-468 cells, but were not cytotoxic to MNK45 cells (Nectin4^{low}Trop2⁻). Payloads Exatecan and MMAE were able to kill the 2 cell lines effectively. The results are shown in FIGs. 15A-15B. V-hu21-VA-Exd, V-hu23-VA-Exd, and V-hu24-VA-Exd were cytotoxic to MDA-MB-468 cells (FIG. 15C). V-hu23-Glu-Exd and V-hu24-Glu-Exd were cytotoxic to MDA-MB-468 cells (FIG. 15D).

### Method 2

The target cells NCI-H292 (human mucoepidermoid pulmonary carcinoma, CBP60187, Nanjing Cobioer Biosciences Co., Ltd.) were plated at 5000 cells/well and at 100 µL/well, and incubated in the 5% CO₂ incubator at 37 °C overnight. rProtein G-MMAE (Levena; LEV-GME-100) was diluted to 200 nM with a complete medium. The target antibody (including the V-hu23-Fc antibody, the parental anti-Nectin4 antibody, and the isotype antibody involved in Table 22) was diluted to 200 nM with a complete medium. The target antibody and rProtein G-MMAE were mixed in a volume ratio of 1:1 (the molar concentration ratio was 1:1), and the mixture was incubated at room temperature for about 1 h. The mixed solution of the target antibody and rProtein G-MMAE was diluted in a ratio of 1:2.5 with a complete medium, diluted in gradient, and added to the plated 96-well culture plate at 100 µL/well. The plate was incubated in the 5% CO₂ incubator at 37 °C for 3-6 days. CCK-8 (Cell Counting Kit-8) was added at 20 µL/well, and the plate was incubated in the 5% CO₂ incubator at 37 °C for 1-5 h. The OD450 was read.

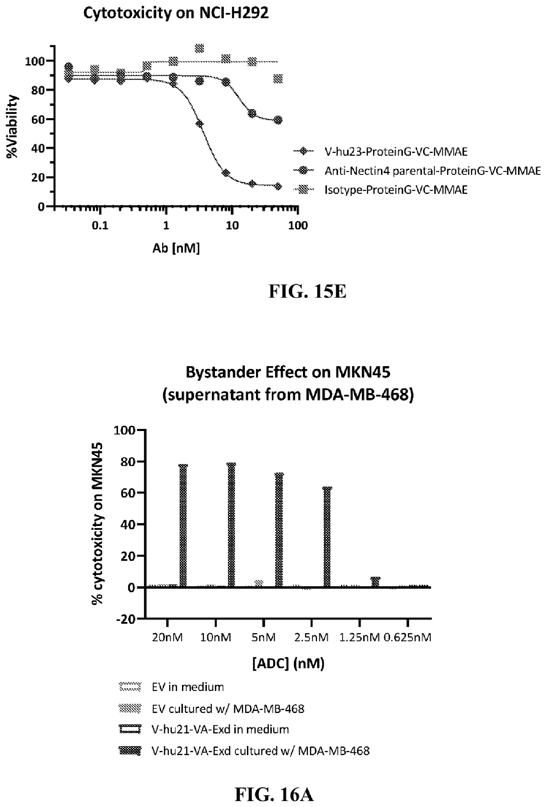

As can be seen from FIG. 15E, the killing ability of V-hu23-Protein G-MMAE against the Trop2/Nectin4 double-positive tumor cell line NCI-H292 was superior to that of the anti-Nectin4 parental control molecule, indicating a synergistic killing effect of anti-Trop2 and anti-Nectin4 on double-positive tumor cells.

### Example 3.3 Bystander Effect

Experiment 1: An ADC at a specified concentration was added to target antigen-positive (Ag+) cells (MDA-MB-468) or a medium, and the cells were cultured for 4 days. 100 µL of the culture supernatant was transferred to target antigen-negative (Ag-) cells (MKN45) in 100 µL of the medium. The Ag- cells were cultured in a 5% CO₂ incubator at 37 °C for 3-4 days. The viability of the Ag- cells was determined by adding CCK8.

For the target antigen-negative tumor cells (MKN45), V-hu21-VA-Exd had a stronger bystander effect than EV (i.e., Enfortumab Vedotin). At 2.5 nM-20 nM, the killing ratio was 60%-80% VS < 10%. The results are shown in FIG. 16A. In the figure, "EV in medium" and "V-hu21-VA-Exd in medium" represent supernatants from the media to which the corresponding ADC drugs were added, respectively; "EV cultured w/ MDA-MB-468" and "V-hu21-VA-Exd cultured w/ MDA-MB-468" represent supernatants from the MDA-MB-468 cell media to which the corresponding ADC drugs were added, respectively.

Experiment 2: After Ag+ cells T47D (TCHu 87, the Cell Bank of Type Culture Collection Committee of the Chinese Academy of Sciences) and Ag- cells MKN45 were stained with fluorescent dyes CFSE (Invitrogen, 65-0850-84) and CellTrace Violet (Thremo, C34557 A), respectively, positive cells T47D were plated at 75000 cells/250 µL/well, and negative cells MKN45 were plated at 25000 cells/250 µL/well. The two types of cells were mixed for culture and incubated in a 5% CO2 incubator at 37 °C overnight. The samples to be tested were diluted to 4 nM and 2 nM with a complete medium and added to the plated 24-well culture plates at 500 µL/well, and the plates were incubated in the 5% CO2 incubator at 37 °C for 3 days. Subsequently, all cells were collected into deep-well plates, and counting beads (invitrogen, C36995) were added at 3 µL/well. The plates were centrifuged at 400 g for 5 min, and then the cells were washed twice with DPBS. The collected cells were stained with Live/Dead dye (Thremo, L34976). After staining was completed, the cells were washed twice with 2% FBS-DPBS buffer, and the cells were then resuspended and detected on a flow cytometer (Beckman, CytoFLEX). Fluorescence signals in the V450/B525/R780 channel were recorded.

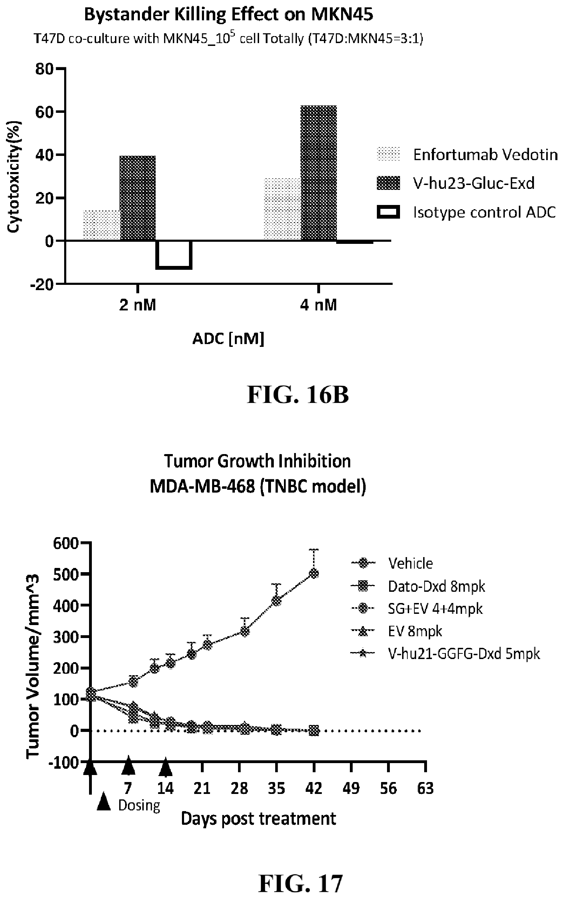

The results are shown in FIG. 16B. The toxic small molecule released by enzymatic digestion after V-hu23-Glu-Exd was endocytosed by positive cells T47D demonstrated a significant bystander killing effect. The bystander killing effect of V-hu23-Glu-Exd was significantly superior to that of the control enfortumab vedotin (EV).

### Example 4. In Vivo Effect Assay of ADCs

The abbreviations used have the following meanings unless otherwise specified and contradicted by context.

| Abbreviation | Full Name |
|---|---|
| ANOVA | Analysis of variance |
| BIW | Twice a week |
| QW | Once a week |
| BW | Body weight |
| D | Day |
| *i.v.* | Intravenous injection |
| *G* | Group |
| mpk | mg (drug) per kg (body weight) |
| N/A | Not available or not applicable |
| PBMC | Peripheral blood mononuclear cell |
| SD | Standard deviation |
| SEM | Standard error |
| SPF | Specific pathogen-free |
| TBD | To be determined |
| TGI | Tumor growth inhibition |
| T-Test | Independent sample T test |
| TV | Tumor volume |
| TW | Tumor weight |

### Example 4.1 Anti-Tumor Effect of ADCs in MDA-MB-468 Subcutaneous Xenograft Mouse Model

This experiment was used to evaluate the efficacy of the test sample (including the ADCs of the present invention) in a human breast cancer cell MDA-MB-468 subcutaneous xenograft model of NCG mice.

### Experiment A

### Preparation information of test samples

| **Administration group** | **Administration dose (mg/kg)** | **Drug concentration (mg/mL)** | **Preparation method** |
|---|---|---|---|
| Dato-Dxd | 8 | 0.8 | Diluted with sodium glutamate buffer |
| SG+EV | 4+4 | 0.4 | Diluted with sodium glutamate buffer |
| EV | 8 | 0.8 | Diluted with sodium glutamate buffer |
| V-hu21-GGFG-Dxd | 5 | 0.5 | Diluted with sodium glutamate buffer |

### Model construction and grouping

**Model construction:** Female NCG mice aged 7-8 weeks (Balb/c nu/nu, from Jiangsu GemPharmatech Co., Ltd.) were taken, acclimatized for 1 week, and injected with MDA-MB-468 (TCHu136, the Cell Bank of the Chinese Academy of Sciences) PBS-resuspended cell solution (1 × 10⁷ cells/animal) at the right scapula site. When the tumor volume reached 100-200 cm³, the mice were randomly divided into 5 groups (n = 4) according to the average tumor volume. The day of grouping was defined as D0. The test sample was administered intravenously on D1. The administration was performed once a week for 3 expected times.

**Administration volume:** It was adjusted according to the mouse body weight (mouse administration volume = 5 µL/g or 10 µL/g × mouse body weight (g)).

**Data acquisition:** After the start of administration, the mice were weighed twice a week, the tumor volume was measured twice a week, and the animals were observed twice a day.

### Experimental endpoint:

The endpoint was determined based on the tumor volume (1500-2000 cm³) or the states of the animals. All living animals were euthanized at the endpoint, and the tumors were collected, photographed, and weighed for subsequent treatment.

### Endpoint analysis

At the end of the experiment, the following indexes were analyzed:
Change in tumor volume (TGI_{TV})
Tumor weight (TGI_{TW})
Change in body weight
Calculation formula of TGI_{TV:} ${TGI}_{TV} = \left\{1-\left[\left({V}_{t}-{V}_{0}\right)/\left({C}_{t}-{C}_{0}\right)\right]\right\} \times 100 %$
*Vₜ* means the average tumor volume of the mice in the test sample administration group on day t;
*V*₀ means the average tumor volume of the mice in the test sample administration group on day 0;
*Cₜ* means the average tumor volume of the mice in the vehicle group on day t;
*C*₀ means the average tumor volume of the mice in the vehicle group on day 0.

### Statistical analysis

The data were analyzed, processed, and reported. The quantitative indexes were described using Mean±standard error (Mean±SEM/SD). Quantitative indexes were analyzed using one-way ANOVA, two-way ANOVA, or the like. Comparisons between groups were made using T test, and p < 0.05 was considered statistically significant. The results were analyzed with both statistical and biological senses taken into account. The plotting software was GraphPad Prism 8.

The V-hu21-GGFG-Dxd 5 mpk group showed comparable efficacy to that of the control drug EV (EV-PADCEV^{®} (Enfortumab vedotin)), combined administration of SG (SG- Trodelvy^{®} (Sacituzumab govitecan)) and EV, and Dato-Dxd. The experimental results are shown in FIG. 17. It should be understood that, unless otherwise specified, the control drugs EV and SG in the other experiments of the present disclosure were the same products as EV (EV- Padcev^{™} (enfortumab vedotin); manufacturer: Astellas/Seagen; Lot No. 3PA0031) and SG (Trodelvy^{™} (Sacituzumab govitecan); manufacturer: Gilead; Lot No. S22B002D) of this experiment.

### Experiment B

### Model construction and grouping

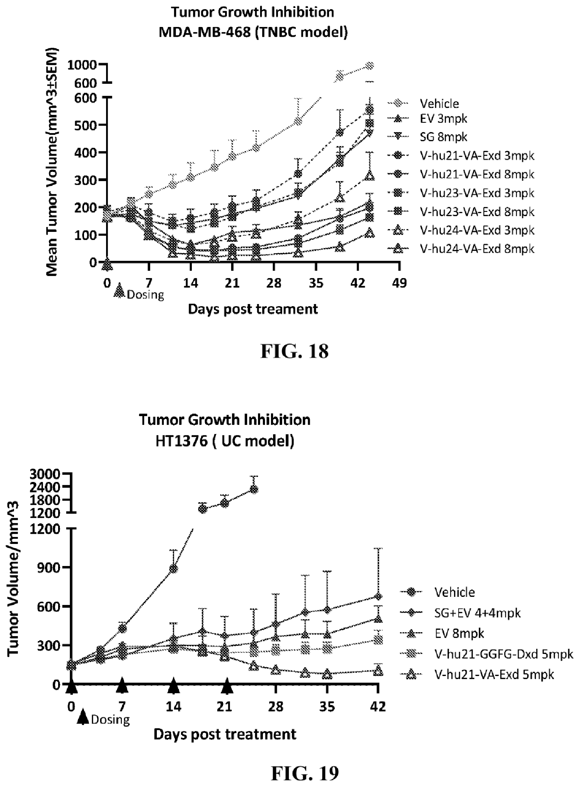

Model construction: Female NCG mice aged 7-8 weeks (Balb/c nu/nu, from Jiangsu GemPharmatech Co., Ltd.) were acclimatized for 1 week and injected with MDA-MB-468 PBS-resuspended cell solution (1 × 10⁷ cells/animal) at the right scapula site. When the tumor volume reached 100-200 cm³, the mice were randomly divided into 9 groups (n = 4) according to the average tumor volume. The day of grouping was defined as D0. The test sample was administered intravenously on D1 in a single dose. See FIG. 18 for the dose administered to each group.

Administration volume: It was adjusted according to the mouse body weight (mouse administration volume = 5 µL/g or 10 µL/g × mouse body weight (g)).

Data acquisition: After the start of administration, the mice were weighed twice a week, the tumor volume was measured twice a week, and the animals were observed twice a day.

Data processing was performed with reference to experiment A. The results are shown in FIG. 18.

Compared with the control drug SG, V-hu21-VA-Exd, V-hu23-VA-Exd, and V-hu24-VA-Exd all had better efficacy at the same dose.

### Example 4.2 Anti-tumor Effects of V-ADCs in HT1376 Model

Model construction: Female NCG mice aged 7-8 weeks (Balb/c nu/nu, from Jiangsu GemPharmatech Co., Ltd.) were acclimatized for 1 week and injected with HT-1376 (CBP60310, Nanjing Cobioer Biosciences Co., Ltd.) PBS-resuspended cell solution (5 × 10⁶ cells/animal) at the right scapula site. When the tumor volume reached 100-200 mm³, the mice were randomly divided into 5 groups (n = 4) according to the average tumor volume. The day of grouping was defined as D0. The test sample was administered intravenously on D1. The administration was performed once a week for a total of 4 times. See FIG. 19 for the dose administered to each group.

Administration volume: It was adjusted according to the mouse body weight (mouse administration volume = 5 µL/g or 10 µL/g × mouse body weight (g)).

Data acquisition: After the start of administration, the mice were weighed twice a week, the tumor volume was measured twice a week, and the animals were observed twice a day.

Data processing was performed with reference to Example 4.1. The results are shown in FIG. 19.

V-hu21-GGFG-Dxd and V-hu21-VA-Exd both showed more excellent effects than that of the control drugs EV (EV-PADCEV^{®} (Enfortumab vedotin)) and combined administration of SG (SG- Trodelvy^{®} (Sacituzumab govitecan)) and EV.

### Example 4.3 Anti-Tumor Effects of V-ADCs in HT1197 Model

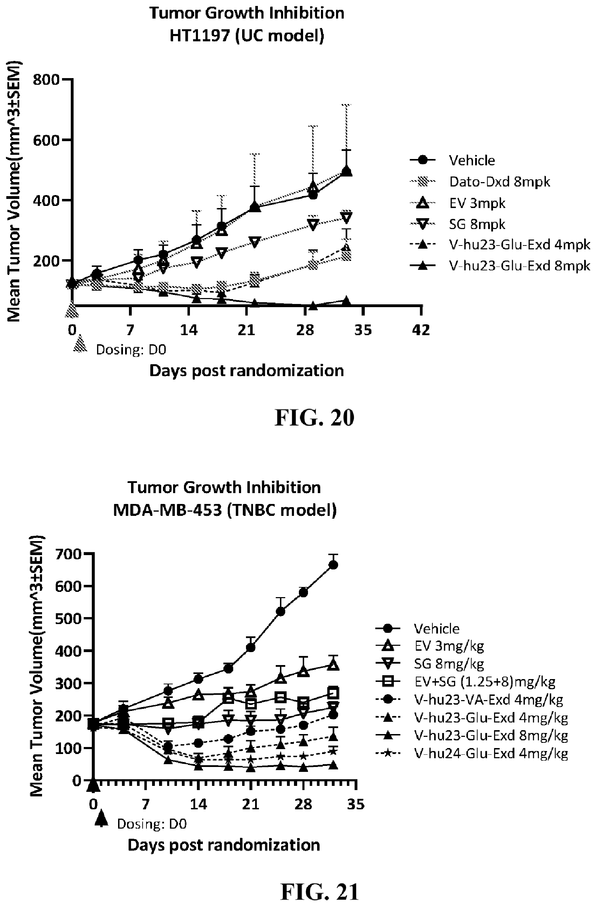

HT1197 model construction: Female BALB/c nu/nu mice aged 7-8 weeks (from Shanghai Lingchang Biotechnology Co., Ltd.) were acclimatized for 3-7 days and injected with HT-1197 (CBP60673, Nanjing Cobioer Biosciences Co., Ltd.) PBS along with 30% matrigel-resuspended cell solution (1 × 10^7 cells/animal) at the right scapula site. When the tumor volume reached 100-200 mm^3, the mice were randomly divided into 6 groups (n = 4) according to the average tumor volume. The day of grouping was defined as D0. The test sample was administered intravenously on D0 with a total of 1 administration. The dose administered to each group is shown in FIG. 20. Reference was made to Example 4.1 for administration and data acquisition and processing. The experimental results show that the cell line HT1197 used in the model expressed a low level of Nectin4 antigen. Therefore, the model also shows resistance to EV. The efficacy of V-hu23-Glu-Exd 4 mpk was comparable to that of Dato-Dxd 8 mpk, but both were superior to those of EV 3 mpk (MTD dose) and SG 8 mpk.

### Example 4.4 Anti-Tumor Effects of V-ADCs in MDA-MB-453 Model

MDA-MB-453 model construction: Female NSG mice aged 7-8 weeks (from Shanghai Model Organisms Center, Inc.) were acclimatized for 3-7 days and injected with MDA-MB-453 (CBP60386, Nanjing Cobioer Biosciences Co., Ltd.) PBS along with 30% matrigel-resuspended cell solution (1 × 10^7 cells/animal) at the right scapula site. When the tumor volume reached 110-210 mm^3, the mice were randomly divided into 8 groups (n = 4) according to the average tumor volume. The day of grouping was defined as D0. The test sample was administered intravenously on D0 with a total of 1 administration. See FIG. 21 for the dose administered to each group. Reference was made to Example 4.1 for data acquisition and processing. The experimental results show that the efficacy of V-hu23-VA-Exd, V-hu23-Glu-Exd, and V-hu24-Glu-Exd at the dose of 4 mpk was better than that of EV 3 mpk (MTD dose), SG 8 mpk, and EV + SG (1.25 + 8 mpk, clinical dose).

### Example 4.5 Anti-Tumor Effects of V-ADCs in Human Head and Neck Cancer In Vivo Xenograft Tumor LD1-0023-200615 Model

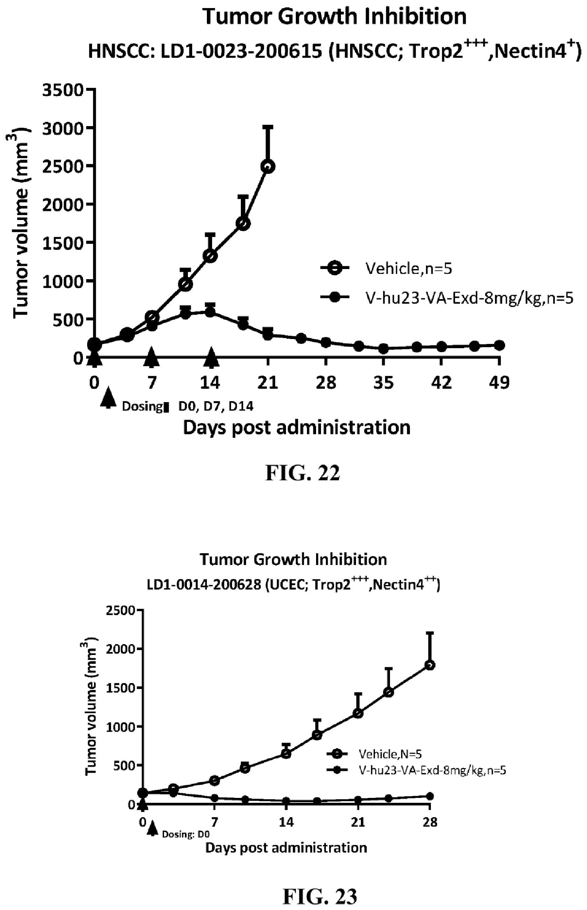

Model construction: Female NU/NU mice aged 7-8 weeks (Beijing Vital River Laboratory Animal Technology Co., Ltd.) were acclimatized for 1 week, and then the established human tumor tissues (Shanghai LIDE Biotech., Co. Ltd.) were subcutaneously inoculated into immunodeficient mice. The mice were continuously observed until tumorigenesis was achieved and the tumor volume reached about 500-800 mm³. The tumor tissues were separated and uniformly cut into tissue pieces of about 3 mm × 3 mm × 3 mm. Then, the tumor tissue pieces were inoculated subcutaneously into the mice, and the tumor growth was observed. When the average tumor volume reached about 100-200 mm³ (average value: 120±30 mm³), the tumor-bearing mice were randomly divided into 2 groups (n = 5). The day of grouping was defined as D0. The test sample was administered intravenously on D0 by QW×3. See FIG. 22 for the dose administered to each group.

Data processing was performed with reference to Example 4.1. The results are shown in FIG. 22. At a dose of 8 mg/kg, the efficacy of V-hu23-VA-Exd could reach TGI 94.56% on day 28 after administration, and the tumor-inhibiting effect could be maintained for at least 49 days.

### Example 4.6 Anti-Tumor Effects of V-ADCs in Human Endometrial Cancer In Vivo Xenograft Tumor LD1-0014-200628 Model

Model construction: Female NU/NU mice aged 7-8 weeks (Beijing Vital River Laboratory Animal Technology Co., Ltd.) were acclimatized for 1 week, and then the established human tumor tissues (Shanghai LIDE Biotech., Co. Ltd.) were subcutaneously inoculated into immunodeficient mice. The mice were continuously observed until tumorigenesis was achieved and the tumor volume reached about 500-800 mm³. The tumor tissues were separated and uniformly cut into tissue pieces of about 3 mm × 3 mm × 3 mm. Then, the tumor tissue pieces were inoculated subcutaneously into the mice, and the tumor growth was observed. When the average tumor volume reached about 100-200 mm³ (average value: 120±30 mm³), the tumor-bearing mice were randomly divided into 2 groups (n = 5). The day of grouping was defined as D0. The test sample was administered intravenously on D0 at a single dose. The dose administered to each group is shown in FIG. 23.

Data processing was performed with reference to Example 4.1. The results are shown in FIG. 23. At a dose of 8 mg/kg, the efficacy of V-hu23-VA-Exd could reach TGI 130.47% on day 28 after administration.

### Example 4.7 Anti-Tumor Effects of V-ADCs in Human Cervical Cancer In Vivo Xenograft Tumor LD1-0010-200615 Model

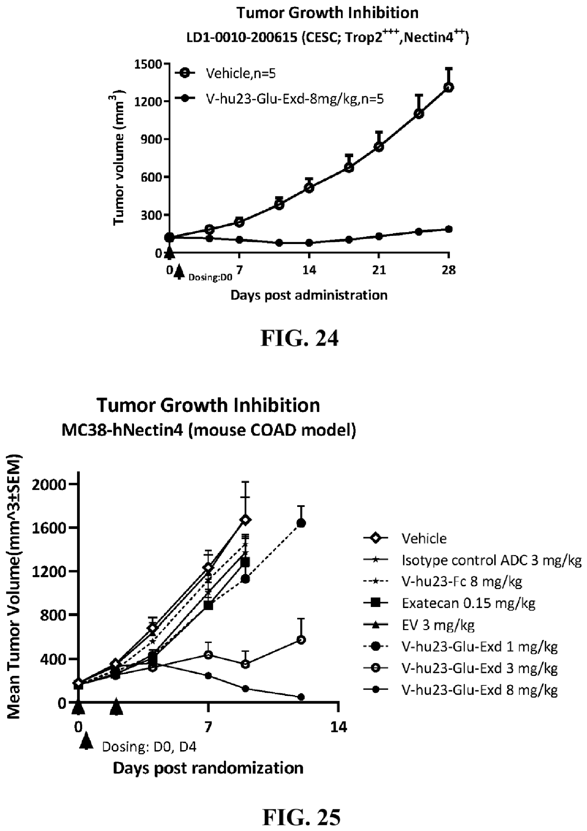

Model construction: Female NU/NU mice aged 7-8 weeks (Beijing Vital River Laboratory Animal Technology Co., Ltd.) were acclimatized for 1 week, and then the established human tumor tissues (Shanghai LIDE Biotech., Co. Ltd.) were subcutaneously inoculated into immunodeficient mice. The mice were continuously observed until tumorigenesis was achieved and the tumor volume reached about 500-800 mm³. The tumor tissues were separated and uniformly cut into tissue pieces of about 3 mm × 3 mm × 3 mm. Then, the tumor tissue pieces were inoculated subcutaneously into the mice, and the tumor growth was observed. When the average tumor volume reached about 100-200 mm³ (average value: 120±30 mm³), the tumor-bearing mice were randomly divided into 2 groups (n = 5). The day of grouping was defined as D0. The test sample was administered intravenously on D0 at a single dose. The dose administered to each group is shown in FIG. 24.

Data processing was performed with reference to Example 4.1. The results are shown in FIG. 24. At a dose of 8 mg/kg, the efficacy of V-hu23-Glu-Exd could reach TGI 94.37% on day 28 after administration.

### Example 4.8 Anti-Tumor Effects of V-ADCs in Murine MC38-hNectin4 Model

Model construction: Female BALB/c nude mice aged 7-8 weeks (Beijing Vital River Laboratory Animal Technology Co., Ltd.) were acclimated for 1 week and injected with MC38-hNectin4 (MC38 cells stably expressing human Nectin4, CBPG0035, Nanjing Cobioer Biosciences Co., Ltd.) PBS-resuspended cell solution (1 × 10⁶ cells/animal) at the right scapula site. When the tumor volume reached 100-250 mm³, the mice were grouped according to the average tumor volume.

The preparation information of the test samples is shown in the table below:

| **Drug** | **Preparation method** | **Administration concentration (mg/mL)** |
|---|---|---|
| Vehicle group | 10 mM glutamate, 8% (w/v) sucrose, pH 5.0 | -- |
| V-hu23-Glu-Exd | Be diluted with vehicle to target concentration | 0.1, 0.3, and 0.8 |
| EV | Be diluted with 10 mM His buffer (pH 6.2) to target concentration | 0.3 |
| Isotype-control ADC | Be diluted with vehicle to target concentration | 0.3 |
| V-hu23-Fc | Be diluted with vehicle to target concentration | 0.8 |
| Exatecan | 10%DMSO/5%Tween80/85% dd water | 0.03 |

Administration volume: It was adjusted according to the mouse body weight (mouse administration volume = 5 or 10 µL/g × mouse body weight (g)). Data acquisition: After the start of administration, the mice were weighed 2-3 times a week, the tumor volume was measured 2-3 times a week, and the animals were observed twice a day.

The tumor-bearing mice were randomly divided into 8 groups (n = 5). The day of grouping was defined as D0. The test sample was administered intravenously on D0 and D4 with a total of 2 administrations. See FIG. 25 for the dose administered to each group.

Data processing was performed with reference to Example 4.1. The results are shown in FIG. 25. After administration, the animals in all dose groups were well tolerated; MC38-hNectin4 cells expressed a high level of multi-drug resistance protein MDR1, and were drug-resistant cells for EV. Therefore, EV did not show a significant anti-tumor effect. Meanwhile, neither the isotype control ADC, the small molecule exatecan (Cat. No. HY-13631, MedChemExpress LLC, with the same amount of exatecan as V-hu23-Glu-Exd 8 mpk contained) nor the naked antibody V-hu23-Fc showed significant anti-tumor effects. However, the ADC V-hu23-Glu-Exd high-, medium-, and low-dose groups of the present invention all had significant tumor-inhibiting effects, which were dose-dependent. Under the test conditions, V-hu23-Glu-Exd had significantly higher potency than EV.

### Example 5. In Vivo Imaging to Study Biodistribution of Fluorescently Labeled Bispecific Antibodies

Fluorescence *in vivo* imaging experiments were performed using the IVIS Lumina Series III system to evaluate the differences in biodistribution and tumor targeting ability between the bispecific antibodies of the present invention and conventional monoclonal antibodies. These experiments were performed in Balb/c nu/nu nude mice bearing the human urothelial tumor cell line HT1376 implanted subcutaneously on one side. During the experiment, female mice (25-32 g) were 6-8 weeks old, and the tumor volume was about 500 mm³.

Preparation of test sample: The buffer of the test drug was replaced with PBS at pH 8.5 using a centrifugal filter (Millipore). Then, the labeling reaction was performed by immediately mixing Sulfo-Cy7 NHS Ester (Duofluor, 10 mg/mL) with antibodies (V-15 and Enfortumab)) at a molar ratio of 6:1 (dye/protein), and the reaction was performed at 20-25 °C for 5 h in the dark. After labeling, the conjugate was purified using a centrifugal filter (3K) to remove unbound free dye. After the binding reaction, the dye/protein molar ratios of V-15-Cy7 and Enfortumab-Cy7 were 1.2 and 2.4, respectively. The *in vitro* binding affinity was assayed by FACS, and it was evaluated that the binding did not affect protein properties after the drug was incubated with the cells for 1 h. Subsequently, 1 nmol dye-labeled protein was injected into the tumor-bearing mice, and the concentration was based on the concentration of the dye. *In vivo* imaging was performed using the IVIS Lumina III system at the time points of 30 min, 1 h, 2 h, 3 h, 4 h, 5 h, 6 h, 8 h, 24 h, 48 h, and 72 h. Data were analyzed using the *in vivo* imaging software (version: 4.7.4).

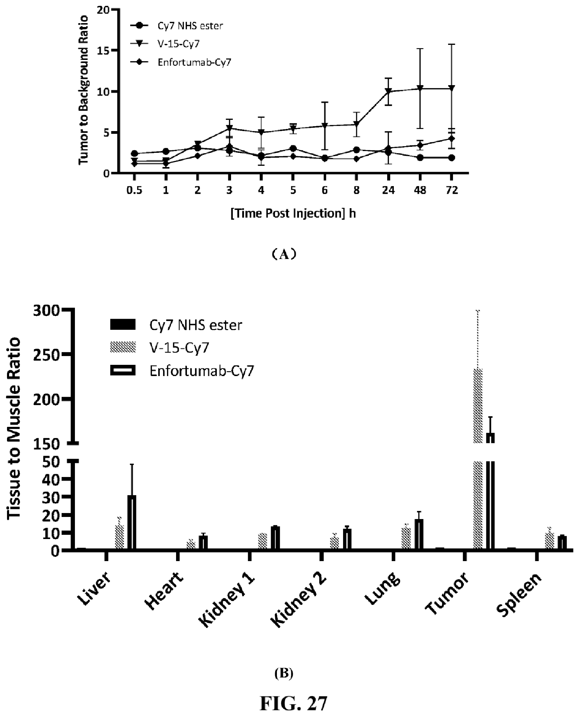

The results are shown in FIG. 27A. Over time, V-15-Cy7 accumulated in the tumor tissues of mice, and the accumulation in the tumor tissues of mice was greater than that of Enfortumab-Cy7. FIG. 27B shows that 72 h after the fluorescent dye-labeled protein was injected into tumor-bearing mice, the drug was distributed to multiple tissues with different tissue penetration abilities. The tumor-to-muscle tissue accumulation ratios of V-15-Cy7 and Enfortumab-Cy7 at 72 h were 233 and 162, respectively, indicating that V-15-Cy7 has better tumor accumulation. However, in other normal tissues (except kidney tissues), the accumulation of V-15-Cy7 was slightly lower than that of Enfortumab-Cy7, indicating that the molecule of the present invention has better tumor targeting property.

### Sequence listing overview

| SEQ ID NO: | Sequence description | Sequence |
|---|---|---|
| | | Variable region sequence of reference antibody |
| 1 | Datopotamab VH | QVQLVQSGAEVKKPGASVKVSCKASGYTFTTAGMQWVR QAPGQGLEWMGWINTHSGVPKYAEDFKGRVTISADTSTST AYLQLSSLKSEDTAVYYCARSGFGSSYWYFDVWGQGTLV TVSS |
| 2 | Datopotamab VL | DIQMTQSPSSLSASVGDRVTITCKASQDVSTAVAWYQQKP GKAPKLLIYSASYRYTGVPSRFSGSGSGTDFTLTISSLQPED FAVYYCQQHYITPLTFGQGTKLEIK |
| 142 | Datopotamab heavy chain | QVQLVQSGAEVKKPGASVKVSCKASGYTFTTAGMQWVR QAPGQGLEWMGWINTHSGVPKYAEDFKGRVTISADTSTST AYLQLSSLKSEDTAVYYCARSGFGSSYWYFDVWGQGTLV TVSSASTKGPSVFPLAPSSKSTSGGTAALGCLVKDYFPEPV TVSWNSGALTSGVHTFPAVLQSSGLYSLSSVVTVPSSSLGT QTYICNVNHKPSNTKVDKRVEPKSCDKTHTCPPCPAPELLG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRE EMTKNQVSLTCLVKGFYPSDIAVEWESNGQPENNYKTTPP VLDSDGSFFLYSKLTVDKSRWQQGNVFSCSVMHEALHNH YTQKSLSLSPGK |
| 143 | Datopotamab light chain | DIQMTQSPSSLSASVGDRVTITCKASQDVSTAVAWYQQKP GKAPKLLIYSASYRYTGVPSRFSGSGSGTDFTLTISSLQPED FAVYYCQQHYITPLTFGQGTKLEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSSP VTKSFNRGEC |
| 3 | Enfortumab VH | EVQLVESGGGLVQPGGSLRLSCAASGFTFSSYNMNWVRQ APGKGLEWVSYISSSSSTIYYADSVKGRFTISRDNAKNSLSL QMNSLRDEDTAVYYCARAYYYGMDVWGQGTTVTVSS |
| 4 | Enfortumab VL | DIQMTQSPSSVSASVGDRVTITCRASQGISGWLAWYQQKP GKAPKFLIYAASTLQSGVPSRFSGSGSGTDFTLTISSLQPEDF ATYYCQQANSFPPTFGGGTKVEIK |
| 144 | Enfortumab heavy chain | EVQLVESGGGLVQPGGSLRLSCAASGFTFSSYNMNWVRQ APGKGLEWVSYISSSSSTIYYADSVKGRFTISRDNAKNSLSL QMNSLRDEDTAVYYCARAYYYGMDVWGQGTTVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKRVEPKSCDKTHTCPPCPAPELLGGPSVFL FPPKPKDTLMISRTPEVTCVVVDVSHEDPEVKFNWYVDGV EVHNAKTKPREEQYNSTYRVVSVLTVLHQDWLNGKEYKC KVSNKALPAPIEKTISKAKGQPREPQVYTLPPSREEMTKNQ VSLTCLVKGFYPSDIAVEWESNGQPENNYKTTPPVLDSDGS FFLYSKLTVDKSRWQQGNVFSCSVMHEALHNHYTQKSLS LSPGK |
| 145 | Enfortumab light chain | DIQMTQSPSSVSASVGDRVTITCRASQGISGWLAWYQQKP GKAPKFLIYAASTLQSGVPSRFSGSGSGTDFTLTISSLQPEDF ATYYCQQANSFPPTFGGGTKVEIKRTVAAPSVFIFPPSDEQL KSGTASVVCLLNNFYPREAKVQWKVDNALQSGNSQESVT EQDSKDSTYSLSSTLTLSKADYEKHKVYACEVTHQGLSSP VTKSFNRGEC |
| | | Anti-Trop2 alpaca VHH sequence and CDR sequence defined by the AbM scheme |
| 5 | A04 VHH | QLQLVESGGGLVQPGGSLRLSCAASALTFKDYAMTWFRQ APGKEREGVSCVSSGGATTYADSVKGRFTISRDNAKNTVY LQMNSLQPEDTAVYYCAASWGYGLRSLTRKEYDYWGQG TQVTVSS |
| 6 | A04 CDR1 | ALTF**K**DYA**M**T |
| 7 | A04 CDR2 | **C**VSSGGATT |
| 8 | A04 CDR3 | S**W**GYGLRSLTR**K**EYDY |
| 9 | G08 VHH | EVQLVESGGGLVQPGGSLTLSCAAPERIFSVYRMYWYRQA PGKQREWVAAITTSGSADYKDSVKGRFTISRDNAKNMAY LQMNSLKPDDTAVYYCNAGGGPGYYGQGTQVTVSS |
| 10 | G08 CDR1 | ERIFSVYR**M**Y |
| 11 | G08 CDR2 | AITTSGSAD |
| 12 | G08 CDR3 | GGGPGY |
| 13 | H03 VHH | QLQLVESGGGLVQPGGSLRLSCAASGFTFSTYAMSWARQA PGKGLEWVSGIYGDGSGTYYADSVKGRFTISRDNAKNTLY LQMNSLKPEDTALYYCAQGYSDYDRRVGEYDYWGQGTQ VTVSS |
| 14 | H03 CDR1 | GFTFSTYA**M**S |
| 15 | H03 CDR2 | GIY**GDG**SGTY |
| 16 | H03 CDR3 | GYSDYDRRVGEYDY |
| | | Anti-Nectin4 alpaca VHH sequence and CDR sequence defined by the AbM scheme |
| 17 | F9 VHH | QVQLVESGGGLVQPGGSLRLSCAASGSTFGINVMGWYRQ APGKQREYVASITRDGSINYADSVRGRFTISRDNAKSTVYL QMNSLKPDDTAVYYCNADRLGPTGWGSRIELWGQGTQVT VSS |
| 18 | F9 CDR1 | GSTFGINV**M**G |
| 19 | F9 CDR2 | SITR**DG**SIN |
| 20 | F9 CDR3 | DRLGPTG**W**GSRIEL |
| 21 | F12 VHH | QVQLVESGGGLVQPGGSLRLSCAASGSLFSINVMGWYRQP PGKKRELVATISSGGSTKYADSVKGRFTISGDNAKNTYLQ MNTLKPEDTAVYYCHAYSDWDFEEYWGQGTQVTVSS |
| 22 | F12 CDR1 | GSLFSINV**M**G |
| 23 | F12 CDR2 | TISSGGST**K** |
| 24 | F12 CDR3 | YSD**W**DFEEY |
| 25 | D9 VHH | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSS |
| 26 | D9 CDR1 | GSIFSSAP**M**A |
| 27 | D9 CDR2 | RITSGGSIY |
| 28 | D9 CDR3 | P**W**GS**W**YEDHD**M**DY |
| | | PTM-removed and humanized anti-Trop2 VHH sequence |
| 29 | A04.m1 VHH | QLQLVESGGGLVQPGGSLRLSCAASALTFKDYAMTWFRQ APGKEREGVSSVSSGGATTYADSVKGRFTISRDNAKNTVY LQMNSLQPEDTAVYYCAASWGYGLRSLTRKEYDYWGQG TQVTVSS |
| 98 | A04.m1.m10 VHH | EVQLVESGGGLVQPGGSLRLSCAASALTFKDYAMTWFRQ APGKEREGVSSVSSGGATTYADSVKGRFTISRDNSKNTVY LQMNSLRAEDTAVYYCAASWGYGLRSLTRKEYDYWGQG TLVTVSS |
| 99 | A04.m1.m11 VHH | QLQLVESGGGLVQPGGSLRLSCAASALTFKDYAMTWFRQ APGKEREGVSSVSSGGATTYADSVKGRFTISRDNSKNTVY LQMNSLRAEDTAVYYCAASWGYGLRSLTRKEYDYWGQG TLVTVS |
| 30 | A04.m1.m12 VHH | QLQLVESGGGLVQPGGSLRLSCAASALTFKDYAMTWFRQ APGKEREGVSSVSSGGATTYADSVKGRFTISRDNSKNTVY LQMNSLQPEDTAVYYCAASWGYGLRSLTRKEYDYWGQG TLVTVSS |
| | | A04.m1, A04.m1.m10, A04.m1.m11, and A04.m1.m12 have the same CDR sequences defined by the AbM scheme, wherein CDR1 and CDR3 are the same as those of A04 |
| 31 | A04.m1 CDR1 | ALTF**K**DYA**M**T |
| 32 | A04.m1 CDR2 | SVSSGGATT |
| 33 | A04.m1 CDR3 | S**W**GYGLRSLTR**K**EYDY |
| | | PTM-removed and humanized anti-Nectin4 VHH sequence and CDR sequence thereof, wherein CDR1 and CDR3 are the same as those of F09 |
| 100 | F09.m1 VHH | EVQLVESGGGLVQPGGSLRLSCAASGSTFGINVMGWYRQ APGKQREYVASITRDGSINYADSVKGRFTISRDNSKNTVYL QMNSLRAEDTAVYYCNADRLGPTGWGSRIELWGQGTLVT VSS |
| 101 | F09.m2 VHH | EVQLVESGGGLVQPGGSLRLSCAASGSTFGINVMGWYRQ APGKQREYVASITRDGSINYADSVRGRFTISRDNSKNTVYL QMNSLRAEDTAVYYCNADRLGPTGWGSRIELWGQGTLVT VSS |
| 102 | F09.m5 VHH | QVQLVESGGGLVQPGGSLRLSCAASGSTFGINVMGWYRQ APGKQREYVASITREGSINYADSVRGRFTISRDNAKSTVYL QMNSLKPDDTAVYYCNADRLGPTGWGSRIELWGQGTQVT VSS |
| 103 | F09.m6 VHH | QVQLVESGGGLVQPGGSLRLSCAASGSTFGINVMGWYRQ APGKQREYVASITRQGSINYADSVRGRFTISRDNAKSTVYL QMNSLKPDDTAVYYCNADRLGPTGWGSRIELWGQGTQVT VSS |
| 104 | F09.m1.m5 VHH | EVQLVESGGGLVQPGGSLRLSCAASGSTFGINVMGWYRQ APGKQREYVASITREGSINYADSVKGRFTISRDNSKNTVYL QMNSLRAEDTAVYYCNADRLGPTGWGSRIELWGQGTLVT VSS |
| 105 | F09.m1.m6 VHH | EVQLVESGGGLVQPGGSLRLSCAASGSTFGINVMGWYRQ APGKQREYVASITRQGSINYADSVKGRFTISRDNSKNTVYL QMNSLRAEDTAVYYCNADRLGPTGWGSRIELWGQGTLVT VSS |
| 34 | F09.m2m5 VHH | EVQLVESGGGLVQPGGSLRLSCAASGSTFGINVMGWYRQ APGKQREYVASITREGSINYADSVRGRFTISRDNSKNTVYL QMNSLRAEDTAVYYCNADRLGPTGWGSRIELWGQGTLVT VSS |
| 35 | F09.m2m6 VHH | EVQLVESGGGLVQPGGSLRLSCAASGSTFGINVMGWYRQ APGKQREYVASITRQGSINYADSVRGRFTISRDNSKNTVYL QMNSLRAEDTAVYYCNADRLGPTGWGSRIELWGQGTLVT VSS |
| 36 | F09.m2m5 CDR2 | SITREGSIN |
| 37 | F09.m2m6 CDR2 | SITRQGSIN |
| 38 | F09.m2m5-2 VHH | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT VSS |
| 106 | F09.m2m5-4 VHH | EVQLVESGGGLVQPGGSLRLSCAASGSTFGINVMGWYRQ APGKQREYVASITREGSINYADSVRGRFTISRDNAKNTVYL QMNSLKPEDTAVYYCNADRLGPTGWGSRIELWGQGTLVT VSS |
| | | Half-life extension domain (HLE), anti-serum albumin VHH, with CDR regions underlined |
| 39 | MSA21 (also called HLE1) | QVQLQESGGGLVQPGGSLRLSCEASGFTFSRFGMTWVRQA PGKGVEWVSGISSLGDSTLYADSVKGRFTISRDNAKNTLYL QMNSLKPEDTAVYYCTIGGSLNPGGQGTQVTVSS |
| 40 | ALB-8 (also called HLE2) | |
| | | Single-chain multispecific antibody molecule, peptide linker: G4S |
| 41 | V-1, chain composition G08-F12 Peptide linker: G4S | EVQLVESGGGLVQPGGSLTLSCAAPERIFSVYRMYWYRQA PGKQREWVAAITTSGSADYKDSVKGRFTISRDNAKNMAY LQMNSLKPDDTAVYYCNAGGGPGYYGQGTQVTVSSGGG GSQVQLVESGGGLVQPGGSLRLSCAASGSLFSINVMGWYR QPPGKKRELVATISSGGSTKYADSVKGRFTISGDNAKNTYL QMNTLKPEDTAVYYCHAYSDWDFEEYWGQGTQVTVSS |
| 42 | V-2, chain composition G08-F12-MSA21 | EVQLVESGGGLVQPGGSLTLSCAAPERIFSVYRMYWYRQA PGKQREWVAAITTSGSADYKDSVKGRFTISRDNAKNMAY LQMNSLKPDDTAVYYCNAGGGPGYYGQGTQVTVSSGGG GSQVQLVESGGGLVQPGGSLRLSCAASGSLFSINVMGWYR QPPGKKRELVATISSGGSTKYADSVKGRFTISGDNAKNTYL QMNTLKPEDTAVYYCHAYSDWDFEEYWGQGTQVTVSSG GGGSQVQLQESGGGLVQPGGSLRLSCEASGFTFSRFGMTW VRQAPGKGVEWVSGISSLGDSTLYADSVKGRFTISRDNAK NTLYLQMNSLKPEDTAVYYCTIGGSLNPGGQGTQVTVSS |
| 43 | V-3, chain composition H03-D9 | QLQLVESGGGLVQPGGSLRLSCAASGFTFSTYAMSWARQA PGKGLEWVSGIYGDGSGTYYADSVKGRFTISRDNAKNTLY LQMNSLKPEDTALYYCAQGYSDYDRRVGEYDYWGQGTQ VTVSSGGGGSQLQLVESGGGSVQPGGSLRLSCEAFGSIFSS APMAWYRQAPGKQRELVARITSGGSIYYADSVKGRFTISR DNAKNTAYLQMNSLKPEDTAVYYCNVPWGSWYEDHDM DYWGKGTLVTVSS |
| 44 | V-4, chain composition H03-D9-MSA21 | QLQLVESGGGLVQPGGSLRLSCAASGFTFSTYAMSWARQA PGKGLEWVSGIYGDGSGTYYADSVKGRFTISRDNAKNTLY LQMNSLKPEDTALYYCAQGYSDYDRRVGEYDYWGQGTQ VTVSSGGGGSQLQLVESGGGSVQPGGSLRLSCEAFGSIFSS APMAWYRQAPGKQRELVARITSGGSIYYADSVKGRFTISR DNAKNTAYLQMNSLKPEDTAVYYCNVPWGSWYEDHDM DYWGKGTLVTVSSGGGGSQVQLQESGGGLVQPGGSLRLS CEASGFTFSRFGMTWVRQAPGKGVEWVSGISSLGDSTLYA DSVKGRFTISRDNAKNTLYLQMNSLKPEDTAVYYCTIGGS LNPGGQGTQVTVSS |
| 45 | V-5, chain composition D9-H03-D9 | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSGGGGSQLQLVESGGGLVQPGGSLRLSCAASGFTFSTY AMSWARQAPGKGLEWVSGIYGDGSGTYYADSVKGRFTIS RDNAKNTLYLQMNSLKPEDTALYYCAQGYSDYDRRVGEY DYWGQGTQVTVSSGGGGSQLQLVESGGGSVQPGGSLRLS CEAFGSIFSSAPMAWYRQAPGKQRELVARITSGGSIYYADS |
| | | VKGRFTISRDNAKNTAYLQMNSLKPEDTAVYYCNVPWGS WYEDHDMDYWGKGTLVTVSS |
| 46 | V-6, chain composition F12-G08-F12 | QVQLVESGGGLVQPGGSLRLSCAASGSLFSINVMGWYRQP PGKKRELVATISSGGSTKYADSVKGRFTISGDNAKNTYLQ MNTLKPEDTAVYYCHAYSDWDFEEYWGQGTQVTVSSGG GGSEVQLVESGGGLVQPGGSLTLSCAAPERIFSVYRMYWY RQAPGKQREWVAAITTSGSADYKDSVKGRFTISRDNAKN MAYLQMNSLKPDDTAVYYCNAGGGPGYYGQGTQVTVSS GGGGSQVQLVESGGGLVQPGGSLRLSCAASGSLFSINVMG WYRQPPGKKRELVATISSGGSTKYADSVKGRFTISGDNAK NTYLQMNTLKPEDTAVYYCHAYSDWDFEEYWGQGTQVT VSS |
| 47 | V-7, chain composition D9-G08-D9 | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSGGGGSEVQLVESGGGLVQPGGSLTLSCAAPERIFSVYR MYWYRQAPGKQREWVAAITTSGSADYKDSVKGRFTISRD NAKNMAYLQMNSLKPDDTAVYYCNAGGGPGYYGQGTQ VTVSSGGGGSQLQLVESGGGSVQPGGSLRLSCEAFGSIFSS APMAWYRQAPGKQRELVARITSGGSIYYADSVKGRFTISR DNAKNTAYLQMNSLKPEDTAVYYCNVPWGSWYEDHDM DYWGKGTLVTVSS |
| 48 | V-8, chain composition G08-F12-F12 | EVQLVESGGGLVQPGGSLTLSCAAPERIFSVYRMYWYRQA PGKQREWVAAITTSGSADYKDSVKGRFTISRDNAKNMAY LQMNSLKPDDTAVYYCNAGGGPGYYGQGTQVTVSSGGG GSQVQLVESGGGLVQPGGSLRLSCAASGSLFSINVMGWYR QPPGKKRELVATISSGGSTKYADSVKGRFTISGDNAKNTYL QMNTLKPEDTAVYYCHAYSDWDFEEYWGQGTQVTVSSG GGGSQVQLVESGGGLVQPGGSLRLSCAASGSLFSINVMGW YRQPPGKKRELVATISSGGSTKYADSVKGRFTISGDNAKNT YLQMNTLKPEDTAVYYCHAYSDWDFEEYWGQGTQVTVS S |
| 49 | V-9, chain composition H03-D9-D9 | QLQLVESGGGLVQPGGSLRLSCAASGFTFSTYAMSWARQA PGKGLEWVSGIYGDGSGTYYADSVKGRFTISRDNAKNTLY LQMNSLKPEDTALYYCAQGYSDYDRRVGEYDYWGQGTQ VTVSSGGGGSQLQLVESGGGSVQPGGSLRLSCEAFGSIFSS APMAWYRQAPGKQRELVARITSGGSIYYADSVKGRFTISR DNAKNTAYLQMNSLKPEDTAVYYCNVPWGSWYEDHDM DYWGKGTLVTVSSGGGGSQLQLVESGGGSVQPGGSLRLS CEAFGSIFSSAPMAWYRQAPGKQRELVARITSGGSIYYADS VKGRFTISRDNAKNTAYLQMNSLKPEDTAVYYCNVPWGS WYEDHDMDYWGKGTLVTVSS |
| 50 | V-hu2 (V-F1S1T1.2) chain composition: A04.m1-F09.m2m5-ALB8 | QLQLVESGGGLVQPGGSLRLSCAASALTFKDYAMTWFRQ APGKEREGVSSVSSGGATTYADSVKGRFTISRDNAKNTVY LQMNSLQPEDTAVYYCAASWGYGLRSLTRKEYDYWGQG TQVTVSSGGGGSGGGGSEVQLVESGGGLVQPGGSLRLSCA ASGSTFGINVMGWYRQAPGKQREYVASITREGSINYADSV RGRFTISRDNSKNTVYLQMNSLRAEDTAVYYCNADRLGPT GWGSRIELWGQGTLVTVSSGGGGSGGGGSEVQLVESGGG LVQPGNSLRLSCAASGFTFSSFGMSWVRQAPGKGLEWVSS |
| | Peptide linker (G4S)2 | ISGSGSDTLYADSVKGRFTISRDNAKTTLYLQMNSLRPEDT AVYYCTIGGSLSRSSQGTLVTVSS |
| 51 | V-hu8 (V-F1F1S1T1.8) chain composition: A04.m1-ALB8-F09.m2m5-F09.m2m5 | QLQLVESGGGLVQPGGSLRLSCAASALTFKDYAMTWFRQ APGKEREGVSSVSSGGATTYADSVKGRFTISRDNAKNTVY LQMNSLQPEDTAVYYCAASWGYGLRSLTRKEYDYWGQG TQVTVSSGGGGSGGGGSEVQLVESGGGLVQPGNSLRLSCA ASGFTFSSFGMSWVRQAPGKGLEWVSSISGSGSDTLYADS VKGRFTISRDNAKTTLYLQMNSLRPEDTAVYYCTIGGSLSR SSQGTLVTVSSGGGGSGGGGSEVQLVESGGGLVQPGGSLR LSCAASGSTFGINVMGWYRQAPGKQREYVASITREGSINY ADSVRGRFTISRDNSKNTVYLQMNSLRAEDTAVYYCNAD RLGPTGWGSRIELWGQGTLVTVSSGGGGSGGGGSEVQLVE SGGGLVQPGGSLRLSCAASGSTFGINVMGWYRQAPGKQR EYVASITREGSINYADSVRGRFTISRDNSKNTVYLQMNSLR AEDTAVYYCNADRLGPTGWGSRIELWGQGTLVTVSS |
| 52 | V-hu22 (V-F1F1S1S1T1.22) chain composition: A04.m1-A04.m1-ALB8-F09.m2m5-F09.m2m5 | QLQLVESGGGLVQPGGSLRLSCAASALTFKDYAMTWFRQ APGKEREGVSSVSSGGATTYADSVKGRFTISRDNAKNTVY LQMNSLQPEDTAVYYCAASWGYGLRSLTRKEYDYWGQG TQVTVSSGGGGSGGGGSQLQLVESGGGLVQPGGSLRLSCA ASALTFKDYAMTWFRQAPGKEREGVSSVSSGGATTYADS VKGRFTISRDNAKNTVYLQMNSLQPEDTAVYYCAASWGY GLRSLTRKEYDYWGQGTQVTVSSGGGGSGGGGSEVQLVE SGGGLVQPGNSLRLSCAASGFTFSSFGMSWVRQAPGKGLE WVSSISGSGSDTLYADSVKGRFTISRDNAKTTLYLQMNSLR PEDTAVYYCTIGGSLSRSSQGTLVTVSSGGGGSGGGGSEVQ LVESGGGLVQPGGSLRLSCAASGSTFGINVMGWYRQAPGK QREYVASITREGSINYADSVRGRFTISRDNSKNTVYLQMNS LRAEDTAVYYCNADRLGPTGWGSRIELWGQGTLVTVSSG GGGSGGGGSEVQLVESGGGLVQPGGSLRLSCAASGSTFGI NVMGWYRQAPGKQREYVASITREGSINYADSVRGRFTISR DNSKNTVYLQMNSLRAEDTAVYYCNADRLGPTGWGSRIE LWGQGTLVTVSS |
| | | Double-chain symmetric bispecific antibody molecule having two identical chains, and LALA mutations are introduced into both Fc regions |
| 53 | V-hu24-Fc chain composition: F09.m2m5-2-A04.m1m12-Fc directly linked to Fc | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT VSSGGGGSGGGGSQLQLVESGGGLVQPGGSLRLSCAASAL TFKDYAMTWFRQAPGKEREGVSSVSSGGATTYADSVKGR FTISRDNSKNTVYLQMNSLQPEDTAVYYCAASWGYGLRSL TRKEYDYWGQGTLVTVSSEPKSSDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALPAPIEKTISKAKGQPREPQVYTLPPSRDE LTKNQVSLTCLVKGFYPSDIAVEWESNGQPENNYKTTPPV LDSDGSFFLYSKLTVDKSRWQQGNVFSCSVMHEALHNHY TQKSLSLSPGK |
| 54 | V-hu25-Fc chain composition: F09.m2m5-2-A04.m1m12-Fc linked to Fc via a peptide linker G4S | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT |
| | | VSSGGGGSGGGGSQLQLVESGGGLVQPGGSLRLSCAASAL TFKDYAMTWFRQAPGKEREGVSSVSSGGATTYADSVKGR FTISRDNSKNTVYLQMNSLQPEDTAVYYCAASWGYGLRSL TRKEYDYWGQGTLVTVSSGGGGSEPKSSDKTHTCPPCPAP EAAGGPSVFLFPPKPKDTLMISRTPEVTCVVVDVSHEDPEV KFNWYVDGVEVHNAKTKPREEQYNSTYRVVSVLTVLHQD WLNGKEYKCKVSNKALPAPIEKTISKAKGQPREPQVYTLPP SRDELTKNQVSLTCLVKGFYPSDIAVEWESNGQPENNYKT TPPVLDSDGSFFLYSKLTVDKSRWQQGNVFSCSVMHEALH NHYTQKSLSLSPGK |
| | | Double-chain asymmetric bispecific antibody molecule having two different chains, wherein a knob mutation is introduced into the first chain, a corresponding hole mutation is introduced into the second chain, and LALA mutations are introduced into Fc regions of both chains |
| 55 | V-10 first chain G08-Fc | EVQLVESGGGLVQPGGSLTLSCAAPERIFSVYRMYWYRQA PGKQREWVAAITTSGSADYKDSVKGRFTISRDNAKNMAY LQMNSLKPDDTAVYYCNAGGGPGYYGQGTQVTVSSDKT HTCPPCPAPEAAGGPSVFLFPPKPKDTLMISRTPEVTCVVV DVSHEDPEVKFNWYVDGVEVHNAKTKPREEQYNSTYRVV SVLTVLHQDWLNGKEYKCKVSNKALPAPIEKTISKAKGQP REPQVYTLPPSREEMTKNQVSLYCLVKGFYPSDIAVEWES NGQPENNYKTTPPVLDSDGSFFLYSKLTVDKSRWQQGNVF SCSVMHEALHNHYTQKSLSLSPGK |
| 56 | V-10 second chain F12-Fc | QVQLVESGGGLVQPGGSLRLSCAASGSLFSINVMGWYRQP PGKKRELVATISSGGSTKYADSVKGRFTISGDNAKNTYLQ MNTLKPEDTAVYYCHAYSDWDFEEYWGQGTQVTVSSDK THTCPPCPAPEAAGGPSVFLFPPKPKDTLMISRTPEVTCVVV DVSHEDPEVKFNWYVDGVEVHNAKTKPREEQYNSTYRVV SVLTVLHQDWLNGKEYKCKVSNKALPAPIEKTISKAKGQP REPQVYTLPPSREEMTKNQVSLTCLVKGFYPSDIAVEWESN GQPENNYKTTPPVLDSDGSFFLTSKLTVDKSRWQQGNVFS CSVMHEALHNHYTQKSLSLSPGK |
| 57 | V-13 first chain H03-Fc | QLQLVESGGGLVQPGGSLRLSCAASGFTFSTYAMSWARQA PGKGLEWVSGIYGDGSGTYYADSVKGRFTISRDNAKNTLY LQMNSLKPEDTALYYCAQGYSDYDRRVGEYDYWGQGTQ VTVSSDKTHTCPPCPAPEAAGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTI SKAKGQPREPQVYTLPPSREEMTKNQVSLYCLVKGFYPSDI AVEWESNGQPENNYKTTPPVLDSDGSFFLYSKLTVDKSRW QQGNVFSCSVMHEALHNHYTQKSLSLSPGK |
| 58 | V-13 second chain D9-Fc | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSDKTHTCPPCPAPEAAGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTISK AKGQPREPQVYTLPPSREEMTKNQVSLTCLVKGFYPSDIAV EWESNGQPENNYKTTPPVLDSDGSFFLTSKLTVDKSRWQQ GNVFSCSVMHEALHNHYTQKSLSLSPGK |
| 59 | V-14 first chain D9-H03-Fc | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSGGGGSQLQLVESGGGLVQPGGSLRLSCAASGFTFSTY AMSWARQAPGKGLEWVSGIYGDGSGTYYADSVKGRFTIS RDNAKNTLYLQMNSLKPEDTALYYCAQGYSDYDRRVGEY DYWGQGTQVTVSSDKTHTCPPCPAPEAAGGPSVFLFPPKP KDTLMISRTPEVTCVVVDVSHEDPEVKFNWYVDGVEVHN AKTKPREEQYNSTYRVVSVLTVLHQDWLNGKEYKCKVSN KALPAPIEKTISKAKGQPREPQVYTLPPSREEMTKNQVSLY CLVKGFYPSDIAVEWESNGQPENNYKTTPPVLDSDGSFFLY SKLTVDKSRWQQGNVFSCSVMHEALHNHYTQKSLSLSPG K |
| 60 | V-14 second chain D9-Fc | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSDKTHTCPPCPAPEAAGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTISK AKGQPREPQVYTLPPSREEMTKNQVSLTCLVKGFYPSDIAV EWESNGQPENNYKTTPPVLDSDGSFFLTSKLTVDKSRWQQ GNVFSCSVMHEALHNHYTQKSLSLSPGK |
| 61 | V-15 first chain F12-G08-Fc | QVQLVESGGGLVQPGGSLRLSCAASGSLFSINVMGWYRQP PGKKRELVATISSGGSTKYADSVKGRFTISGDNAKNTYLQ MNTLKPEDTAVYYCHAYSDWDFEEYWGQGTQVTVSSGG GGSEVQLVESGGGLVQPGGSLTLSCAAPERIFSVYRMYWY RQAPGKQREWVAAITTSGSADYKDSVKGRFTISRDNAKN MAYLQMNSLKPDDTAVYYCNAGGGPGYYGQGTQVTVSS DKTHTCPPCPAPEAAGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSREEMTKNQVSLYCLVKGFYPSDIAVEW ESNGQPENNYKTTPPVLDSDGSFFLYSKLTVDKSRWQQGN VFSCSVMHEALHNHYTQKSLSLSPGK |
| 62 | V-15 second chain F12-Fc | QVQLVESGGGLVQPGGSLRLSCAASGSLFSINVMGWYRQP PGKKRELVATISSGGSTKYADSVKGRFTISGDNAKNTYLQ MNTLKPEDTAVYYCHAYSDWDFEEYWGQGTQVTVSSDK THTCPPCPAPEAAGGPSVFLFPPKPKDTLMISRTPEVTCVVV DVSHEDPEVKFNWYVDGVEVHNAKTKPREEQYNSTYRVV SVLTVLHQDWLNGKEYKCKVSNKALPAPIEKTISKAKGQP REPQVYTLPPSREEMTKNQVSLTCLVKGFYPSDIAVEWESN GQPENNYKTTPPVLDSDGSFFLTSKLTVDKSRWQQGNVFS CSVMHEALHNHYTQKSLSLSPGK |
| 63 | V-16 first chain D9-G08-Fc | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSGGGGSEVQLVESGGGLVQPGGSLTLSCAAPERIFSVYR MYWYRQAPGKQREWVAAITTSGSADYKDSVKGRFTISRD NAKNMAYLQMNSLKPDDTAVYYCNAGGGPGYYGQGTQ VTVSSDKTHTCPPCPAPEAAGGPSVFLFPPKPKDTLMISRTP EVTCVVVDVSHEDPEVKFNWYVDGVEVHNAKTKPREEQY |
| | | NSTYRVVSVLTVLHQDWLNGKEYKCKVSNKALPAPIEKTI SKAKGQPREPQVYTLPPSREEMTKNQVSLYCLVKGFYPSDI AVEWESNGQPENNYKTTPPVLDSDGSFFLYSKLTVDKSRW QQGNVFSCSVMHEALHNHYTQKSLSLSPGK |
| 64 | V-16 second chain D9-Fc | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSDKTHTCPPCPAPEAAGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTISK AKGQPREPQVYTLPPSREEMTKNQVSLTCLVKGFYPSDIAV EWESNGQPENNYKTTPPVLDSDGSFFLTSKLTVDKSRWQQ GNVFSCSVMHEALHNHYTQKSLSLSPGK |
| 65 | V-17 first chain F12-F12-Fc | QVQLVESGGGLVQPGGSLRLSCAASGSLFSINVMGWYRQP PGKKRELVATISSGGSTKYADSVKGRFTISGDNAKNTYLQ MNTLKPEDTAVYYCHAYSDWDFEEYWGQGTQVTVSSGG GGSQVQLVESGGGLVQPGGSLRLSCAASGSLFSINVMGWY RQPPGKKRELVATISSGGSTKYADSVKGRFTISGDNAKNTY LQMNTLKPEDTAVYYCHAYSDWDFEEYWGQGTQVTVSS DKTHTCPPCPAPEAAGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSREEMTKNQVSLYCLVKGFYPSDIAVEW ESNGQPENNYKTTPPVLDSDGSFFLYSKLTVDKSRWQQGN VFSCSVMHEALHNHYTQKSLSLSPGK |
| 66 | V-17 second chain G08-Fc | EVQLVESGGGLVQPGGSLTLSCAAPERIFSVYRMYWYRQA PGKQREWVAAITTSGSADYKDSVKGRFTISRDNAKNMAY LQMNSLKPDDTAVYYCNAGGGPGYYGQGTQVTVSSDKT HTCPPCPAPEAAGGPSVFLFPPKPKDTLMISRTPEVTCVVV DVSHEDPEVKFNWYVDGVEVHNAKTKPREEQYNSTYRVV SVLTVLHQDWLNGKEYKCKVSNKALPAPIEKTISKAKGQP REPQVYTLPPSREEMTKNQVSLTCLVKGFYPSDIAVEWESN GQPENNYKTTPPVLDSDGSFFLTSKLTVDKSRWQQGNVFS CSVMHEALHNHYTQKSLSLSPGK |
| 67 | V-18 first chain D9-D9-Fc | QLQLVESGGGSVQPGGSLRLSCEAFGSIFSSAPMAWYRQA PGKQRELVARITSGGSIYYADSVKGRFTISRDNAKNTAYLQ MNSLKPEDTAVYYCNVPWGSWYEDHDMDYWGKGTLVT VSSGGGGSQLQLVESGGGSVQPGGSLRLSCEAFGSIFSSAP MAWYRQAPGKQRELVARITSGGSIYYADSVKGRFTISRDN AKNTAYLQMNSLKPEDTAVYYCNVPWGSWYEDHDMDY WGKGTLVTVSSDKTHTCPPCPAPEAAGGPSVFLFPPKPKDT LMISRTPEVTCVVVDVSHEDPEVKFNWYVDGVEVHNAKT KPREEQYNSTYRVVSVLTVLHQDWLNGKEYKCKVSNKAL PAPIEKTISKAKGQPREPQVYTLPPSREEMTKNQVSLYCLV KGFYPSDIAVEWESNGQPENNYKTTPPVLDSDGSFFLYSKL TVDKSRWQQGNVFSCSVMHEALHNHYTQKSLSLSPGK |
| 68 | V-18 second chain H03-Fc | QLQLVESGGGLVQPGGSLRLSCAASGFTFSTYAMSWARQA PGKGLEWVSGIYGDGSGTYYADSVKGRFTISRDNAKNTLY LQMNSLKPEDTALYYCAQGYSDYDRRVGEYDYWGQGTQ VTVSSDKTHTCPPCPAPEAAGGPSVFLFPPKPKDTLMISRTP EVTCVVVDVSHEDPEVKFNWYVDGVEVHNAKTKPREEQY |
| | | NSTYRVVSVLTVLHQDWLNGKEYKCKVSNKALPAPIEKTI SKAKGQPREPQVYTLPPSREEMTKNQVSLTCLVKGFYPSDI AVEWESNGQPENNYKTTPPVLDSDGSFFLTSKLTVDKSRW QQGNVFSCSVMHEALHNHYTQKSLSLSPGK |
| 69 | V-hu21-Fc first chain: F09.m2m5-A04.m1-Fc | EVQLVESGGGLVQPGGSLRLSCAASGSTFGINVMGWYRQ APGKQREYVASITREGSINYADSVRGRFTISRDNSKNTVYL QMNSLRAEDTAVYYCNADRLGPTGWGSRIELWGQGTLVT VSSGGGGSGGGGSQLQLVESGGGLVQPGGSLRLSCAASAL TFKDYAMTWFRQAPGKEREGVSSVSSGGATTYADSVKGR FTISRDNAKNTVYLQMNSLQPEDTAVYYCAASWGYGLRS LTRKEYDYWGQGTQVTVSSEPKSSDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRE EMTKNQVSLWCLVKGFYPSDIAVEWESNGQPENNYKTTPP VLDSDGSFFLYSKLTVDKSRWQQGNVFSCSVMHEALHNH YTQKSLSLSPGK |
| 70 | V-hu21-Fc second chain: F09.m2m5-Fc | EVQLVESGGGLVQPGGSLRLSCAASGSTFGINVMGWYRQ APGKQREYVASITREGSINYADSVRGRFTISRDNSKNTVYL QMNSLRAEDTAVYYCNADRLGPTGWGSRIELWGQGTLVT VSSEPKSSDKTHTCPPCPAPEAAGGPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPAPI EKTISKAKGQPREPQVYTLPPSREEMTKNQVSLSCAVKGFY PSDIAVEWESNGQPENNYKTTPPVLDSDGSFFLVSKLTVDK SRWQQGNVFSCSVMHEALHNHYTQKSLSLSPGK |
| 71 | V-hu23-Fc first chain: F09.m2m5-2-A04.m1m12-Fc | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT VSSGGGGSGGGGSQLQLVESGGGLVQPGGSLRLSCAASAL TFKDYAMTWFRQAPGKEREGVSSVSSGGATTYADSVKGR FTISRDNSKNTVYLQMNSLQPEDTAVYYCAASWGYGLRSL TRKEYDYWGQGTLVTVSSEPKSSDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALPAPIEKTISKAKGQPREPQVYTLPPSRDE LTKNQVSLWCLVKGFYPSDIAVEWESNGQPENNYKTTPPV LDSDGSFFLYSKLTVDKSRWQQGNVFSCSVMHEALHNHY TQKSLSLSPGK |
| 72 | V-hu23-Fc second chain: F09.m2m5-2-Fc | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT VSSEPKSSDKTHTCPPCPAPEAAGGPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPAPI EKTISKAKGQPREPQVYTLPPSRDELTKNQVSLSCAVKGFY PSDIAVEWESNGQPENNYKTTPPVLDSDGSFFLVSKLTVDK SRWQQGNVFSCSVMHEALHNHYTQKSLSLSPGK |
| 73 | V-hu28-Fc first chain: F09.m2m5-2-F09.m2m5-2-Fc | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT |
| | | VSSGGGGSGGGGSEVQLVESGGGLVQPGGSLRLSCAASGS TFGINVMGWYRQAPGKQREYVASITREGSINYADSVRGRF TISRDNAKNTVYLQMNSLRPEDTAVYYCNADRLGPTGWG SRIELWGQGTLVTVSSEPKSSDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALPAPIEKTISKAKGQPREPQVYTLPPSRDELTK NQVSLWCLVKGFYPSDIAVEWESNGQPENNYKTTPPVLDS DGSFFLYSKLTVDKSRWQQGNVFSCSVMHEALHNHYTQK SLSLSPGK |
| 74 | V-hu28-Fc second chain: A04.m1m12-Fc | QLQLVESGGGLVQPGGSLRLSCAASALTFKDYAMTWFRQ APGKEREGVSSVSSGGATTYADSVKGRFTISRDNSKNTVY LQMNSLQPEDTAVYYCAASWGYGLRSLTRKEYDYWGQG TLVTVSSEPKSSDKTHTCPPCPAPEAAGGPSVFLFPPKPKDT LMISRTPEVTCVVVDVSHEDPEVKFNWYVDGVEVHNAKT KPREEQYNSTYRVVSVLTVLHQDWLNGKEYKCKVSNKAL PAPIEKTISKAKGQPREPQVYTLPPSRDELTKNQVSLSCAVK GFYPSDIAVEWESNGQPENNYKTTPPVLDSDGSFFLVSKLT VDKSRWQQGNVFSCSVMHEALHNHYTQKSLSLSPGK |
| 75 | V-hu29-Fc first chain: A04.m1m12-Fc | QLQLVESGGGLVQPGGSLRLSCAASALTFKDYAMTWFRQ APGKEREGVSSVSSGGATTYADSVKGRFTISRDNSKNTVY LQMNSLQPEDTAVYYCAASWGYGLRSLTRKEYDYWGQG TLVTVSSEPKSSDKTHTCPPCPAPEAAGGPSVFLFPPKPKDT LMISRTPEVTCVVVDVSHEDPEVKFNWYVDGVEVHNAKT KPREEQYNSTYRVVSVLTVLHQDWLNGKEYKCKVSNKAL PAPIEKTISKAKGQPREPQVYTLPPSREEMTKNQVSLWCLV KGFYPSDIAVEWESNGQPENNYKTTPPVLDSDGSFFLYSKL TVDKSRWOOGNVFSCSVMHEALHNHYTOKSLSLSPGK |
| 76 | V-hu29-Fc second chain: F09.m2m5-2-Fc | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT VSSEPKSSDKTHTCPPCPAPEAAGGPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPAPI EKTISKAKGQPREPQVYTLPPSREEMTKNQVSLSCAVKGFY PSDIAVEWESNGQPENNYKTTPPVLDSDGSFFLVSKLTVDK SRWQQGNVFSCSVMHEALHNHYTQKSLSLSPGK |
| | | Control multispecific antibody |
| 77 | V-11 LC light chain | DIQMTQSPSSLSASVGDRVTITCKASQDVSTAVAWYQQKP GKAPKLLIYSASYRYTGVPSRFSGSGSGTDFTLTISSLQPED FAVYYCQQHYITPLTFGQGTKLEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSSP VTKSFNRGEC |
| 78 | V-11 knob heavy chain | QVQLVQSGAEVKKPGASVKVSCKASGYTFTTAGMQWVR QAPGQGLEWMGWINTHSGVPKYAEDFKGRVTISADTSTST AYLQLSSLKSEDTAVYYCARSGFGSSYWYFDVWGQGTLV TVSSASTKGPSVFPLAPSSKSTSGGTAALGCLVKDYFPEPV TVSWNSGALTSGVHTFPAVLQSSGLYSLSSVVTVPSSSLGT QTYICNVNHKPSNTKVDKKVEPKSCDKTHTCPPCPAPEAA GGPSVFLFPPKPKDTLMISRTPEVTCVVVDVSHEDPEVKFN |
| | | WYVDGVEVHNAKTKPREEQYNSTYRVVSVLTVLHQDWL NGKEYKCKVSNKALPAPIEKTISKAKGQPREPQVYTLPPSR DELTKNQVSLYCLVKGFYPSDIAVEWESNGQPENNYKTTP PVLDSDGSFFLYSKLTVDKSRWQQGNVFSCSVMHEALHN HYTQKSLSLSPGK |
| 79 | V-11 hole heavy chain | EVQLVESGGGLVQPGGSLRLSCAASGFTFSSYNMNWVRQ APGKGLEWVSYISSSSSTIYYADSVKGRFTISRDNAKNSLSL QMNSLRDEDTAVYYCARAYYYGMDVWGQGTTVTVSSGG GGSGGGGSGGGGSDIQMTQSPSSVSASVGDRVTITCRASQ GISGWLAWYQQKPGKAPKFLIYAASTLQSGVPSRFSGSGS GTDFTLTISSLQPEDFATYYCQQANSFPPTFGGGTKVEIKEP KSSDKTHTCPPCPAPEAAGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLTSKLTVDKSRWQQ GNVFSCSVMHEALHNHYTQKSLSLSPGK |
| 80 | V-12 LC light chain | DIQMTQSPSSVSASVGDRVTITCRASQGISGWLAWYQQKP GKAPKFLIYAASTLQSGVPSRFSGSGSGTDFTLTISSLQPEDF ATYYCQQANSFPPTFGGGTKVEIKRTVAAPSVFIFPPSDEQL KSGTASVVCLLNNFYPREAKVQWKVDNALQSGNSQESVT EQDSKDSTYSLSSTLTLSKADYEKHKVYACEVTHQGLSSP VTKSFNRGEC |
| 81 | V-12 Knob heavy chain | EVQLVESGGGLVQPGGSLRLSCAASGFTFSSYNMNWVRQ APGKGLEWVSYISSSSSTIYYADSVKGRFTISRDNAKNSLSL QMNSLRDEDTAVYYCARAYYYGMDVWGQGTTVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALPAPIEKTISKAKGQPREPQVYTLPPSRDELTKN QVSLYCLVKGFYPSDIAVEWESNGQPENNYKTTPPVLDSD GSFFLYSKLTVDKSRWQQGNVFSCSVMHEALHNHYTQKS LSLSPGK |
| 82 | V-12 hole heavy chain | QVQLVQSGAEVKKPGASVKVSCKASGYTFTTAGMQWVR QAPGQGLEWMGWINTHSGVPKYAEDFKGRVTISADTSTST AYLQLSSLKSEDTAVYYCARSGFGSSYWYFDVWGQGTLV TVSSGGGGSGGGGSGGGGSDIQMTQSPSSLSASVGDRVTIT CKASQDVSTAVAWYQQKPGKAPKLLIYSASYRYTGVPSRF SGSGSGTDFTLTISSLQPEDFAVYYCQQHYITPLTFGQGTKL EIKEPKSSDKTHTCPPCPAPEAAGGPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPAPI EKTISKAKGQPREPQVYTLPPSRDELTKNQVSLTCLVKGFY PSDIAVEWESNGQPENNYKTTPPVLDSDGSFFLTSKLTVDK SRWQQGNVFSCSVMHEALHNHYTQKSLSLSPGK |
| 107 | Parental anti-Trop2 Knob heavy chain | QVQLQDSGGGLVQAGDRLSLSCAASGRTFSSYDMAWFRQ APGKEREFVAAITTSEGTWYGDAGKGRFTIARVNAKNTVY LHMNRLKPEDTAVYYCAASNQGGSLQISTNYNYWGQGTQ VTVSSGGGGSGGGGSQVQLQDSGGGLVQAGDRLSLSCAA |
| | | SGRTFSSYDMAWFRQAPGKEREFVAAITTSEGTWYGDAG KGRFTIARVNAKNTVYLHMNRLKPEDTAVYYCAASNQGG SLQISTNYNYWGQGTQVTVSSEPKSSDKTHTCPPCPAPEAA GGPSVFLFPPKPKDTLMISRTPEVTCVVVDVSHEDPEVKFN WYVDGVEVHNAKTKPREEQYNSTYRVVSVLTVLHQDWL NGKEYKCKVSNKALPAPIEKTISKAKGQPREPQVYTLPPCR DELTKNQVSLWCLVKGFYPSDIAVEWESNGQPENNYKTTP PVLDSDGSFFLYSKLTVDKSRWQQGNVFSCSVMHEALHN HYTQKSLSLSPGK |
| 108 | Parental anti-Trop2 Hole heavy chain | QLQLVESGGGLVQPGGSLRLSCAASALTFKDYAMTWFRQ APGKEREGVSSVSSGGATTYADSVKGRFTISRDNSKNTVY LQMNSLQPEDTAVYYCAASWGYGLRSLTRKEYDYWGQG TLVTVSSEPKSSDKTHTCPPCPAPEAAGGPSVFLFPPKPKDT LMISRTPEVTCVVVDVSHEDPEVKFNWYVDGVEVHNAKT KPREEQYNSTYRVVSVLTVLHQDWLNGKEYKCKVSNKAL PAPIEKTISKAKGQPREPQVCTLPPSRDELTKNQVSLSCAVK GFYPSDIAVEWESNGQPENNYKTTPPVLDSDGSFFLVSKLT VDKSRWQQGNVFSCSVMHEALHNHYTQKSLSLSPGK |
| 109 | Parental anti-Nectin4 antibody chain F09.m2m5-2-Fc | EVQLVESGGGLVQPGGSLRLSCAASGSTFGINVMGWYRQ APGKQREYVASITREGSINYADSVRGRFTISRDNAKNTVYL QMNSLRPEDTAVYYCNADRLGPTGWGSRIELWGQGTLVT VSSEPKSSDKTHTCPPCPAPEAAGGPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPAPI EKTISKAKGQPREPQVYTLPPSRDELTKNQVSLTCLVKGFY PSDIAVEWESNGQPENNYKTTPPVLDSDGSFFLYSKLTVDK SRWQQGNVFSCSVMHEALHNHYTQKSLSLSPGK |
| 110 | Isotype/isotype control | QVQLQDSGGGLVQAGDRLSLSCAASGRTFSSYDMAWFRQ APGKEREFVAAITTSEGTWYGDAGKGRFTIARVNAKNTVY LHMNRLKPEDTAVYYCAASNQGGSLQISTNYNYWGQGTQ VTVSSEPKSSDKTHTCPPCPAPEAAGGPSVFLFPPKPKDTL MISRTPEVTCVVVDVSHEDPEVKFNWYVDGVEVHNAKTK PREEQYNSTYRVVSVLTVLHQDWLNGKEYKCKVSNKALP APIEKTISKAKGQPREPQVYTLPPSRDELTKNQVSLTCLVK GFYPSDIAVEWESNGQPENNYKTTPPVLDSDGSFFLYSKLT VDKSRWQQGNVFSCSVMHEALHNHYTQKSLSLSPGK |
| | | Human IgG Fc region sequence |
| 83 | Human IgG1 Fc EM | EPKSSDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTI SKAKGQPREPQVYTLPPSREEMTKNQVSLTCLVKGFYPSDI AVEWESNGQPENNYKTTPPVLDSDGSFFLYSKLTVDKSRW QQGNVFSCSVMHEALHNHYTQKSLSLSPGK |
| 84 | Human IgG1 Fc DL | EPKSSDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTI SKAKGQPREPQVYTLPPSRDELTKNQVSLTCLVKGFYPSDI AVEWESNGQPENNYKTTPPVLDSDGSFFLYSKLTVDKSRW QQGNVFSCSVMHEALHNHYTQKSLSLSPGK |
| 85 | Human IgG1 Fc with a portion of hinge region | DKTHTCPPCPAPELLGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSREEMTKNQVSLTCLVKGFYPSDIAVEWE SNGQPENNYKTTPPVLDSDGSFFLYSKLTVDKSRWQQGNV FSCSVMHEALHNHYTQKSLSLSPGK |
| 86 | Human IgG1 Fc with a portion of hinge region | DKTHTCPPCPAPELLGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSRDELTKNQVSLTCLVKGFYPSDIAVEWE SNGQPENNYKTTPPVLDSDGSFFLYSKLTVDKSRWQQGNV FSCSVMHEALHNHYTQKSLSLSPGK |
| 87 | Human IgG4 sequence | ESKYGPPCPSCPAPEFLGGPSVFLFPPKPKDTLMISRTPEVT CVVVDVSQEDPEVQFNWYVDGVEVHNAKTKPREEQFNST YRVVSVLTVLHQDWLNGKEYKCKVSNKGLPSSIEKTISKA KGQPREPQVYTLPPSQEEMTKNQVSLTCLVKGFYPSDIAVE WESNGQPENNYKTDPPVLDSDGSFFLYSRLTVDKSRWQEG NVFSCSVMHEALHNHYTQKSLSLSLGK |
| 111 | Human IgG1 Fc knob chain T366Y+LALA | DKTHTCPPCPAPEAAGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSREEMTKNQVSLYCLVKGFYPSDIAVEW ESNGQPENNYKTTPPVLDSDGSFFLYSKLTVDKSRWQQGN VFSCSVMHEALHNHYTQKSLSLSPGK |
| 112 | Human IgG1 Fc hole chain Y407T+LALA | DKTHTCPPCPAPEAAGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSREEMTKNQVSLTCLVKGFYPSDIAVEWE SNGQPENNYKTTPPVLDSDGSFFLTSKLTVDKSRWQQGNV FSCSVMHEALHNHYTQKSLSLSPGK |
| 113 | Human IgG1 Fc knob chain T366W+LALA | EPKSSDKTHTCPPCPAPEAAGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTI SKAKGQPREPQVYTLPPSREEMTKNQVSLWCLVKGFYPSD IAVEWESNGQPENNYKTTPPVLDSDGSFFLYSKLTVDKSR WQQGNVFSCSVMHEALHNHYTQKSLSLSPGK |
| 114 | Human IgG1 Fc hole chain T366S,L368A,Y 407V+LALA | EPKSSDKTHTCPPCPAPEAAGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTI SKAKGQPREPQVYTLPPSREEMTKNQVSLSCAVKGFYPSDI AVEWESNGQPENNYKTTPPVLDSDGSFFLVSKLTVDKSRW QQGNVFSCSVMHEALHNHYTQKSLSLSPGK |
| 115 | Human IgG1 Fc knob chain T366W+LALA | EPKSSDKTHTCPPCPAPEAAGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTI SKAKGQPREPQVYTLPPSRDELTKNQVSLWCLVKGFYPSDI AVEWESNGQPENNYKTTPPVLDSDGSFFLYSKLTVDKSRW QQGNVFSCSVMHEALHNHYTQKSLSLSPGK |
| 116 | Human IgG1 Fc hole chain T366S,L368A,Y 407V+LALA | EPKSSDKTHTCPPCPAPEAAGGPSVFLFPPKPKDTLMISRTP EVTCVVVDVSHEDPEVKFNWYVDGVEVHNAKTKPREEQY |
| | | NSTYRVVSVLTVLHQDWLNGKEYKCKVSNKALPAPIEKTI SKAKGQPREPQVYTLPPSRDELTKNQVSLSCAVKGFYPSDI AVEWESNGQPENNYKTTPPVLDSDGSFFLVSKLTVDKSRW QQGNVFSCSVMHEALHNHYTQKSLSLSPGK |
| 117 | Human IgG1 Fc chain LALA | NSTYRVVSVLTVLHQDWLNGKEYKCKVSNKALPAPIEKTI SKAKGQPREPQVYTLPPSRDELTKNQVSLSCAVKGFYPSDI AVEWESNGQPENNYKTTPPVLDSDGSFFLVSKLTVDKSRW QQGNVFSCSVMHEALHNHYTQKSLSLSPGK EPKSSDKTHTCPPCPAPEAAGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTI SKAKGQPREPQVYTLPPSRDELTKNQVSLTCLVKGFYPSDI AVEWESNGQPENNYKTTPPVLDSDGSFFLYSKLTVDKSRW QQGNVFSCSVMHEALHNHYTQKSLSLSPGK |
| | | Peptide linker |
| 88 | G4S | GGGGS |
| 89 | (G4S)2 | GGGGSGGGGS |
| 90 | (G4S)3 | GGGGSGGGGSGGGGS |
| 91 | (G4S)n | (GGGGS)n, wherein n is an integer equal to or greater than 1 |
| 92 | TS(G4S)n | TS(GGGGS)n, wherein n is an integer equal to or greater than 1 |
| 93 | G(G4S)n | G(GGGGS)n, wherein n is an integer equal to or greater than 1 |
| 94 | (G4)n | (GGGG)n, wherein n is an integer equal to or greater than 2 |
| 95 | (GRPGS)n | (GRPGS)n, wherein n is an integer equal to or greater than 2 |
| | | Hinge region |
| 96 | Human IgG1 hinge region | EPKSSDKTHTCPPCP |
| 97 | Human IgG1 hinge region, a portion | DKTHTCPPCP |
| | | Other peptide linkers |
| 118 | Peptide linker | GGGSG |
| 119 | Peptide linker | GGSGG |
| 120 | Peptide linker | GSGGG |
| 121 | Peptide linker | GSGGGP |
| 122 | Peptide linker | GGEPS |
| 123 | Peptide linker | GGEGGGP |
| 124 | Peptide linker | GGEGGGSEGGGS |
| 125 | Peptide linker (G₃S)₂ | GGGSGGGS |
| 126 | Peptide linker (G₃S)₃ | GGGSGGGSGGGS |
| 127 | Peptide linker (G₃S)₄ | GGGSGGGSGGGSGGGS |
| 128 | Peptide linker (G₄S)₄ | GGGGSGGGGSGGGGSGGGGS |
| 129 | Peptide linker (G₃S)₅ | GGGSGGGSGGGSGGGSGGGS |
| 130 | Peptide linker (G₄S)₅ | GGGGSGGGGSGGGGSGGGGSGGGGS |
| 131 | Peptide linker (G₃S)₆ | GGGSGGGSGGGSGGGSGGGSGGGS |
| 132 | Peptide linker | GGG |
| 133 | Peptide linker | DGGGS |
| 134 | Peptide linker | TGEKP |
| 135 | Peptide linker | GGRR |
| 136 | Peptide linker | EGKSSGSGSESKVD |
| 137 | Peptide linker | KESGSVSSEQLAQFRSLD |
| 138 | Peptide linker | GGRRGGGS |
| 139 | Peptide linker | LRQRDGERP |
| 140 | Peptide linker | LRQKDGGGSERP |
| 141 | Peptide linker | GSTSGSGKPGSGEGSTKG |

## Claims

1. A multispecific antibody binding to Trop2 and Nectin4, wherein the antibody comprises at least one antigen-binding domain specifically binding to Trop2 and at least one antigen-binding domain specifically binding to Nectin4.

2. The multispecific antibody according to claim 1, wherein the antibody has one or more characteristics selected from the following:
(a) the Trop2 and Nectin4 antigen-binding domains are VHH domains;
(b) the Trop2 and Nectin4 antigen-binding domains each have an antigen-binding affinity K_{D} value of 50 × 10⁻⁸ M to 0.5 × 10⁻⁸ M;
(c) the Trop2 antigen-binding domain and the Nectin4 antigen-binding domain in the antibody have a valence ratio of 1:1 or 1:2; and
(d) the antibody is a trivalent or tetravalent bispecific antibody; and
preferably, the antibody further has one or more characteristics selected from the following: compared with Nectin4 and Trop2 single-target drugs, the antibody exhibits, on a target tumor cell expressing human Nectin4 and Trop2, (e) a synergistic binding effect; (f) a synergistic endocytosis effect; and (g) improved targeting distribution to the tumor cell.

3. The multispecific antibody according to any one of claims 1-2, wherein the antigen-binding domain specifically binding to Trop2 is a VHH domain, and
wherein the VHH domain comprises CDR1, CDR2, and CDR3 sequences in the amino acid sequence selected from SEQ ID NOs: 5, 9, 13, 29, 30, 98, and 99;
still preferably, the CDR1, CDR2, and CDR3 sequences:
(i) comprise or consist of the amino acid sequences of SEQ ID NOs: 6, 7, and 8, respectively;
(ii) comprise or consist of the amino acid sequences of SEQ ID NOs: 10, 11, and 12, respectively;
(iii) comprise or consist of the amino acid sequences of SEQ ID NOs: 14, 15, and 16, respectively; or
(iv) comprise or consist of the amino acid sequences of SEQ ID NOs: 31, 32, and 33, respectively;
still preferably, the VHH domain comprises the amino acid sequence selected from SEQ ID NOs: 5, 9, 13, 29, 30, 98, and 99, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence; and
most preferably, the VHH domain comprises or consists of the amino acid sequence selected from SEQ ID NOs: 5, 9, 13, 29, 30, 98, and 99.

4. The multispecific antibody according to any one of claims 1-3, wherein the antigen-binding domain specifically binding to Nectin4 is a VHH domain, and
wherein the VHH domain comprises CDR1, CDR2, and CDR3 sequences in the amino acid sequence selected from SEQ ID NOs: 17, 21, 25, 34, 35, 38, and 100-106;
still preferably, the CDR1, CDR2, and CDR3 sequences:
(i) comprise or consist of the amino acid sequences of SEQ ID NOs: 18, 19, and 20, respectively;
(ii) comprise or consist of the amino acid sequences of SEQ ID NOs: 22, 23, and 24, respectively;
(iii) comprise or consist of the amino acid sequences of SEQ ID NOs: 26, 27, and 28, respectively;
(iv) comprise or consist of the amino acid sequences of SEQ ID NOs: 18, 36, and 20, respectively; or
(v) comprise or consist of the amino acid sequences of SEQ ID NOs: 18, 37, and 20, respectively;
still preferably, the VHH domain comprises the amino acid sequence selected from SEQ ID NOs: 17, 21, 25, 34, 35, 38, and 100-106, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence; and
most preferably, the VHH domain comprises or consists of the amino acid sequence selected from SEQ ID NOs: 17, 21, 25, 34, 35, 38, and 100-106.

5. The multispecific antibody according to any one of claims 1-4, wherein the antibody further comprises:
(a) a half-life extension domain, wherein preferably, the half-life extension domain is selected from a serum albumin binding peptide and an immunoglobulin Fc region; and/or
(b) a peptide linker; preferably, the peptide linker is 5-15 amino acids in length, and more preferably, the peptide linker comprises the amino acid sequence (G₄S)ₙ, wherein n = 1, 2, or 3.

6. The multispecific antibody according to any one of claims 1-5, wherein the antibody is a single-chain or double-chain antibody comprising at least one (preferably 1 or 2) Nectin4-binding domain and at least one (preferably 1 or 2) Trop2-binding domain.

7. The multispecific antibody according to any one of claims 1-6, wherein the antibody comprises two identical polypeptide chains, wherein the polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
wherein n1, n2, n3, and n4 are each independently selected from integers of 0, 1, and 2; preferably, n3 and n4 are 0, and n1 and n2 are 1;
wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain; the symbol "-" represents linkage via a peptide linker or direct linkage.

8. The multispecific antibody according to claim 7, wherein the polypeptide chain comprises, from the N-terminus to the C-terminus, VHH^{A}-VHH^{B}-HLE, and wherein VHH^{A} represents a Nectin4-binding domain and VHH^{B} represents a Trop2-binding domain.

9. The multispecific antibody according to any one of claims 1-6, wherein the antibody comprises two different polypeptide chains, wherein
the first polypeptide chain comprises, from the N-terminus to the C-terminus, (VHH^{A})ₙ₁-(VHH^{B})ₙ₂-HLE-(VHH^{A})ₙ₃-(VHH^{B})ₙ₄,
the second polypeptide chain comprises, from the N-terminus to the C-terminus, (VHH^{B})ₘ₁-(VHH^{A})ₘ₂-HLE-(VHH^{B})ₘ₃-(VHH^{A})ₘ₄,
wherein n1, n2, n3, and n4, as well as m1, m2, m3, and m4 are each independently selected from integers of 0, 1, and 2, and preferably, wherein n3, n4, m3, and m4 are 0, n1 + m2 = 1 or 2, n2 + m1 = 1 or 2; still preferably, m1 = 0 and m2 = 1 or m2 = 0 and m1 = 1;
wherein VHH^{A} and VHH^{B} represent VHH domains binding to an antigen A and an antigen B, respectively, wherein A and B are different from each other and are each independently selected from Nectin4 and Trop2; HLE represents an immunoglobulin Fc region as a half-life extension domain, and preferably, comprises a knob-into-hole mutation; the symbol "-" represents linkage via a peptide linker or direct linkage.

10. The multispecific antibody according to claim 9, wherein
the first polypeptide chain comprises, from the N-terminus to the C-terminus, VHH^{A}-VHH^{B}-HLE,
the second polypeptide chain comprises, from the N-terminus to the C-terminus, VHH^{A}-HLE,
wherein VHH^{A} represents a Nectin4-binding domain and VHH^{B} represents a Trop2-binding domain.

11. The multispecific antibody according to any one of claims 7-10, wherein the immunoglobulin Fc region is an Fc region from IgG, such as an Fc region from human IgG1 or IgG4; preferably, the Fc region comprises a mutation that reduces or eliminates the binding of the Fc region to FcγR.

12. The multispecific antibody according to any one of claims 1-6, wherein the antibody comprises a single polypeptide chain, wherein the polypeptide chain comprises, from the N-terminus to the C-terminus, (VHH^{A})ₙ₁-(VHH^{B})ₙ₂-(HLE)ₙ₃-(VHH^{A})ₙ₄-(VHH^{B})ₙ₅-(HLE)ₙ₆,
wherein n1, n2, n3, n4, n5, and n6 are each independently selected from integers of 0, 1, and 2, and preferably, n1 + n4 = 1 or 2, n2 + n5 = 1 or 2, and n3 + n6 = 0 or 1,
wherein VHH^{A} and VHH^{B} represent VHH domains binding to an antigen A and an antigen B, respectively, wherein A and B are different from each other and are each independently selected from Nectin4 and Trop2; HLE represents a serum albumin binding peptide as a half-life extension domain; the symbol "-" represents linkage via a peptide linker or direct linkage,
preferably, the polypeptide chain comprises, from the N-terminus to the C-terminus:
(i) VHH^{A}-VHH^{B},
(ii) VHH^{A}-VHH^{B}-HLE,
(iii) VHH^{B}-VHH^{A}-VHH^{B},
(iv) VHH^{A}-VHH^{B}-VHH^{B},
(v) VHH^{A}-HLE-VHH^{B}-VHH^{B}, or
(vi) VHH^{A}-VHH^{A}-HLE-VHH^{B}-VHH^{B}, wherein preferably, A represents Trop2 and B represents Nectin4;
still preferably, the HLE represents an anti-serum albumin VHH domain, and is optionally selected from the amino acid sequences of SEQ ID NOs: 39 and 40, or amino acid sequences having at least 85%, 90%, 95%, or 99% identity thereto or having one or more (preferably 1-10, and more preferably 1-5) amino acid additions, deletions, and/or substitutions.

13. The multispecific antibody according to any one of claims 1-12, wherein
(a) the antigen-binding domain specifically binding to Nectin4 comprises the amino acid sequence of SEQ ID NO: 25, and the antigen-binding domain specifically binding to Trop2 comprises the amino acid sequence of SEQ ID NO: 13;
(b) the antigen-binding domain specifically binding to Nectin4 comprises the amino acid sequence of SEQ ID NO: 21, and the antigen-binding domain specifically binding to Trop2 comprises the amino acid sequence of SEQ ID NO: 9;
(c) the antigen-binding domain specifically binding to Nectin4 comprises the amino acid sequence of SEQ ID NO: 25, and the antigen-binding domain specifically binding to Trop2 comprises the amino acid sequence of SEQ ID NO: 9;
(d) the antigen-binding domain specifically binding to Nectin4 comprises the amino acid sequence of SEQ ID NO: 35, and the antigen-binding domain specifically binding to Trop2 comprises the amino acid sequence selected from SEQ ID NOs: 29 and 30; or
(e) the antigen-binding domain specifically binding to Nectin4 comprises the amino acid sequence selected from SEQ ID NOs: 34 and 38, and the antigen-binding domain specifically binding to Trop2 comprises the amino acid sequence selected from SEQ ID NOs: 29 and 30.

14. The multispecific antibody according to any one of claims 1-6, wherein the antibody is in a double-chain form, wherein
(a) the antibody is in a symmetric double-chain form, and the polypeptide chain of the antibody comprises the amino acid sequence of SEQ ID NO: 53 or 54 or an amino acid sequence having at least 95%, 96%, 97%, 98%, or 99% identity thereto, and preferably comprises the amino acid sequence of SEQ ID NO: 53; or
(b) the antibody is in an asymmetric double-chain form, and the antibody comprises a first polypeptide chain and a second polypeptide chain selected from the group consisting of:
(i) first and second polypeptide chains comprising the amino acid sequences set forth in SEQ ID NOs: 55 and 56 or amino acid sequences having at least 95%, 96%, 97%, 98%, or 99% identity thereto, respectively,
(ii) first and second polypeptide chains comprising the amino acid sequences set forth in SEQ ID NOs: 57 and 58 or amino acid sequences having at least 95%, 96%, 97%, 98%, or 99% identity thereto, respectively,
(iii) first and second polypeptide chains comprising the amino acid sequences set forth in SEQ ID NOs: 59 and 60 or amino acid sequences having at least 95%, 96%, 97%, 98%, or 99% identity thereto, respectively,
(iv) first and second polypeptide chains comprising the amino acid sequences set forth in SEQ ID NOs: 61 and 62 or amino acid sequences having at least 95%, 96%, 97%, 98%, or 99% identity thereto, respectively,
(v) first and second polypeptide chains comprising the amino acid sequences set forth in SEQ ID NOs: 63 and 64 or amino acid sequences having at least 95%, 96%, 97%, 98%, or 99% identity thereto, respectively,
(vi) first and second polypeptide chains comprising the amino acid sequences set forth in SEQ ID NOs: 65 and 66 or amino acid sequences having at least 95%, 96%, 97%, 98%, or 99% identity thereto, respectively,
(vii) first and second polypeptide chains comprising the amino acid sequences set forth in SEQ ID NOs: 67 and 68 or amino acid sequences having at least 95%, 96%, 97%, 98%, or 99% identity thereto, respectively,
(viii) first and second polypeptide chains comprising the amino acid sequences set forth in SEQ ID NOs: 69 and 70 or amino acid sequences having at least 95%, 96%, 97%, 98%, or 99% identity thereto, respectively,
(ix) first and second polypeptide chains comprising the amino acid sequences set forth in SEQ ID NOs: 71 and 72 or amino acid sequences having at least 95%, 96%, 97%, 98%, or 99% identity thereto, respectively,
(x) first and second polypeptide chains comprising the amino acid sequences set forth in SEQ ID NOs: 73 and 74 or amino acid sequences having at least 95%, 96%, 97%, 98%, or 99% identity thereto, respectively, and
(xi) first and second polypeptide chains comprising the amino acid sequences set forth in SEQ ID NOs: 75 and 76 or amino acid sequences having at least 95%, 96%, 97%, 98%, or 99% identity thereto, respectively;
preferably, the first and second polypeptide chains comprise the amino acid sequences of SEQ ID NOs: 61/62, 69/70, or 71/72, respectively.

15. The multispecific antibody according to any one of claims 1-6, wherein the antibody is in a single-chain form, wherein the polypeptide chain of the antibody comprises the amino acid sequence selected from SEQ ID NOs: 41-52 or an amino acid sequence having at least 95%, 96%, 97%, 98%, or 99% identity thereto.

16. A VHH domain specifically binding to Trop2,
wherein the VHH domain comprises CDR1, CDR2, and CDR3 sequences in the amino acid sequence selected from SEQ ID NOs: 5, 9, 13, 29, 30, 98, and 99;
still preferably, the CDR1, CDR2, and CDR3 sequences:
(i) comprise or consist of the amino acid sequences of SEQ ID NOs: 6, 7, and 8, respectively;
(ii) comprise or consist of the amino acid sequences of SEQ ID NOs: 10, 11, and 12, respectively;
(iii) comprise or consist of the amino acid sequences of SEQ ID NOs: 14, 15, and 16, respectively; or
(iv) comprise or consist of the amino acid sequences of SEQ ID NOs: 31, 32, and 33, respectively;
still preferably, the VHH domain comprises the amino acid sequence selected from SEQ ID NOs: 5, 9, 13, 29, 30, 98, and 99, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence; and
most preferably, the VHH domain comprises or consists of the amino acid sequence selected from SEQ ID NOs: 5, 9, 13, 29, 30, 98, and 99.

17. A VHH domain specifically binding to Nectin4,
wherein the VHH domain comprises CDR1, CDR2, and CDR3 sequences in the amino acid sequence selected from SEQ ID NOs: 17, 21, 25, 34, 35, 38, and 100-106;
preferably, the CDR1, CDR2, and CDR3 sequences:
(i) comprise or consist of the amino acid sequences of SEQ ID NOs: 18, 19, and 20, respectively;
(ii) comprise or consist of the amino acid sequences of SEQ ID NOs: 22, 23, and 24, respectively;
(iii) comprise or consist of the amino acid sequences of SEQ ID NOs: 26, 27, and 28, respectively;
(iv) comprise or consist of the amino acid sequences of SEQ ID NOs: 18, 36, and 20, respectively; or
(v) comprise or consist of the amino acid sequences of SEQ ID NOs: 18, 37, and 20, respectively;
still preferably, the VHH domain comprises the amino acid sequence selected from SEQ ID NOs: 17, 21, 25, 34, 35, 38, and 100-106, or an amino acid sequence having at least 85%, 90%, 95%, or 99% identity with respect to the amino acid sequence, or an amino acid sequence having one or more (preferably 1-10, and more preferably 1-5) amino acid additions, deletions, and/or substitutions with respect to the amino acid sequence; and
most preferably, the VHH domain comprises or consists of the amino acid sequence selected from SEQ ID NOs: 17, 21, 25, 34, 35, 38, and 100-106.

18. An antibody, comprising the VHH domain specifically binding to Trop2 according to claim 16 and/or the VHH domain specifically binding to Nectin4 according to claim 17.

19. An antigen-binding molecule, comprising the antibody according to claim 18.

20. A polynucleotide encoding the antibody according to any one of claims 1-15, the VHH domain according to claim 16 or 17, the antibody according to claim 18, or the antigen-binding molecule according to claim 19.

21. A vector, preferably an expression vector, comprising the polynucleotide according to claim 20.

22. A host cell, comprising the polynucleotide according to claim 20 or the vector according to claim 21, wherein, for example, the host cell is a mammalian cell.

23. A method for producing the antibody according to any one of claims 1-15 or 18, comprising:
culturing a host cell comprising a polynucleotide encoding a polypeptide chain under conditions suitable for the expression of the polypeptide chain of the antibody; and assembling the polypeptide chain to produce the antibody under conditions suitable for the assembly of the polypeptide chain into the antibody.

24. An immunoconjugate or immunofusion comprising the antibody according to any one of claims 1-15 or 18.

25. An antibody-drug conjugate represented by formula (I):
Ab-(L-D)ₚ (I)
or a pharmaceutically acceptable salt or solvate thereof,
wherein:
Ab is the antibody according to any one of claims 1-15 or 18 or the antigen-binding molecule according to claim 19;
L is a linker;
D is a drug, preferably an anti-tumor compound; and
p is an integer selected from 1 to 16, e.g., 1, 2, 3, 4, 5, 6, 7, 8, 9, or 10.

26. The antibody-drug conjugate or the pharmaceutically acceptable salt or solvate thereof according to claim 25, wherein the anti-tumor compound is a cytotoxic agent, such as a camptothecin compound and an auristatin compound, such as exatecan, Dxd, or MMAE.

27. The antibody-drug conjugate or the pharmaceutically acceptable salt or solvate thereof according to claim 25, wherein D has a structure represented by formula (D-1a) or formula (D-1b): 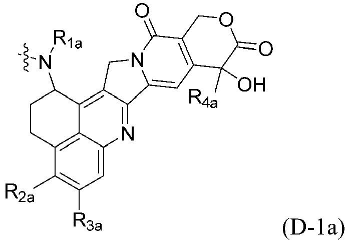
wherein R₁ₐ is selected from H and C₁-C₆ alkyl;
R₂ₐ is selected from H, halogen, C₁-C₆ alkyl, C₁-C₆ haloalkyl, -OR₅ₐ, and -SR₅ₐ; R₃ₐ is selected from H, halogen, CN, C₁-C₆ alkyl, C₁-C₆ haloalkyl, and -OR₅ₐ; and R₄ₐ and R₅ₐ are independently selected from H and C₁-C₄ alkyl;
or 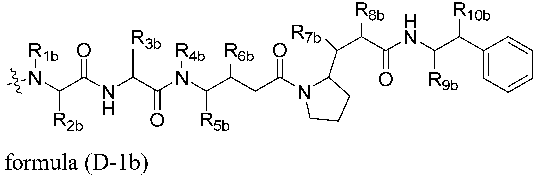
wherein R_{1b}, R_{2b}, R_{3b}, R_{4b}, R_{5b}, and R_{8b} are each independently selected from C₁₋₈ alkyl, such as methyl, ethyl, n-propyl, isopropyl, n-butyl, isobutyl, or *sec*-butyl;
R_{6b} and R_{7b} are each independently selected from C₁₋₈ alkoxy, such as methoxy, ethoxy, or propoxy;
R_{9b} is selected from C₁₋₈ alkyl and COOH, such as methyl, ethyl, n-propyl, isopropyl, n-butyl, isobutyl, or *sec*-butyl; and
R_{10b} is selected from OH and H.

28. The antibody-drug conjugate or the pharmaceutically acceptable salt or solvate thereof according to claim 27, wherein
R₁ₐ is H; R₂ₐ is C₁-C₆ alkyl; R₃ₐ is halogen, preferably -F; R₄ₐ is C₁-C₄ alkyl, preferably ethyl; R_{1b}, R_{4b}, and R_{8b} are each independently selected from C₁₋₂ alkyl, preferably methyl;
R_{2b}, R_{3b}, and R_{5b} are each independently selected from C₃₋₄ alkyl;
R_{6b} and R_{7b} are each independently selected from C₁₋₂ alkoxy; and
R_{9b} is selected from C₁₋₄ alkyl, and R_{10b} is OH; or R_{9b} is COOH, and R_{10b} is H.

29. The antibody-drug conjugate or the pharmaceutically acceptable salt or solvate thereof according to claim 27, wherein D has a structure represented by formula (D-2a) or formula (D-2b): 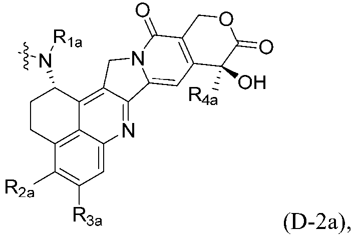
wherein R₁ₐ, R₂ₐ, R₃ₐ, and R₄ₐ are as defined in claim 27 or 28; 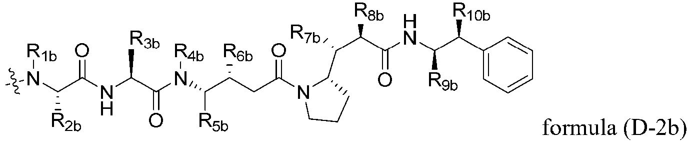
wherein R_{1b}, R_{2b}, R_{3b}, R_{4b}, R_{5b}, R_{6b}, R_{7b}, R_{8b}, R_{9b}, and R_{10b} are as defined in claim 27 or 28.

30. The antibody-drug conjugate or the pharmaceutically acceptable salt or solvate thereof according to claim 27, wherein D has a structure represented by formula (D-3a) or (D-3b):

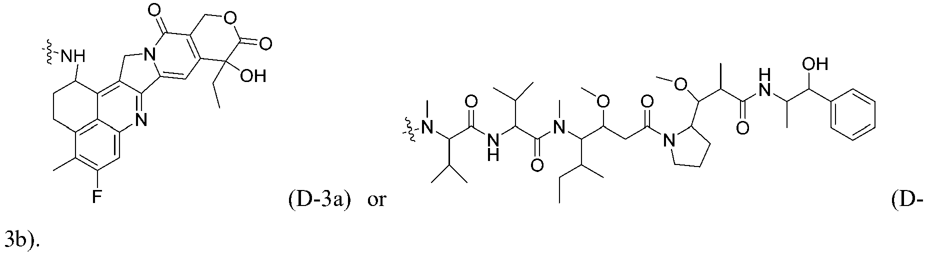

31. The antibody-drug conjugate or the pharmaceutically acceptable salt or solvate thereof according to claim 27, wherein D has a structure represented by formula (D-4a) or (D-4b):

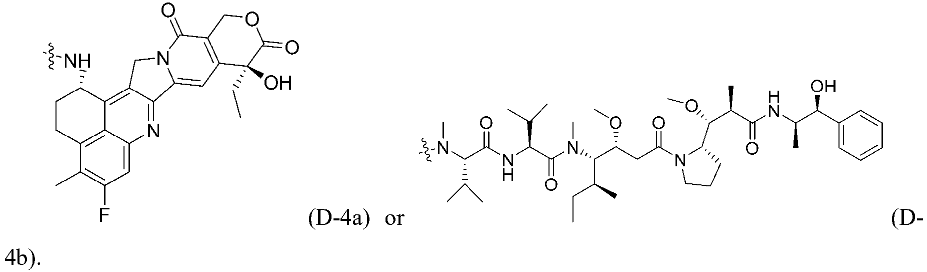

32. The antibody-drug conjugate or the pharmaceutically acceptable salt or solvate thereof according to any one of claims 25-31, wherein -L- has the following structure: -Z-L₁-L₂-L₃-, wherein
Z is selected from 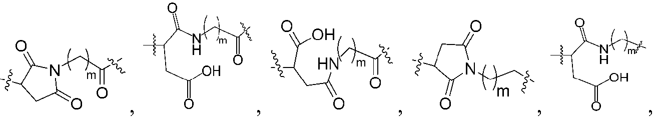 and 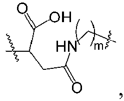 wherein m is independently an integer selected from 1-10, such as 1, 2, 3, 4, 5, 6, 7, or 8;
L₁ is selected from absence, 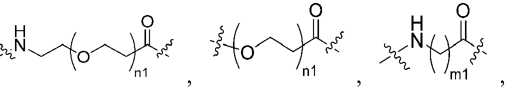 and 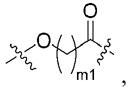 wherein n1 and m1 are each independently an integer selected from 0-20, for example, an integer selected from 0-12, such as 1, 2, 3, 4, 5, 6, 7, or 8;
L₂ is an amino acid residue or a peptide residue consisting of 2-8 amino acids; and
L₃ is selected from: 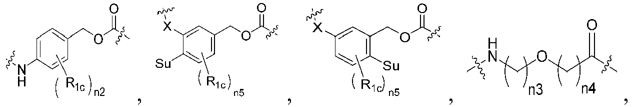 and 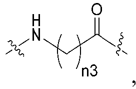 wherein X is selected from -NH-, -O-, and -S-; each R_{1c} is independently selected from C₁₋₈ alkyl, C₁₋₈ haloalkyl-, C₁₋₈ alkoxy, halogen, nitro, and cyano; each Su is independently selected from pentose, penturonic acid, hexose, and hexuronic acid; n2 is 0, 1, 2, 3, or 4; n5 is 0, 1, 2, or 3; n3 and n4 are independently 1, 2, 3, 4, 5, or 6.

33. The antibody-drug conjugate or the pharmaceutically acceptable salt or solvate thereof according to claim 32,
wherein Z is selected from 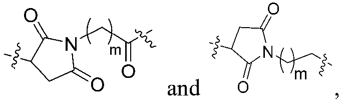 wherein m is 1, 2, 3, 4, 5, 6, 7, or 8; and
preferably, Z is selected from 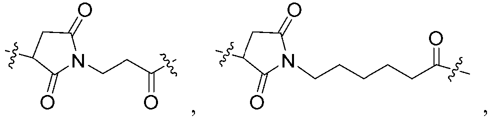 and

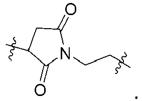

34. The antibody-drug conjugate or the pharmaceutically acceptable salt or solvate thereof according to any one of claims 32-33,
wherein L₁ is selected from absence, 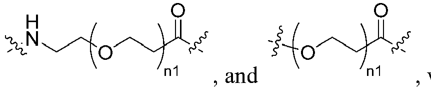 wherein n1 is independently an integer selected from 0-12, such as 1, 2, 3, 4, 5, 6, 7, or 8; and
preferably, L ₁ is absent or

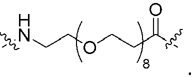

35. The antibody-drug conjugate or the pharmaceutically acceptable salt or solvate thereof according to any one of claims 32-34,
wherein L₂ is an amino acid residue or a peptide residue consisting of 2, 3, 4, 5, 6, or 7 amino acids; preferably, the amino acid residues or the amino acids constituting the peptide residue are each independently selected from glycine (Gly), valine (Val), alanine (Ala), lysine (Lys), citrulline (Cit), glutamine (Gln), glutamic acid (Glu), phenylalanine (Phe), leucine (Leu), tyrosine (Tyr), serine (Ser), aspartic acid (Asp), asparagine (Asn), isoleucine (Ile), arginine (Arg), proline (Pro), methionine (Met), tryptophan (Trp), cysteine (Cys), histidine (His), and threonine (Thr), wherein the amino acid residues or the amino acids are optionally substituted with one or more C₁₋₆ alkyl;
more preferably, the amino acid residues or the amino acids constituting the peptide residue are each independently selected from glycine (Gly), valine (Val), alanine (Ala), phenylalanine (Phe), glutamic acid (Glu), and citrulline (Cit); and
still more preferably, L₂ is selected from -Ala-, -Val-, -Gly-, -Val-Ala-, -Gly-Gly-Phe-Gly-, - Val-Cit-, and -Glu-Val-Cit-.

36. The antibody-drug conjugate or the pharmaceutically acceptable salt or solvate thereof according to any one of claims 32-35,
wherein L₃ is selected from: 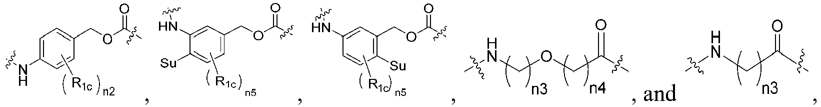
wherein each R _{1c} is independently selected from C₁₋₈ alkyl, C₁₋₈ haloalkyl-, C₁₋₈ alkoxy, halogen, nitro, and cyano; each Su is independently selected from 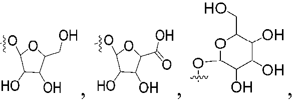 and 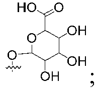
n2 is 0, 1, 2, 3, or 4; n5 is 0, 1, 2, or 3; and n3 and n4 are independently 1, 2, 3, 4, 5, or 6; and
preferably, L₃ is selected from: 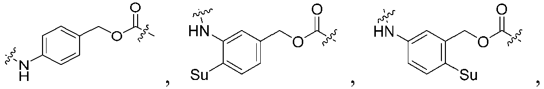 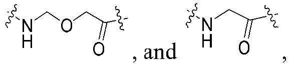
wherein the variables are as defined above.

37. The antibody-drug conjugate or the pharmaceutically acceptable salt or solvate thereof according to any one of claims 32-36,
wherein each Su is independently: 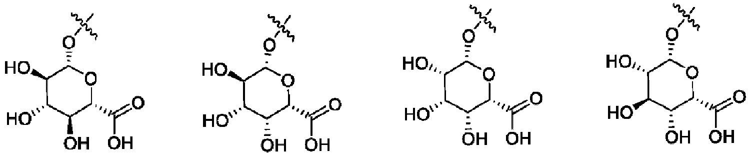 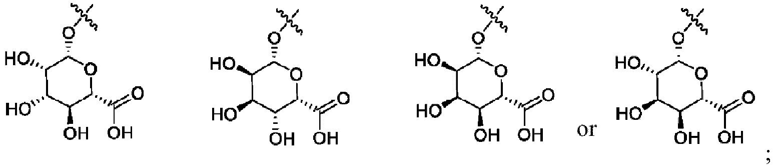
more preferably, each Su is independently 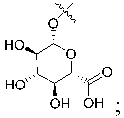 and
still more preferably, each Su is independently

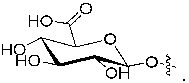

38. The antibody-drug conjugate or the pharmaceutically acceptable salt or solvate thereof according to any one of claims 32-36,
wherein L₃ is selected from: 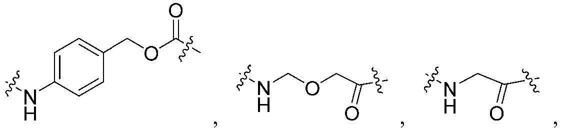 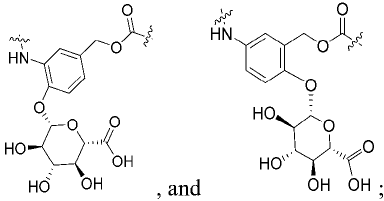 and
preferably, L₃ is selected from:

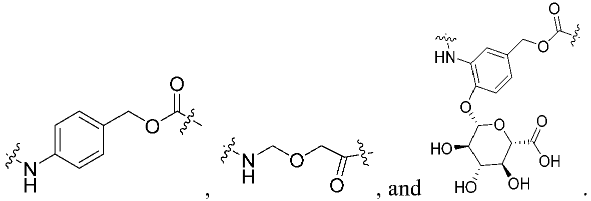

39. The antibody-drug conjugate or the pharmaceutically acceptable salt or solvate thereof according to claim 35,
wherein Z is selected from 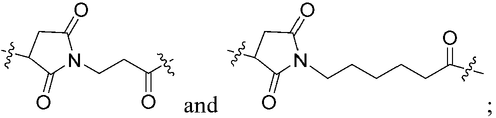
L₁ is selected from absence and 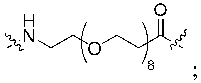
L₂ is selected from -Gly-, -Val-Ala-, and -Gly-Gly-Phe-Gly; and
L₃ is selected from 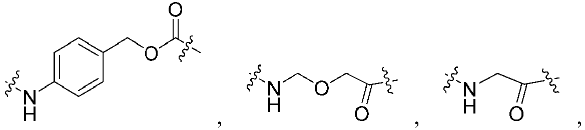 and

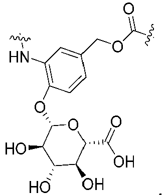

40. The antibody-drug conjugate or the pharmaceutically acceptable salt or solvate thereof according to claim 35,
wherein -Z-L₁-L₂-L₃- is selected from the following structure 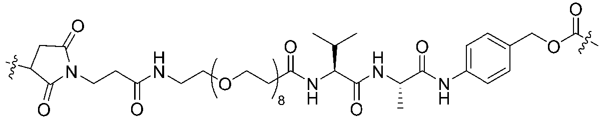 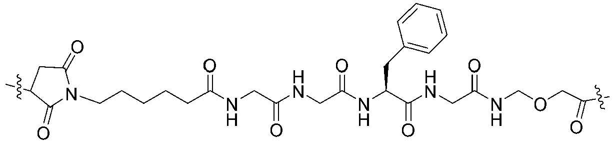 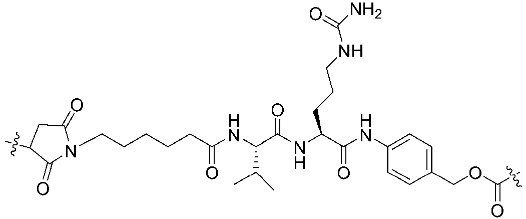 or

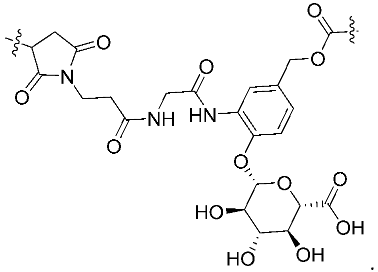

41. The antibody-drug conjugate or the pharmaceutically acceptable salt or solvate thereof according to any one of claims 25-40, having an average DAR value of 2-10, e.g., 4-8.

42. The antibody-drug conjugate or the pharmaceutically acceptable salt or solvate thereof according to claim 25, wherein the antibody-drug conjugate is selected from: 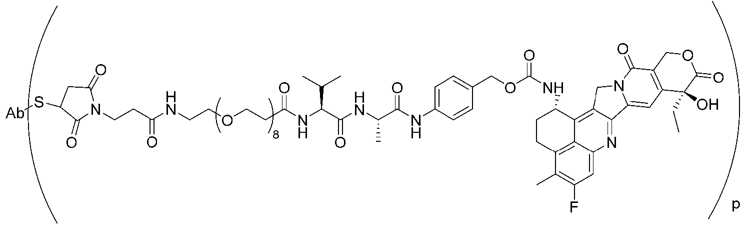 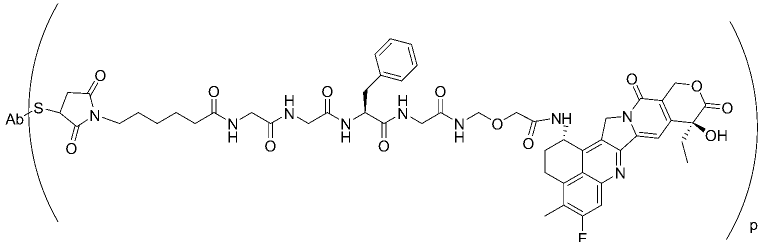 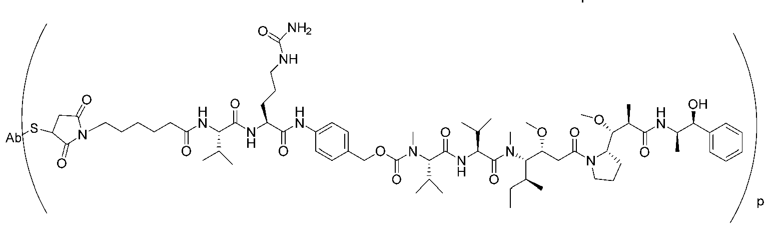 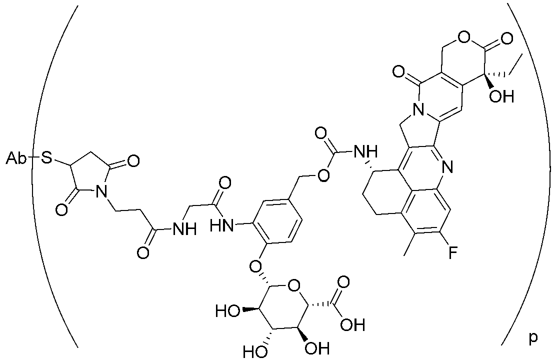
wherein Ab and p are as defined in claim 25;
preferably, Ab is a double-chain antibody, wherein
(a) the antibody is in a symmetric double-chain form, and the polypeptide chain of the antibody comprises the amino acid sequence of SEQ ID NO: 53; or
(b) the antibody is in an asymmetric double-chain form, and the antibody comprises first and second polypeptide chains comprising the amino acid sequences of SEQ ID NOs: 61/62, 69/70, or 71/72, respectively; and
preferably, the antibody-drug conjugate has an average DAR of 2-10 or 4-8.

43. A pharmaceutical composition, comprising the antibody according to any one of claims 1-15 and 18, the antigen-binding molecule according to claim 19, the immunoconjugate or immunofusion according to claim 24, or the antibody-drug conjugate or the pharmaceutically acceptable salt or solvate thereof according to any one of claims 25-42, and a pharmaceutically acceptable carrier.

44. Use of the antibody according to any one of claims 1-15 and 18, the antigen-binding molecule according to claim 19, the immunoconjugate or immunofusion according to claim 24, or the antibody-drug conjugate or the pharmaceutically acceptable salt or solvate thereof according to any one of claims 25-42 as a medicament or in the manufacture of a medicament.

45. The use according to claim 44, wherein the medicament is used for treating and/or preventing cancer in an individual, wherein the cancer is a Trop2-positive, Nectin4-positive, or Trop2/Nectin4 double-positive solid tumor selected from, for example, urothelial cancer; bladder cancer (e.g., BLCA); cervical cancer (e.g., CESC); head and neck cancer (e.g., HNSC); lung cancer (e.g., LUSC and LUAD); breast cancer (e.g., BRCA); pancreatic cancer (e.g., PAAD); esophageal cancer (e.g., ESCA); prostate cancer (e.g., PRAD); bile duct cancer (e.g., CHOL); endometrioid cancer (e.g., UCEC); thyroid cancer (e.g., THCA); ovarian cancer (e.g., OV); colorectal cancer (e.g., COAD and READ); and gastric cancer (e.g., STAD), including primary, recurrent, and refractory cancers thereof.
